# FASCICULE DE BREVET EUROPEEN

(11) **EP 2 518 067 B1**
(45) Date de publication et mention de la délivrance du brevet: **05.03.2014**
(21) Numéro de dépôt: 11306144.4
(22) Date de dépôt: 13.09.2011
(51) Int. Cl.: C07D 403/12, C07D 403/14, C07D 405/14, C07D 409/14, C07D 413/14, C07D 471/04, C07D 471/08, C07D 487/04, C07D 487/08, A61K 31/455, A61K 31/439, A61K 31/497, A61K 31/501, A61K 31/506, A61P 9/00, C07D 401/14

(54) **DÉRIVES DE N-[(1H-PYRAZOL-1-YL)ARYL]-1H-INDOLE OU 1H- INDAZOLE-3-CARBOXAMIDE ET LEURS APPLICATIONS EN THÉRAPEUTIQUE COMME ANTAGONISTES DE P2Y12.**
DERIVATE VON N-[(1H-PYRAZOL-1-YL)ARYL]-1H-INDOL ODER 1H-INDAZOL-3-CARBOXAMID UND IHRE THERAPEUTISCHEN ANWENDUNGEN ALS P2Y12 ANTAGONISTEN
DERIVATIVES OF N-[(1H-PYRAZOL-1-YL)ARYL]-1H-INDOLE OR 1H- INDAZOLE-3-CARBOXAMIDE AND THEIR THERAPEUTIC USES AS P2Y12 ANTAGONISTS

(30) Priorité: 29.04.2011 FR 1153659
(43) Date de publication de la demande: 31.10.2012
(73) Titulaire: SANOFI, 75008 Paris (FR)
(72) Inventeur: Badorc, Alain, 75013 Paris (FR); Boldron, Christophe, 75013 Paris (FR); Delesque, Nathalie, 75013 Paris (FR); Fossey, Valérie, 75013 Paris (FR); Lassalle, Gilbert, 75013 Paris (FR); Yvon, Xavier, 75013 Paris (FR)
(74) Mandataire: Werner, Alain Henri

(56) Documents cités:
- WO-A1-2005/070920
- WO-A2-2007/002635
- WO-A2-2009/080226

## Description

La présente invention a pour objet de nouveaux dérivés de N-[(1*H*-pyrazol-1-yl)aryl]-1*H*-indole ou 1*H*-indazole-3-carboxamide, leur préparation et leur application en thérapeutique.

Les composés selon la présente invention sont des antagonistes réversibles du récepteur purinergique P2Y12. Ces composés sont des anti-agrégants plaquettaires, exerçant un puissant effet anti-thrombotique. Ils peuvent être utilisés pour le traitement et la prophylaxie de troubles cardiovasculaires tels que les maladies thromboemboliques ou les resténoses.

Dans le monde industrialisé, les complications médicales liées à la survenue d'une thrombose représentent une des principales causes de mortalité. Quelques exemples de pathologies associées au développement d'une thrombose incluent l'infarctus aigu du myocarde, l'angine (de poitrine) instable et l'angine stable chronique, les attaques ischémiques passagères, les accidents vasculaires cérébraux, la maladie vasculaire périphérique, la pré-éclampsie et l'éclampsie, la thrombose veineuse profonde, les embolies (cérébrale, pulmonaire, coronarienne, rénale ..), la coagulation intravasculaire disséminée, ou le purpura thrombotique thrombocytopénique. Il existe également des risques de complications thrombotiques et resténotiques pendant et à la suite de procédures chirurgicales invasives, telles que l'angioplastie, l'endartériectomie carotidienne, le pontage aorto-coronarien par greffe, ou encore le placement de stents ou de prothèses endovasculaires.

Les thromboses artérielles peuvent survenir à la suite d'une lésion de la paroi d'un vaisseau ou d'une rupture d'une plaque d'athérosclérose. Les plaquettes jouent un rôle essentiel dans la formation de ces thromboses. Les plaquettes peuvent être activées soit par des médiateurs libérés dans la circulation sanguine par des cellules circulantes ou par des cellules endothéliales endommagées présentes le long de la paroi du vaisseau, soit par des molécules thrombogènes de la matrice sous-endothéliale - comme le collagène - exposées lors de lésions vasculaires. En outre, les plaquettes peuvent également être activées dans des conditions de flux sanguin à contrainte de cisaillement élevée que l'on retrouve dans des vaisseaux sténosés. Après activation, les plaquettes circulantes adhèrent et s'accumulent au niveau de la lésion vasculaire pour former un thrombus. Pendant ce processus, les thrombi générés peuvent être suffisamment volumineux pour bloquer en partie ou complètement la circulation sanguine dans le vaisseau.

Dans les veines, des thrombi peuvent aussi se former au niveau de zones de stase ou de circulation sanguine ralentie. De par leur nature, ces thrombi veineux peuvent produire des emboles qui se déplacent dans le système vasculaire. Ces emboles sont alors capables de bloquer la circulation sanguine dans des vaisseaux plus distants, tels que les artères pulmonaire ou coronaire.

De nombreuses études ont démontré que l'adénosine 5'-diphosphate (ADP) est un médiateur capital de l'activation et de l'agrégation plaquettaire, jouant un rôle clef dans l'initiation et la progression de la formation d'un thrombus (Maffrand et al, Thromb. Haemostas. (1988) 59, 225-230; Herbert et al, Arterioscl. Thromb. (1993) 13, 1171-1179).

L'ADP est libéré dans la circulation par les globules rouges lésés et les cellules endothéliales de la paroi athéroscléreuse, et plus spécifiquement sécrété par les plaquettes activées où l'ADP est stocké dans les granules denses à très forte concentration. L'agrégation plaquettaire induite à l'ADP est déclenchée par sa liaison à deux récepteurs purinergiques spécifiques, exprimés sur la membrane cellulaire des plaquettes humaines: P2Y1 et P2Y12. Le récepteur P2Y1, couplé à la stimulation de la PLCβ via Gαq, est responsable de la mobilisation des stocks internes de calcium, du changement de forme des plaquettes, et d'une agrégation transitoire à l'ADP. Le récepteur P2Y12, couplé à l'inhibition de l'adénylcyclase via Gαi2 et à l'activation de la PI-3 kinase, est responsable de l'amplification des réponses et de la stabilisation de l'agrégation (Gachet, Thromb. Haemost. (2001) 86, 222-232; Andre et al, J. Clin. Invest. (2003) 112, 398-406). L'utilisation de souris transgéniques P2Y1-/- (Gachet et al, J Clin Invest (1999) 104, 1731-1737; Gachet et al, Circulation (2001) 103, 718-723 ; Gachet et al, Haematologia (2002) 87, 23) et P2Y12-/- (Conley et al, Nature (2001) 409, 202-207) a permis de mettre en évidence l'importance de ces deux récepteurs dans le développement des thromboses *in vivo.* Chez l'Homme, des déficits génétiques sur P2Y12 ont été décrits comme étant associés à un phénotype hémorragique et à une altération prononcée de l'agrégation plaquettaire induite à l'ADP (Kanakura et al, J Thromb Haemost. (2005) 3, 2315-2323).

L'utilisation du Clopidogrel en clinique humaine a fourni la preuve que le blocage du récepteur P2Y12 par un antagoniste représente une stratégie thérapeutique clef dans le traitement des maladies cardiovasculaires. Le Clopidogrel est une pro-drogue de la famille des thiénopyridines dont le métabolite actif se lie de façon covalente au récepteur P2Y12, conduisant à une inhibition irréversible de l'activité plaquettaire *in vivo.* (Savi et al, Biochem Biophys Res Commun (2001) 283, 379-383 ; Savi et al, Proc Natl Acad Sci (2006) 103, 11069-11074). Cette molécule avait initialement montré son efficacité dans plusieurs essais cliniques en réduisant le risque des accidents cardiovasculaires chez des patients à risque ("A randomised, blinded, trial of clopidogrel versus aspirin in patients at risk of ischaemic events (CAPRIE)" CAPRIE steering committee Lancet (1996) 348, 1329-1339 ; "The Clopidogrel in Unstable Angina to Prevent Recurrent Events (CURE). Effects of Clopidogrel in Addition to Aspirin in Patients with Acute Coronary Syndromes without ST-Segment Elevation" CURE steering committee N Engl J Med (2001) 345, 7, 494-502).

WO 2009/080226 décrit de derivés du pyrazole et leur utilisation comme antagonistes du récepteur P2Y12.

Des antagonistes synthétiques du récepteur P2Y12 qui présentent une activité anti-plaquettaire et anti-thrombotique ont été décrits. Néanmoins, le besoin de nouveaux antagonistes possédant des propriétés supérieures subsiste, notamment le besoin d'antagonistes réversibles présentant un meilleur rapport bénéfice sur risque.

La présente invention a pour objet des composés répondant à la formule (I) : 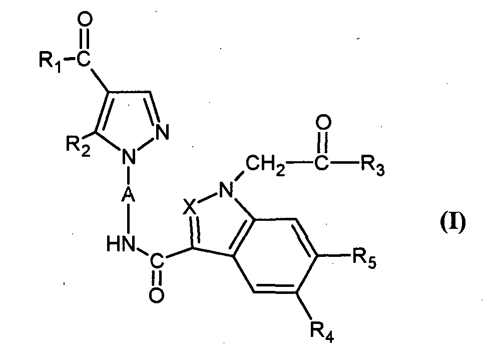 dans laquelle :
- A représente un radical aromatique bivalent choisi parmi : 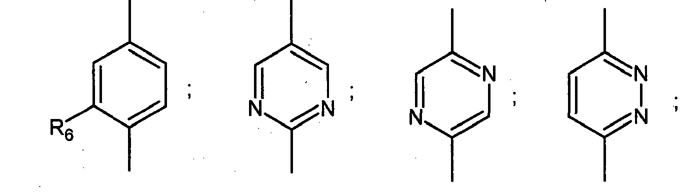
- X représente un groupe -CH- ou un atome d' azote ;
- R₁ représente un (C₁-C₄)alkyle ou un (C₁-C₄)alcoxy ;
- R₂ représente un groupe Alk ;
- R₃ représente un hydroxy ou un groupe -NR₇R₈ ;
- R₄ représente un atome d'hydrogène, un atome d'halogène, un cyano, un phényle, un groupe Alk, un groupe OAlk ou un groupe -NR₉R₁₀ ;
- R₅ représente un atome d'hydrogène, un atome d'halogène ou un groupe Alk ;
- R₆ représente un atome d'hydrogène, un atome d'halogène, un cyano, un groupe -COOAlk ou un groupe -CONH₂ ;
- R₇ représente un atome d'hydrogène ou un (C₁-C₄)alkyle ;
- R₈ représente :
   a) un atome d'hydrogène ;
   b) un (C₁-C₄)alkyle non substitué ou substitué par :
      (i) un hydroxy ;
      (ii) un groupe OAlk ;
      (iii) un groupe -NR₉R₁₀ ;
      (iv) un (C₃-C₆)hétérocycloalkyle non substitué ou substitué par un (C₁-C₄)alkyle ou par un groupe -COOAlk ;
      (v) un hétéroaryle non substitué ou substitué par un (C₁-C₄)alkyle ;
   c) un (C₃-C₇)cycloalkyle ;
   d) un (C₃-C₆)hétérocycloalkyle non substitué ou substitué par un (C₁-C₄)alkyle, un groupe -COOAlk ou par un ou deux oxo ;
   e) un groupe -SO₂Alk ;
- ou bien R₇ et R₈ ensemble avec l'atome d'azote auquel ils sont liés constituent un hétérocycle, saturé ou insaturé, mono- ou polycyclique, condensé ou ponté, comprenant de 4 à 10 chainons et pouvant contenir un, deux ou trois autres atomes d'azote ou un autre hétéroatome choisi parmi l'atome d'oxygène ou de soufre ; ledit hétérocycle étant non substitué ou substitué une, deux ou trois fois par des substituants choisis indépendamment parmi :
   a) un atome d'halogène ;
   b) un hydroxy ;
   c) un groupe -OR₁₁ ;
   d) un oxo ;
   e) un groupe -NR₉R₁₀ ;
   f) un groupe -NR₁₂COR₁₃ ;
   g) un groupe -NR₁₂COOR₁₃ ;
   h) un groupe -COR₁₃ ;
   i) un groupe -COOR₁₃ ;
   j) un groupe -CONR₁₄R₁₅ ;
   k) un (C₃-C₇)cycloalkyle non substitué ou substitué par un hydroxy ou par un (C₁-C₄)alkyle ;
   l) un (C₃-C₆)hétérocycloalkyle non substitué ou substitué par un ou deux oxo ;
   m) un phényle non substitué ou substitué une ou plusieurs fois par des substituants choisis indépendamment parmi un atome d'halogène, un groupe Alk ou un groupe OAlk ;
   n) un pyridinyle ;
   o) un (C₁-C₄)alkyle non substitué ou substitué une ou plusieurs fois par des substituants choisis indépendamment parmi :
      (i) un atome d'halogène ;
      (ii) un hydroxy ;
      (iii) un groupe -OR₁₁ ;
      (iv) un groupe -NR₉R₁₀ ;
      (v) un groupe -NR₁₂COR₁₃ ;
      (vi) un groupe -COOR₁₃ ;
      (vii) un groupe -CONR₁₄R₁₅ ;
      (viii) un groupe -SO₂Alk ;
      (ix) un (C₃-C₇)cycloalkyle ;
      (x) un (C₃-C₆)hétérocycloalkyle ;
      (xi) un phényle non substitué ou substitué une ou plusieurs fois par des substituants choisis indépendamment parmi un atome d'halogène, un groupe Alk ou un groupe OAlk ;
      (xii) un hétéroaryle non substitué ou substitué par un (C₁-C₄)alkyle ;
- R₉ et R₁₀ représentent chacun indépendamment un atome d'hydrogène ou un (C₁-C₄)alkyle ;
- R₁₁ représente un groupe Alk, un (C₁-C₄)alkylène-OH ou un (C₁-C₄)alkylène-OAlk ;
- R₁₂ représente un atome d'hydrogène ou un (C₁-C₄)alkyle ;
- R₁₃ représente un (C₁-C₄)alkyle ;
- R₁₄ et R₁₅ représentent chacun indépendamment un atome d'hydrogène, un (C₁-C₄)alkyle ou un (C₃-C₇)cycloalkyle ;
- ou bien R₁₄ et R₁₅ ensemble avec l'atome d'azote auquel ils sont liés constituent un radical hétérocyclique choisi parmi : l'azétidin-1-yle, la pyrrolidin-1-yle, la pipéridin-1-yle, la morpholin-4-yle ou la pipérazin-1-yle ;
- Alk représente un (C₁-C₄)alkyle non substitué ou substitué une ou plusieurs fois par un atome de fluor .

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques font partie de l'invention.

Les composés de formule (I) peuvent exister à l'état de bases ou salifiés par des acides ou des bases, notamment des acides ou des bases pharmaceutiquement acceptables. De tels sels d'addition font parties de l'invention.

Ces sels sont avantageusement préparés avec des acides ou des bases pharmaceutiquement acceptables, mais les sels d'autres acides ou bases utiles, par exemple, pour la purification ou l'isolement des composés de formule (I), font également partie de l'invention.

Par atome d'halogène, on entend un atome de brome, de chlore, de fluor ou d'iode.

Par (C₁-C₄)alkyle, on entend un radical alkyle linéaire ou ramifié de un à quatre atomes de carbone, tel que le radical méthyle, éthyle, propyle, isopropyle, butyle, isobutyle, sec-butyle, *tert*-butyle.

Par (C₁-C₄)alkylène, on entend un radical bivalent de un à quatre atomes de carbone tel que le radical méthylène, éthylène, triméthylène ou tétraméthylène.

Par (C₁-C₄)alcoxy, on entend un radical alcoxy linéaire ou ramifié de un à quatre atomes de carbone, tel que le radical méthoxy, éthoxy, propoxy, isopropoxy, butoxy, isobutoxy, sec-butoxy, *tert*-butoxy.

Par (C₃-C₇)cycloalkyle, on entend un radical carboné cyclopropyle, cyclobutyle, cyclopentyle, cyclohexyle ou cycloheptyle.

Par (C₃-C₆)hétérocycloalkyle, on entend un cycle saturé, monocyclique, comprenant de 3 à 6 chaînons et un à plusieurs hétéroatomes tels que les atomes d'azote, d'oxygène ou de soufre. A titre d'exemple, un hétérocycloalkyle peut être une aziridine, une azétidine, une pyrrolidine, une morpholine, une pipérazine, une pipéridine, une imidazolidine, une pyrazolidine, une thiomorpholine, un oxétane, un tétrahydrofurane, un tétrahydro-2H-pyrane, un tétrahydrothiophène .

Par hétéroaryle, on entend un système aromatique monocyclique comprenant de 5 à 6 chaînons, et comprenant un à plusieurs hétéroatomes tels que les atomes d'azote, d'oxygène ou de soufre. Les atomes d'azote peuvent être sous forme de N-oxydes. A titre d'exemple d'hétéroaryles monocycliques on peut citer le thiazole, le thiadiazole, le thiophène, l'imidazole, le triazole, le tétrazole, la pyridine, le furane, l'oxazole, l'isoxazole, l'oxadiazole, le pyrrole, le pyrazole, la pyrimidine, la pyridazine et la pyrazine.

Par hétérocycle, saturé ou insaturé, mono- ou polycyclique, condensé ou ponté, comprenant de 4 à 10 chainons on entend un hétérocycloalkyle tel que, par exemple, une aziridine, une azétidine, une pyrrolidine, une pipéridine, une pipérazine, une morpholine, une thiomorpholine, ainsi que, par exemple, les hétérocycles suivants : un octahydro-2*H*-pyrido[1,2-*a*]pyrazine, un 5,6,7,8-tétrahydro[1,2,4]triazolo[4,3-*a*]pyrazine, un 2,3-dihydro-1*H*-pyrrolo[3,4-*c*]pyridine, un octahydropyrrolo[1,2-*a*]pyrazine, un 1,4-diazépane, un 5,6,7,8-tétrahydroimidazo[1,2-*a*]pyrazine, un 4,5,6,7-tétrahydro-1*H*-pyrazolo[4,3-*c*]pyridine, un 1,4,5,6-tétrahydropyrrolo[3,4-*c*]pyrazole, un 6,7-dihydro-5*H*-pyrrolo[3,4-*b*]pyridine, un 3,8-diazabicyclo[3.2.1]octane, un 4,5,6,7-tétrahydro-1*H*-imidazo[4,5-*c*]pyridine, un 4,5,6,7-tétrahydro-1*H*-pyrazolo[3,4-*c*]pyridine, un 2,5-diazabicyclo[2.2.2]octane, un 2,5-diazabicyclo[2.2.1]heptane, un octahydropyrrolo[3,4-*b*]pyrrole, un octahydropyrrolo[3,4-*c*]pyrrole, un octahydro-2*H-*pyrazino[1,2-*a*]pyrazine.

Selon la présente invention, on distingue :
- les composés de formule (I) dans laquelle R₃ représente un hydroxy (référencé IA) et les autres substituants A, X, R₁, R₂, R₄ et R₅ sont tels que définis pour un composé de formule (I) ;
- les composés de formule (I) dans laquelle R₃ représente un groupe -NR₇R₈ (référencé IB) et les autres substituants A, X, R₁, R₂, R₄, R₅, R₇ et R₈ sont tels que définis pour un composé de formule (I) ;
à l'état de base ou de sels d'addition à des acides ou à des bases.

Selon la présente invention, on distingue les composés de formule (I) dans laquelle
- A représente un radical aromatique bivalent choisi parmi : 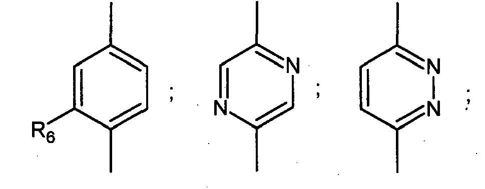
- X représente un groupe -CH- ou un atome d' azote ;
- R₁ représente un radical méthyle, éthyle, n-propyle ou un radical éthoxy;
- R₂ représente un radical méthyle;
- R₃ représente un hydroxy ou un groupe -NR₇R₈ ;
- R₄ représente un atome d'hydrogène, un atome de brome, de chlore ou de fluor, un cyano, un phényle, un radical méthyle, un radical trifluorométhyle, un radical méthoxy ou un groupe diméthylamino ;
- R₅ représente un atome d'hydrogène, un atome de brome, de chlore ou de fluor ou un radical méthyle ;
- R₆ représente un atome d'hydrogène, un atome de brome, un cyano, un groupe méthoxycarbonyle ou un groupe -CONH₂ ;
- R₇ représente un atome d'hydrogène ou un radical méthyle ;
- R₈ représente :
   a) un atome d'hydrogène ;
   b) un radical méthyle, un 2-hydroxyéthyle, un 3-hydroxypropyle, un 2-méthoxyéthyle, un 2-(méthylamino)éthyle, un 2-(diméthylamino)éthyle, un 2-(diméthylamino)propyle, un 2-(diméthylamino)-1-méthyléthyle, un 2-(diméthylamino)-2-méthylpropyl, un 3-(diméthylamino)propyle, un (1-méthylpipéridin-3-yl)méthyle, un tétrahydrofuran-3-ylméthyle, un tétrahydro-2*H-*pyran-4-ylméthyle, un 2-furylméthyle, un (3-méthyl-1*H*-1,2,4-triazol-5-yl)méthyle, 1-(1*H*-tétrazol-5-yl)éthyle, un 2-(1*H*-pyrrol-1-yl)éthyle, un 2-(1*H-*imidazol-1-yl)éthyle ;
   c) un cyclopropyle ;
   d) un 1,1-dioxidotétrahydro-3-thiényle, un pyrrolidin-3-yle, un 1-méthylpyrrolidin-3-yle, un 1-(*tert*-butoxycarbonyl)pyrrolidin-3-yle, un pipéridin-3-yle, un 1-méthylpipéridin-3-yle, un 1-(*tert*-butoxycarbonyl)pipéridin-3-yle, un 1-méthylpipéridin-4-yle ;
   e) un groupe méthylsulfonyle ;
- ou bien R₇ et R₈ ensemble avec l'atome d'azote auquel ils sont liés constituent un hétérocycle choisi parmi : une azétidine, une pyrrolidine, une pipéridine, une pipérazine, une morpholine, une thiomorpholine, un octahydro-2*H*-pyrido[1,2-*a*]pyrazine, un 5,6,7,8-tétrahydro[1,2,4]triazolo[4,3-*a*]pyrazine, un 2,3-dihydro-1*H-*pyrrolo[3,4-*c*]pyridine, un octahydropyrrolo[1,2-*a*]pyrazine, un 1,4-diazépane, un 5,6,7,8-tétrahydroimidazo[1,2-*a*]pyrazine, un 4,5,6,7-tétrahydro-1*H*-pyrazolo[4,3-*c*]pyridine, un 1,4,5,6-tétrahydropyrrolo[3,4-*c*]pyrazole, un 6,7-dihydro-5*H*-pyrrolo[3,4-*b*]pyridine, un 3,8-diazabicyclo[3.2.1]octane, un 4,5,6,7-tétrahydro-1*H*-imidazo[4,5-*c*]pyridine, un 4,5,6,7-tétrahydro-1*H*-pyrazolo[3,4-c]pyridine, un 2,5-diazabicyclo[2.2.2]octane, un 2,5-diazabicyclo[2.2.1]heptane, un octahydropyrrolo[3,4-*b*]pyrrole, un octahydropyrrolo[3,4-*c*]pyrrole, un octahydro-2*H*-pyrazino[1,2-*a*]pyrazine; ledit hétérocycle étant non substitué ou substitué une, deux ou trois fois par des substituants choisis indépendamment parmi :
   a) un atome de fluor ;
   b) un hydroxy ;
   c) un radical méthoxy, un 2-hydroxyéthoxy, un 2-méthoxyéthoxy ;
   d) un oxo ;
   e) un groupe amino, un méthylamino, un diméthylamino ;
   f) un groupe acétamido ;
   g) un groupe (*tert*-butoxycarbonyl)amino, un (*tert*-butoxycarbonyl)(méthyl)amino ;
   h) un groupe acétyle ;
   i) un groupe méthoxycarbonyle, un *tert*-butoxycarbonyle ;
   j) un groupe diméthylcarbamoyle, un cyclopropylcarbamoyle, un cyclobutylcarbamoyle ;
   k) un radical cyclopropyle, un cyclobutyle, un 2-hydroxycyclopentyle ;
   l) un oxetan-3-yle, un 1,1-dioxidotétrahydro-3-thiényle, une pipéridin-1-yle, une morpholin-4-yle ;
   m) un 4-fluorophényle;
   n) un pyridin-2-yle ;
   o) un radical méthyle, un éthyle, un n-propyle, un isopropyle, un n-butyle, un trifluorométhyle, un 2-fluoroéthyle, un 2,2-difluoroéthyle, un 2,2,2-trifluoroéthyle, un 3,3,3-trifluoropropyle, un 3,3,3-trifluoro-2-hydroxypropyle, un hydroxyméthyle, un 2-hydroxyéthyle, un 2-hydroxy-1,1-diméthyléthyle, un méthoxyméthyle, un 2-méthoxyéthyle, un 2-méthoxy-1-méthyléthyle, un 2-méthoxy-1,1-diméthyléthyle, un 2-éthoxyéthyle, un 2-(trifluorométhoxy)éthyle, un 2-(2-hydroxyéthoxy)éthyle, un 2-(2-méthoxyéthoxy)éthyle, un 2-(diméthylamino)éthyle, un 2-acétamidoéthyle, un 2-[acétyl(méthyl)amino]éthyle, un 2-méthoxy-2-oxoéthyle, un 2-éthoxy-2-oxoéthyle, un 3-éthoxy-3-oxopropyle, un 2-amino-2-oxoéthyle, un 2-(méthylamino)-2-oxoéthyle, un 2-(isopropylamino)-2-oxoéthyle, un 2-(diméthylamino)-2-oxoéthyle, un 2-(cyclopropylamino)-2-oxoéthyle, un 2-oxo-2-pyrrolidin-1-yléthyle, un 2-(méthylsulfonyl)éthyle, un cyclopropylméthyle, un pyrrolidin-1-ylméthyle, un tétrahydrofuran-2-ylméthyle, un 2-thiénylméthyle, un 4-chlorobenzyle, un 4-fluorobenzyle, un 2-méthoxybenzyle, un 3-fluoro-4-méthoxybenzyle, un pyridin-4-ylméthyle, un (5-méthyl-1,2,4-oxadiazol-3-yl)méthyle, un (5-méthylisoxazol-3-yl)méthyle ;
à l'état de base ou de sels d'addition à des acides ou à des bases.

Selon la présente invention, on distingue les composés de formule (I) dans laquelle
- A représente un radical aromatique bivalent choisi parmi : 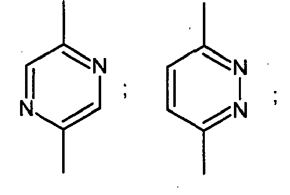
- X représente un groupe -CH-;
- R₁ représente un radical n-propyle;
- R₂ représente un radical méthyle;
- R₃ représente un hydroxy ou un groupe -NR₇R₈ ;
- R₄ représente un atome de chlore;
- R₅ représente un atome d'hydrogène ;
- R₇ représente un atome d'hydrogène ou un radical méthyle ;
- R₈ représente :
   b) un radical méthyle, un 2-hydroxyéthyle, un 3-hydroxypropyle, un 2-méthoxyéthyle, un 2-(diméthylamino)éthyle, un 2-(diméthylamino)propyle, un 2-(diméthylamino)-2-méthylpropyl, un 3-(diméthylamino)propyle, un tétrahydrofuran-3-ylméthyle, un tétrahydro-2*H*-pyran-4-ylméthyle, un 2-furylméthyle, un (3-méthyl-1*H*-1,2,4-triazol-5-yl)méthyle, 1-(1*H*-tétrazol-5-yl)éthyle, un 2-(1*H*-pyrrol-1-yl)éthyle;
   c) un cyclopropyle ;
   d) un 1-méthylpyrrolidin-3-yle, un 1-(*tert*-butoxycarbonyl)pipéridin-3-yle, un 1-méthylpipéridin-4-yle ;
- ou bien R₇ et R₈ ensemble avec l'atome d'azote auquel ils sont liés constituent un hétérocycle choisi parmi : une azétidine, une pyrrolidine, une pipéridine, une pipérazine, une morpholine, une thiomorpholine, un octahydro-2*H*-pyrido[1,2-*a*]pyrazine, un 5,6,7,8-tétrahydro[1,2,4]triazolo[4,3-*a*]pyrazine, un 2,3-dihydro-1*H*-pyrrolo[3,4-*c*]pyridine, un octahydropyrrolo[1,2-*a*]pyrazine, un 1,4-diazépane; ledit hétérocycle étant non substitué ou substitué une, deux ou trois fois par des substituants choisis indépendamment parmi :
   b) un hydroxy ;
   c) un radical méthoxy;
   d) un oxo ;
   h) un groupe acétyle ;
   i) un groupe méthoxycarbonyle;
   j) un groupe diméthylcarbamoyle;
   k) un radical un cyclobutyle;
   l) un 1,1-dioxidotétrahydro-3-thiényle, une pipéridin-1-yle, une morpholin-4-yle ; m) un 4-fluorophényle;
   o) un radical méthyle, un éthyle, un n-propyle, un isopropyle, un n-butyle, un trifluorométhyle, un 3,3,3-trifluoropropyle, un hydroxyméthyle, un 2-hydroxyéthyle, un méthoxyméthyle, un 2-méthoxyéthyle, un 2-éthoxyéthyle, un 2-(trifluorométhoxy)éthyle, un 2-(diméthylamino)éthyle, un 2-méthoxy-2-oxoéthyle, un 2-éthoxy-2-oxoéthyle, un 2-(isopropylamino)-2-oxoéthyle, un 2-(diméthylamino)-2-oxoéthyle, un 2-(cyclopropylamino)-2-oxoéthyle, un 2-oxo-2-pyrrolidin-1-yléthyle, un tétrahydrofuran-2-ylméthyle, un 4-fluorobenzyle, un 3-fluoro-4-méthoxybenzyle, un pyridin-4-ylméthyle, un (5-méthyl-1,2,4-oxadiazol-3-yl)méthyle,;
à l'état de base ou de sels d'addition à des acides ou à des bases.

Selon la présente invention, on distingue les composés de formule (I) dans laquelle :
- A représente un radical aromatique bivalent : 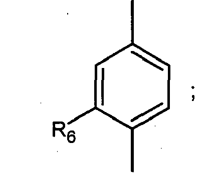
- X représente un groupe -CH- ou un atome d' azote ;
- R₁ représente un radical méthyle, éthyle, n-propyle ou un radical éthoxy;
- R₂ représente un radical méthyle;
- R₃ représente un hydroxy ou un groupe -NR₇R₈ ;
- R₄ représente un atome d'hydrogène, un atome de brome, de chlore ou de fluor, un cyano, un phényle, un radical méthyle, un radical trifluorométhyle, un radical méthoxy ou un groupe diméthylamino ;
- R₅ représente un atome d'hydrogène, un atome de brome, de chlore ou de fluor ou un radical méthyle ;
- R₆ représente un atome d'hydrogène, un atome de brome, un cyano, un groupe méthoxycarbonyle ou un groupe -CONH₂ ;
- R₇ représente un atome d'hydrogène ou un radical méthyle ;
- R₈ représente :
   b) un radical méthyle, un 2-hydroxyéthyle, un 2-méthoxyéthyle, un 2-(méthylamino)éthyle, un 2-(diméthylamino)éthyle, un 2-(diméthylamino)-1-méthyléthyle, un (1-méthylpipéridin-3-yl)méthyle, un 2-(1H-imidazol-1-yl)éthyle ;
   d) un 1,1-dioxidotétrahydro-3-thiényle, un pyrrolidin-3-yle, un 1-méthylpyrrolidin-3-yle, un 1-(*tert*-butoxycarbonyl)pyrrolidin-3-yle, un pipéridin-3-yle, un 1-méthylpipéridin-3-yle, un 1-(*tert*-butoxycarbonyl)pipéridin-3-yle ;
   e) un groupe méthylsulfonyle ;
- ou bien R₇ et R₈ ensemble avec l'atome d'azote auquel ils sont liés constituent un hétérocycle choisi parmi : une azétidine, une pyrrolidine, une pipéridine, une pipérazine, une morpholine, un 5,6,7,8-tétrahydro[1,2,4]triazolo[4,3-*a*]pyrazine, un octahydropyrrolo[1,2-*a*]pyrazine, un 1,4-diazepane, un 5,6,7,8-tétrahydroimidazo[1,2-*a*]pyrazine, un 4,5,6,7-tétrahydro-1*H*-pyrazolo[4,3-c]pyridine, un 1,4,5,6-tétrahydropyrrolo[3,4-*c*]pyrazole, un 6,7-dihydro-5H-pyrrolo[3,4-b]pyridine, un 3,8-diazabicyclo[3.2.1]octane, un 4,5,6,7-tétrahydro-1*H*-imidazo[4,5-*c*]pyridine, un 4,5,6,7-tétrahydro-1*H*-pyrazolo[3,4-*c*]pyridine, un 2,5-diazabicyclo[2.2.2]octane, un 2,5-diazabicyclo[2.2.1]heptane, un octahydropyrrolo[3,4-*b*]pyrrole, un octahydropyrrolo[3,4-*c*]pyrrole, un octahydro-2*H*-pyrazino[1,2-*a*]pyrazine; ledit hétérocycle étant non substitué ou substitué une, deux ou trois fois par des substituants choisis indépendamment parmi :
   a) un atome de fluor ;
   b) un hydroxy ;
   c) un 2-hydroxyéthoxy, un 2-méthoxyéthoxy ;
   d) un oxo ;
   e) un groupe amino, un méthylamino, un diméthylamino ;
   f) un groupe acétamido ;
   g) un groupe (*tert*-butoxycarbonyl)amino, un (*tert*-butoxycarbonyl)(méthyl)amino ;
   i) un groupe méthoxycarbonyle, un *tert*-butoxycarbonyle ;
   j) un groupe , un cyclopropylcarbamoyle, un cyclobutylcarbamoyle ;
   k) un radical cyclopropyle, un cyclobutyle, un 2-hydroxycyclopentyle ;
   l) un oxetan-3-yle, une morpholin-4-yle ;
   n) un pyridin-2-yle ;
   o) un radical méthyle, un éthyle, un n-propyle, un isopropyle, un n-butyle, un trifluorométhyle, un 2-fluoroéthyle, un 2,2-difluoroéthyle, un 2,2,2-trifluoroéthyle, un 3,3,3-trifluoro-2-hydroxypropyle, un hydroxyméthyle, un 2-hydroxyéthyle, un 2-hydroxy-1,1-diméthyléthyle, un méthoxyméthyle, un 2-méthoxyéthyle, un 2-méthoxy-1-méthyléthyle, un 2-méthoxy-1,1-diméthyléthyle, un 2-éthoxyéthyle, un 2-(trifluorométhoxy)éthyle, un 2-(2-hydroxyéthoxy)éthyle, un 2-(2-méthoxyéthoxy)éthyle, un 2-(diméthylamino)éthyle, un 2-acétamidoéthyle, un 2-[acétyl(méthyl)amino]éthyle, un 2-éthoxy-2-oxoéthyle, un 3-éthoxy-3-oxopropyle, un 2-amino-2-oxoéthyle, un 2-(méthylamino)-2-oxoéthyle, un 2-(diméthylamino)-2-oxoéthyle, un 2-oxo-2-pyrrolidin-1-yléthyle, un 2-(méthylsulfonyl)éthyle, un cyclopropylméthyle, un pyrrolidin-1-ylméthyle, un 2-thiénylméthyle, un 4-chlorobenzyle, un 4-fluorobenzyle, un 2-méthoxybenzyle, un (5-méthylisoxazol-3-yl)méthyle ;
à l'état de base ou de sels d'addition à des acides ou à des bases.

Selon la présente invention, on distingue les composés de formule (I) dans laquelle
- A représente un radical aromatique bivalent choisi parmi : 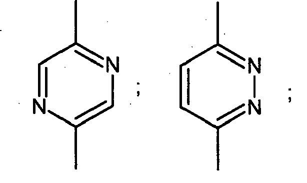
- X représente un groupe -CH-;
- R₁ représente un radical n-propyle;
- R₂ représente un radical méthyle;
- R₃ représente un hydroxy ou un groupe -NR₇R₈ choisi parmi : 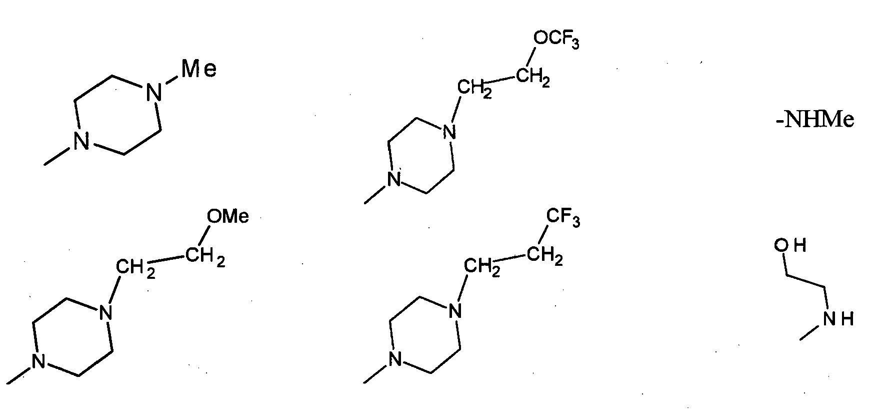 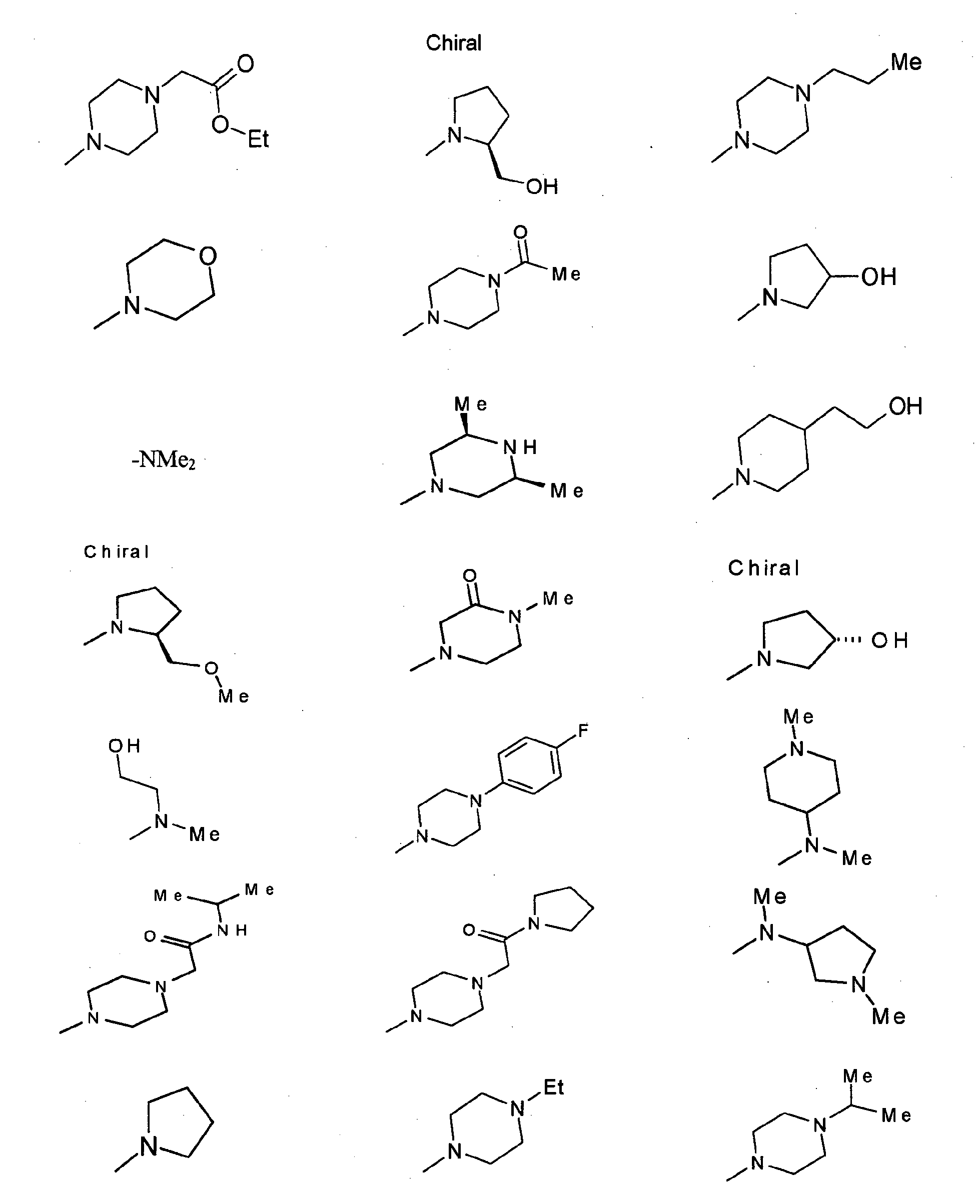 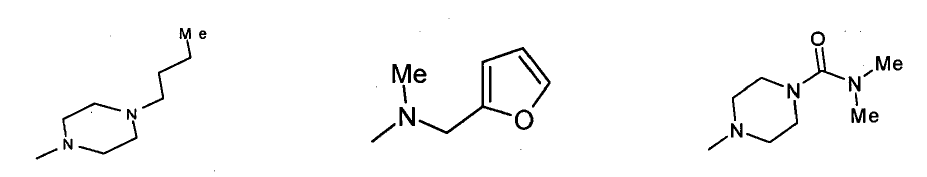 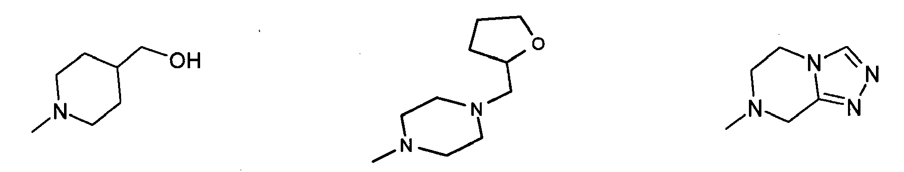 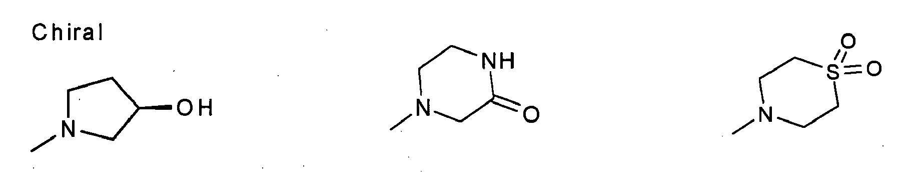 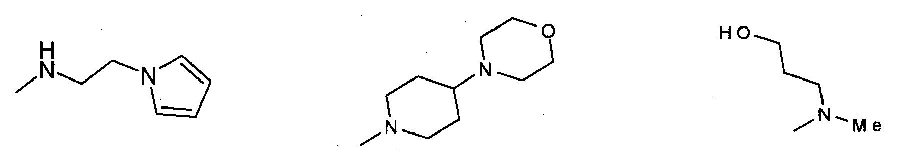 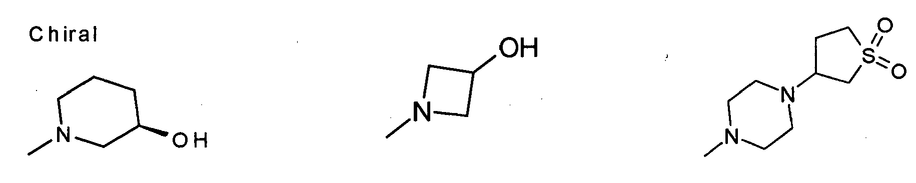 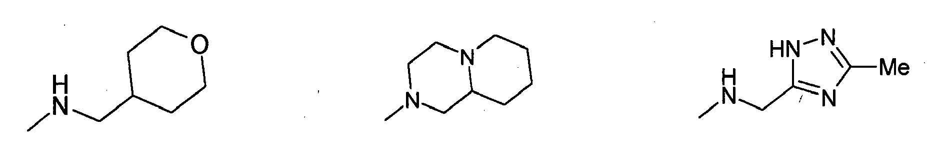 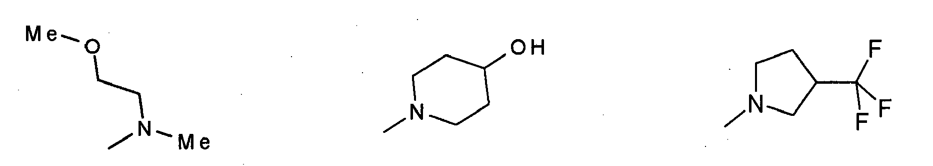 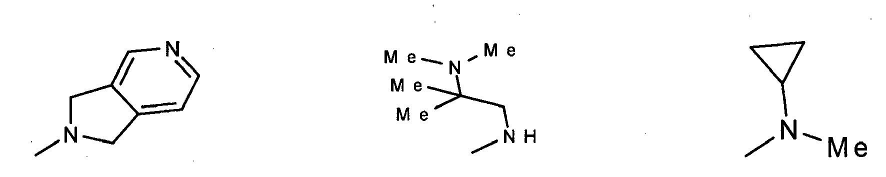 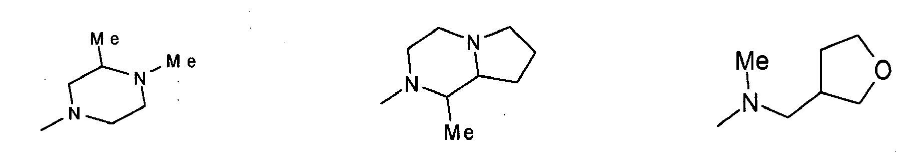 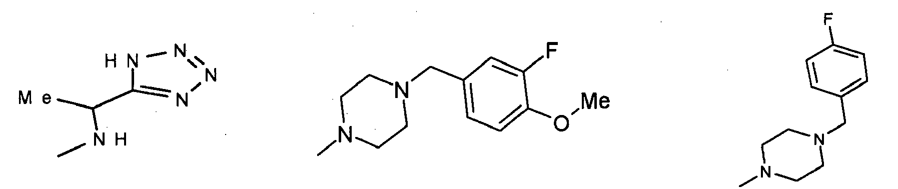 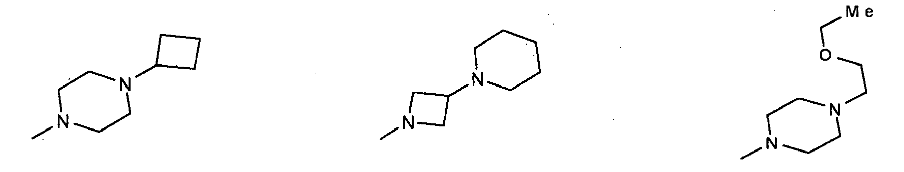 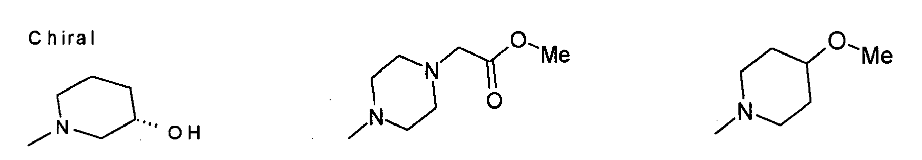 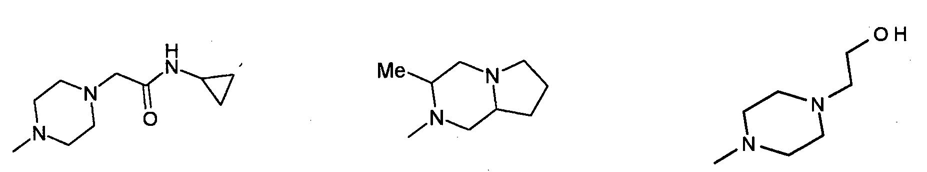 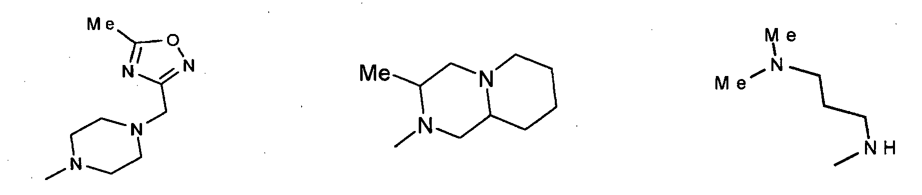 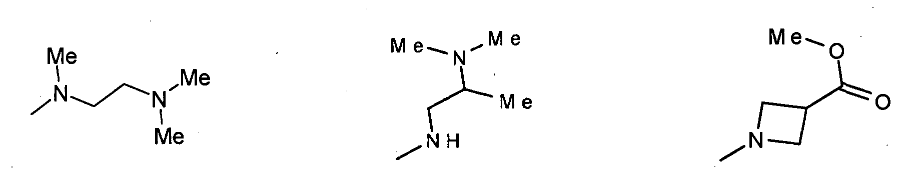 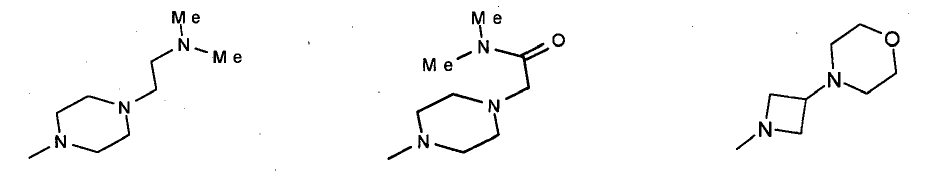 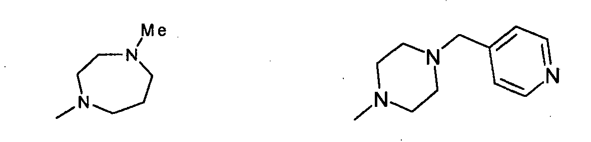
- R₄ représente un atome de chlore ;
- R₅ représente un atome d'hydrogène ; à l'état de base ou de sels d'addition à des acides ou à des bases.

Selon la présente invention, on distingue les composés de formule (I) dans laquelle
- A représente un radical aromatique bivalent : 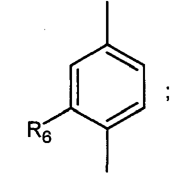
- X représente un groupe -CH- ou un atome d' azote ;
- R₁ représente un radical méthyle, éthyle, n-propyle ou un radical éthoxy ;
- R₂ représente un radical méthyle ;
- R₃ représente un hydroxy ou un groupe -NR₇R₈ choisi parmi : 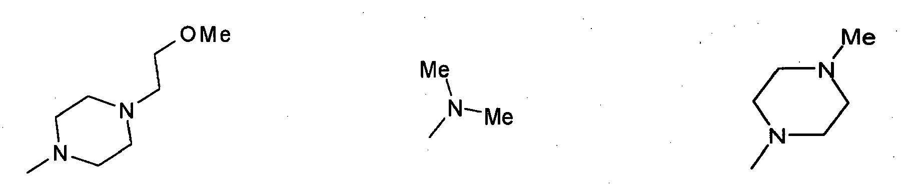 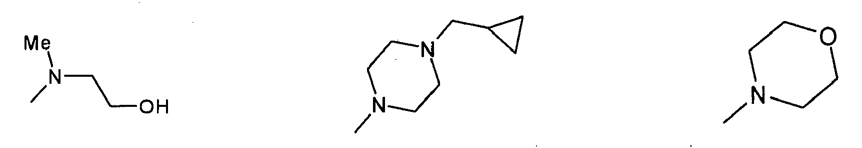 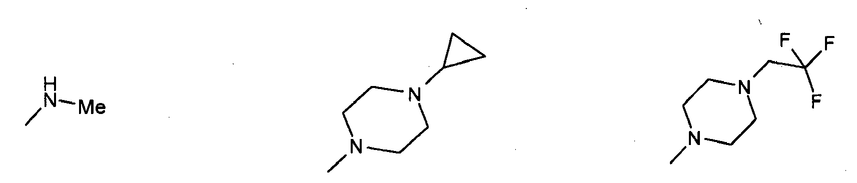 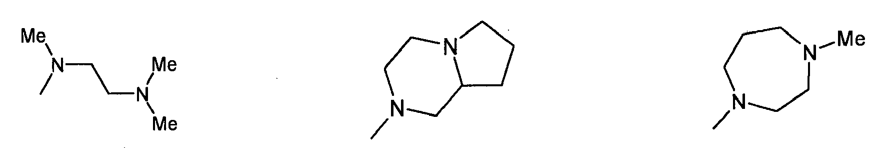 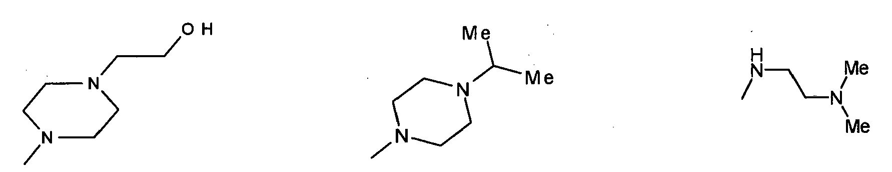 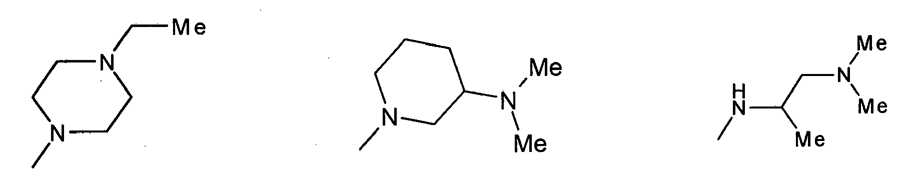 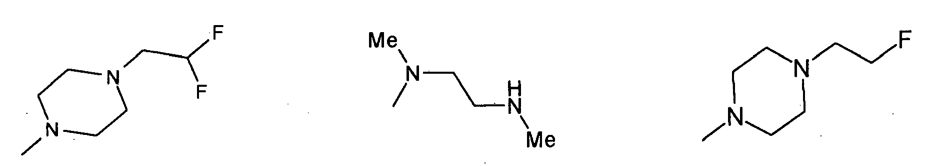 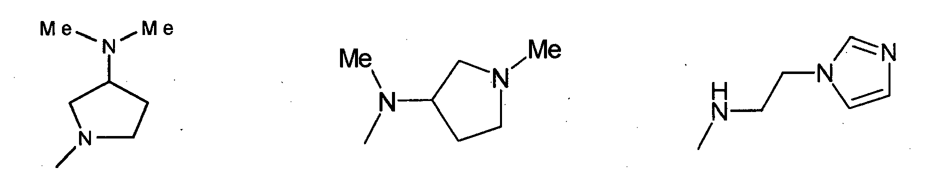 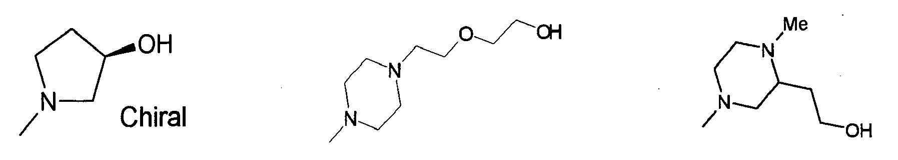 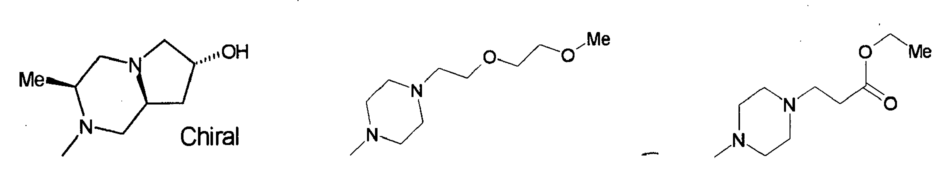 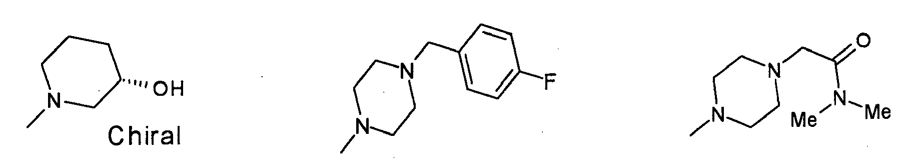 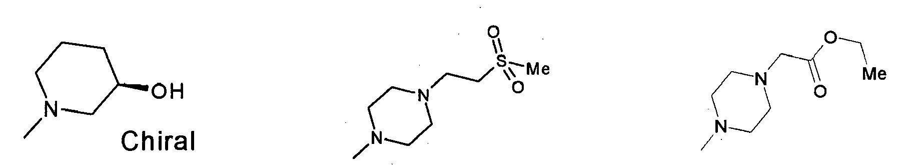 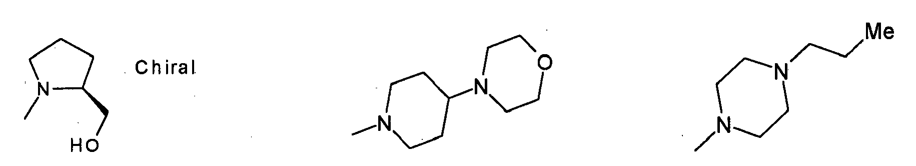 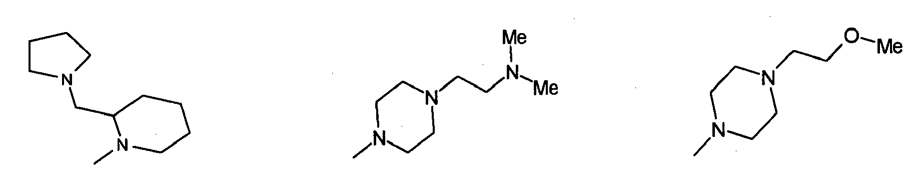 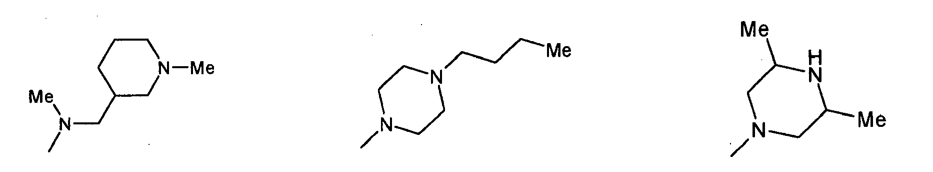 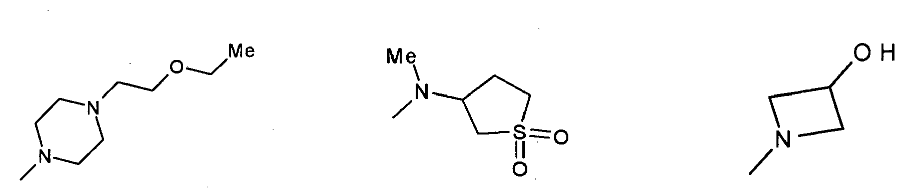 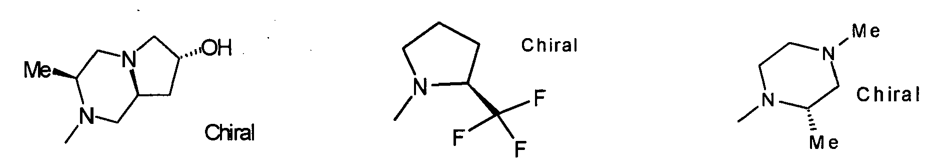 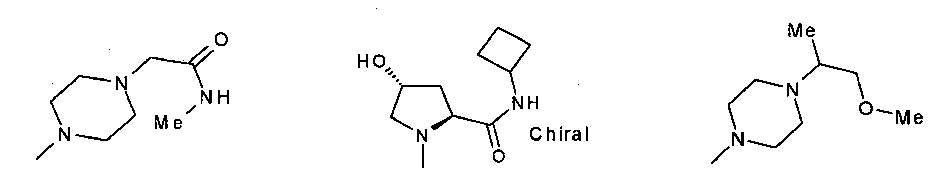 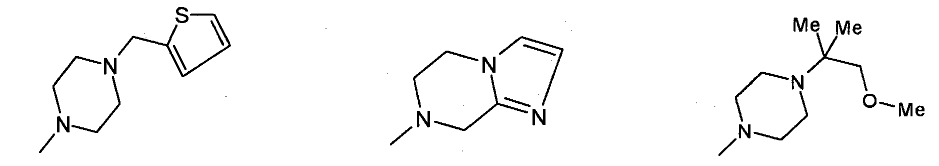 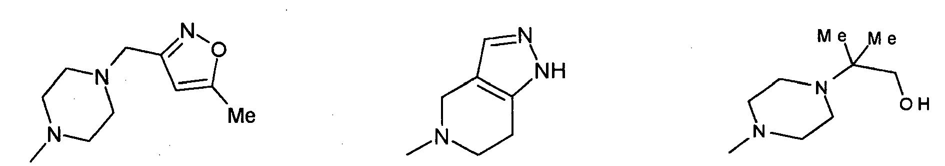 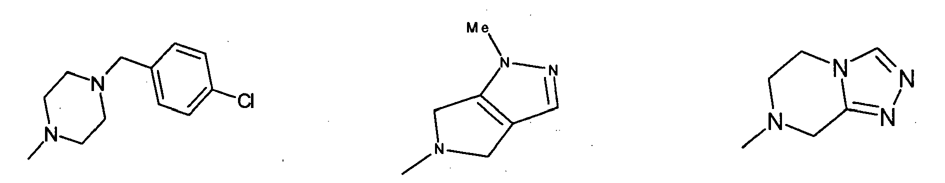 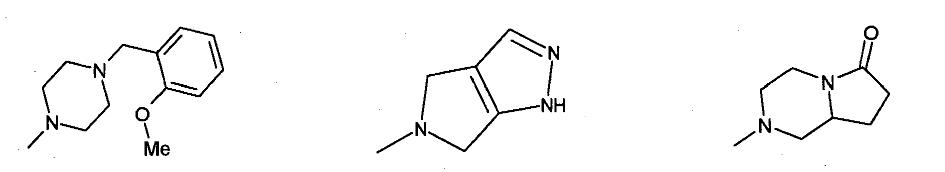 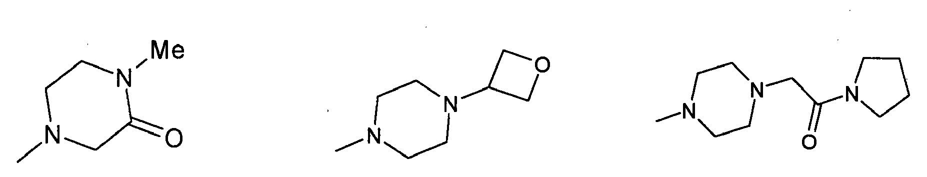 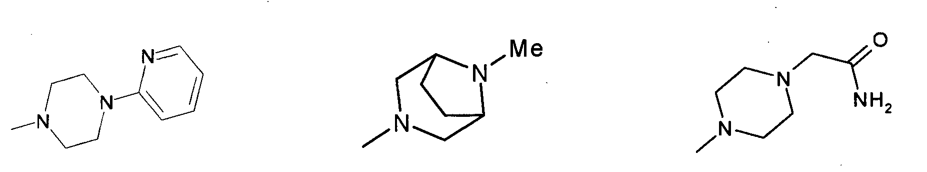 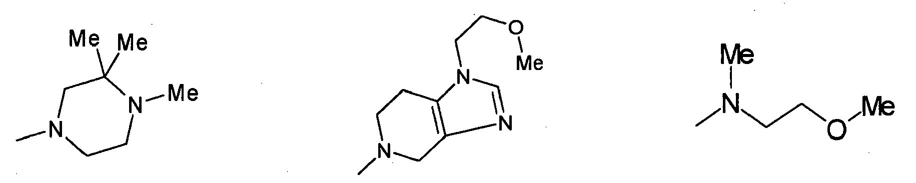 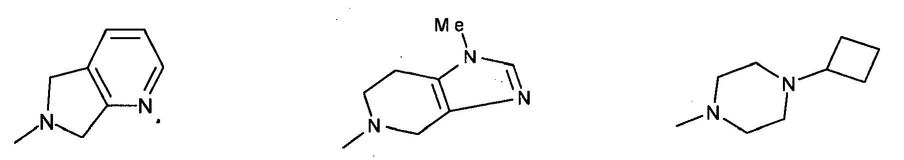 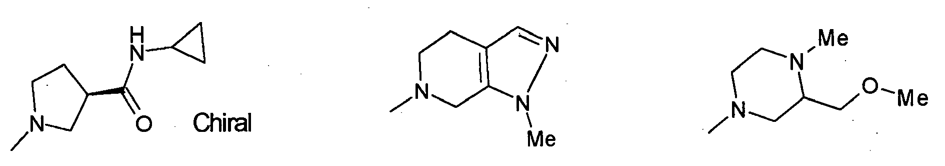 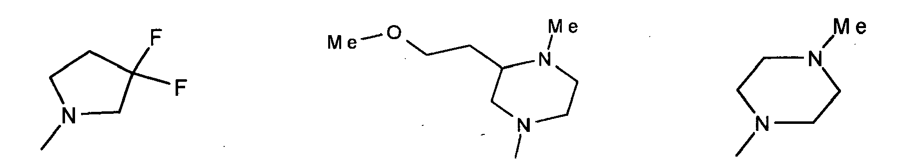 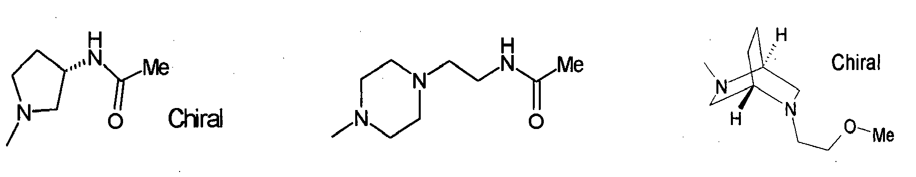 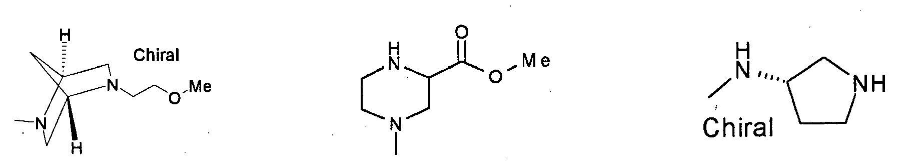 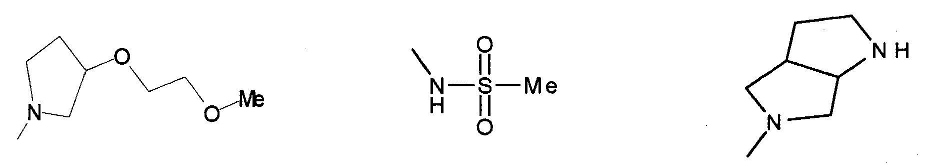 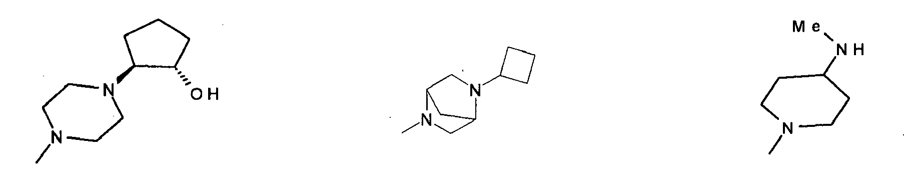 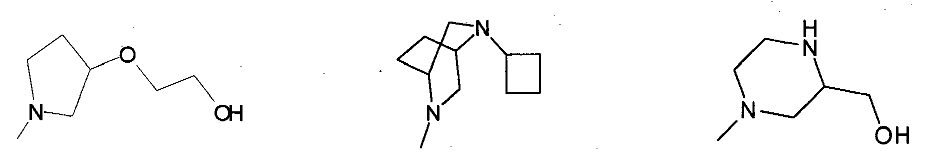 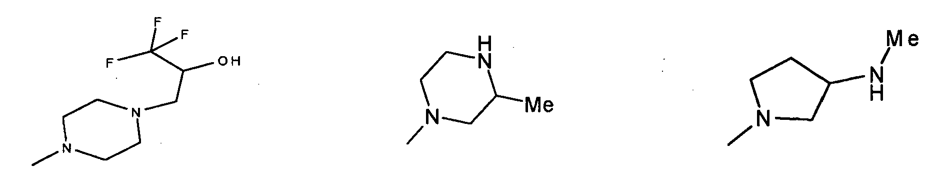 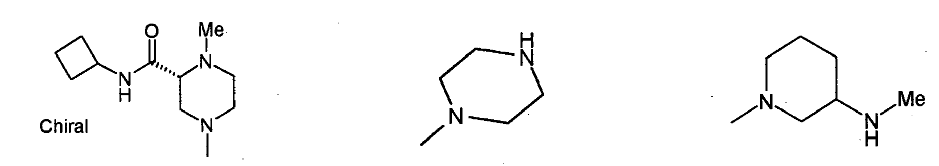 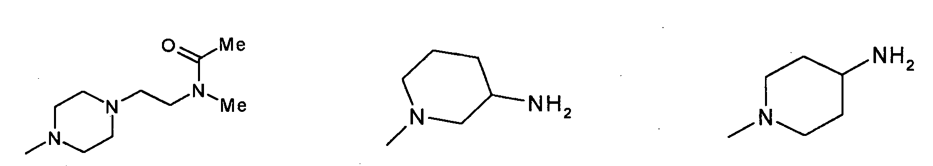 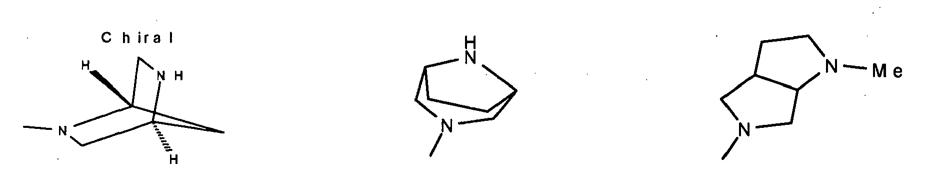 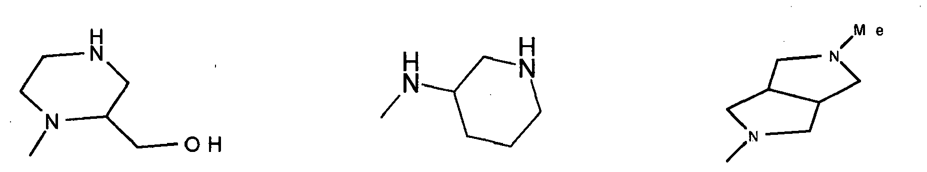 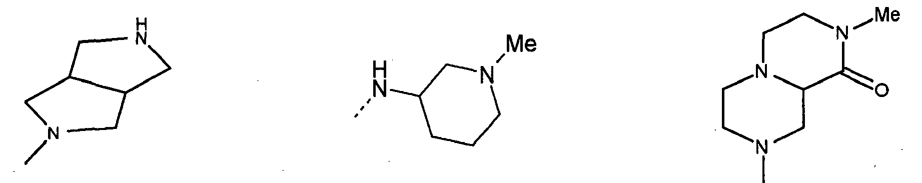 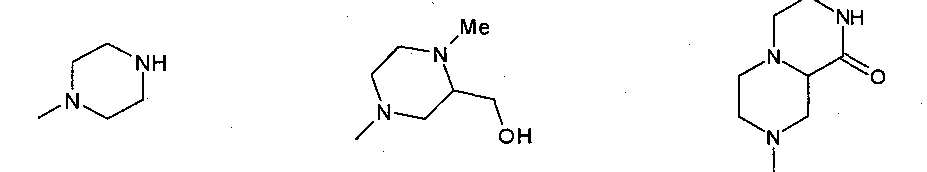 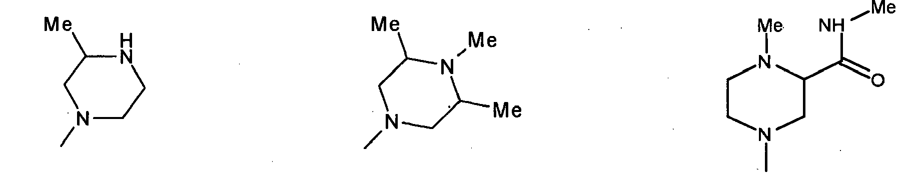 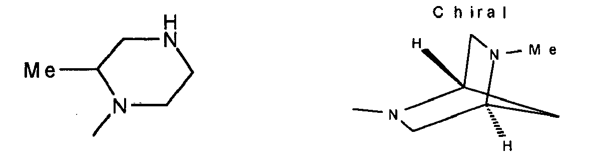
- R₄ représente un atome d'hydrogène, un atome de brome, de chlore ou de fluor, un cyano, un phényle, un radical méthyle, un radical trifluorométhyle, un radical méthoxy ou un groupe diméthylamino ;
- R₅ représente un atome d'hydrogène, un atome de brome, de chlore ou de fluor ou un radical méthyle ;
- R₆ représente un atome d'hydrogène, un atome de brome, un cyano, un groupe méthoxycarbonyle ou un groupe -CONH₂ ; à l'état de base ou de sels d'addition à des acides ou à des bases.

Selon la présente invention, on distingue les composés de formule (I) dans laquelle
- A représente un radical aromatique bivalent choisi parmi : 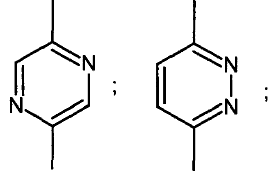
- X représente un groupe -CH- ;
- R₁ représente un radical n-propyle ;
- R₂ représente un radical méthyle ;
- R₃ représente un hydroxy ou un groupe -NR₇R₈ choisi parmi : 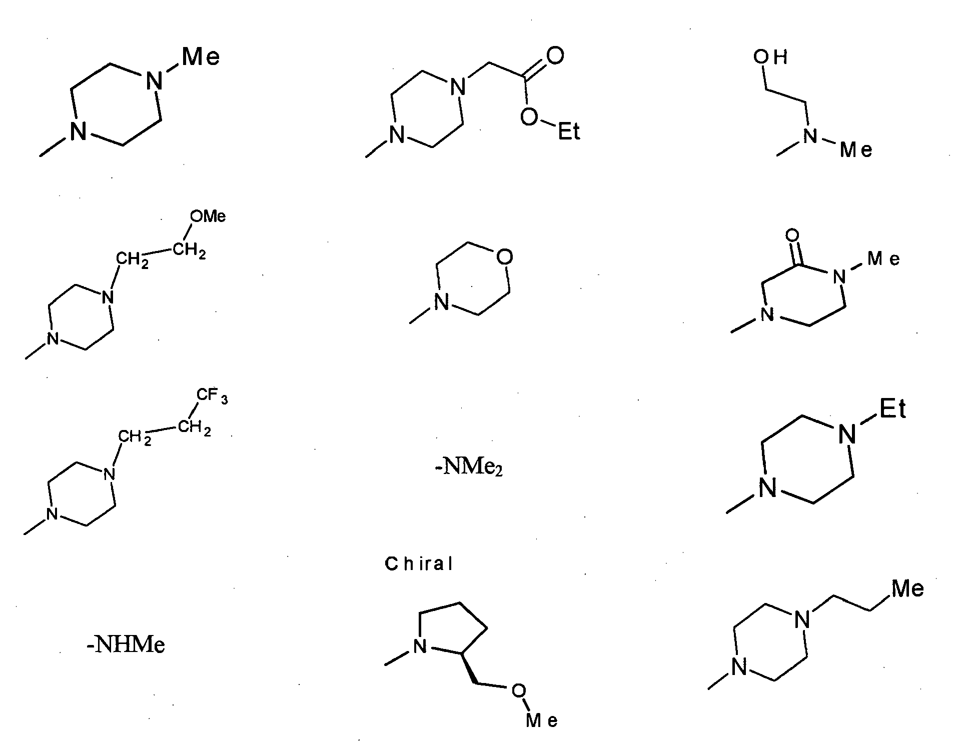 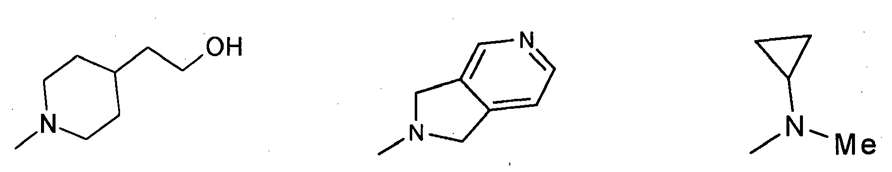 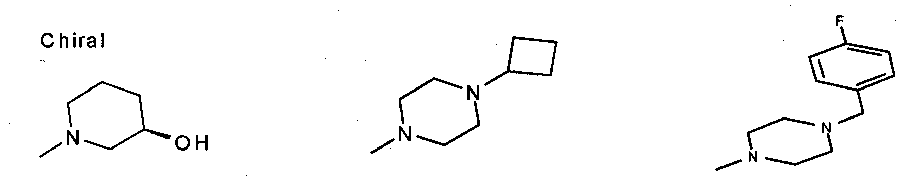 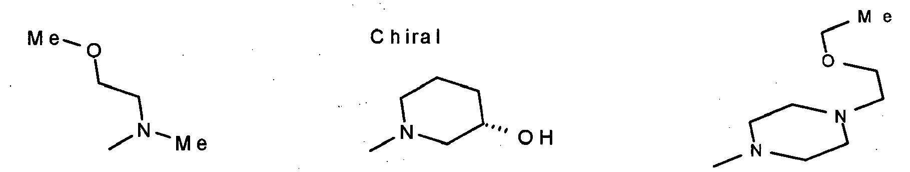 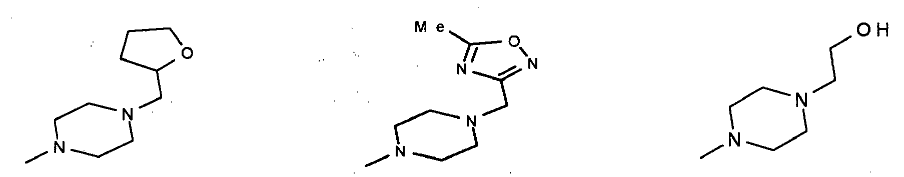 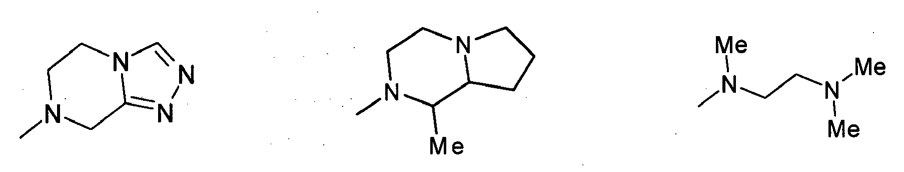 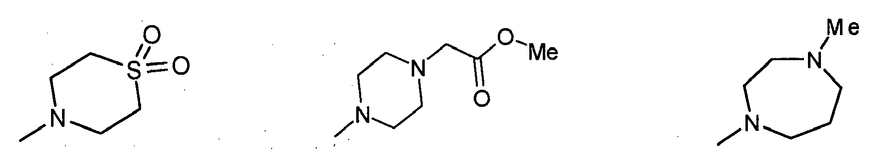
- R₄ représente un atome de chlore ;
- R₅ représente un atome d'hydrogène ;
à l'état de base ou de sels d'addition à des acides ou à des bases.

Selon la présente invention, on distingue les composés de formule (I) dans laquelle
- A représente un radical aromatique bivalent : 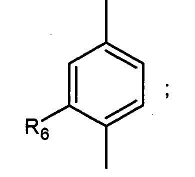
- X représente un groupe -CH- ou un atome d' azote ;
- R₁ représente un radical méthyle, éthyle, n-propyle ou un radical éthoxy ;
- R₂ représente un radical méthyle ;
- R₃ représente un hydroxy ou un groupe -NR₇R₈ choisi parmi : 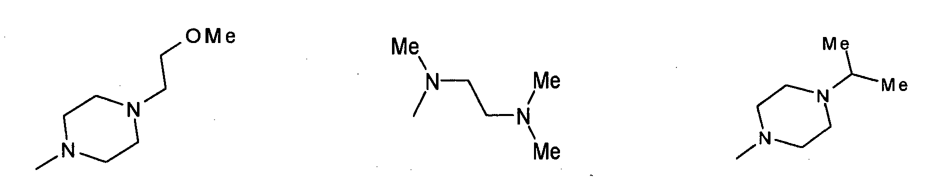 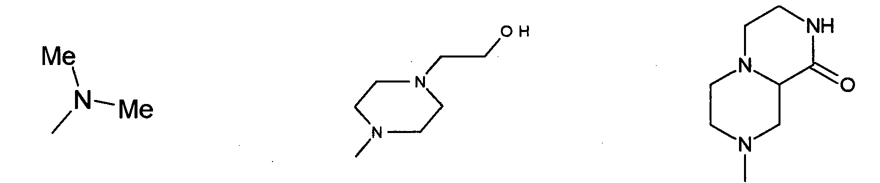 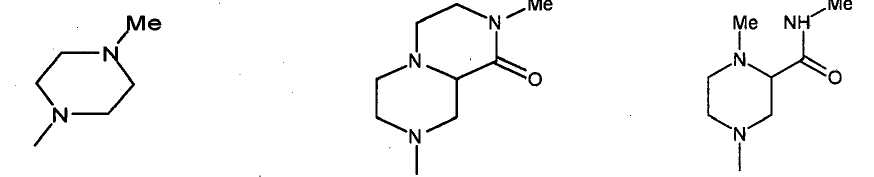 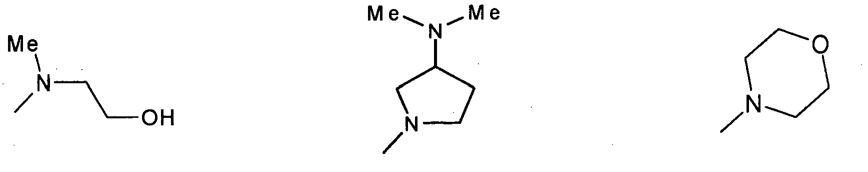 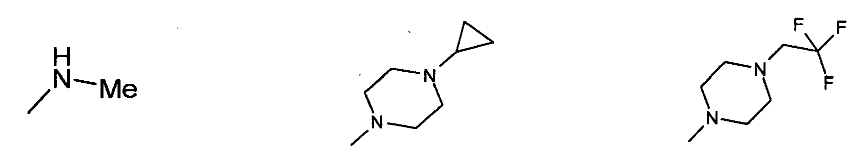 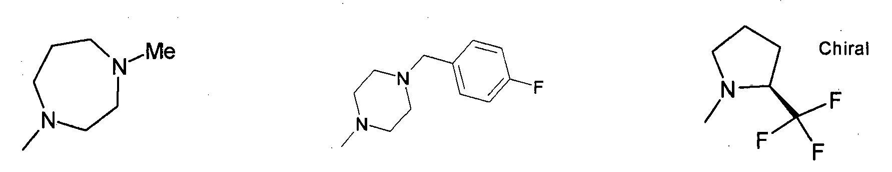 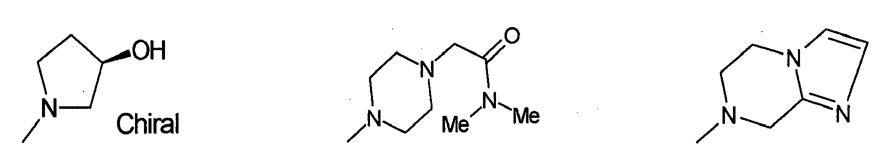 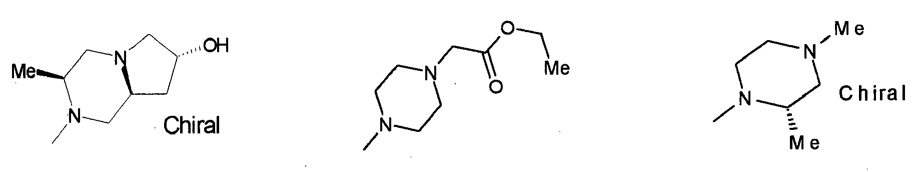 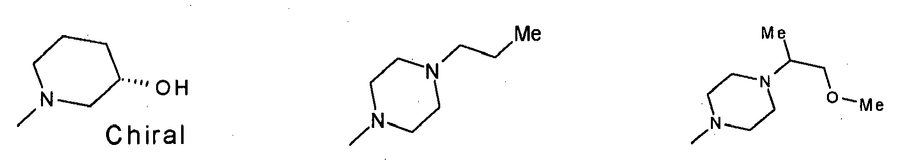 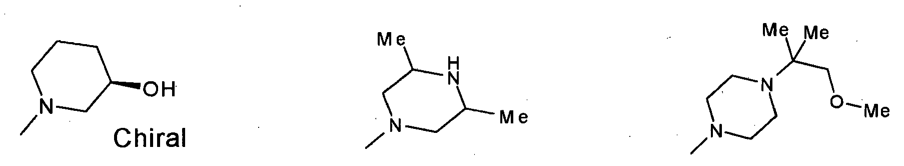 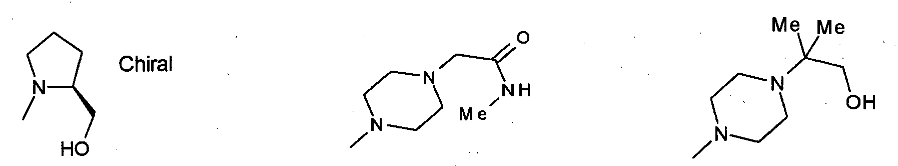 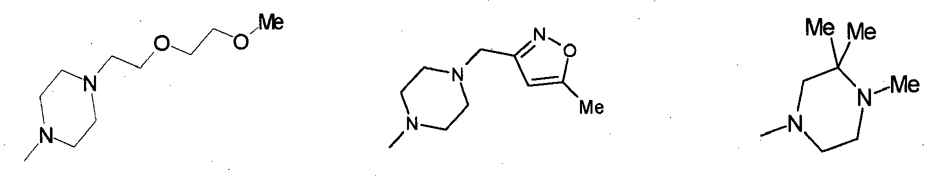 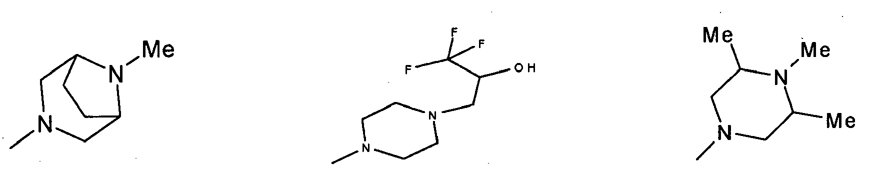 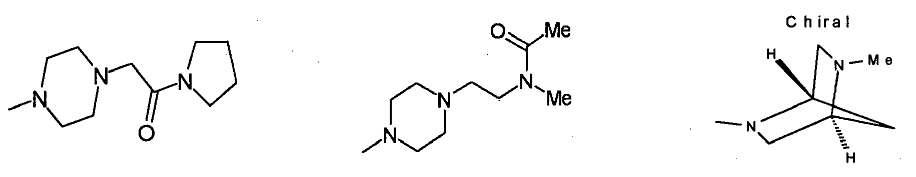 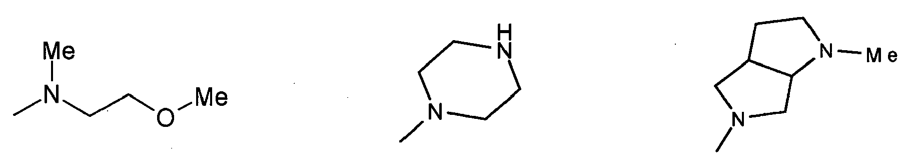 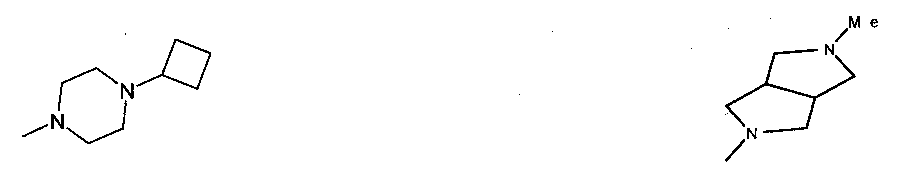 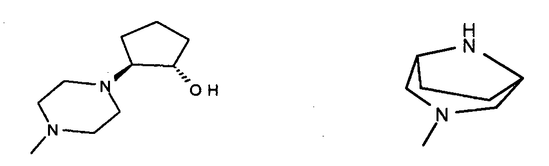
- R₄ représente un atome d'hydrogène, un atome de brome, de chlore ou de fluor, un cyano, un phényle, un radical méthyle, un radical trifluorométhyle, un radical méthoxy ou un groupe diméthylamino ;
- R₅ représente un atome d'hydrogène, un atome de brome, de chlore ou de fluor ou un radical méthyle ;
- R₆ représente un atome d'hydrogène, un atome de brome, un cyano, un groupe méthoxycarbonyle ou un groupe -CONH₂ ;
à l'état de base ou de sels d'addition à des acides ou à des bases.

Parmi les composés de formule (I), objets de l'invention, on peut notamment citer les composés suivants :
- Acide (3-{[4-(4-butyryl-5-méthyl-1*H*-pyrazol-1-yl)phényl]carbamoyl}-5-méthyl-1*H*-indol-1-yl)acétique ;
- Acide (3-{[4-(4-butyryl-5-méthyl-1*H*-pyrazol-1-yl)phényl]carbamoyl}-5,6-diméthyl -1*H*-indol-1-yl)acétique ;
- Acide (3-{[6-(4-butyryl-5-méthyl-1*H*-pyrazol-1-yl)pyridazin-3-yl]carbamoyl}-5-chloro-1*H*-indol-1-yl)acétique ;
- N-[5-(4-butyryl-5-méthyl-1*H*-pyrazol-1-yl)pyrazin-2-yl]-5-chloro-1-[2-(4-méthylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-[2-(4-méthylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-{2-[4-(2-méthoxyéthyl)pipérazin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-oxo-2-[4-(3,3,3-trifluoropropyl)pipérazin-1-yl]éthyl}-1*H*-indole-3-carboxamide ;
- N-[6-(4-butyryl-5-méthyl-1*H*-1-pyrazol-1-yl)pyridazin-3-yl]-5-chloro-1-[2-(méthylamino)-2-oxoéthyl]-1*H*-indole-3-carboxamide ;
- N-[6-(4-butyryl-5-méthyl-1*H*-pyrazol-1-yl)pyridazin-3-yl]-5-chloro-1-(2-morpholin-4-yl-2-oxoéthyl)-1*H*-indole-3-carboxamide ;
- N-[6-(4-butyryl-5-méthyl-1*H*-1-pyrazol-1-yl)pyridazin-3-yl]-5-chloro-1-[2-(diméthylamino)-2-oxoéthyl]-1*H*-indole-3-carboxamide ;
- N-[6-(4-butyryl-5-méthyl-1*H*-pyrazol-1-yl)pyridazin-3-yl]-5-chloro-1-{2-[(2S)-2-(méthoxyméthyl)pyrrolidin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-[2-(4-éthylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-[2-(4-propylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-{2-[(2-méthoxyéthyl)(méthyl)amino]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-oxo-2-[4-(teétrahydrofuran-2-ylméthyl)pipérazin-1-yl]éthyl}-1*H*-indole-3-carboxamide ;
- N-[6-(4-butyryl-5-méthyl-1*H*-pyrazol-1-yl)pyridazin-3-yl]-5-chloro-1-[2-(5,6-dihydro[1,2,4]triazolo[4,3-a]pyrazin-7(8*H*)-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-[2-(4-cyclobutylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-(2-{4-[(5-méthyl-1,2,4-oxadiazol-3-yl)méthyl]pipérazin-1-yl}-2-oxoéthyl)-1*H*-indole-3-carboxamide ;
- {4-[(3-{[6-(4-butyryl-5-méthyl-1*H*-pyrazol-1-yl)pyridazin-3-yl]carbamoyl}-5-chloro-1*H*-indol-1-yl)acétyl]pipérazin-1-yl} acétate de méthyle ;
- N-[6-(4-butyryl-5-méthyl-1*H*-pyrazol-1-yl)pyridazin-3-yl]-5-chloro-1-{2-[cyclopropyl(méthyl)amino]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-[4-(4-fluorobenzyl)pipérazin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-[4-(2-éthoxyéthyl)pipérazin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-[4-(2-hydroxyéthyl)pipérazin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-(2-{[2-(diméthylamino)éthyl](méthyl)amino}-2-oxoéthyl)-1*H*-indole-3-carboxamide ;
- N-[6-(4-butyryl-5-méthyl-1*H*-pyrazol-1-yl)pyridazin-3-yl]-5-chloro-1-[2-(4-méthyl-1,4-diazépan-1-yl)-2-oxoéthyl]-1*H*-indole-3-carboxamide ;
- N-[4-(4-butyryl-5-méthyl-1*H*-pyrazol-1-yl)phényl]-5-chloro-1-[2-(8-méthyl-9-oxooctahydro-2*H*-pyrazino[1,2-a]pyrazin-2-yl)-2-oxoéthyl]-1*H*-indole-3-carboxamide ;
- N-[4-(4-butyryl-5-méthyl-1*H*-pyrazol-1-yl)-2-cyanophényl]-5-chloro-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-1-{2-[4-(4-fluorobenzyl)pipérazin-1-yl]-2-oxoéthyl}-1*H*-indole-3-carboxamide ;
- N-[4-(4-butyryl-5-méthyl-1*H*-pyrazol-1-yl)phényl]-5-chloro-1-{2-[4-(2-méthoxyéthyl)pipérazin-1-yl]-2-oxoéthyl}-1*H*-indole-3-carboxamide ;
- N-[4-(4-butyryl-5-méthyl-1*H*-pyrazol-1-yl)phényl]-1-{2-[4-(2-methoxyéthyl)pipérazin-1-yl]-2-oxoéthyl}-5-méthyl-1*H*-indole-3-carboxamide ;
- N-[4-(4-butyryl-5-méthyl-1*H*-pyrazol-1-yl)phényl]-5-chloro-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-1-{2-[4-(2-méthoxy-1-méthyléthyl)pipérazin-1-yl]-2-oxoéthyl}-1*H*-indole-3-carboxamide ;
- N-[4-(4-butyryl-5-méthyl-1*H*-pyrazol-1-yl)phényl]-5-méthyl-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-6-fluoro-1-{2-[4-(2-methoxyéthyl)pipérazin-1-yl]-2-oxoéthyl}-1*H*-indole-3-carboxamide ;
- N-[4-(4-butyryl-5-méthyl-1*H*-pyrazol-1-yl)phényl]-5-méthoxy-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-1-{2-[4-méthyl-3-(méthylcarbamoyl)pipérazin-1-yl]-2-oxoéthyl}-1*H*-indole-3-carboxamide ;
- N-[4-(4-butyryl-5-méthyl-1*H*-pyrazol-1-yl)phényl]-5,6-diméthyl-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-méthyl-1-[2-(4-méthylpipérazin-1-yl)-2-oxoéthyl]-1*H*-indazole-3-carboxamide ;
- N-[4-(4-butyryl-5-méthyl-1*H*-pyrazol-1-yl)phényl]-5-méthyl-1-(2-oxo-2-pipérazin-1-yléthyl)-1*H*-indole-3-carboxamide ;
- N-[4-(4-butyryl-5-méthyl-1*H*-pyrazol-1-yl)phényl]-5-chloro-6-fluoro-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-1-(2-{4-[(5-méthylisoxazol-3-yl)méthyl]pipérazin-1-yl}-2-oxoéthyl)-1*H*-indole-3-carboxamide ;
- N-[4-(4-butyryl-5-méthyl-1*H*-pyrazol-1-yl)phényl]-5-méthyl-1-(2-morpholin-4-yl-2-oxoéthyl)-1*H*-indole-3-carboxamide ;
- N-[4-(4-butyryl-5-méthyl-1*H*-pyrazol-1-yl)phényl]-5-chloro-1-(2-oxo-2-pipérazin-1-yléthyl)-1*H*-indole-3-carboxamide ;
- N-[4-(4-butyryl-5-méthyl-1*H*-pyrazol-1-yl)phényl]-5-chloro-1-{2-[4-(2-méthoxy-1,1-diméthyléthyl)pipérazin-1-yl]-2-oxoéthyl}-1*H*-indole-3-carboxamide ;
- N-[4-(4-butyryl-5-méthyl-1*H*-pyrazol-1-yl)phényl]-5-chloro-1-{2-[4-(2-hydroxy-1,1-diméthyléthyl)pipérazin-1-yl]-2-oxoéthyl}-1*H*-indole-3-carboxamide ;
- N-[4-(4-butyryl-5-méthyl-1*H*-pyrazol-1-yl)phényl]-5-chloro-1-[2-(8-méthyl-3,8-diazabicyclo[3.2.1]oct-3-yl)-2-oxoéthyl]-1*H*-indole-3-carboxamide ;
- N-[4-(4-butyryl-5-méthyl-1*H*-pyrazol-1-yl)phényl]-1-[2-(4-méthylpipérazin-1-yl)-2-oxoéthyl]-5-(trifluorométhyl)-1*H*-indole-3-carboxamide ;
- N-[4-(4-butyryl-5-méthyl-1*H*-pyrazol-1-yl)phényl]-5-chloro-1-[2-oxo-2-(9-oxooctahydro-2H-pyrazino[1,2-a]pyrazin-2-yl)éthyl]-1H-indole-3-carboxamide ;
à l'état de base ou de sel d'addition à un acide ou à une base.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans "Protective Group in Organic Synthesis", Green et al., 4th Edition, John Wiley & Sons, Inc., New York, 2007.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans "Advanced Organic Chemistry", M.B. Smith and J. March, 6th Edition, Wiley Interscience, 2007, p. 496-501.

Conformément à l'invention, on peut préparer les composés de formule (I) dans laquelle R₃ = -OH (composé IA) selon un procédé qui est caractérisé en ce que :
on hydrolyse, en milieu acide ou basique, un composé de formule (II) : 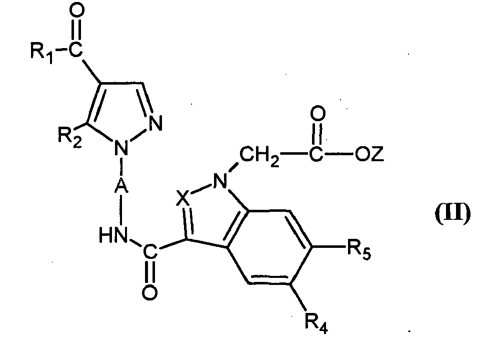
dans laquelle A, X, R₁, R₂, R₄ et R₅ sont tels que définis pour un composé de formule (I) et Z représente un (C₁-C₄) alkyle.

Eventuellement, on transforme le composé de formule (I) en l'un de ses sels avec des bases minérales ou organiques.

La réaction s'effectue en milieu acide par action d'un acide fort, comme, par exemple, l'acide chlorhydrique ou l'acide sulfurique, dans un solvant tel que, par exemple, le dioxane ou l'eau et à une température comprise entre -10°C et 110°C.

La réaction s'effectue en milieu basique par action d'une base alcaline, comme, par exemple, l'hydroxyde de potassium, l'hydroxyde de lithium ou l'hydroxyde de sodium, dans un solvant tel que, par exemple, le dioxane, le tétrahydrofurane, l'eau, le méthanol, l'éthanol ou un mélange de ces solvants et à une température comprise entre -10°C et la température de reflux du solvant.

Conformément à l'invention, on peut préparer les composés de formule (I) dans laquelle R₃ = -NR₇R₈ (composé IB) selon un procédé qui est caractérisé en ce que :
on fait réagir un composé de formule (IA) : 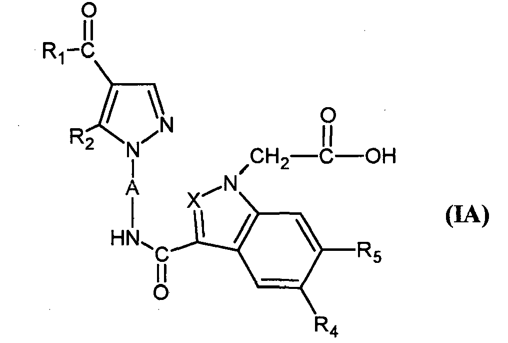
dans laquelle A, X, R₁, R₂, R₄ et R₅ sont tels que définis pour un composé de formule (I) avec une amine de formule (III) :

HNR₇R₈ **(III)**

dans laquelle R₇ et R₈ sont tels que définis pour un composé de formule (I).

Eventuellement, on transforme le composé de formule (I) en l'un de ses sels avec des acides minéraux ou organiques.

La réaction s'effectue en présence d'une base comme, par exemple, la triéthylamine, la 4-diméthylaminopyridine, la N-méthylmorpholine, la N-éthylmorpholine ou la pyridine et en présence d'un agent de couplage comme, par exemple, le chlorure de bis(2-oxo-1,3-oxazolidin-3-yl)phosphinique, le chloroformate d'isobutyle, le 1,1'-carbonyldiimidazole, le chlorohydrate de 1-(3-diméthylaminopropyl)-3-éthylcarbodiimide, le 2-cyano-2-(hydroxyimino)acétate d'éthyle. Le solvant utilisé est, par exemple, le dichlorométhane, le 1,2-dichloroéthane ou le N,N-diméthylformamide. La température de la réaction est comprise entre -10°C et la température de reflux du solvant.

De façon particulière, on peut préparer certains composés de formule (I) à partir d'autres composés de formule (I).

Les composés de formule (I) ainsi obtenus peuvent être ultérieurement séparés du milieu réactionnel et purifiés selon les méthodes classiques, par exemple, par cristallisation ou chromatographie sur colonne de silice.

Les composés de formule (I) ainsi obtenus sont isolés sous forme de base libre ou de sel, selon les techniques classiques.

Les composés de formule (II) peuvent se préparer par réaction d'un acide ou d'un dérivé fonctionnel activé de cet acide de formule : 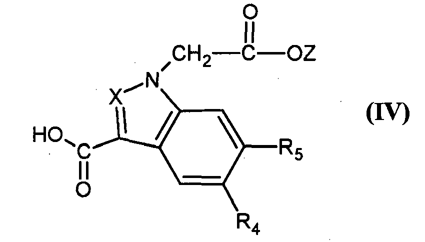 dans laquelle X, R₄ et R₅ sont tels que définis pour un composé de formule (I) et Z représente un (C₁-C₄)alkyle, avec un composé de formule : 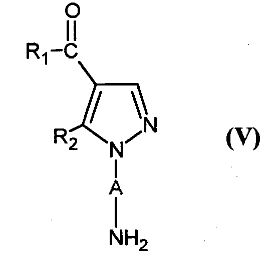 dans laquelle A, R₁ et R₂ sont tels que définis pour un composé de formule (I).

Lorsqu'on traite un composé de formule (V) avec l'acide de formule (IV) lui-même, on opère en présence d'un agent de couplage utilisé en chimie peptidique tel que le 1,3-dicyclohexylcarbodiimide ou l'hexafluorophosphate de benzotriazol-1-yl-oxytris(diméthylamino)phosphonium ou l'hexafluorophosphate de benzotriazol-1-yl-oxytris(pyrrolidino)phosphonium ou le tétrafluoroborate de 2-(1*H*-benzotriazol-1-yl)-1,1,3,3-tétraméthyluronium ou le chlorhydrate de 1-(3-diméthylaminopropyl)-3-éthylcarbodiimide, en présence d'une base telle que la triéthylamine, la N,N-diisopropyléthylamine ou la 4-diméthylaminopyridine, dans un solvant tel que le dichlorométhane, le 1,2-dichloroéthane, le N-N-diméthylformamide ou le tétrahydrofurane à une température comprise entre -10°C et la température de reflux du solvant.

Comme dérivé fonctionnel activé de l'acide (IV) on peut utiliser le chlorure d'acide, l'anhydride, un anhydride mixte, un ester alkylique en C₁-C₄ dans lequel l'alkyle est linéaire ou ramifié, un ester activé, par exemple l'ester de *p*-nitrophényle.

Ainsi, on peut, par exemple, faire réagir le chlorure de l'acide obtenu par réaction du chlorure de thionyle ou du chlorure d'oxalyle sur l'acide de formule (IV), avec le composé de formule (V), dans un solvant, tel qu'un solvant chloré (le dichlorométhane, le 1,2-dichloroéthane, le chloroforme par exemple), un éther (tétrahydrofurane, dioxane par exemple), un amide (N,N-diméthylformamide par exemple) ou la pyridine, sous une atmosphère inerte, à une température comprise entre 0°C et la température de reflux du solvant, en présence d'une amine tertiaire telle que la triéthylamine, la N-méthylmorpholine, la pyridine, la 4-diméthylaminopyridine ou le 1,8-diazabicyclo[5.4.0]undec-7-ène.

Les composés de formule (II) peuvent également se préparer par réaction d'un composé de formule : 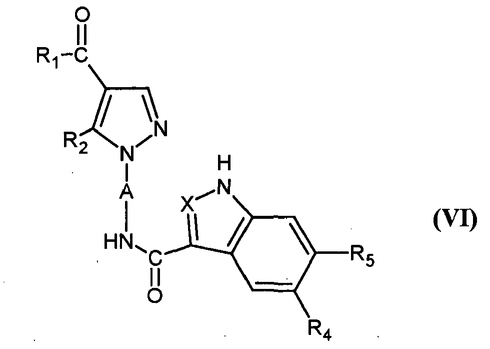 dans laquelle A, X, R₁, R₂, R₄ et R₅ sont tels que définis pour un composé de formule (I), avec un composé de formule : 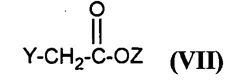 dans laquelle Y représente un groupe partant tel qu'un atome d'halogène, un méthanesulfonate, un benzènesulfonate, un p-toluène sulfonate ou un triflate et Z représente un (C₁-C₄)alkyle.

La réaction s'effectue en présence d'une base telle que, par exemple, le carbonate de sodium ou de potassium, dans un solvant tel que, par exemple, le N,N-diméthylformamide et à une température comprise entre -20°C et la température de reflux du solvant.

De façon particulière, on peut préparer certains composés de formule (II) à partir d'autres composés de formule (II).

Ainsi, par exemple, à partir des composés de formule (II) dans laquelle R₄ = Br on peut préparer les composés de formule (II) :
- dans laquelle R₄ = phényle, par réaction avec l'acide phénylboronique en présence d'un catalyseur au palladium tel que le tétrakis(triphénylphosphine)palladium (0) ;
- dans laquelle R₄ = -NR₉R₁₀, par réaction avec une amine HNR₉R₁₀ en présence d'un catalyseur au cuivre (I) tel que l'iodure de cuivre (I) ;
- dans laquelle R₄ = CN, par réaction avec du cyanure de zinc (II) et en présence d'un catalyseur tel que le tétrakis(triphénylphosphine)palladium (0).

A partir des composés de formule (II) dans laquelle : 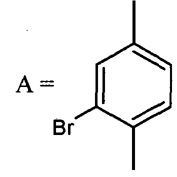 on peut préparer les composés de formule (II) dans laquelle : 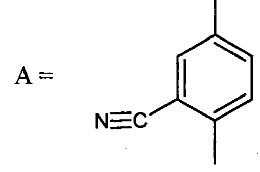 par action du cyanure de zinc (II) en présence de tétrakis (triphénylphosphine)palladium (0).

Les composés de formule (III) sont connus, commercialisés ou se préparent selon des méthodes connues de l'homme de l'art telles que, par exemple, celles décrites dans WO 95/18105.
Les composés de formule (IV) se préparent par réaction d'un composé de formule: 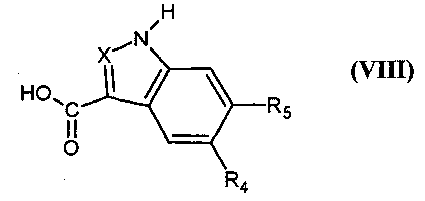 dans laquelle X, R₄ et R₅ sont tels que définis pour un composé de formule (I), avec un composé de formule (VII) : 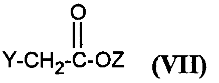 dans laquelle Y représente un groupe partant tel qu'un atome d'halogène, un méthanesulfonate, un benzènesulfonate, un p-toluènesulfonate ou un triflate et Z représente un (C₁-C₄)alkyle.

La réaction s'effectue en présence de deux équivalents d'une base forte comme, par exemple, l'hydrure de sodium, dans un solvant comme, par exemple, le N,N-diméthylformamide et à une température comprise entre -30°C et la température de reflux du solvant.

Les composés de formule (V) se préparent par réaction d'un composé de formule : 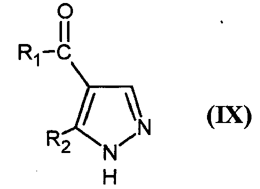 dans laquelle R₁ et R₂ sont tels que définis pour un composé de formule (I), avec un composé de formule :

Y-A-NH₂ **(X)**

dans laquelle A est tel que défini pour un composé de formule (I) et Y représente un groupe partant tel qu'un atome d'halogène, un méthanesulfonate, un benzène sulfonate, un p-toluènesulfonate, un triflate ou un acétate.

La réaction s'effectue en présence d'une base comme, par exemple, le carbonate de potassium ou le carbonate de césium, en mélange avec de la proline. La réaction s'effectue en présence d'un agent métallique comme, par exemple, l'iodure de cuivre, dans un solvant tel que, par exemple, le diméthylsulfoxyde, le dioxane ou le tétrahydrofurane et à une température comprise entre 0°C et 150°C.

Les composés de formule (V) peuvent également se préparer par réduction d'un composé de formule : 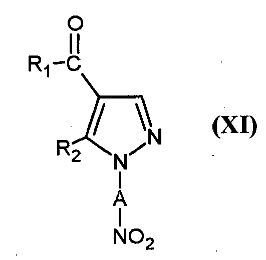 dans laquelle R₁ et R₂ sont tels que définis pour un composé de formule (I).

La réaction de réduction peut s'effectuer, par exemple, en présence d'un métal tel que le fer (0), le zinc ou l'étain avec un acide tel que, par exemple, l'acide acétique, dans un solvant tel que, par exemple, le méthanol, l'éthanol, l'eau ou un mélange de ces solvants et à une température comprise entre 0°C et la température de reflux du solvant. On peut également effectuer une réduction sous pression d'hydrogène en présence d'un catalyseur tel que, par exemple, le palladium.

Les composés de formule (V) dans laquelle R₁ représente un (C₁-C₄)alkyle peuvent également se préparer par hydrolyse acide d'un composé de formule : 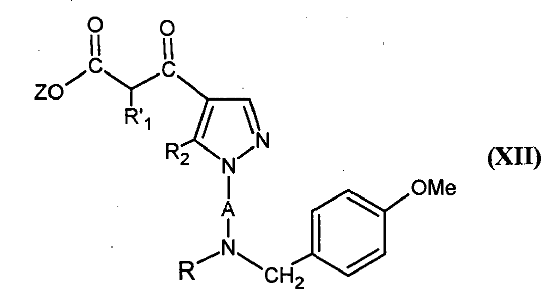 dans laquelle A et R₂ sont tels que définis pour un composé de formule (I), Z représente un (C₁-C₄) alkyle, R'₁ représente un atome d'hydrogène ou un (C₁-C₃) alkyle et R représente un *tert*-butoxycarbonyle ou un atome d'hydrogène.

La réaction s'effectue par action d'un acide fort comme, par exemple, l'acide chlorhydrique dans un solvant tel que l'eau et à une température comprise entre la température ambiante et la température de reflux du milieu réactionnel.

De façon particulière, on peut préparer certains composés de formule (V) à partir d'autres composés de formule (V).

Ainsi, par exemple, à partir des composés de formule (V) dans laquelle A représente un phényle non substitué, on peut préparer les composés de formule (V) dans laquelle : 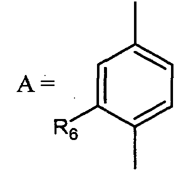 avec R₆ = Br, par réaction avec du N-bromosuccinimide;

A partir de ces composés de formule (V, R₆ = Br) ainsi obtenus, on prépare les composés de formule (V) dans laquelle : 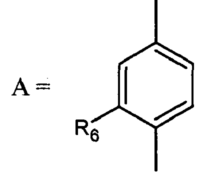 avec R₆ = CN, par réaction avec du cyanure de zinc;

A partir de ces composés de formule (V, R₆ = CN) ainsi obtenus, par hydrolyse basique, on prépare les composés de formule (V) dans laquelle : 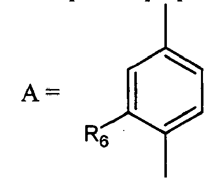 avec R₆ = COOH ou CONH₂

A partir de ces composés de formule (V, R6 = COOH) ainsi obtenus, on prépare, par alkylation, les composés de formule (V) dans laquelle : 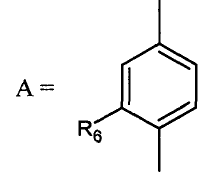 avec R₆ = COOAlk

Les composés de formule (VI) peuvent se préparer selon le SCHEMA 1 ci-après, dans lequel A, X, R₁, R₂, R₄ et R₅ sont tels que définis pour un composé de formule (I), et Pg représente un groupe N-protecteur, de préférence un groupe acétyle.

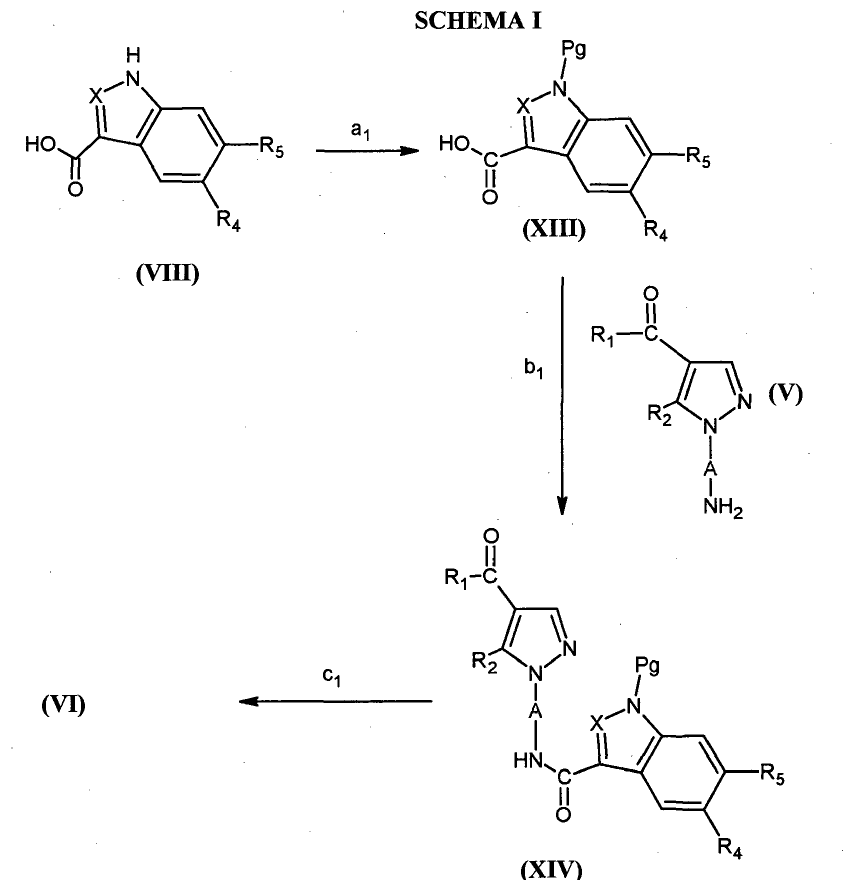

A l'étape a₁ du SCHEMA I, on protège sélectivement l'atome d'azote des composés de formule (VIII) à l'aide d'un agent d'acylation.

A l'étape b₁, on fait réagir l'acide ou un dérivé fonctionnel de cet acide de formule (XIII) avec un composé de formule (V) pour obtenir le composé de formule (XIV). La réaction s'effectue selon les méthodes précédemment décrites par la réaction d'un composé de formule (IV) avec le composé de formule (V).

A l'étape c₁, on déprotège le composé de formule (XIV) ainsi obtenu selon les méthodes classiques.

De façon particulière, on peut préparer certains composés de formule (VI) à partir d'autres composés de formlule (VI). Ainsi par exemple, à partir des composés de formule (VI) dans laquelle R₄ = Br on peut préparer les composés de formule (VI) dans laquelle R₄ = Cl par réaction avec du chlorure de nickel (II) selon les méthodes classiques.

Les composés de formule (VII) sont connus, commercialisés ou préparés selon des méthodes connues.

Les composés de formule (VIII) dans laquelle X = -CH- peuvent se préparer selon une adaptation de la méthode décrite dans Journal of Fluorine Chemistry (1977) 10, 437-445 et illustrée dans le SCHEMA II ci-après dans lequel R₄ et R₅ sont tels que définis pour un composé de formule (I).

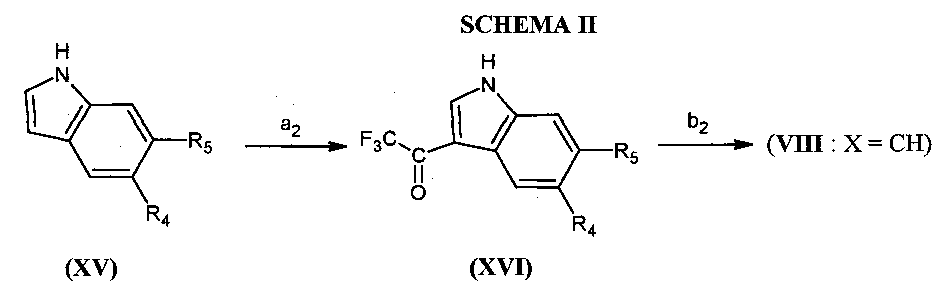

A l'étape a₂ du SCHEMA II, on fait réagir un composé de formule (XV) avec de l'anhydride trifluoroacétique, dans un solvant tel que, par exemple, l'éther diéthylique et à une température inférieure ou égale à 0°C.

A l'étape b₂, on hydrolyse le composé de formule (XVI) ainsi obtenu par action d'une base forte telle que, par exemple, l'hydroxyde de sodium ou l'hydroxyde de potassium. La réaction s'effectue dans un solvant comme, par exemple, l'eau ou l'éthanol et à une température comprise entre la température ambiante et la température de reflux du solvant.

Les composés de formule (VIII) dans laquelle X = -CH- peuvent également se préparer selon une adaptation de la méthode décrite dans Synthesis (1990) 215-218 et illustrée dans le SCHEMA III ci-après dans lequel R₄ et R₅ sont tels que définis pour un composé de formule (I) et Hal représente un atome d'halogène, de préférence le brome ou l'iode.

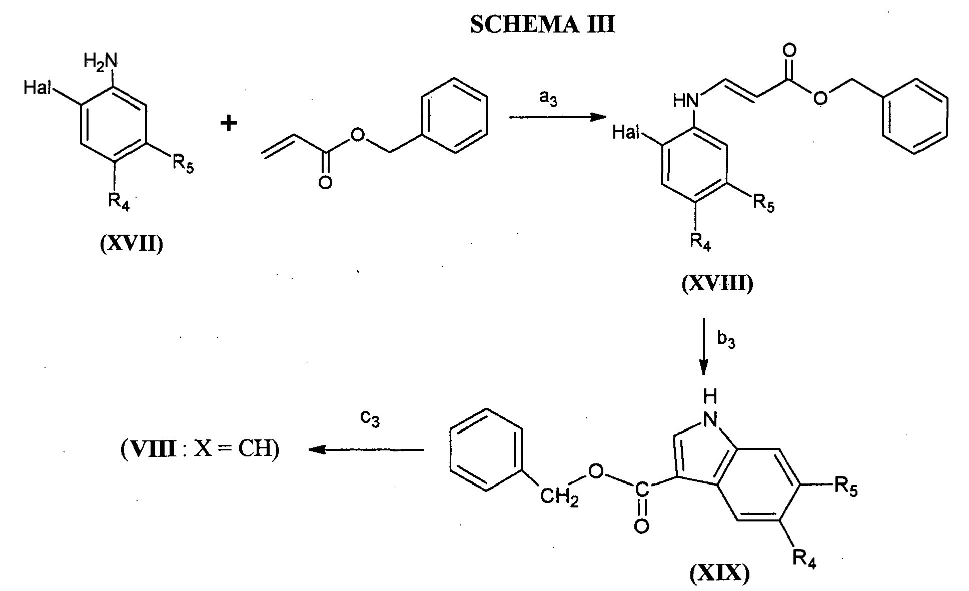

A l'étape a₃ du SCHEMA III, on fait réagir un composé de formule (XVII) avec de l'acrylate de benzyle, en présence de 1,4-benzoquinone, de chlorure de lithium et d'acétate de palladium. La réaction s'effectue dans un solvant comme, par exemple, le tétrahydrofurane et à une température comprise entre 0°C et la température de reflux du solvant.

A l'étape b₃, on cyclise le composé de formule (XVIII) ainsi obtenu en présence d'une base telle que, par exemple, la triéthylamine ou le 1,4-diazabicyclo[2.2.2]octane et en présence d'un complexe du palladium tel que, par exemple, l'acétate de palladium. La réaction s'effectue dans un solvant comme, par exemple, le N,N-diméthylformamide et à une température comprise entre la température ambiante et 130°C.

A l'étape c₃, le composé de formule (XIX) ainsi obtenu est débenzylé selon les méthodes connues (Protective Group in Organic Synthesis, Green et al., 4th Edition, John Wiley & Sons, Inc., New York, 2007) pour donner l'acide de formule (VIII) attendu.

Les composés de formule (VIII) dans laquelle X = N peuvent être préparés selon la méthode décrite dans Synthetic Communications (2005) 35(20), 2681-2684 et illustrée dans le SCHEMA IV ci-après dans lequel R₄ et R₅ sont tels que définis pour un composé de formule (I).

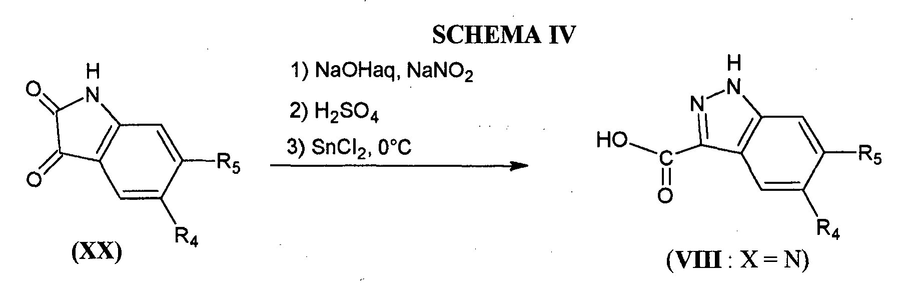

Les composés de formule (IX) dans laquelle R₁ = (C₁-C₄)alkyle peuvent se préparer selon le SCHEMA V ci-après dans lequel R₂ est tel que défini pour un composé de formule (I), Pg représente un groupe protecteur, de préférence un groupe trityle.

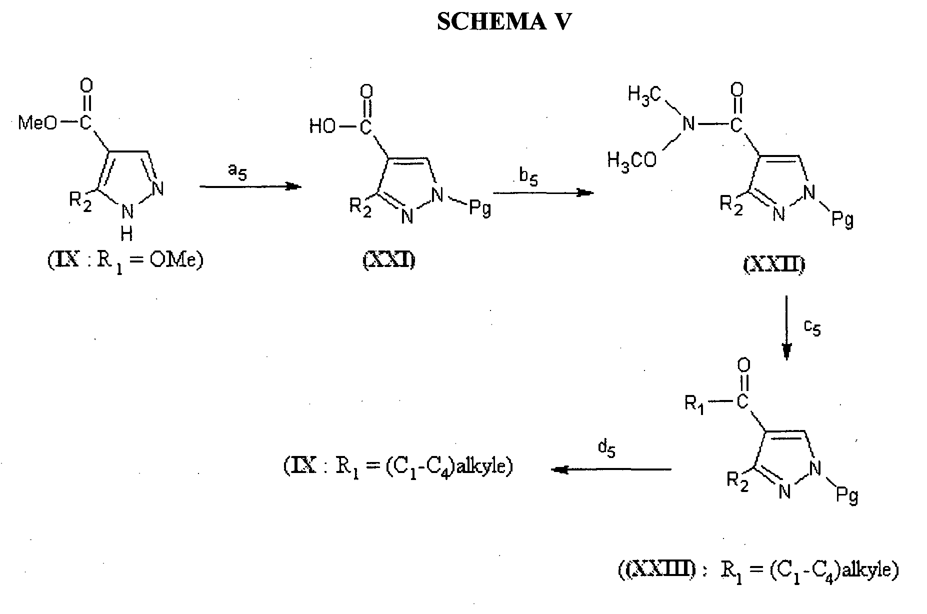

A l'étape a₅ du SCHEMA V, on protège l'atome d'azote du composé de formule (IX), en particulier par un groupe trityle, puis hydrolyse en milieu basique le composé intermédiaire obtenu.

A l'étape b₅, le composé de formule (XXI) ainsi obtenu est mis en réaction avec la N-méthoxyméthanamine, en présence d'un agent de couplage tel que, par exemple, le chlorure de bis(2-oxo-1,3-oxazolidin-3-yl)phosphinique et en présence d'une base telle que, par exemple, la 4-diméthylaminopyridine. La réaction s'effectue dans un solvant comme, par exemple, le dichlorométhane et à une température comprise entre 0°C et la température ambiante.

Le composé de formule (XXII) ainsi obtenu est mis à réagir à l'étape c₅ avec un composé organométallique tel qu'un halogénure de (C₁-C₄)alkylmagnésium, dans un solvant tel que l'éther diéthylique ou le tétrahydrofurane et à une température comprise entre -70°C et la température ambiante.

Le composé de formule (XXIII) ainsi obtenu est déprotégé à l'étape d₅ selon les méthodes classiques (Protective Group in Organic Synthesis, Green et al., 4th Edition, John Wiley & Sons, Inc., New York, 2007).

Les composés de formule (IX) dans laquelle R₁ = (C₁-C₄)alkyle peuvent également se préparer selon le SCHEMA VI ci-après dans lequel R₂ est tel que défini pour un composé de formule (I), Pg représente un groupe protecteur, de préférence un groupe trityle, Z représente un (C₁-C₄)alkyle et R'₁ représente un atome d'hydrogène ou un (C₁-C₃)alkyle.

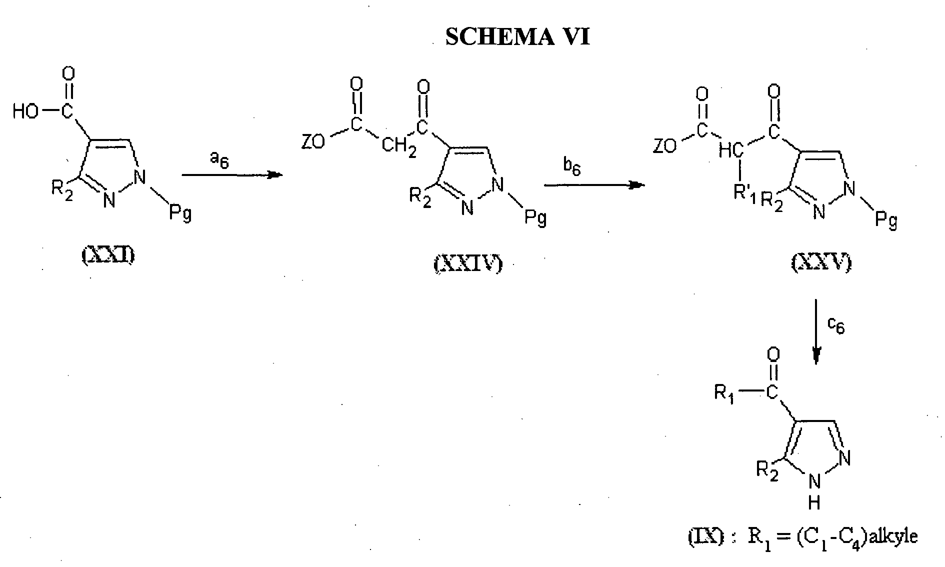

A l'étape a₆ du SCHEMA VI, on fait réagir le composé de formule (XXI) avec du 1,1'-carbonyldiimidazole puis, sans isoler, on traite le composé intermédiaire ainsi obtenu avec un sel de magnésium d'un hémi-ester de l'acide malonique selon la méthode décrite dans Angew. Chem. Int. Ed. Engl (1979) 18(1), 72-74.

A l'étape b₆, on alkyle le composé de formule (XXIV) ainsi obtenu par réaction d'un halogénure, d'un mésylate ou d'un tosylate de (C₁-C₃)alkyle, en présence d'une base forte telle que, par exemple, l'hydrure de sodium, dans un solvant comme, par exemple, le tétrahydrofurane et à une température comprise entre 0°C et la température ambiante.

A l'étape c₆, on hydrolyse en milieu acide le composé de formule (XXV) ainsi obtenu. La réaction s'effectue par action d'un acide fort tel que, par exemple, l'acide chlorhydrique, dans un solvant tel que l'eau et à une température comprise entre la température ambiante et 105°C. La décarboxylation spontanée qui s'ensuit permet de générer le composé de formule (IX).

Les composés de formule (IX) dans laquelle R₁ = (C₁-C₄)alcoxy sont connus, commercialisés ou préparés selon des méthodes connues (Synlett (2004) 4, 703-707).

Les composés de formule (X) sont connus, commercialisés ou préparés selon des méthodes connues (Tetrahedron (1988) 44(10), 2977-2983 ; J. Org. Chem. (2008) 73(23), 9326-9333).

Les composés de formule (XI) dans laquelle R₁ = (C₁-C₄)alkyle se préparent selon le SCHEMA VII ci-après dans lequel A et R₂ sont tels que définis pour un composé de formule (I), Z représente un (C₁-C₄)alkyle et R'₁ représente un atome d'hydrogène ou un (C₁-C₃)alkyle.

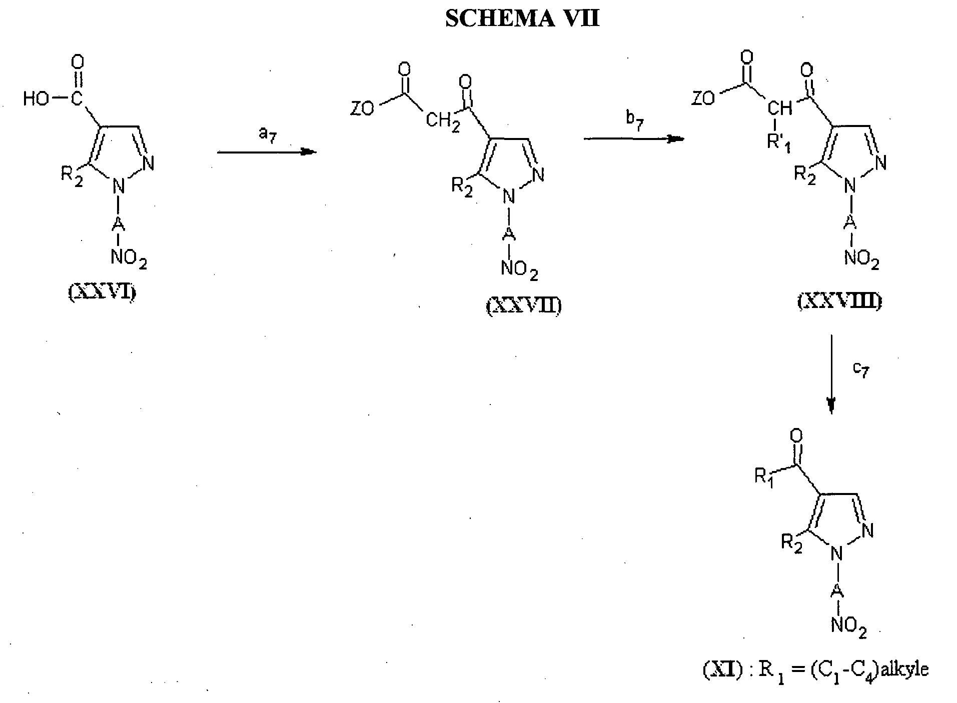

Les étapes a₇, b₇ et c₇ du SCHEMA VII s'effectuent selon les mêmes protocoles que ceux décrits aux étapes a₆, b₆ et c₆ du SCHEMA VI.

Les composés de formule (XII) peuvent également se préparer selon le SCHEMA VIII ci-après dans lequel A et R₂ sont tels que définis pour un composé de formule (I), Z représente un (C₁-C₄)alkyle, R'₁ représente un atome d'hydrogène ou un (C₁-C₃)alkyle, Y représente un groupe partant tel que défini précédemment et Boc représente un *tert*-butoxycarbonyle.

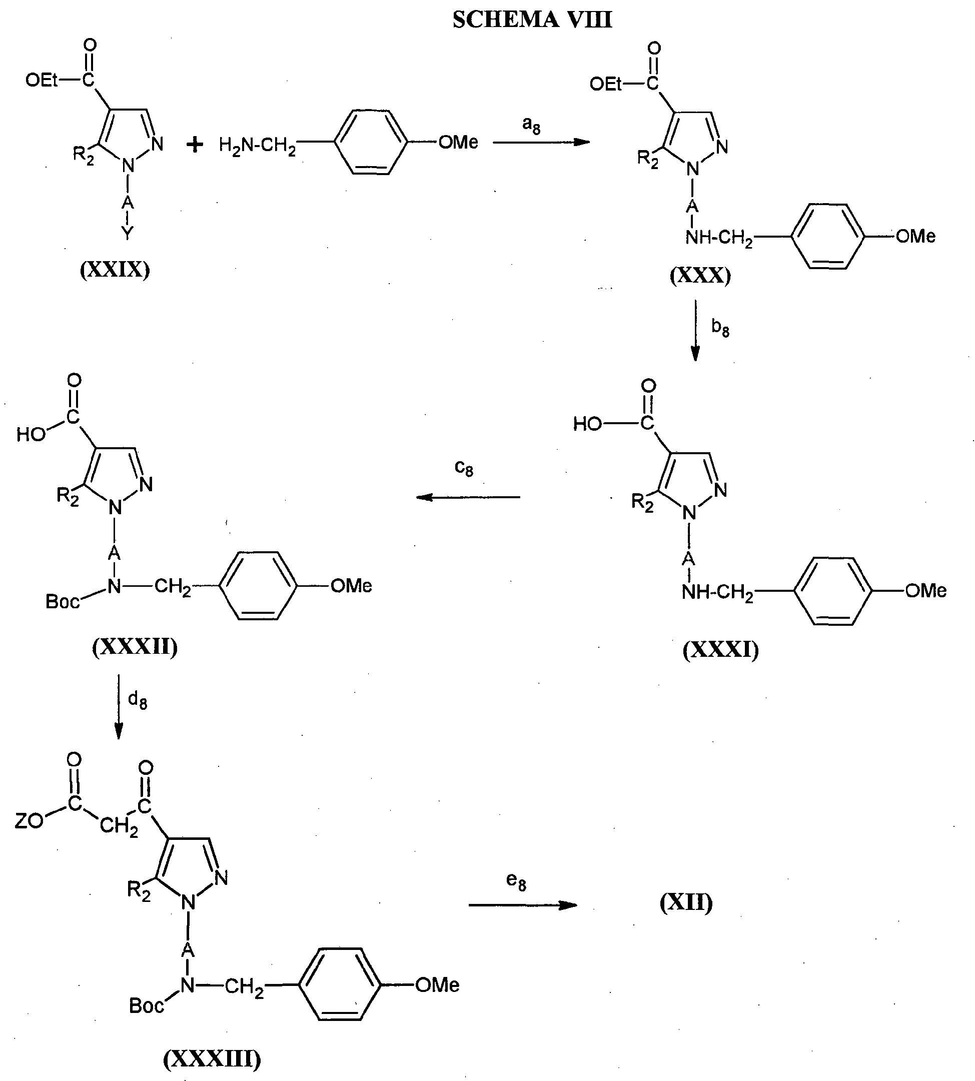

A l'étape a₈ du SCHEMA VIII, on fait réagir le composé de formule (XXIX) avec la 4-méthoxybenzylamine dans un solvant comme le dioxane et à la température de reflux du solvant.

A l'étape b₈, le composé de formule (XXX) ainsi obtenu est hydrolyse en milieu basique, avec une base telle que l'hydroxyde de potassium pour donner l'acide de formule (XXXI).

A l'étape c₈, le composé de formule (XXXI) est protégé par un groupement Boc, en présence de bases telles que la triéthylamine ou la 4-diméthylaminopyridine dans un solvant comme le DMF.

Les étapes d₈ et e₈ s'effectuent selon les mêmes protocoles que ceux décrits aux étapes a₆ et b₆ du SCHEMA VI.

Les composés de formule (XV), (XVII), (XX) sont connus, commercialisés ou préparés selon des méthodes connues.

Les composés de formule (XXVI) sont connus, commercialisés ou préparés selon des méthodes décrites dans EP 1 176 140.

Les composés de formule (XXIX) dans laquelle A représente un radical pyridazinyle se préparent selon la méthode décrites dans J. Heterocyclic. Chem. (2009) 46, 584-590.

L'invention, selon un autre de ses aspects, a également pour objet les composés nouveaux de formule (II). Ces composés sont utiles comme intermédiaire de synthèse des composés de formule (I).

Ainsi, l'invention a pour objet des composés de formule : 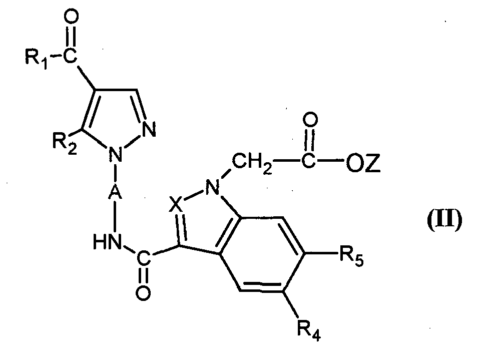 dans laquelle :
- A représente un radical aromatique bivalent choisi parmi : 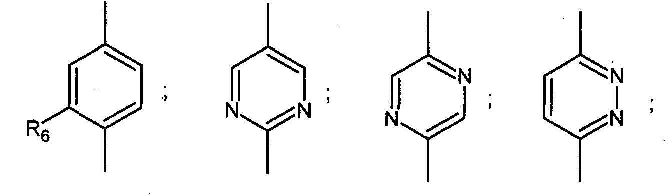
- X représente un groupe -CH- ou un atome d' azote ;
- R₁ représente un (C₁-C₄)alkyle ou un (C₁-C₄)alcoxy ;
- R₂ représente un groupe Alk ;
- R₄ représente un atome d'hydrogène, un atome d'halogène, un cyano, un phényle, un groupe Alk, un groupe OAlk ou un groupe -NR₉R₁₀ ;
- R₅ représente un atome d'hydrogène, un atome d'halogène ou un groupe Alk ;
- R₆ représente un atome d'hydrogène, un atome d'halogène, un cyano, un groupe -COOAlk ou un groupe -CONH₂ ;
- R₉ et R₁₀ représentent chacun indépendamment un atome d'hydrogène ou un (C₁-C₄)alkyle ;
- Z représente un (C₁-C₄)alkyle ;
- Alk représente un (C₁-C₄)alkyle non substitué ou substitué une ou plusieurs fois par un atome de fluor.

L'invention, selon un autre de ses aspects, a également pour objet les composés de formule I tels quels ou sous forme radiomarquée pour l'utilisation, comme outils pharmacologiques chez l'homme ou chez l'animal, pour la détection et le marquage du récepteur purinergique P2Y12.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans les

TABLEAUX X à XV ci-après, qui illustrent les structures chimiques et les propriétés physiques de quelques composés selon l'invention.

Dans les Préparations et dans les Exemples on utilise les abréviations suivantes :
Me : méthyle
Et : éthyle
n-Pr : n-propyle
Ph : phényle
éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
DMAP : 4-diméthylaminopyridine
DIPEA : diisopropyléthylamine
HOAT : 1-hydroxy-7-azabenzotriazole
HOBT : 1-hydroxybenzotriazole
TFA : acide trifluoroacétique
BOP : benzotriazol-1-yloxytris(diméthylamino)phosphonium hexafluoro phosphate
BOP-Cl : chlorure de bis-(2-oxo-1,3-oxazolidin-3-yl)phosphinique
EDC : chlorhydrate de 1-(3-diméthylaminopropyl)-3-éthylcarbodiimide
NaOH : hydroxyde de sodium
KOH : hydroxyde de potassium
HCl : acide chlorhydrique
NaBH₄ : borohydrure de sodium
NaHCO₃ : hydrogénocarbonate de sodium
NaH : hydrure de sodium
Na₂SO₄ : sulfate de sodium
Ether chlorhydrique 1N ou 2N : solution 1N ou 2N d'acide chlorhydrique dans l'éther diéthylique
HCl 1N (ou 2N) dans l'éther : solution 1N (ou 2N) d'acide chlorhydrique dans l'éther diéthylique
HCl 4N dans le dioxane : solution 4N d'acide chlorhydrique dans le dioxane
F : point de fusion
TA : température ambiante
Eb : température d'ébullition
CLHP : chromatographie liquide haute performance
Saumure : solution saturée de chlorure de sodium dans l'eau.

Les spectres de résonance magnétique nucléaire du proton (RMN ¹H) sont enregistrés sur des spectromètres Bruker (250 et 400 MHz) dans le DMSO-d₆. Les déplacements chimiques δ sont exprimés en parties par million (ppm). Pour l'interprétation des spectres, on utilise les abréviations suivantes : s : singulet, d : doublet, t : triplet, q : quadruplet : m : massif, mt : multiplet, se : singulet élargi, dd : doublet dédoublé, br : pic large.

Les composés selon l'invention sont analysés par couplage HPLC-UV-MS (chromatographie liquide/détection UV/détection de masse).

L'appareil utilisé est composé d'une chaine chromatographique équipée d'un détecteur à barrette de diodes et d'un spectromètre de masse quadripôlaire. On mesure le pic moléculaire (MH⁺) et le temps de rétention (tr) en minutes.
Methode A : WatersXBridge C18, 4,6 x 50 mm, 2,5µm
Solvant A : eau + 0,05% TFA
Solvant B : MeCN + 0,05 % TFA
1,3 ml/min ; 40 °C ; Waters LCT classic TOF-MS

| Gradient (minutes) | A | B |
|---|---|---|
| 0 | 95 | 5 |
| 0.3 | 95 | 5 |
| 3.5 | 5 | 95 |
| 4 | 5 | 95 |

Methode B: Waters XBridge C18 4,6 x 50 mm, 2,5 µm,
Solvant A : eau + 0,1% d'acide formique
Solvant B : MeCN + 0,08% d'acide formique
1,3 ml/min ; 20 °C ; Waters Ultima Triple Quad MS

| Gradient (minutes) | A | B |
|---|---|---|
| 0 | 97 | 3 |
| 3.5 | 40 | 60 |
| 4 | 2 | 98 |
| 5 | 2 | 98 |
| 5.2 | 97 | 3 |
| 6.5 | 97 | 3 |

Methode C: YMC-Pack Jsphere H80 33 x 2,1 mm, 4,0 µm Solvant A : eau + 0,05% TFA
Solvant B : méthanol + 0,05% TFA
1,0 ml/min ; 20 °C ; Waters LCT classic TOF-MS, 8-channel Mux

| Gradient (minutes) | A | B |
|---|---|---|
| 0 | 98 | 2 |
| 1 | 98 | 2 |
| 5 | 5 | 95 |
| 6.25 | 5 | 95 |

Methode D: WatersXBridge C18, 4,6x50 mm, 2,5 µm
Solvant A : eau + 0,05% TFA
Solvant B : MeCN + 0,05% TFA
1,7 ml/min, 40 °C ; Waters LCT classic TOF-MS

| Gradient (minutes) | A | B |
|---|---|---|
| 0 | 95 | 5 |
| 0.2 | 95 | 5 |
| 2.4 | 5 | 95 |
| 3.2 | 5 | 95 |
| 3.3 | 95 | 5 |
| 4 | 95 | 5 |

Methode E: Waters XBridge C18 4,6 x 50 mm ; 2,5 µm
Solvant A : eau + 0,1% d'acide formique
Solvant B : MeCN + 0,1% d'acide formique
1,3 ml/min, 45 °C ; Waters ZQ Single Quadrupol

| Gradient (minutes) | A | B |
|---|---|---|
| 0 | 97 | 3 |
| 3.5 | 40 | 60 |
| 4 | 2 | 98 |
| 5 | 2 | 98 |
| 5.2 | 97 | 3 |
| 6.5 | 97 | 3 |

Methode F: Merck Chromolith FastGrad. RP-18e, 50 x 2 mm
Solvant A : eau + 0,05% TFA
Solvant B : MeCN + 0,05% TFA
2,4 ml/min, 50 °C ; Waters LCT classic TOF-MS

| Gradient (minutes) | A | B |
|---|---|---|
| 0 | 98 | 2 |
| 0.2 | 98 | 2 |
| 2.4 | 2 | 98 |
| 3.2 | 2 | 98 |
| 3.3 | 98 | 2 |
| 4 | 98 | 2 |

Méthode G : Agilent 1100 series. Symmetry C18 3,5 µm (2,1 x 50 mm, Waters)
Solvant A : eau + 0,005% TFA
Solvant B : MeCN + 0,005% TFA
0,4ml/min, 25°C ; MSD SL (Agilent) ESI⁺.

| Gradient (minutes) | A | B |
|---|---|---|
| 0 | 100 | 0 |
| 10 | 0 | 100 |
| 15 | 0 | 100 |

Methode H : Agilent 1100 series. Symmetry C18 3,5 µm (2,1 x 50 mm, Waters)
Solvant A : eau + 0,005% TFA
Solvant B : MeCN + 0,005% TFA
0,4ml/min, 25°C ; MSD SL (Agilent) ESI⁺.

| Gradient (minutes) | A | B |
|---|---|---|
| 0 | 100 | 0 |
| 30 | 0 | 100 |
| 35 | 0 | 100 |

Méthode I : Agilent 1100 series. X Terra C18 3,5µm (2,1 x 50 mm, Waters)
Solvant A : Tampon acétate d'ammonium 10 mM pH 7
Solvant B : MeCN
0,4ml/min, 30°C ; MSD SL (Agilent) ESI⁺.

| Gradient (minutes) | A | B |
|---|---|---|
| 0 | 100 | 0 |
| 10 | 10 | 90 |
| 15 | 10 | 90 |

Méthode J : Waters UPLC BEH. C18 2,1 x 50 mm; 1.7um
Solvant A : eau + 0,05% d'acide formique
Solvant B : MeCN + 0,035% d'acide formique
0.9 ml/min ; 55°C

| Gradient (minutes) | A | B |
|---|---|---|
| 0 | 95 | 5 |
| 1.1 | 5 | 95 |
| 1.7 | 5 | 95 |
| 1.8 | 95 | 5 |
| 2 | 95 | 5 |

L'enregistrement des spectres de masse est effectué en mode électrospray (ESI) positif, afin d'observer les ions issus de la protonation de composés analysés (MH⁺), ou de la formation d'adduits avec d'autres cations tels que Na⁺, K⁺, etc.

### PREPARATIONS

### 1. Préparations des composés de formule (XVI).

### Préparation 1.1

### 1-(5-Bromo-1H-indol-3-yl)-2,2,2-trifluoroéthanone.

### (XVI) : R₄ = Br ; R₅ = H.

A une solution de 20 g de 5-bromoindole dans 250 ml d'éther refroidi à -5°C, on ajoute, goutte à goutte, une solution de 21,3 ml d'anhydride trifluoroacétique dans 70 ml d'éther. On laisse 2 heures sous agitation à -5°C. On essore le précipité formé et le lave à l'éther. On obtient 24,5 g du composé attendu sous forme d'une poudre blanche.

### F = 254°C

RMN ¹H : DMSO-d₆ (250 MHz) : δ (ppm) : 7,50 (1H, d) ; 7,58 (1H, d) ; 8,31 (1H,s) ; 8,55 (1H, s) ; 12,85 (1H, br).

En procédant selon le mode opératoire décrit à la préparation 1.1, on prépare les 3-trifluoroacétylindoles de formule (XVI) rassemblés dans le TABLEAU I ci-après :

**TABLEAU I**

| | | | |
|---|---|---|---|
| 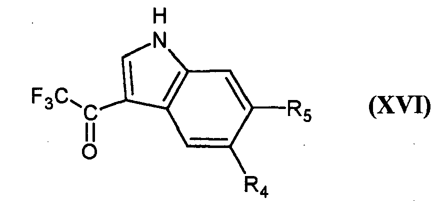 | | | |

| | R₄ | R₅ | RMN ¹H : DMSO-d₆ (250 MHz) : δ (ppm) |
|---|---|---|---|
| Préparation 1.2 | Me | Br | 2,47 (3H, s) ; 7,81 (1H, s) ; 8,14 (1H, s) ; 8,47 (1H, s) ; 12,70 (1H, br) |
| Préparation 1.3 | Br | Me | 2,47 (3H, s) ; 7,58 (1H, s) ; 8,33 (1H, s) ; 8,48 (1H, s) ; 12,70 (1H, br) |
| Préparation 1.4 | Cl | F | 7,63 (1H, d) ; 8,23 (1H, d) ; 8,58 (1H, s) ; 12,90 (1H, br) |

### 2. Préparations des composés de formule (VIII : X = CH).

### Préparation 2.1

### Acide 5- Bromo-1H-indole-3-carboxylique

### (VIII) : X = CH ; R4 = Br ; R5 = H.

A une solution de 46,1 g d'hydroxyde de potassium dans 25 ml d'eau, on ajoute 24 g du composé obtenu à la Préparation 1.1 et chauffe pendant 4 heures à reflux. On refroidit le milieu réactionnel et le lave à l'éther. On refroidit la phase aqueuse à 5°C puis neutralise à l'aide d'une solution de tampon phosphate préchargé avec de l'acide chlorhydrique 35%. On essore le précipité formé et le lave à l'eau. On obtient 15,6 g du composé attendu sous forme d'une poudre blanche.

### F = 234°C.

RMN ¹H : DMSO-d₆ (250 MHz) : δ (ppm) : 7,32 (1H, d) ; 7,46 (1H, d) ; 8,06 (1H,s) ; 8,14 (1H, s) ; 12,00 (2H, br).

En procédant selon le mode opératoire décrit à la préparation 2.1, on prépare les 3-carboxyindoles de formule (VIII : X = CH) rassemblés dans le TABLEAU II ci-après :

**TABLEAU II**

| | | | |
|---|---|---|---|
| 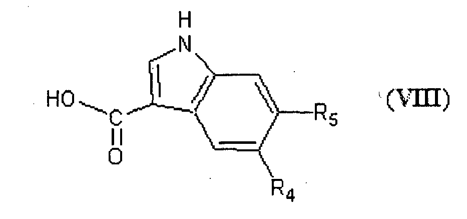 | | | |

| | R₄ | R₅ | RMN ¹H : DMSO-d₆ (250 MHz) : δ (ppm) |
|---|---|---|---|
| Préparation 2.2 | Me | Br | 2,44 (3H, S) ; 7,68 (1H, s) ; 7,95 (1H, s) ; 7,98 (1H, s) ; 11,70 (1H, br) ; 12,00 (1H, br) |
| Préparation 2.3 | Br | Me | 2,44 (3H, s) ; 7,45 (1H, s) ; 7,98 (1H, s) ; 8,15 (1H, s) ; 11,90 (1H, br) ; 12,10 (1H, br) |
| Préparation 2.4 | Cl | Cl | 7,73 (1H, s) ; 8,10 (1H, s) ; 8,13 (1H, s) ; 12,00 (2H, br) |
| Préparation 2.5 | Cl | F | 7,49 (1H, d) ; 8,00-8,08 (2H, m) ; 12,00 (1H, br) ; 12,20 (1H, br) |

### Préparation 2.6

### Acide 5,6-diméthyl-1H-indole-3-carboxylique.

### (VIII) : X = CH; R4 = Me ; R₅ = Me.

### Etape 1 : 3-[(2-Iodo-4,5-diméthylphényl)amino]acrylate de benzyle (XVIII).

A une solution de 15,5 g de [1,4]-Benzoquinone dans 350 ml de THF, on ajoute 60,8 g de chlorure de lithium et dégaze à l'azote. On ajoute 3,2 g d'acétate de palladium et 23,7 g d'acrylate de benzyle et dégaze à l'azote pendant environ 30 minutes. On ajoute ensuite une solution de 35,1 g de 2-iodo-4,5-diméthylaniline (préparé selon J. Med. Chem 2001, 44, 3856-3871) dans 150 ml de THF et laisse une nuit sous agitation. On filtre et évapore le filtrat. On triture à l'éther le résidu solide ainsi obtenu. On filtre, lave le filtrat avec une solution de NaOH 0,5N puis à l'eau et à la saumure. On évapore puis purifie le résidu solide par chromatographie sur gel de silice en éluant avec un mélange de cyclohexane/AcOEt (8/2 ; v/v). On obtient 57,6 g du composé attendu sous forme d'une poudre blanche.

RMN ¹H : DMSO-d₆ (250 MHz) : δ (ppm) : 2,14 (3H, s) ; 2,19 (3H,s) ; 4,93 (1H, d) ; 5,18 (2H, s) ; 7,23 (1H, s) ; 7,30-7,45 (5H, m) ; 7,59 (1H,s) ; 7,72 (1H, dd) ; 10,05 (1H, d).

En procédant selon le même mode opératoire, on prépare le composé de formule (XVIII) ci-après :

| R₄ | R₅ | RMN ¹H : DMSO-d₆ (250 MHz) : δ (ppm) : |
|---|---|---|
| CF₃ | H | 5,15 (1H, d) ; 5,21 (2H, s) ; 7,30-7,50 (5H, m) ; 7,70 (2H, m) ; 7,88 (1H, dd) ; 8,02 (1H, s) ; 10,50 (1H, d). |

### Etape 2 : 5,6-diméthyl-1H-indole-3-carboxylate de benzyle (XIX).

A une solution de 25,5 g du composé obtenu à l'étape 1 dans 120 ml de DMF, on ajoute 8,60 g de 1,4-diazabicyclo[2.2.2]octane. On dégaze à l'azote puis ajoute 0,703 g d'acétate de palladium et chauffe le milieu réactionnel à 120°C pendant 7 heures. On ajoute de l'AcOEt puis lave à l'eau, à la saumure, sèche sur Na₂SO₄ et évapore. Après trituration du résidu solide à l'éther iso, on obtient 12,3 g du composé attendu sous forme d'une poudre beige.

RMN ¹H : DMSO-d₆ (250 MHz) : δ (ppm) : 2,29 (3H, s) ; 2,30 (3H,s) ; 5,32 (2H, s) ; 7,24 (1H, s) ; 7,30-7,50 (5H, m) ; 7,76 (1H,s) ; 7,98 (1H, d) ; 11,70 (1H, br).

En procédant selon le même mode opératoire, on prépare le composé de formule (XIX) ci-après :

| R₄ | R₅ | RMN ¹H : DMSO-d₆ (250 MHz) : δ (ppm) : |
|---|---|---|
| CF₃ | H | 5,38 (2H, s) ; 7,35-7,55 (6H, m) ; 7,71 (1H, d) ; 8,33 (1H, s) ; 8,35 (1H, s) ; 12,35 (1H, br). |

### Etape 3 : acide 5,6-diméthyl-1H-indole-3-carboxylique.

A une solution de 5 g du composé obtenu à l'étape 2 dans 120 ml de MeOH, on ajoute 0,54 g de Pd/C 10% et 16,9 g de formiate d'ammonium. On chauffe à reflux pendant 2 heures. On filtre sur talc et évapore le filtrat.

Le résidu solide est extrait à l'AcOEt. On lave avec une solution d'HCl 0,1N, sèche sur Na₂SO₄ et évapore à sec. On obtient 2,87 g du composé attendu sous forme d'une poudre blanche.

RMN ¹H : DMSO-d₆ (250 MHz) : δ (ppm) : 2,31 (6H, s) ; 7,23 (1H, s) ; 7,77 (1H, s) ; 7,85 (1H,s) ; 11,50 (1H, br) ; 11,80 (1H, br).

En procédant selon le même mode opératoire, on prépare le composé de formule (VIII : X = CH) ci-après :

| | R₄ | R₅ | RMN ¹H : DMSO-d₆ (250 MHz) : δ (ppm) : |
|---|---|---|---|
| Préparation 2.7 | CF₃ | H | 7,48 (1H, d) ; 7,79 (1H, d) ; 8,06 (1H,s) ; 8,69 (1H, s) ; 12,05 (2H, br). |

Les 3-carboxyindazoles (composés de formule VIII, X=N) sont synthétisés à partir des isatines commerciales selon la méthode décrite dans Synthetic Communications (2005), 2681-2684.

### 3. Préparations des composés de formule (IV).

### Préparation 3.1

### Acide 5-chloro-1-(2-méthoxy-2-oxoéthyl)-1H-indole-3-carboxylique.

### (IV) : X = CH ; R₄ = Cl ; R₅ = H ; Z = Me.

A un mélange de 4,50 g de NaH (60% dans l'huile) dans 400 ml de DMF à -10°C, on ajoute, goutte à goutte, 110 ml d'une solution contenant 10 g d'acide 5-chloro-1*H-*indole-3-carboxylique (commercial) dans le DMF. Après retour à TA, on laisse sous agitation pendant 1 heure. On refroidit le mélange réactionnel à -20°C. On ajoute, goutte à goutte, 4,86 ml de bromoacétate de méthyle, retourne à TA sur une période de 5 heures et laisse pendant 15 heures sous agitation. On ajoute le milieu réactionnel à IL d'un mélange AcOEt/HCl 1N, collecte la phase organique et extrait la phase aqueuse à l'AcOEt. On rassemble les phases organiques, lave à l'eau, à la saumure puis sèche sur Na₂SO₄ et évapore à sec. On obtient, 8,9 g du composé attendu sous forme d'une poudre blanche.

RMN ¹H : DMSO-d₆ (250 MHz) : δ (ppm) : 3,70 (3H, s) ; 7,26 (1H, d) ; 7,57 (1H, d) ; 7,98 (1H,s) ; 8,12 (1H, s) ; 12,3 (1H, br).

En procédant selon le mode opératoire décrit à la préparation 3.1, on prépare les composés de formule (IV) dans laquelle X = CH et Z = Me rassemblés dans le TABLEAU III ci-après :

**TABLEAU III**

| | | | |
|---|---|---|---|
| 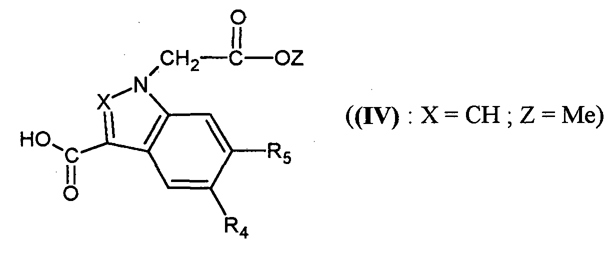 | | | |

| | R₄ | R₅ | RMN ¹H : DMSO-d₆ (250 MHz) : δ (ppm) |
|---|---|---|---|
| Préparation 3.2 | Me | H | 2.42 (3H, s) ; 3.70 (3H, s) ; 5.21 (2H, s) ; 7.06 (1H, d) ; 7.37 (1H, d) ; 7.83 (1H, s) ; 8.00 (1H, s) ; 12.00 (1H, br). |
| Préparation 3.3 | OMe | H | 3.69 (3H, s) ; 3.79 (3H, s) ; 5.20 (2H, s) ; 6.86 (1H, d) ; 7.39 (1H, d) ; 7.50 (1H, s) ; 7.99 (1H, s) ; 12.05 (1H, br). |
| Préparation 3.4 | CF₃ | H | 3.72 (3H, s) ; 5.35 (2H, s) ; 7.55 (1H, d) ; 7.78 (1H, d) ; 8.26 (1H, s) ; 8.36 (1H, s) ; 12.50 (1H, br). |
| Préparation 3.5 | Br | H | 3.71 (3H, s) ; 5.27 (2H, s) ; 7.37 (1H, d) ; 7.53 (1H, d) ; 8.14 (2H, m) ; 12.25 (1H, br). |
| Préparation 3.6 | Me | Me | 2.32 (6H, s) ; 3.70 (3H, s) ; 5.18 (2H, s) ; 7.26 (1H, s) ; 7.79 (1H, s) ; 7.92 (1H, s) ; 11.90 (1H, br). |
| Préparation 3.7 | Cl | Cl | 3.71 (3H, s) ; 5.27 (2H, s) ; 8.00 (1H, s) ; 8.14 (1H, s) ; 8.16 (1H, s) ; 12.45 (1H, br). |

### 4. Préparations des composés de formule (XIII).

### Préparation 4.1

### Acide 1-acétyl-5-chloro-6-fluoro-1H-indole-3-carboxylique.

### (XIII) : X = CH ; R₄ = Cl ; R₅ = F ; Pg = -COMe.

On refroidit à 0°C une solution de 4,4 g du composé de la Préparation 2.5 dans 100 ml de DCM, ajoute 6,28 ml de triéthylamine et 0,51 g de DMAP puis, goutte à goutte, 1,47 ml de chlorure d'acétyle et laisse 3 heures sous agitation. On lave le mélange réactionnel par une solution d'HCl 1N puis à l'eau, par une solution saturée de NaCl, sèche la phase organique sur Na₂SO₄ et évapore le solvant sous vide. On obtient 4,82 g du composé attendu sous forme de poudre blanche.

RMN ¹H : DMSO-d₆ (250 MHz) : δ (ppm) : 2,74 : s : 3H ; 8,13 : d : 1H ; 8,25 : d : 1H ; 8,52 : s : 1H ; 13,10 : br : 1H.

En procédant selon le mode opératoire décrit à la préparation 4.1, on prépare les composés de formule (XIII) dans laquelle Pg = -COMe rassemblés dans le TABLEAU IV ci-après :

**TABLEAU IV**

| | | | | |
|---|---|---|---|---|
| 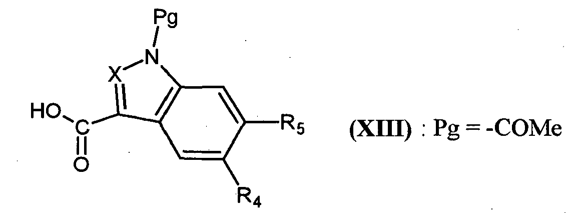 | | | | |

| | R₄ | R₅ | X | RMN ¹H : DMSO-d₆ (250 MHz) : δ (ppm) |
|---|---|---|---|---|
| Préparation 4.2 | H | Me | CH | 2,49 (3H, s) ; 2,73 (3H, s) ; 7,21 (1H, d) ; 7,96 (1H, d) ; 8,20 (1H, s) ; 8,81 (1H, s) ; 12,75 (1H, br) |
| Préparation 4.3 | F | H | CH | 2,75 (3H, s) ; 7,27 (1H, dd) ; 7,76 (1H, d) ; 8,38 (1H, dd) ; 8,52 (1H, s) ; 12,90 (1H, br) |
| Préparation 4.4 | Me | Br | CH | 2,46 (3H, s) ; 2,72 (3H, s) ; 8,01 (1H, s) ; 8,42 (1H, s) ; 8,53 (1H, s) ; 12,85 (1H, br) |
| Préparation 4.5 | Br | Me | CH | 2,48 (3H, s) ; 2,73 (3H, s) ; 8,22 (1H, s) ; 8,35 (1H, s) ; 8,42 (1H, s) ; 12,95 (1H, br) |
| Préparation 4.6 | Me | H | N | 2,50 (3H, s) ; 2,76 (3H, s) ; 7,52 (1H, d) ; 7,98 (1H, s) ; 8,25 (1H, d) ; 13,85 (1H, br) |

### 5. Préparations des composés de formule (IX).

### Préparation 5.1

### 1-(5-méthyl-1H-pyrazol-4-yl)butan-1-one.

### (IX) : R₁ = n-Pr ; R₂ = Me.

### Etape 1 : acide 3-méthyl-1-trityl-1H-pyrazole-4-carboxylique (XXI).

A une solution de 6,60 g de 5-méthyl-1*H*-pyrazole-4-carboxylate de méthyle dans 50 ml de DMF, on ajoute 8,46 g de carbonate de potassium et 15,8 g de chlorure de trityle. Après 5 jours à TA, on ajoute de l'AcOEt, lave à l'eau, à la saumure, sèche sur Na₂SO₄ et évapore à sec. On dissout le résidu huileux ainsi obtenu dans 100 ml d'un mélange EtOH/eau (50/50 ; v/v), ajoute 9,95 g d'hydroxyde de potassium et chauffe au reflux pendant 6 heures. On filtre le milieu réactionnel à chaud, concentre le filtrat et acidifie avec une solution d' HCl 1N. On essore le précipité formé, sèche sous vide puis lave avec un mélange AcOEt/éther iso (50/50 ; v/v) et sèche sous vide. On obtient 9,70 g du composé attendu sous forme d'une poudre blanche.

RMN ¹H : DMSO-d₆ (250 MHz) : δ (ppm) : 2,33 (3H, s) ; 7,03-7,09 (6H, m) ; 7,34-7,43 (9H, m) ; 7,61 (1H, s).

### Etape 2 : N-méthoxy-N,3-diméthyl-1-trityl-1H-pyrazole-4-carboxamide (XXII).

A une solution de 9,70 g du composé obtenu à l'étape précédente, dans 100 ml de DCM, on ajoute 10,3 g de DMAP et 10,3 g de BOP-Cl. On ajoute ensuite par portion 3,85 g de chlorhydrate de N-méthoxyméthanamine et agite pendant 1 heure à TA. On concentre le milieu réactionnel sous vide, extrait le résidu à l'AcOEt, lave à l'eau, à la saumure, sèche sur Na₂SO₄ puis évapore à sec. On triture à l'éther iso, filtre et sèche à l'étuve à vide. On obtient 10,4 g du composé attendu sous forme d'une poudre blanche.

RMN ¹H : DMSO-d₆ (250 MHz) : δ (ppm) : 2,34 (3H, s) ; 3,13 (3H, s) ; 3,39 (3H, s) ; 7,05-7,15 (6H, m) ; 7,33-7,44 (9H, m) ; 7,71 (1H, s).

### Etape 3 : 1-(3-méthyl-1-trityl-1H-pyrazol-4-yl)butan-1-one (XXIII).

A une solution de 10,4 g du composé obtenu à l'étape précédente, dans 130 ml de THF à -30°C, on ajoute, goutte à goutte, 32,7 ml d'une solution 2M de chlorure de n-propylmagnésium dans l'éther. On revient à TA puis agite pendant 4 heures. On refroidit le milieu réactionnel à -30°C puis ajoute (goutte à goutte au début) 50 ml d'eau. On revient à TA, ajoute 250 ml d'une solution d'HCl 1N puis extrait avec de l'AcOEt. On lave à l'eau, à la saumure, sèche sur Na₂SO₄ puis évapore à sec. On triture dans l'éther iso, filtre et sèche à l'étuve à vide. On obtient 8,4 g du composé attendu sous forme d'une poudre blanche.

RMN ¹H : DMSO-d₆ (400 MHz) : δ (ppm) : 0,84 (3H, t) ; 1,52 (2H, sext) ; 2,34 (3H, s) ; 2,62 (2H, t) ; 7,04-7,12 (6H, m) ; 7,33-7,45 (9H, m) ; 7,93 (1H, s).

### Etape 4 : 1-(5-méthyl-1H-pyrazol-4-yl)butan-1-one.

On agite une suspension de 8,4 g du composé obtenu à l'étape précédente, dans 50 ml de HCl 4N dans le dioxane pendant 6 heures. On évapore à sec, triture à l'éther iso, filtre et sèche à l'étuve à vide. On obtient 3,1 g du composé attendu sous forme d'une gomme incolore.

RMN ¹H : DMSO-d₆ (250 MHz) : δ (ppm) : 0,90 (3H, t) ; 1,58 (2H, sext) ; 2,39 (3H, s) ; 2,71 (2H, t) ; 8,15 (1H, s).

### 6. Préparations des composés de formule (V).

### Préparation 6.1

### 1-[1-(5-Aminopyrazin-2-yl)-5-méthyl-1H-pyrazol-4-yl]butan-1-one.

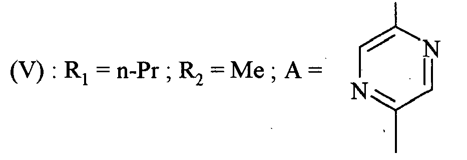

On chauffe à 130°C pendant 20 heures un mélange de 0,60 g du composé de la Préparation 5.1, 1,37 g de 5-Bromo-pyrazin-2-ylamine (X), 1,36 g de carbonate de potassium, 0,38 g de proline et 0,23 g d'iodure de cuivre (I) dans 15 ml de DMSO. On ajoute 100 ml d'eau et extrait au DCM. On lave la phase organique avec une solution saturée de bicarbonate de sodium puis à la saumure, sèche sur Na₂SO₄ et évapore à sec. On purifie par chromatographie sur gel de silice en éluant avec un gradient du mélange DCM/AcOEt de (80/20 ; v/v) à (40/60 ; v/v). On obtient 0,17 g du composé attendu sous forme de poudre.

RMN ¹H : DMSO-d₆ (250 MHz) : δ (ppm) : 0,93 (3H, t) ; 1,62 (2H, sext) ; 2,55 (3H, s) ; 2,80 (2H, t) ; 6,86 (2H, br) ; 7,80 (1H, s) ; 8,19-8,22 (2H, m).

### Préparation 6.2

### 1-[1-(6-Aminopyridazin-3-yl)-5-méthyl-1H-pyrazol-4-yl]butan-1-one.

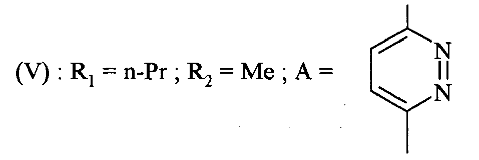

### Etape 1 : 1-[6-(4-méthoxybenzylamino)-pyridazin-3-yl]-5-méthyl-1H-pyrazole-4-carboxylate d'éthyle (XXX).

A une solution de 0,30 g de 1-(6-chloropyridazin-3-yl)-5-méthyl-1*H*-pyrazole-4-carboxylate d'éthyle (synthétisé selon la méthode décrite dans J. Heterocyclic Chem. 2009, 46, 584-590) dans 2 ml de dioxane, on ajoute 0,31 ml de 4-méthoxybenzylamine puis chauffe à 130°C pendant 2 heures. On évapore à sec, triture avec de l'eau, essore le précipité et sèche sous vide. On reprend le précipité avec de l'éther iso, essore et sèche sous vide. On obtient 0,30 g du produit attendu sous forme de poudre.

RMN ¹H : DMSO-d₆ (250 MHz) : δ (ppm) : 1.30 (3H, t) ; 2.69 (3H, s) ; 3.74 (3H, s) ; 4.26 (2H, q) ; 4.55 (2H, d) ; 6.91 (2H, d) ; 7.09 (1H, d) ; 7.32 (2H, d) ; 7.62 (1H, d) ; 7.67 (1H, t) ; 8.03 (1H, s).

### Etape 2 : Acide 1-[6-(4-méthoxybenzylamino)-pyridazin-3-yl]-5-méthyl-1H-pyrazole-4-carboxylique (XXXI).

A une solution de 3,82 g de KOH dans 50 ml d'eau, on ajoute une solution de 5 g du composé obtenu à l'étape précédente dans 50 ml d'EtOH. On chauffe à 80°C pendant 2 heures. On évapore à sec puis reprend le résidu avec 100 ml d'eau. On ajoute, goutte à goutte, 68 ml d'une solution d'HCl 1N sous agitation. On essore le précipité formé, le lave à l'eau puis le sèche à l'étuve à vide. On obtient 4,5 g du composé attendu sous forme d'une poudre blanche.

RMN ¹H : DMSO-d₆ (250 MHz) : δ (ppm) : 2.71 (3H, s) ; 3.75 (3H, s) ; 4.57 (2H, s) ; 6.93 (2H, d) ; 7.30-7.37 (3H, m) ; 7.81 (1H, d) ; 8.04 (1H, s) ; 8.42 (1H, br).

### Etape 3 : Acide 1-{6-[(tert-Butoxycarbonyl)-(4-méthoxybenzyl)-amino]-pyridazin-3-yl}-5-méthyl-1H-pyrazole-4-carboxylique (XXXII).

A une solution de 1,87 g du composé de l'étape précédente dans 22 ml de DMF, on ajoute 0,17 g de DMAP, 2,32 ml de triéthylamine et 3 g de Di-*tert*-butyldicarbonate puis agite pendant 20 heures à TA. On ajoute 0,47 g d'hydroxyde de potassium en solution dans 10 ml d'eau, puis agite pendant 20 heures. On ajoute 500 ml d'eau et lave à l'éther. On tamponne la phase aqueuse avec du tampon phosphate préchargé avec de l'acide chlorhydrique 1N. On essore le précipité formé, le lave à l'eau et le sèche à l'étuve à vide à 50°C. On obtient 1,35 g du composé attendu sous forme de poudre.

RMN ¹H : DMSO-d₆ (250 MHz) : δ (ppm) : 1.43 (9H, s) ; 2.83 (3H, s) ; 3.71 (3H, s) ; 5.19 (2H, br) ; 6.88 (1H, d) ; 7.25 (1H, d) ; 8.05-8.21 (3H, m) ; 12.7 (1H, br).

### Etape 4 : 3-(1-{6-[(tert-butoxycarbonyl)-(4-méthoxybenzyl)amino]pyridazin-3-yl}-5-méthyl-1H-pyrazol-4-yl)-3-oxo-propanoate d'éthyle (XXXIII).

A une solution de 1,1 g du composé obtenu à l'étape précédente dans 17 ml de THF, on ajoute 0,53 g de 1,1'-carbonyldiimidazole et agite pendant 20 heures à TA. On ajoute 0,82 g de magnésium bis (3-éthoxy-3-oxopropanoate) et agite à 55°C pendant 20 heures. On ajoute 50 ml d'AcOEt, lave à la soude 0,1 N, à la saumure, sèche sur Na₂SO₄ et évapore à sec. On purifie par chromatographie sur gel de silice par élution au DCM puis par élution avec un mélange DCM/MeOH (95/5 ; v/v). On obtient 1,18 g du composé attendu sous forme d'une poudre blanche.

RMN ¹H : DMSO-d₆ (250 MHz) : δ (ppm) : 1.21 (3H, t) ; 1.43 (9H, s) ; 2.82 (3H, s) ; 3.72 (3H, s) ; 4.04 (2H, s) ; 4.13 (2H, q) ; 5.19 (2H, s) ; 6.88 (2H, d) ; 7.25 (2H, d) ; 8.09 (1H, d) ; 8.22 (1H, d) ; 8.41 (1H, s).

### Etape 5 : 2-{1-[6-(4-méthoxybenzylamino)-pyridazin-3-yl]-5-méthyl-1H-pyrazole-4-carbonyl}butanoate d'éthyle (XII).

A une solution de 1,15 g du composé obtenu à l'étape précédente dans 23 ml de THF, on ajoute 1,25 g de carbonate de potassium, 0,98 g de bromure de tétrabutylammonium et 0,44 ml d'iodoéthane puis agite à 55°C pendant 20 heures. Après retour à TA, on ajoute 150 ml d'AcOEt, lave à l'eau, à la saumure, sèche sur Na₂SO₄ et évapore à sec. On purifie par chromatographie sur gel de silice en éluant avec un mélange DCM/MeOH (97/3 ; v/v). On obtient 1,18 g du composé attendu sous forme d'une poudre blanche.

RMN ¹H : DMSO-d₆ (250 MHz) : δ (ppm) : 0.92 (3H, t) ; 1.15 (3H, t) ; 1.43 (9H, s) ; 1.88 (2H, quint) ; 2.82 (3H, s) ; 3.72 (3H, s) ; 4.10 (2H, q) ; 4.28 (1H, t) ; 5.19 (2H, s) ; 6.88 (2H, d) ; 7.25 (2H, d) ; 8.09 (1H, d) ; 8.22 (1H, d) ; 8.49 (1H, s).

### Etape 6 : 1-[1-(6-Amino-pyridazin-3-yl)-5-méthyl-1H-pyrazol-4-yl]butan-1-one.

On chauffe à reflux pendant 6 heures 1,18 g du composé de l'étape précédente dans 5 ml d'HCl 6N. Après retour à TA, on ajoute 50 ml d'eau, lave à l'AcOEt, puis évapore à sec la phase aqueuse. On ajoute 50 ml d'une solution de NaOH 0,2 N et extrait à l'AcOEt. On lave la phase organique à la saumure, sèche sur Na₂SO₄ et évapore à sec. On obtient 0,42 g du composé attendu sous forme d'une poudre blanche.

RMN ¹H : DMSO-d₆ (250 MHz) : δ (ppm) : 0.93 (3H, t) ; 1.62 (2H, sext) ; 2.65 (3H, s) ; 2.82 (2H, t) ; 6.76 (2H, br) ; 7.00 (1H, d) ; 7.59 (1H, d) ; 8.27 (1H, s).

### Préparation 6.3

### 1-[1-(4-Aminophényl)-5-méthyl-1H-pyrazol-4-yl]butan-1-one.

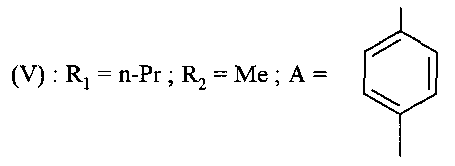

### Etape 1 : 3-[5-Méthyl-1-(4-nitrophényl)-1H-pyrazol-4-yl]-3-oxopropanoate d'éthyle (XXVII).

A une solution de 3 g d'acide 5-méthyl-1-(4-nitrophényl)-1*H*-pyrazole-4-carboxylique (XXVI) dans 120 ml de THF, on ajoute 2,95 g de 1,1'-carbonyldiimidazole et laisse 20 heures sous agitation à TA. On ajoute 5,2 g de magnésium bis (3-éthoxy-3-oxopropanoate) (synthétisé selon la méthode décrite dans Angew. Chem. Int. Ed. Engl., 1979, 18, 72-74) et chauffe à 45°C pendant 16 heures. On concentre le mélange réactionnel sous vide, extrait le résidu à l'AcOEt, lave la phase organique par une solution saturée de Na₂CO₃, à l'eau, sèche sur MgSO₄ et évapore le solvant sous vide. On chromatographie le résidu sur gel de silice en éluant au DCM puis par le mélange DCM/EtOH (99,5/0,5 ; v/v). On obtient 2,82 g du composé attendu.

RMN ¹H : CDCl₃ (250 MHz) : δ (ppm) : 1.28 (3H, t) ; 2.70 (3H, s) ; 3.88 (2H, s) ; 4.26 (2H, q) ; 7.70 (2H, d) ; 8.09 (1H, s) ; 8.42 (2H, d).

### Etape 2 : 2-{[5-Méthyl-1-(4-nitrophényl)-1H-pyrazol-4-yl]carbonyl}butanoate d'éthyle (XXVIII).

On refroidit à 0°C une solution de 2,44 g du composé de l'étape précédente dans 25 ml de THF, ajoute, par portions, 0,34 g de NaH (60% dans l'huile) et laisse 30 minutes sous agitation. On ajoute ensuite 0,92 ml d'iodoéthane et laisse 24 heures sous agitation à TA. On ajoute lentement de l'eau puis concentre le mélange réactionnel sous vide. On extrait le résidu au DCM, lave la phase organique à l'eau, sèche sur MgSO₄ et évapore le solvant sous vide. On chromatographie le résidu sur gel de silice en éluant avec le mélange AcOEt/cyclohexane (gradient de 10 à 20% d'AcOEt). On obtient 2,19 g du composé attendu.

RMN ¹H : CDCl₃ (250 MHz) : δ (ppm) : 1.02 (3H, t) ; 1.28 (3H, t) ; 2.06 (3H, m) ; 2.72 (3H, s) ; 3.97 (1H, t) ; 4.23 (2H, q) ; 7.70 (2H, d) ; 8.18 (1H, s) ; 8.42 (2H, d).

### Etape 3: 1-[5-Méthyl-1-(4-nitrophényl)-1H-pyrazol-4-yl]butan-1-one (XI).

On chauffe à 105°C pendant 2 heures 2,4 g du composé de l'étape précédente dans 58 ml d'une solution aqueuse de HCl à 37%. On refroidit le mélange réactionnel à 0°C, ajoute lentement 60 ml de NaOH à 35%, extrait à l'AcOEt, lave la phase organique à l'eau, sèche sur MgSO₄ et évapore le solvant sous vide. On obtient 1,8 g du composé attendu.

RMN ¹H : CDCl₃ (250 MHz) : δ (ppm) : 1.02 (3H, t) ; 1.78 (2H, m) ; 2.75 (3H, s) ; 2.85 (2H, t) ; 7.70 (2H, d) ; 8.10 (1H, s) ; 8.42 (2H, d).

### Etape 4: 1-[1-(4-Aminophényl)-5-méthyl-1H-pyrazol-4-yl]butan-1-one.

On coule, sous flux maitrisé d'argon, 450 ml d'EtOH sur 5 g de palladium/charbon. On ajoute ensuite une suspension de 48,7 g du composé de l'étape précédente dans 50 ml d'EtOH puis 181 ml de cyclohexène et chauffe à reflux pendant 4 heures. Après refroidissement à TA, on filtre le mélange réactionnel sur Célite®, lave à l'AcOEt et concentre le filtrat sous vide. On reprend le résidu à l'éther de pétrole, essore le précipité formé, le lave à l'éther de pétrole et le sèche sous vide à 50°C. On obtient 41,3 g du composé attendu.

RMN ¹H : DMSO-d₆ (250 MHz) : δ (ppm) : 0.93 (3H, t) ; 1.60 (2H, m) ; 2.41 (3H, s) ; 2.77 (2H, t) ; 5.6 (2H, br) ; 6.66 (2H, d) ; 7.10 (2H, d) ; 8.14 (1H, s).

### Préparation 6.4

### 1-[1-(4-Aminophényl)-5-méthyl-1H-pyrazol-4-yl]propan-1-one.

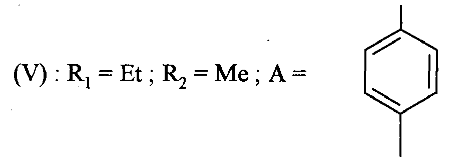

### Etape 1 : 2-Méthyl-3-[5-méthyl-1-(4-nitrophényl)-1H-pyrazol-4-yl]-3-oxo-propanoate d'éthyle.

A 0,50 g du composé obtenu à l'étape 1 de la Préparation 6.3 dans 5 ml de THF à 0°C, on ajoute lentement par petites portions 0,07 g de NaH (60% dans l'huile). On laisse agiter 30 minutes puis additionne 0,15 ml d'iodométhane. Après 24 h à TA, on ajoute lentement de l'eau et on évapore le THF. On extrait le résidu au DCM et lave la phase organique à l'eau, sèche sur Na₂SO₄ et évapore à sec. On recristallise le résidu dans du cyclohexane pour obtenir 0,5 g d'un solide jaune.

RMN ¹H : DMSO-d₆ (250 MHz) : δ (ppm) : 1,16 (3H, t) ; 1,32 (1H, d) ; 2,61 (3H, s) ; 4,09 (2H, q) ; 4,44 (1H, q) ; 7,90 (2H, d) ; 8,38-8,44 (3H, m).

### Etape 2 : 1-[5-Méthyl-1-(4-nitrophényl)-1H-pyrazol-4-yl]propan-1-one.

On chauffe 0,5 g du composé obtenu à l'étape précédente dans 13 ml de HCl 35% à 105°C pendant 2 heures. On refroidit à 0°C et on additionne lentement 14 ml de NaOH 35 %. On extrait à l'AcOEt, lave à l'eau, à la saumure, sèche sur Na₂SO₄ et évapore à sec. On chromatographie le résidu sur gel de silice en éluant avec un mélange AcOEt/cyclohexane (gradient de 0 à 20% d'AcOEt). On obtient 0,39 g d'un solide beige.

RMN ¹H : DMSO-d₆ (250 MHz) : δ (ppm) : 1,08 (3H, t) ; 2,62 (3H, s) ; 2,89 (2H, q) ; 7,88 (2H, d) ; 8,36-8,43 (3H, m).

### Etape 3 : 1-[1-(4-Aminophényl)-5-méthyl-1H-pyrazol-4-yl]propan-1-one.

On dissout 0,5 g du composé de l'étape précédente dans 20 ml d'un mélange AcOEt/MeOH (50/50 ; v/v). On réduit à l'aide de l'appareil d'hydrogénation en continu H-Cube (Cartouche Pd 10%, Mode Full H2, 50°C, débit 1ml/min). On concentre à sec, reprend le résidu solide à l'éther iso, essore le précipité formé et obtient 0,25 g d'une poudre blanche.

RMN ¹H : DMSO-d₆ (250 MHz) : δ (ppm) : 1,07 (3H, t) ; 2,42 (3H, s) ; 2,82 (2H, q) ; 5,44 (2H, br) ; 6,64 (2H, d) ; 7,07 (2H,d) ; 8,12 (1H, s).

### Préparation 6.5

### 1-[1-(4-Aminophényl)-5-méthyl-1H-pyrazol-4-yl]éthanone.

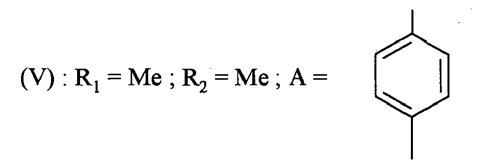

A 0,94 g de 1-[5-Méthyl-1-(4-nitrophényl)-1*H*-pyrazol-4-yl]éthanone (Journal of Chemical Research, Synopses (1986), (5), 166-7) dans 60 ml d'un mélange EtOH/eau (65/35 ; v/v), on ajoute 2 ml d'acide acétique et 0,64 g de Fer (0). On chauffe à 75°C pendant 2 heures. On concentre, filtre sur Célite®, neutralise le filtrat à l'aide de Na₂CO₃ saturé et extrait au DCM. On lave la phase organique à l'eau, à la saumure, sèche sur Na₂SO₄ et évapore. On obtient 0,70 g de poudre jaune.

RMN ¹H : DMSO-d₆ (250 MHz) : δ (ppm) : 2,41 (3H, s) ; 5,47 (2H, br) ; 6,65 (2H, d) ; 7,08 (2H,d) ; 8,12 (1H, s).

### Préparation 6.6

### 1-(4-Aminophényl)-5-méthyl-1H-pyrazole-4-carboxylate d'éthyle.

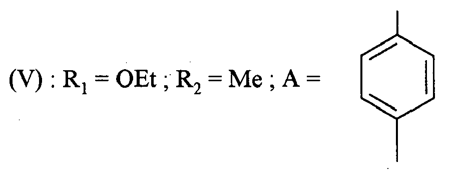

On dissout 0,1 g de 5-Méthyl-1-(4-nitrophényl)-1*H*-pyrazole-4-carboxylate d'éthyle (Russian Journal of Organic Chemistry English (2000) 36, 2, 191-194) dans 20 ml d'un mélange AcOEt/MeOH (50/50 ; v/v). On réduit à l'aide de l'appareil d'hydrogénation en continu (Cartouche Pd 10%, Mode Full H2, 50°C, débit 1ml/min). On concentre à sec, reprend le résidu solide à l'éther iso et essore le précipité formé. On obtient 0,08 g d'une poudre blanche.

RMN ¹H : DMSO-d₆ (250 MHz) : δ (ppm) : 1,41 (3H, t) ; 4,35 (2H, q) ; 6,74 (2H, d) ; 7,20 (2H, d) ; 8,03 (1H, s).

### Préparation 6.7

### 1-[1-(4-Amino-3-bromophényl)-5-méthyl-1H-pyrazol-4-yl]butan-1-one.

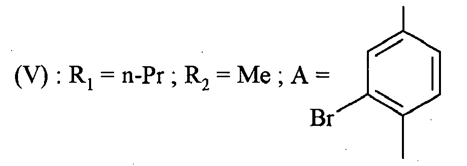

A 3 g du composé de la Préparation 6.3 dans 50 ml d'acétonitrile, on ajoute 2,2 g de N-bromosuccinimide puis chauffe à reflux pendant 1 heure. Après retour à TA, on ajoute une solution de NaHCO₃ 1N, extrait à l'AcOEt, lave à l'eau, à la saumure, sèche sur Na₂SO₄ et évapore à sec. On reprend le résidu à l'éther de pétrole, essore le précipité formé pour obtenir 3,5 g d'une poudre beige.

RMN ¹H : DMSO-d₆ (250 MHz) : δ (ppm) : 0,92 (3H, t) ; 1,62 (2H, sext) ; 2,44 (3H, s) ; 2,78 (2H, t) ; 5,69 (2H, br) ; 6,89 (1H, d) ; 7,19 (1H, d) ; 7,49 (1H, s) ; 8,17 (1H, s).

### Préparation 6.8

### 2-Amino-5-(4-butyryl-5-méthyl-1H-pyrazol-1-yl)benzonitrile.

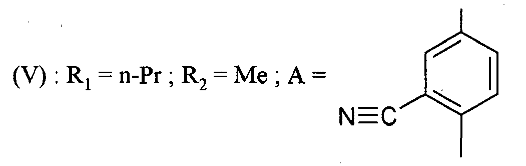

A 1 g du composé de la Préparation 6,7 dans 20 ml de DMF dans un tube scellé, on ajoute 0,44 g de cyanure de zinc et 0,18 g de Tétrakis(triphénylphosphine)palladium et chauffe à 100°C pendant 7 heures. On verse le milieu réactionnel sur une solution de NaHCO₃ saturée et on extrait à l'AcOEt. On lave à l'eau, à la saumure, sèche sur Na₂SO₄ et évapore à sec. On reprend le résidu à l'éther iso et essore le précipité formé pour obtenir 0,71 g d'une poudre blanche.

RMN ¹H : DMSO-d₆ (250 MHz) : δ (ppm) : 0,92 (3H, t) ; 1,61 (2H, sext) ; 2,44 (3H, s) ; 2,78 (2H, t) ; 6,45 (2H, br) ; 6,90 (1H, d) ; 7,42 (1H, d) ; 7,58 (1H, s) ; 8,19 (1H, s).

### Préparation 6.9

### 2-Amino-5-(4-butyryl-5-méthyl-1H-pyrazol-1-yl)benzoate de méthyle.

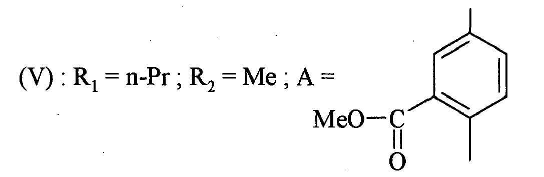

### Etape 1 : Acide 2-Amino-5-(4-butyryl-5-méthyl-1H-pyrazol-1-yl)benzoique.

A 1,5 g du composé de la Préparation 6.8 on ajoute 17 ml de NaOH 2N et chauffe à 100°C pendant 8 heures. On ajoute de l'eau, lave au DCM puis acidifie avec HCl 2N. On essore le précipité, lave à l'eau et sèche à l'étuve à vide. On obtient 1,4 g du composé attendu sous forme d'une poudre blanche.

RMN ¹H : DMSO-d₆ (250 MHz) : δ (ppm) : 0,93 (3H, t) ; 1,62 (2H, sext) ; 2,44 (3H, s) ; 2,78 (2H, t) ; 6,88 (1H, d) ; 7,33 (1H, d) ; 7,69 (1H, s) ; 8,17 (1H, s).

### Etape 2 : 2-Amino-5-(4-butyryl-5-méthyl-pyrazol-1-yl)-benzoate de méthyle.

A 1 g du composé de l'étape précédente dans 20 ml de DMF, on ajoute 0,38 g de bicarbonate de potassium puis 0,24 ml d'iodure de méthyle. Après 3 heures à TA, on ajoute de l'eau et extrait à l'AcOEt. On lave la phase organique à l'eau, à la saumure, sèche sur Na2SO4 et évapore à sec pour obtenir 0,89 g d'une poudre beige.

RMN ¹H : DMSO-d₆ (250 MHz) : δ (ppm) : 0,93 (3H, t) ; 1,62 (2H, sext) ; 2,44 (3H, s) ; 2,78 (2H, t) ; 3,80 (3H, s) ; 6,91 (1H, d) ; 6,97 (2H, br) ; 7,38 (1H, d) ; 7,72 (1H, s) ; 8,17 (1H, s).

### Préparation 6.10

### 2-Amino-5-(4-butyryl-5-méthyl-1H-pyrazol-1-yl)benzamide.

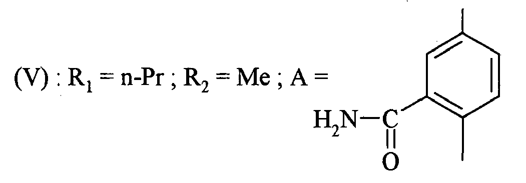

A 0,2 g du composé de la Préparation 6.8 dans 5 ml de dioxane, on ajoute 2,2 ml de NaOH 2N puis chauffe à 100°C pendant 20h. Après retour à TA, on ajoute de l'eau et extrait au DCM. On sèche sur Na₂SO₄ et évapore à sec. On purifie par chromatographie sur gel de silice en éluant avec un mélange de DCM/MeOH (jusqu'à 95/5 ; v/v). On obtient 0,14 g d'une poudre blanche.

RMN ¹H : DMSO-d₆ (250 MHz) : δ (ppm) : 0,93 (3H, t) ; 1,62 (2H, sext) ; 2,44 (3H, s) ; 2,78 (2H, t) ; 6,81 (1H, d) ; 6,98 (2H, br) ; 7,18 (1H, br) ; 7,24 (1H, d) ; 7,65 (1H, s) ; 7,81 (1H, br) ; 8,16 (1H, s).

### 7. Préparations des composés de formule (III).

### Préparation 7.1

### Dichlorhydrate de 1-(1-méthoxypropan-2-yl)pipérazine

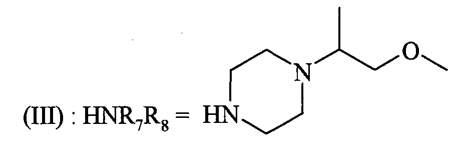

### Etape 1 : 4-(1-méthoxy-1-oxopropan-2-yl)pipérazine-1-carboxylate de tert-butyle.

A une solution de 2 g de pipérazine-1-carboxylate de *tert*-butyle dans 36 ml de DMF, on ajoute 2,97 g de carbonate de potassium et 1,97 g de 2-bromopropanoate de méthyle puis chauffe à 60°C pendant 1 heure. Après retour à TA, on ajoute 150 ml d'AcOEt, lave à l'eau, à la saumure, sèche sur Na₂SO₄ puis évapore à sec. On obtient 2,8 g du composé attendu sous forme d'une huile incolore.

RMN ¹H : DMSO-d₆ (250 MHz) : δ (ppm) : 1.18 (3H, d) ; 1.39 (9H, s) ; 2.49 (4H, m) ; 3.26 (4H, m) ; 3.37 (1H, q) ; 3.62 (3H, s).

### Etape 2 : Acide de 2-[4-(tert-butoxycarbonyl)pipérazin-1-yl]propanoique.

A une solution de 2,3 g du composé obtenu à l'étape précédente dans 30 ml de MeOH, on ajoute 6,33 ml d'une solution de NaOH 2N et agite pendant 20 heures. On ajoute le milieu réactionnel à une solution de chlorure d'ammonium puis évapore à sec. On reprend le résidu solide avec un mélange DCM/MeOH (9/1 ; v/v), filtre et évapore le filtrat. On reprend le résidu à l'éther et essore. On obtient 1,2 g du composé attendu sous forme d'une poudre blanche.

RMN ¹H : DMSO-d₆ (250 MHz) : δ (ppm) : 1.17 (3H, d) ; 1.39 (9H, s) ; 2.50 (m, 4H) ; 3.22-3.32 (m, 5H) ; 7.25 (1H, br).

### Etape 3 : 4-(1-hydroxypropan-2-yl)pipérazine-1-carboxylate de tert-butyle.

A une solution de 1,35 g du composé obtenu à l'étape précédente, dans 15 ml de THF, on ajoute 1,10 g de 1,1'-carbonyldimidazole et agite pendant 30 minutes. Par ailleurs, on prépare à 0°C une solution de 0,33 g de NaBH₄ dans 6 ml d'eau que l'on ajoute goutte à goutte, à la solution initiale d'ester activé, préalablement refroidie à -5°C. On ajoute 30 ml d'eau et extrait à l'AcOEt. On lave à la saumure, sèche sur Na₂SO₄ et évapore à sec. On purifie par chromatographie sur gel de silice par élution avec un mélange de DCM/MeOH (gradient de 2 à 5% de MeOH). On obtient 1 g du composé attendu sous forme d'une huile incolore.

RMN ¹H : DMSO-d₆ (250 MHz) : δ (ppm) : 0.90 (3H, d) ; 1.39 (9H, s) ; 2.42 (m, 4H) ; 2.57 (1H, q) ; 3.22-3.45 (m, 6H) ; 4.24 (1H, t).

### Etape 4 : 4-(1-méthoxypropan-2-yl)pipérazine-1-carboxylate de tert-butyle.

A une suspension de 0,17 g de NaH (60% dans l'huile) dans 5 ml de THF à 0°C, on ajoute une solution de 0,49 g du composé obtenu à l'étape précédente dans 5 ml de THF et agite pendant 30 minutes. On ajoute à 0°C, 0,27 ml d'iodométhane et agite pendant 20 heures. On ajoute 10 ml d'AcOEt, lave à l'eau, à la saumure, sèche sur Na₂SO₄ et évapore à sec. On purifie par chromatographie sur gel de silice par élution avec un mélange de DCM/MeOH (97/3 ; v/v). On obtient 0,41 g du composé attendu sous forme d'une huile incolore.

RMN ¹H : DMSO-d₆ (250 MHz) : δ (ppm) : 0.93 (3H, d) ; 1.39 (9H, s) ; 2.42 (m, 4H) ; 2.70 (1H, q) ; 3.17-3.48 (m, 11H).

### Etape 5 : Dichlorohydrate de 1-(1-méthoxypropan-2-yl)pipérazine.

A une solution de 0,40 g du composé obtenu à l'étape précédente dans 7 ml de DCM, on ajoute 3,88 ml d'une solution d'acide chlorhydrique 4N dans le dioxane. Après 2 heures à TA, on évapore à sec, reprend à l'acétone et filtre. On obtient 0,34 g du composé attendu sous forme d'une poudre blanche.

RMN ¹H : DMSO-d₆ (250 MHz) : δ (ppm) : 1.30 (3H, d) ; 3.31 (3H, s) ; 3.40-4.00 (m, 11H) ; 9.75 (2H, br) ; 11.75 (1H, br).

### Préparation 7.2

### Oxetan-3-ylpipérazine.

A une solution de 2,0 g de pipérazine-1-carboxylate de benzyle dans 20 ml d'acétonitrile on ajoute 2,6 g de 3-oxetanone, 1,0 g de cyanoborohydrure de sodium et 0,16 ml d'acide acétique puis laisse 16 heures sous agitation à TA. On dilue le mélange réactionnel à l'eau et filtre au travers d'une cartouche Chem Elut® en éluant au DCM. Les phases organiques jointes sont séchées sur MgSO₄ et on évapore le solvant sous vide. On purifie le résidu par CLHP préparative et obtient 1,7 g d'un solide blanc. On dissout 0,5 g de ce solide dans 20 ml d'EtOH, ajoute 0,2 g de Pd/C à 10% et agite le mélange sous atmosphère d'hydrogène (4 bars) pendant 3 heures. On filtre le mélange réactionnel sur Célite® et concentre sous vide le filtrat. On obtient le composé attendu.

### Préparation 7.3

### Ditrifluoroacétate de N-méthyl-N-[2-(pipérazin-1-yl)éthyl]acétamide.

A une solution de 0,748 g de N-méthylacétamide dans 80 ml de THF on ajoute 0,341 g de NaH à 60% dans l'huile et laisse 10 minutes sous agitation à TA. On ajoute ensuite 2 g de 4-(2-bromoéthyl)pipérazinecarboxylate de *tert-* butyle et laisse 16 heures sous agitation à TA. On ajoute de l'eau au mélange réactionnel, décante et évapore le solvant organique sous vide. On dissout le résidu dans du DCM, filtre au travers d'une cartouche Chem Elut® en éluant au DCM et évapore les solvants sous vide. On purifie le résidu par CLHP préparative et obtient un solide blanc. On dissout le solide dans 5 ml de DCM, ajoute 1,2 ml de TFA et laisse 16 heures sous agitation à TA. On dilue le mélange réactionnel par ajout de 100 ml de toluène et concentre les solvants sous vide.On obtient 1,5 g du composé attendu.

### Préparation 7.4

### Ditrifluoroacétate de (1S,4S)-2-(2-méthoxyéthyl)-2,5-diazabicyclo[2.2.1]heptane.

A une solution de 1,0 g de chlorhydrate de (1S,4S)-2,5-diazabicyclo[2.2.1]heptane-2-carboxylate de *tert*-butyle dans 10 ml d'EtOH on ajoute 2 ml de DIPEA et 0,52 ml de 1-bromo-2-méthoxyéthane et chauffe à reflux pendant 16 heures. Après refroidissement à TA, on dilue le mélange réactionnel par ajout d'eau et filtre au travers d'une cartouche Chem Elut® en éluant au DCM. On sèche les phases organiques jointes sur MgSO₄ et évapore le solvant sous vide. On dissout le résidu dans 10 ml de DCM, ajoute 15 ml de TFA et laisse 2 heures sous agitation à TA. On ajoute 100 ml de toluène et concentre les solvants sous vide. On obtient 1,45 g du composé attendu.

### Préparation 7.5

### Ditrifluoroacétate de (2R)-2-(cyclobutylcarbamoyl)-1-méthylpipérazine.

A une solution de 1 g de (3R)-pipérazine-1,3-dicarboxylate de *tert*-butyle dans 26 ml de MeOH on ajoute 0,39 ml d'une solution de formaldéhyde à 37% dans l'eau, 0.32 g de cyanoborohydrure de sodium et 0,994 ml d'acide acétique et laisse 3 heures sous agitation à TA. On dilue le mélange réactionnel par ajout d'une solution saturée de K₂CO₃, extrait au DCM, sèche la phase organique sur MgSO₄ et évapore le solvant sous vide. On dissout le résidu dans 4 ml de DMF, ajoute 0,19 g d'HOAT, 0,267 g d'EDC et 0,1 g de cyclobutylamine et laisse 16 heures sous agitation à TA. On dilue le mélange réactionnel par ajout d'une solution saturée de NaHCO₃, extrait au DCM, sèche la phase organique sur MgSO₄ et évapore le solvant sous vide. On purifie le résidu sur CLHP préparative, concentre sous vide les fractions contenant le produit et le lyophilise pour obtenir 0,31 g d'un solide blanc. On dissout le solide dans 2 ml de DCM, ajoute 2,5 ml de TFA et laisse 4 heures sous agitation à TA. On dilue par ajout de 100 ml de toluène et concentre sous vide les solvants. On obtient 0,94 g du composé attendu.

### Préparation 7.6

### 1,1,1-Trifluoro-3-(pipérazin-1-yl)propan-2-ol.

### Etape 1 : 4-(3,3,3-trifluoro-2-hydroxypropyl)pipérazine-1-carboxylate de benzyle.

A une solution de 1 g de pipérazine-1-carboxylate de benzyle dans 100 ml de N-méthylpyrrolidine on ajoute 0,47 ml de 1,1,1-trifluoro-2,3-epoxypropane et chauffe à 80°C pendant 12 heures. On rajoute 0,05 ml de 1,1,1-trifluoro-2,3-époxypropane et chauffe à 80°C pendant 48 heures. On extrait le mélange réactionnel au DCM, lave la phase organique par une solution de LiCl à 4% dans l'eau, sèche sur MgSO4 et évapore sous vide le solvant. On purifie le résidu sur CLHP préparative et obtient 1,7 g du composé attendu.

### Etape 2 : 1,1,1-Trifluoro-3-(pipérazin-1-yl)propan-2-ol.

A une solution de 1,7 g du composé de l'étape précédente dans 50 ml d'EtOH on ajoute, sous atmosphère d'argon, 0,15 g de Pd/C à 10% et laisse 16 heures sous agitation sous atmosphère d'hydrogène (3 bars). On filtre le mélange réactionnel sur Célite®, lave à l'EtOH et concentre sous vide le filtrat. On obtient 1,2 g du composé attendu.

### Préparation 7.7

### 1-(2-méthoxyéthyl)-4,5,6,7-tétrahydro-1H-imidazo[4,5-c]pyridine.

### Etape 1 : 1,4,6,7-tétrahydro-5H-imidazo[4,5-c]pyridine-5-carboxylate de benzyle.

On laisse 16 heures sous agitation à TA un mélange de 2,0 g de dichlorhydrate de 4,5,6,7-tétrahydro-1*H*-imidazo[4,5-c]pyridine, 2,36 g de NaHCO₃ et 2,45 g de N-(benzyloxycarbonyloxy)succinimide dans 80 ml du mélange dioxane/eau (50/50 ;v/v). On extrait le mélange réactionnel à l'AcOEt, lave la phase organique par une solution saturée de NaHCO₃ , par une solution d'HCl 0,1 M, par une solution saturée de NaCl, sèche et évapore sous vide le solvant. On chromatographie le résidu sur gel de silice en éluant par le mélange DCM/MeOH. On obtient 1,9 g du composé attendu.

### Etape 2 : 1-(2-méthoxyéthyl)-1,4,6,7-tétrahydro-5H-imidazo[4,5-c]pyridine-5-carboxylate de benzyle.

A une solution de 0,475 g du composé de l'étape précédente dans 20 ml de toluène et 5 ml de DCM on ajoute 3,6 ml de NaOH 6N, 0,051 g de bromure de tétrabutylammonium et 0,308 g de 1-bromo-2-méthoxyéthane et laisse 12 heures sous agitation à TA. On rajoute 0,308 g de 1-bromo-2-méthoxyéthane et 0,05 g d' iodure de tétrabutylammonium et laisse 36 heures sous agitation à TA. On concentre le mélange réactionnel sous vide, extrait le résidu au DCM, lave la phase organique à l'eau, sèche et évapore sous vide le solvant. On purifie le produit ainsi obtenu par CLHP préparative et obtient 0,24 g du composé attendu.

### Etape 3 : 1-(2-méthoxyéthyl)-4,5,6,7-tétrahydro-1H-imidazo[4,5-c]pyridine.

A une solution de 0,25 g du composé de l'étape précédente dans 50 ml d'EtOH on ajoute sous argon 0,025 g de Pd/C à 10% et laisse 16 heures sous agitation sous atmosphère d'hydrogène (1 bar). On filtre sur Célite®, lave à l'EtOH et concentre sous vide le filtrat.on obtient 0,115 g du composé attendu.

### Préparation 7.8

### Trans-2-(pipérazin-1-yl)cyclopentanol.

### Etape 1 : 4-(trans-2-hydroxycyclopentyl)pipérazine-1-carboxylate de benzyle.

On refroidit à 0°C une solution de 0,524 g de pipérazine-1-carboxylate de benzyle, ajoute 0,2 g de 1,2-époxycyclopentane et chauffe à 80°C pendant quatre jours. On concentre le mélange réactionnel sous vide et purifie le résidu par chromatographie sur gel de silice en éluant par le mélange AcOEt/MeOH. On obtient 0,32 g du composé attendu.

### Etape 2 : Trans-2-(pipérazin-1-yl)cyclopentanol.

A une solution de 0,32 g du composé de l'étape précédente dans 50 ml d'EtOH on ajoute sous argon 0,025 g de Pd/C à 10% et laisse 16 heures sous agitation sous atmosphère d'hydrogène (3 bars). On filtre le mélange réactionnel sur Célite, lave à l'EtOH et concentre sous vide le filtrat. On obtient 0,19 gdu composé attendu.

### Préparation 7.9

### 2-(Pyrrolidin-3-yloxy)éthanol.

### Etape 1 : 3-(2-benzyloxyéthoxy)pyrrolidine-1-carboxylate de benzyle.

On refroidit à 0°C une solution de 1,29 g de 3-hydroxypyrrolidine-1-carboxylate de benzyle dans 80 ml de THF, ajoute 0,245 g de NaH à 60% dans l'huile puis 1,26 g de [(2-bromoéthoxy)méthyl]benzène et 0,108 g d'iodure de tétrabutylammonium et chauffe à 80°C pendant 3 heures. On rajoute 0,28 g de NaH et 0,40 g de [(2-bromoéthoxy)méthyl]benzène et chauffe à 80°C pendant 2 heures. On concentre le mélange réactionnel sous vide, extrait le résidu au DCM, lave la phase organique par une solution d'HCl 0,1 M, par une solution saturée de NaHCO₃ , sèche et évapore le solvant sous vide. On purifie le produit ainsi obtenu par CLHP préparative et obtient 0,46 g du composé attendu.

### Etape 2 : 2-(Pyrrolidin-3-yloxy)éthanol.

A une solution de 0,459 g du composé de l'étape précédente dans 35 ml d'EtOH on ajoute, sous argon, 0,05 g de Pd/C à 10% et laisse 16 heures sous agitation sous atmosphère d'hydrogène (3 bars). On filtre le mélange réactionnel sur Célite, lave à l'EtOH et concentre le filtrat sous vide. On obtient 0,205 g du composé attendu.

### Préparation 7.10

### Trifluoroacétate de (2S,4R)-2-(cyclobutylcarbamoyl)-4-hydroxypyrrolidine.

### Etape 1 : (2S,4R)-2-(cyclobutylcarbamoyl)-4-hydroxypyrrolidine-1-carboxylate de tert-butyle.

A une solution de 2,0 g de (4R)-1-(*tert*-butoxycarbonyl)-4-hydroxy-L-proline dans 20 ml de DMF on ajoute 1,17 g de HOAT, 1,66 g de chlorhydrate de 1-(3-diméthymaminopropyl)-3-éthylcarbodiimide et 0,615 g de cyclobutylamine. On ajoute 1,5 ml d'une solution saturée de NaHCO₃ et filtre le mélange réactionnel sur une cartouche Chem Elut® en éluant au DCM. On concentre sous vide les solvants et purifie le résidu par filtration sur gel de silice en éluant à l'AcOEt. On obtient 1,22 g du composé attendu.

### Etape 2: trifluoroacétate de (2S,4R)-2-(cyclobutylcarbamoyl)-4-hydroxypyrrolidine.

A une solution de 1,22 g du composé de l'étape précédente dans 31 ml de DCM on ajoute 3,2 ml de TFA et laisse 12 heures sous agitation à TA. On concentre sous vide, reprend le résidu au toluène et évapore le solvant sous vide. On obtient 1,51 g du composé attendu.

### Préparation 7.11

### Ditrifluoroacétate de 2-(méthoxyméthyl)-1-méthylpipérazine.

### Etape 1 : 3-(hydroxyméthyl)-4-méthylpipérazine-1-carboxylate de tert-butyle.

A une solution de 0,5 g de 3-(hydroxyméthyl)pipérazine-1-carboxylate de *tert-*butyle dans 50 ml de MeOH on ajoute 0,303 g d'acétate de sodium, 0,375 g de formaldéhyde et 0,218 g de cyanoborohydrure de sodium et laisse 1 heure sous agitation à TA. On ajoute une solution saturée de NaHCO₃, extrait au DCM, sèche la phase organique sur Na₂SO₄ et évapore le solvant sous vide. On chromatographie le résidu sur gel de silice en éluant par le mélange DCM/MeOH. On obtient 0,31 g du composé attendu.

### Etape 2 : 3-(méthoxyméthyl)-4-méthylpipérazine-1-carboxylate de tert-butyle.

On refroidit à 0°C une solution de 0,125 g du composé de l'étape précédente dans 12 ml de DMF, ajoute 0,024 g de NaH (60% dans l'huile) et 0,092 g d'iodométhane et laisse 16 heures sous agitation à TA. On ajoute de l'eau au mélange réactionnel, extrait au DCM, sèche la phase organique sur Na₂SO₄ et évapore le solvant sous vide. On obtient 0,122 g du composé attendu.

### Etape 3 : Ditrifluoroacétate de 2-(méthoxyméthyl)-1-méthylpipérazine.

A une solution de 0,122 g du composé de l'étape précédente dans 5 ml de DCM on ajoute 0,4 ml de TFA et laisse 12 heures sous agitation à TA. On concentre sous vide, reprend le résidu au toluène et évapore le solvant sous vide. On obtient 0,14 g du composé attendu.

### Préparation 7.12

### 3-Méthyl-4,5,6,7-tétrahydro-3H-imidazo[4,5-c]pyridine.

### Etape 1 : 3,4,6,7-tétrahydro-5H-imidazo[4,5-c]pyridine-5-carboxylate de benzyle.

On laisse 16 heures sous agitation à TA un mélange de 2,0 g de dichlorhydrate de 4,5,6,7-tétrahydro-3H-imidazo[4,5-c]pyridine, 2,36 g de NaHCO₃ et 2,45 g de N-(benzyloxycarbonyloxy)succinimide dans 80 ml d'un mélange dioxane/eau (50/50 ;v/v). On extrait le mélange réactionnel à l'AcOEt, lave la phase organique par une solution saturée de NaHCO₃, par une solution 0,1 M d'HCl, par une solution saturée de NaCl, sèche sur Na₂SO₄ et évapore le solvant sous vide. On purifie le résidu par chromatographie sur gel de silice en éluant par le mélange DCM/MeOH. On obtient 1,9 g du composé attendu.

### Etape 2 : 3-méthyl-3,4,6,7-tétrahydro-5H-imidazo[4,5-c]pyridine-5-carboxylate de benzyle.

A une solutionde 0,475 g du composé de l'étape précédente dans 20 ml de toluène et 5 ml de DCM on ajoute 3,6 ml de NaOH 6N, 0,051g de bromure de tétrabutylammonium et 0,313 g d'iodométhane et laisse 12 heures sous agitation à TA. On concentre le mélange réactionnel sous vide, extrait le résidu au DCM, lave la phase organique à l'eau, sèche et évapore le solvant sous vide. On purifie le résidu par CLHP préparative. On obtient 0,32 g du composé attendu.

### Etape 3 : 3-Méthyl-4,5,6,7-tétrahydro-3H-imidazo[4,5-c]pyridine.

A une solution de 0,3 g du composé de l'étape précédente dans 50ml d'EtOH on ajoute, sous argon, 0,025 g de Pd/C à 10% et laisse 16 heures sous agitation sous atmosphère d'hydrogène (1 bar). On filtre le mélange réactionnel sur Célite®, lave à l'EtOH et concentre le filtrat sous vide. On obtient 0,166 g du composé attendu sous forme d'huile.

### 8. Préparations des composés de formule (VI).

### Préparation 8.1

N-[4-(4-Butyryl-5-méthyl-1*H*-pyrazol-1-yl)phényl]-6-méthyl-1*H*-indole-3-carboxamide.

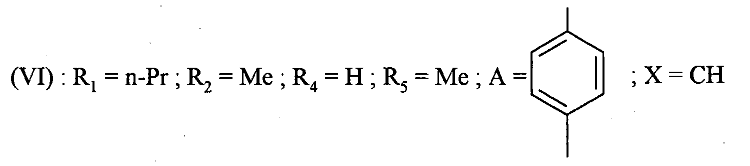

### Etape 1 : 1-acétyl-N-[4-(4-butyryl-5-méthyl-1H-pyrazol-1-yl)phényl]-6-méthyl-1H-indole-3-carboxamide (XIV).

On refroidit à 0°C un mélange de 1,03 g du composé de la Préparation 4.2 dans 20 ml de DCM, ajoute 0,81 g du composé de la Préparation 6.3, 0,62 g de DMAP puis 0,97 g de chlorhydrate de 1-(3-diméthylaminopropyl)-3-éthylcarbodiimide et laisse 2 heures sous agitation en laissant remonter la température à TA. On ajoute 100 ml d'AcOEt, lave la phase organique avec une solution d'HCl 1N, avec une solution de NaOH 1N, à l'eau, par une solution saturée de NaCl, sèche sur Na₂SO₄ et évapore le solvant sous vide. On reprend le résidu à l'éther iso, essore le précipité formé et le sèche sous vide. On obtient 1,39 g du composé attendu.

RMN ¹H : DMSO-d₆ (250 MHz) : δ (ppm) : 0.95 (3H, t) ; 1.65 (2H, m) ; 2.47-2.52 (6H) ; 2.77 (3H, s) ; 2.83 (2H, t) ; 7.24 (1H, d) ; 7.55 (2H, d) ; 7.95 (2H, d) ; 8.13 (1H, d) ; 8.24 (2H, d) ; 8.75 (1H, s) ; 10.30 (1H, s).

En procédant selon le mode opératoire décrit à l'étape 1, on prépare les composés de formule (XIV) rassemblés dans le TABLEAU V ci-après :

**TABLEAU V**

| | | | |
|---|---|---|---|
| 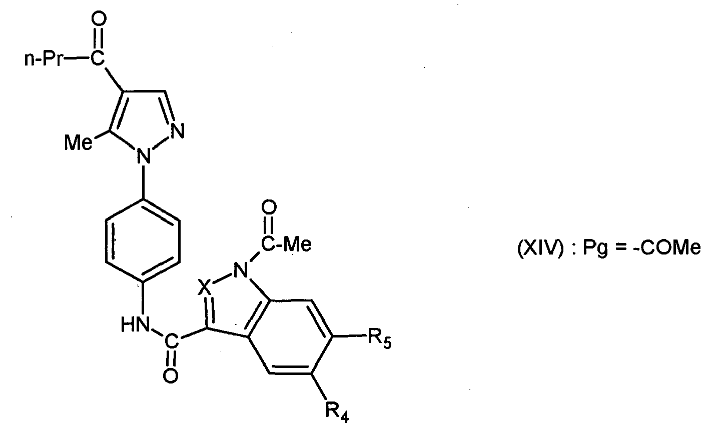 | | | |

| X | R₄ | R₅ | RMN ¹H : DMSO-d₆ (250 MHz) : δ (ppm) |
|---|---|---|---|
| CH | F | H | 0,94 (3H, t) ; 1,64 (2H, m) ; 2,53 (3H, s) ; 2,77 (3H, s) ; 2,82 (2H, t) ; 7,29 (1H, t) ; 7,56 (2H, d) ; 7,95 (3H, m) ; 8,25 (1H, s) ; 8,39 (1H, dd) ; 8,92 (1H, s) ; 10,35 (1H, s). |
| CH | Me | Br | 0,94 (3H, t) ; 1,64 (2H, m) ; 2,48-2,52 (6H) ; 2,76 (3H, s) ; 2,82 (2H, t) ; 7,55 (2H, d) ; 7,94 (2H, d) ; 8,22 (1H, s) ; 8,25 (1H, s) ; 8,58 (1H, s) ; 8,82 (1H, s) ; 10,30 (1H, s). |
| CH | Br | Me | 0,94 (3H, t) ; 1,64 (2H, m) ; 2,52 (3H, s) ; 2,76 (3H, s) ; 2,82 (2H, t) ; 7,55 (2H, d) ; 7,95 (2H, d) ; 8,27 (1H, s) ; 8,38 (1H, s) ; 8,45 (1H, s) ; 8,86 (1H, s) ; 10,35 (1H, s). |
| CH | Cl | F | 0,94 (3H, t) ; 1,64 (2H, m) ; 2,52 (3H, s) ; 2,76 (3H, s) ; 2,82 (2H, t) ; 7,55 (2H, d) ; 7,93 (2H, d) ; 8,20-8,40 (3H, m) ; 8,92 (1H, s) ; 10,35 (1H, s). |
| N | Me | H | 0,95 (3H, t) ; 1,64 (2H, m) ; 2,48-2,52 (6H) ; 2,83 (2H, t) ; 2,90 (3H, s) ; 7,54-7,62 (3H, m) ; 8,03-8,10 (3H, m) ; 8,27-8,30 (2H, m) ; 10,75 (1H, s). |

### Etape 2 : N-[4-(4-Butyryl-5-méthyl-1H-pyrazol-1-yl)phényl]-6-méthyl-1H-indole-3-carboxamide.

A une solution de 1,15 g du composé de l'étape 1 dans 40 ml d'un mélange MeOH/THF (50/50 ; v/v) on ajoute 1,5 g de K₂CO₃ et laisse 4 heures sous agitation à TA. On concentre le mélange réactionnel sous vide, reprend le résidu par un mélange AcOEt/eau, lave la phase organique à l'eau, par une solution saturée de NaCl, sèche sur Na₂SO₄ et évapore le solvant sous vide. On reprend le résidu à l'éther iso et essore le précipité formé. On obtient 0,9 g du composé attendu.

RMN ¹H : DMSO-d₆ (250 MHz) : δ (ppm) : 0,95 : t : 3H ; 1,65 : m : 2H ; 2,42 : s : 3H ; 2,50 : s : 3H ; 2,82 : t : 2H ; 7,00 : d : 1H ; 7,27 : s : 1H ; 7,47 : d : 2H ; 7,95 : d : 2H ; 8,07 : d : 1H ; 8,24 : s : 1H ; 8,25 : s : 1H ; 9,90 : s : 1H ; 11,60 : s : 1H.

En procédant selon le mode opératoire décrit à l'étape 2, on prépare les composés de formule (VI) rassemblés dans le TABLEAU VI ci-après :

**TABLEAU VI**

| | | | | |
|---|---|---|---|---|
| 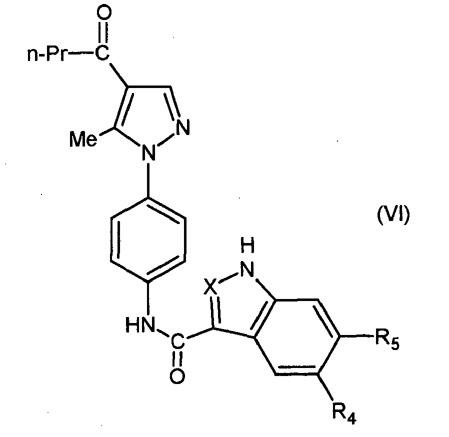 | | | | |

| | X | R₄ | R₅ | RMN ¹H : DMSO-d₆ (250 MHz) : δ (ppm) |
|---|---|---|---|---|
| Préparation 8.2 | CH | F | H | 0,95 (3H, t) ; 1,63 (2H, m) ; 2,52 (3H, s) ; 2,83 (2H, t) ; 7,07 (1H, t) ; 7,47-7,55 (3H, m) ; 7,90 (1H, d) ; 7,96 (2H, d) ; 8,26 (1H, s) ; 8,43 (1H, dd) ; 10,03 (1H, s) ; 11,95 (1H, br). |
| Préparation 8.3 | CH | Me | Br | 0,94 (3H, t) ; 1,64 (2H, m) ; 2,46 (3H, s) ; 2,50 (3H, s) ; 2,81 (2H, t) ; 7,48 (2H, d) ; 7,73 (1H, s) ; 7,94 (2H, d) ; 8,17 (1H, s) ; 8,24 (1H, s) ; 8,32 (1H, s) ; 8,98 (1H, s) ; 11,75 (1H, s). |
| Préparation 8.4 | CH | Br | Me | 0,95 (3H, t) ; 1,65 (2H, m) ; 2,46 (3H, s) ; 2,50 (3H, s) ; 2,82 (2H, t) ; 7,47-7,52 (3H, m) ; 7,95 (2H, d) ; 8,24 (1H, s) ; 8,34 (1H, s) ; 8,39 (1H, s) ; 10,00 (1H, s) ; 11,85 (1H, s). |
| Préparation 8.5 | CH | Cl | F | 0,94 (3H, t) ; 1,64 (2H, m) ; 2,50 (3H, s) ; 2,81 (2H, t) ; 7,46-7,57 (3H, m) ; 7,94 (2H, d) ; 8,24 (1H, s) ; 8,29 (1H, d) ; 8,41 (1H, s) ; 10,10 (1H, s) ; 12,00 (1H, br). |
| Préparation 8.6 | N | Me | H | 0,95 (3H, t) ; 1,64 (2H, m) ; 2,48 (3H, s) ; 2,50 (3H, s) ; 2,83 (2H, t) ; 7,31 (1H, d) ; 7,51 (2H, d) ; 8,04 (1H, d) ; 8,10 (2H, d) ; 8,25 (1H, s) ; 10,55 (1H, s) ; 13,75 (1H, s). |

### Préparation 8.7

### N-[4-(4-Butyryl-5-méthyl-1H-pyrazol-1-yl)phényl]-5-chloro-6-méthyl-1H-indole-3-carboxamide.

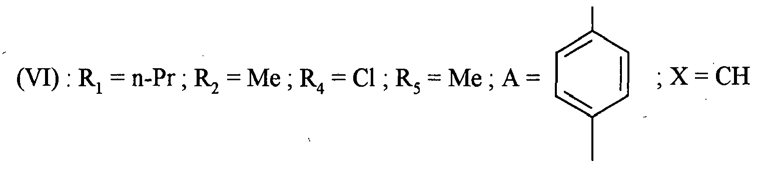

A une solution de 1 g du composé de la Préparation 8.4 dans 6 ml de DMF on ajoute 0,5 g de chlorure de nickel (II) et chauffe au micro-onde pendant 30 minutes à 200°C. Après refroidissement à TA, on ajoute 60 ml d'eau, extrait à l'AcOEt, lave la phase organique par une solution saturée de NaCl, sèche sur Na₂SO₄ et évapore le solvant sous vide. On reprend le résidu dans le mélange acétone/ether iso (50/50 ; v/v) et essore le précipité formé. On obtient 0,66 g du composé attendu.

RMN ¹H : DMSO-d₆ (250 MHz) : δ (ppm) : 0,94 (3H, t) ; 1,64 (2H, m) ; 2,47 (3H, s) ; 2,52 (3H, s) ; 2,82 (2H, t) ; 7,46-7,51 (3H, m) ; 7,94 (2H, d) ; 8,19 (1H, s) ; 8,24 (1H, s) ; 8,34 (1H, s) ; 9,95 (1H, s); 11,80 (1H, br).

### 9. Préparations des composés de formule (II).

### Préparation 9.1

### (3-{[5-(4-Butyryl-5-méthyl-1H-pyrazol-1-yl)pyrazin-2-yl]carbamoyl}-5-chloro-1H-indol-1-yl)acétate de méthyle.

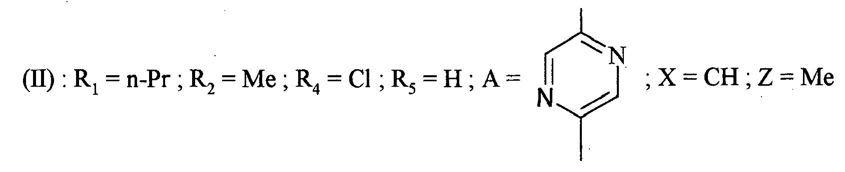

### Etape 1 : [5-Chloro-3-(chlorocarbonyl)-1H-indol-1-yl]acétate de méthyle.

A une solution de 6,00 g du composé de la Préparation 3.1 dans 200 ml de DCM, on ajoute 6,6 ml de chlorure de thionyle et quelques gouttes de DMF. Après 3h au reflux, on évapore à sec et triture le résidu solide avec 80 ml de DCM. Le précipité formé est filtré, lavé au DCM pour obtenir 4,5 g d'une poudre blanche.

### Etape 2 : (3-{[5-(4-Butyryl-5-méthyl-1H-pyrazol-1-yl)pyrazin-2-yl]carbamoyl}-5-chloro-1H-indol-1-yl)acétate de méthyle.

A 0,17 g du composé de la Préparation 6.1 dans 10 ml de 1,2-dichloroéthane, on ajoute 0,16 ml de 1,8-Diazabicyclo[5.4.0]undec-7-ène et 0,26 g du composé de l'étape 1 puis chauffe à 80°C pendant 2 heures. Après retour à TA, on lave le milieu réactionnel à l'eau puis à la saumure et sèche sur Na₂SO₄. On évapore puis purifie le résidu solide par chromatographie sur gel de silice en éluant avec un mélange DCM/ MeOH (gradient de 1 à 2 % de MeOH). Le résidu solide est trituré à l'éther iso puis filtré pour obtenir 0,14 g d'une poudre jaune.

RMN ¹H : DMSO-d₆ (250 MHz) : δ (ppm) : 0.94 (3H, t) ; 1.63 (2H, m) ; 2.78 (3H, s) ; 2.86 (2H, t) ; 3.73 (3H, s) ; 5.32 (2H, s) ; 7.31 (1H, d) ; 7.61 (1H, d) ; 8.25 (1H, s) ; 8.36 (1H, s) ; 8.62 (1H, s) ; 8.86 (1H, s) ; 9.42 (1H, s) ; 11.15 (1H, s).

### Préparation 9.2

### (3-{[6-(4-Butyryl-5-méthyl-1H-pyrazol-1-yl)pyridazin-3-yl]carbamoyl}-5-chloro-1H-indol-1-yl)acétate de méthyle.

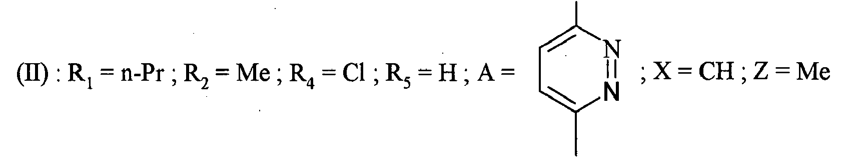

On laisse 30 minutes sous agitation une solution de 0,35 g du composé de la Préparation 6.2 et 0,59 g de DMAP en présence de 1 g de tamis moléculaire 4A dans 20 ml de 1,2-dichloroéthane. On ajoute 0,92 g du composé obtenu à l'étape 1 de la Préparation 9.1, puis chauffe à 80°C pendant 6 heures. Après retour à TA, on élimine le tamis moléculaire par filtration, lave le milieu réactionnel à l'eau puis à la saumure et sèche sur Na₂SO₄. On évapore puis purifie le résidu solide par chromatographie sur gel de silice en éluant avec un mélange de DCM/MeOH (gradient de 0 à 5 % de MeOH) pour obtenir 0,51 g d'une poudre blanche.

RMN ¹H : DMSO-d₆ (250 MHz) : δ (ppm) : 0.94 (3H, t) ; 1.64 (2H, m) ; 2.82 (3H, s) ; 2.87 (2H, t) ; 3.73 (3H, s) ; 5.33 (2H, s) ; 7.32 (1H, d) ; 7.62 (1H, d) ; 8.13 (1H, d) ; 8.23 (1H, s) ; 8.41 (1H, s) ; 8.65 (1H, s) ; 8.71 (1H, d) ; 11.42 (1H, s).

### Préparation 9.3

### (3-{[4-(4-Butyryl-5-méthyl-1H-pyrazol-1-yl)phényl]carbamoyl}-5-chloro-6-fluoro-1H-indol-1-yl)acétate de méthyle.

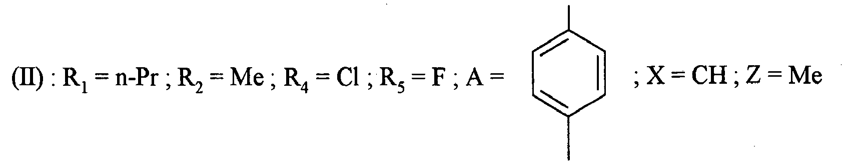

On refroidit à 0°C une solution de 1,25 g du composé de la Préparation 8.5 dans 15 ml de DMF, ajoute 0,47 g de K₂CO₃ puis 0,49 g de bromoacétate de méthyle et laisse 3 heures sous agitation à TA. On ajoute 50 ml d'AcOEt et 100 ml d'eau, décante, lave la phase organique à l'eau, par une solution saturée de NaCl, sèche sur Na₂SO₄ et évapore le solvant sous vide. On obtient 1,38 g d'une poudre blanche.

RMN ¹H : DMSO-d₆ (250 MHz) : δ (ppm) : 0,94 (3H, t) ; 1,64 (2H, m) ; 2,51 (3H, s) ; 2,81 (2H, t) ; 3,73 (3H, s) ; 5,32 (2H, s) ; 7,50 (2H, d) ; 7,78 (1H, d) ; 7,94 (2H, d) ; 8,24 (1H, s) ; 8,30 (1H, d) ; 8,35 (1H, s) ; 10,20 (1H, s).

En procédant selon le mode opératoire décrit à la Préparation 9.3, on prépare les composés de formule (II) rassemblés dans le TABLEAU VII ci-après :

**TABLEAU VII**

| | | | | | |
|---|---|---|---|---|---|
| 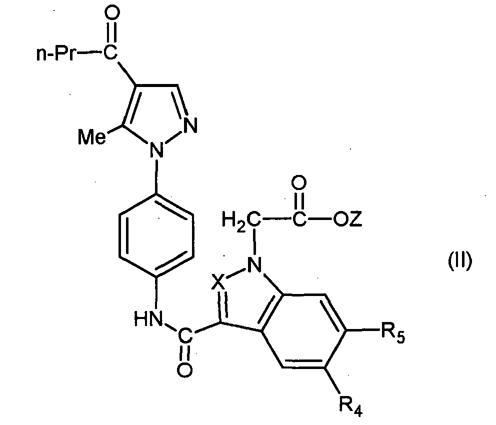 | | | | | |

| | X | R₄ | R₅ | Z | RMN ¹H : DMSO-d₆ (250 MHz) : δ (ppm) |
|---|---|---|---|---|---|
| Préparation 9.4 | CH | F | H | Me | 0,94 (3H, t) ; 1,64 (2H, m) ; 2,51 (3H, s) ; 2,82 (2H, t) ; 3,73 (3H, s) ; 5,33 (2H, s) ; 7,13 (1H, t) ; 7,49 (2H, d) ; 7,56 (1H, dd) ; 7,87-7,97 (3H, m) ; 8,24 (1H, s) ; 8,35 (1H, s) ; 10,10 (1H, s). |
| Préparation 9.5 | CH | H | Me | Me | 0,94 (3H, t) ; 1,64 (2H, m) ; 2,44 (3H, s) ; 2,50 (3H, s) ; 2,81 (2H, t) ; 3,73 (3H, s) ; 5,26 (2H, s) ; 7,06 (1H, d) ; 7,32 (1H, s) ; 7,49 (2H, d) ; 7,95 (2H, d) ; 8,09 (1H, d) ; 8,20 (1H, s) ; 8,24 (1H, s) ; 10,05 (1H, s). |
| Préparation 9.6 | CH | Me | Br | Me | 0,94 (3H, t) ; 1,64 (2H, m) ; 2,47 (3H, s) ; 2,52 (3H, s) ; 2,82 (2H, t) ; 3,73 (3H, s) ; 5,31 (2H, s) ; 7,49 (2H, d) ; 7,89 (1H, s) ; 7,94 (2H, d) ; 8,17 (1H, s) ; 8,24 (1H, s) ; 8,25 (1H, s) ; 10,10 (1H, s). |
| Préparation 9.7 | CH | Br | Me | Me | 0,95 (3H, t) ; 1,64 (2H, m) ; 2,44 (3H, s) ; 2,51 (3H, s) ; 2,83 (2H, t) ; 3,74 (3H, s) ; 5,30 (2H, s) ; 7,50 (2H, d) ; 7,60 (1H, s) ; 7,94 (2H, d) ; 8,26 (1H, s) ; 8,28 (1H, s) ; 8,41 (1H, s) ; 10,15 (1H, s). |
| Préparation 9.8 | CH | Cl | Me | Me | 0,95 (3H, t) ; 1,65 (2H, m) ; 2,45 (3H, s) ; 2,51 (3H, s) ; 2,83 (2H, t) ; 3,74 (3H, s) ; 5,31 (2H, s) ; 7,50 (2H, d) ; 7,59 (1H, s) ; 7,95 (2H, d) ; 8,21 (1H, s) ; 8,26 (1H, s) ; 8,29 (1H, s) ; 10,15 (1H, s). |
| Préparation 9.9 | N | Me | H | Me | 0,94 (3H, t) ; 1,64 (2H, m) ; 2,50 (3H, s) ; 2,53 (3H, s) ; 2,82 (2H, t) ; 3,72 (3H, s) ; 5,55 (2H, s) ; 7,37 (1H, d) ; 7,51 (2H, d) ; 7,71 (1H, d) ; 8,06-8,10 (3H, m) ; 8,25 (1H, s) ; 10,60 (1H, s). |

### Préparation 9.10

### (3-{[4-(4-Butyryl-5-méthyl-1H-pyrazol-1-yl)phényl]carbamoyl}-5-chloro-1H-indol-1-yl)acétate de méthyle.

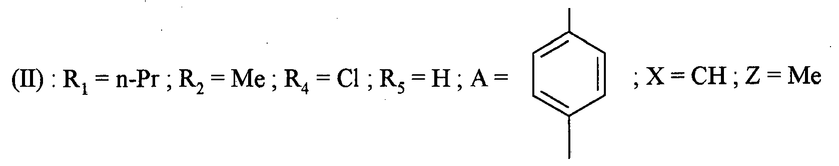

A une solution de 2,70 g du composé de la Préparation 3.1 dans 50 ml de 1,2-dichloroéthane on ajoute 2,17 g de chlorhydrate de 1-(3-diméthylaminopropyl)-3-éthylcarbodiimide, 2,61 g de DMAP et 2,67 g du composé de la Préparation 6.3 et laisse 20 heures sous agitation à TA. On lave le mélange réactionnel par une solution d'HCl 1N, par une solution de NaHCO₃ saturée, à l'eau, sèche la phase organique sur Na₂SO₄ et évapore le solvant sous vide. On reprend le résidu dans l'acétone et essore le précipité formé. On obtient 3,35 g du composé attendu.

RMN ¹H : DMSO-d₆ (250 MHz) : δ (ppm) : 0,94 (3H, t) ; 1,62 (2H, m) ; 2,50 (3H, s) ; 2,82 (2H, t) ; 3,73 (3H, s) ; 5,34 (2H, s) ; 7,29 (1H, d) ; 7,50 (2H, d) ; 7,60 (1H, d) ; 7,94 (2H, d) ; 8,21 (1H, s) ; 8,24 (1H, s) ; 8,35 (1H, s) ; 10,20 (1H, s).

En procédant selon le mode opératoire décrit à la Préparation 9.10, on prépare les composés de formule (II) rassemblés dans le TABLEAU VIII ci-après :

**TABLEAU VIII**

| | | | | |
|---|---|---|---|---|
| 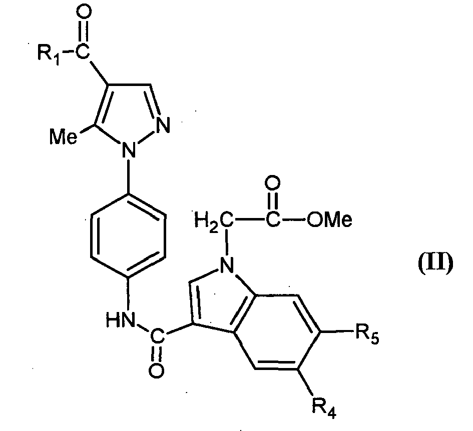 | | | | |

| | R₁ | R₄ | R₅ | RMN ¹H : DMSO-d₆ (250 MHz) : δ (ppm) |
|---|---|---|---|---|
| Préparation 9.11 | OEt | Cl | H | / |
| Préparation 9.12 | Me | Cl | H | 2,45 (3H, s) ; 2,50 (3H, s) ; 3,73 (3H, s) ; 5,34 (2H, s) ; 7,29 (1H, d) ; 7,49 (2H, d) ; 7,60 (1H, d) ; 7,95 (2H, d) ; 8,21 (1H, s) ; 8,23 (1H, s) ; 8,35 (1H, s) ; 10,15 (1H, s) |
| Préparation 9.13 | Et | Cl | H | 1,06 (3H, t) ; 2,50 (3H, s) ; 2,85 (2H, q) ; 3,73 (3H, s) ; 5,26 (2H, s) ; 7,29 (1H, d) ; 7,49 (2H, d) ; 7,60 (1H, d) ; 7,95 (2H, d) ; 8,21 (1H, s) ; 8,23 (1H, s) ; 8,35 (1H, s) ; 10,15 (1H, s) |
| Préparation 9.14 | nPr | Me | H | 0,94 (3H, t) ; 1,64 (2H, m) ; 2,43 (3H, s) ; 2,50 (3H, s) ; 2,82 (2H, t) ; 3,72 (3H, s) ; 5,27 (2H, s) ; 7,08 (1H, d) ; 7,40 (1H, d) ; 7,48 (2H, d) ; 7,95 (2H, d) ; 8,02 (1H, s) ; 8,22 (1H, s) ; 8,24 (1H, s) ; 10,05 (1H, s) |
| Préparation 9.15 | nPr | OMe | H | 0,94 (3H, t) ; 1,64 (2H, m) ; 2,50 (3H, s) ; 2,81 (2H, t) ; 3,72 (3H, s) ; 3,81 (3H, s) ; 5,27 (2H, s) ; 6,89 (1H, d) ; 7,42 (1H, d) ; 7,48 (2H, d) ; 7,73 (1H, s) ; 7,95 (2H, d) ; 8,23 (2H, s) ; 10,05 (1H, s) |
| Préparation 9.16 | nPr | CF₃ | H | 0,94 (3H, t) ; 1,64 (2H, m) ; 2,50 (3H, s) ; 2,82 (2H, t) ; 3,74 (3H, s) ; 5,42 (2H, s) ; 7,50 (2H, d) ; 7,59 (1H, d) ; 7,80 (1H, d) ; 7,96 (2H, d) ; 8,24 (1H, s) ; 8,46 (1H, s) ; 8,58 (1H, s) ; 10,30 (1H, s) |
| Préparation 9.17 | nPr | Br | H | 0,94 (3H, t) ; 1,64 (2H, m) ; 2,50 (3H, s) ; 2,82 (2H, t) ; 3,72 (3H, s) ; 5,35 (2H, s) ; 7,40 (1H, d) ; 7,50 (2H, d) ; 7,54 (1H, d) ; 7,94 (2H, d) ; 8,26 (1H, s) ; 8,33 (1H, s) ; 8,37 (1H, s) ; 10,20 (1H, s) |
| Préparation 9.18 | nPr | Me | Me | 0,95 (3H, t) ; 1,63 (2H, m) ; 2,34 (6H, s) ; 2,51 (3H, s) ; 2,83 (2H, t) ; 3,72 (3H, s) ; 5,24 (2H, s) ; 7,30 (1H, s) ; 7,49 (2H, d) ; 7,93-7,99 (3H, m) ; 8,15 (1H,s) ; 8,25 (1H, s) ; 10,05 (1H, s) |
| Préparation 9.19 | nPr | Cl | Cl | 0,95 (3H, t) ; 1,65 (2H, m) ; 2,34 (6H, s) ; 2,54 (3H, s) ; 2,83 (2H, t) ; 3,75 (3H, s) ; 5,37 (2H, s) ; 7,51 (2H, d) ; 7,95 (2H, d) ; 8,04 (1H, s) ; 8,26 (1H, s) ; 8,38 (1H, s) 8,39 (1H, s) ; 10,25 (1H, s) |

### Préparation 9.20

### (3-[[2-Bromo-4-(4-butyryl-5-méthyl-1H-pyrazol-1-yl)-phényl]carbamoyl]-5-chloro-1H-indol-1-yl)-acétate de méthyle.

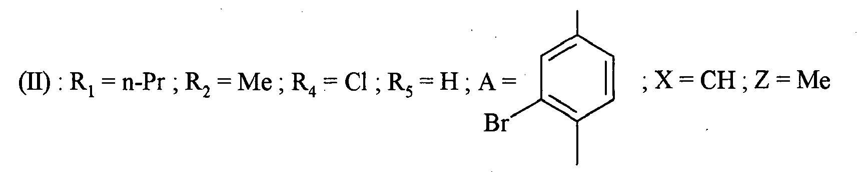

A 1,33 g du composé de l'étape 1 de la Préparation 9.1 dans 25 ml de 1,2-dichloroéthane, on ajoute 0,63 ml de pyridine puis 1 g du composé de la Préparation 6.7. Le milieu réactionnel est placé dans un appareil à microondes à 80°C pendant 30 min (300 W). Après retour à TA, on ajoute 50 ml d'eau et extrait au DCM. Les phases organiques rassemblées sont lavées au bicarbonate de sodium saturé, à l'eau et à la saumure, séchées sur sulfate de sodium et évaporées à sec. Le produit est trituré dans l'acétone puis filtré pour obtenir 0.89 g d'une poudre blanche.

RMN ¹H : DMSO-d₆ (250 MHz) : δ (ppm) : 0,94 (3H, t) ; 1,64 (2H, m) ; 2,58 (3H, s) ; 2,83 (2H, t) ; 3,73 (3H, s) ; 5,34 (2H, s) ; 7,29 (1H, d) ; 7,57-7,63 (2H, m) ; 7,86 (1H, d) ; 7,93 (1H, d) ; 8,18 (1H, s) ; 8,30 (1H, s) ; 8,34 (1H, s) ; 9,69 (1H, s).

En procédant selon le mode opératoire décrit à la Préparation 9.20, on prépare les composés de formule (II) rassemblés dans le TABLEAU IX ci-après :

**TABLEAU IX**

| | | |
|---|---|---|
| 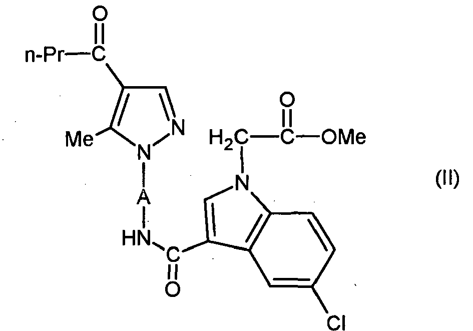 | | |

| | A | RMN ¹H : DMSO-d₆ (250 MHz) : δ (ppm) |
|---|---|---|
| Préparation 9.21 | 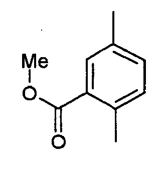 | 0,94 (3H, t) ; 1,64 (2H, m) ; 2,55 (3H, s) ; 2,83 (2H, t) ; 3,73 (3H, s) ; 3,93 (3H, s) ; 5,39 (2H, s) ; 7,33 (1H, d) ; 7,61 (1H, d) ; 7,86 (1H, d) ; 8,08 (1H, s) ; 8,20-8,25 (2H, m) ; 8,38 (1H, s) ; 8,74 (1H, d) ; 11,30 (1H, s). |
| Préparation 9.22 | 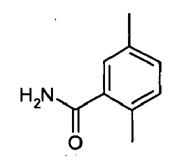 | 0,94 (3H, t) ; 1,64 (2H, m) ; 2,55 (3H, s) ; 2,83 (2H, t) ; 3,72 (3H, s) ; 5,37 (2H, s) ; 7,31 (1H, d) ; 7,62 (1H, d) ; 7,73 (1H, d) ; 7,94 (1H, br) ; 8,04 (1H, s) ; 8,14 (1H, s) ; 8,26 (1H, s) ; 8,29 (1H, s) ; 8,47 (1H, br) ; 8,86 (1H, d) ; 12,70 (1H, s). |

### Préparation 9.23

### 3-{[4-(4-Butyryl-5-méthyl-1H-pyrazol-1-yl)phényl]carbamoyl}-5-phényl-1H-indol-1-yl)-acétate de méthyle.

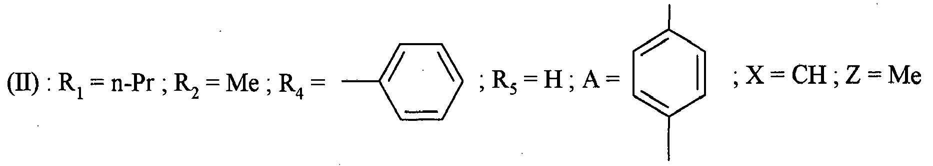

A 0,60 du composé de la Préparation 9.17 dans 10 ml de dioxane, on ajoute 0,18 g d'acide phénylboronique, 0,36 g de K₃PO₄.2H₂O. On dégaze le milieu réactionnel à l'argon, puis ajoute 0,06 g de tétrakis(triphénylphosphine)palladium(0) et chauffe au reflux pendant 5 heures. On ajoute 50 ml d'AcOEt et 50 ml d'eau, récupère la phase organique, puis extrait la phase aqueuse deux fois supplémentaires. On rassemble les phases organiques, lave à l'eau puis à la saumure et sèche sur Na₂SO₄. On évapore puis purifie le résidu solide par chromatographie sur gel de silice en éluant avec un mélange de DCM/MeOH jusqu'à (98/2 ; v/v). On obtient 0,39 g d'une poudre blanche.

RMN ¹H : DMSO-d₆ (250 MHz) : δ (ppm) : 0,96 (3H, t) ; 1,65 (2H, m) ; 2,54 (3H, s) ; 2,83 (2H, t) ; 3,75 (3H, s) ; 5,37 (2H, s) ; 7,33-7,75 (9H, m) ; 7,99 (2H, d) ; 8,25 (1H, s) ; 8,33 (1H, s) ; 8,48 (1H, s) ; 10,20 (1H, s).

### Préparation 9.24

### [3-{[4-(4-Butyryl-5-méthyl-1H-pyrazol-1-yl)-phényl]carbamoyl}-5-(diméthylamino)-1H-indol-1-yl]acétate de méthyle.

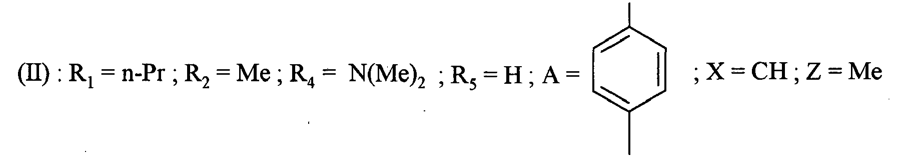

A 1 g du composé de la Préparation 9.17 dans 7 ml de DMSO dans un tube à vis, on ajoute 0,26 g de proline et 0,21 g d'iodure de cuivre (I). On dégaze le milieu réactionnel à l'azote, puis ajoute 7 ml d'une solution de diméthylamine 2M dans le THF. On ferme le tube et chauffe à 120°C pendant 7 heures. On verse le milieu réactionnel sur de l'eau et on extrait à l'AcOEt, sèche la phase organique sur Na₂SO₄ et évapore. On purifie le résidu solide par chromatographie sur gel de silice en éluant avec un mélange de DCM/MeOH jusqu'à (95/5 ; v/v). On obtient 0,11 g de poudre.

RMN ¹H : DMSO-d₆ (250 MHz) : δ (ppm) : 0,94 (3H, t) ; 1,64 (2H, m) ; 2,52 (3H, s) ; 2,83 (2H, t) ; 2,91 (6H, s) ; 3,71 (3H, s) ; 5,22 (2H, s) ; 6,88 (1H, d) ; 7,34 (1H, d) ; 7,47 (2H, d) ; 7,56 (1H, s) ; 7,95 (2H, d) ; 8,16 (1H, s) ; 8,24 (1H, s) ; 9,95 (1H, s).

### Préparation 9.25

### (3-{[4-(4-Butyryl-5-méthyl-1H-pyrazol-1-yl)-phényl]carbamoyl}-5-cyano-1H-indol-1-yl)-acétate de méthyle.

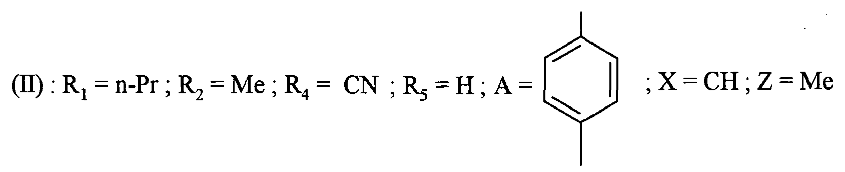

Dans un tube à vis, on dissout 3 g du composé de la Préparation 9.17 dans 40 ml de DMF. On dégaze le milieu réactionnel à l'argon, puis ajoute 0,79 g de cyanure de zinc (II) et 0,32 g de tétrakis(triphénylphosphine)palladium(0). On ferme le tube puis chauffe à 100°C pendant 7 heures. On verse le milieu réactionnel sur une solution diluée de bicarbonate de sodium en s'assurant qu'à la fin de l'ajout le pH de la solution est basique. On extrait à l'AcOEt, sèche la phase organique sur Na₂SO₄ et évapore. On triture le résidu solide dans le MeOH puis filtre pour obtenir 2,2 g de poudre blanche.

RMN ¹H : DMSO-d₆ (250 MHz) : δ (ppm) : 0,94 (3H, t) ; 1,64 (2H, m) ; 2,52 (3H, s) ; 2,82 (2H, t) ; 3,73 (3H, s) ; 5,42 (2H, s) ; 7,52 (2H, d) ; 7,66 (1H, d) ; 7,80 (1H, d) ; 7,95 (2H, d) ; 8,25 (1H, s) ; 8,46 (1H, s) ; 8,61 (1H, s) ; 10,25 (1H, s).

### Préparation 9.26

### (3-{[4-(4-Butyryl-5-méthyl-1H-pyrazol-1-yl)-2-cyano-phényl]carbamoyl}-5-chloro-1H-indol-1-yl)-acétate de méthyle.

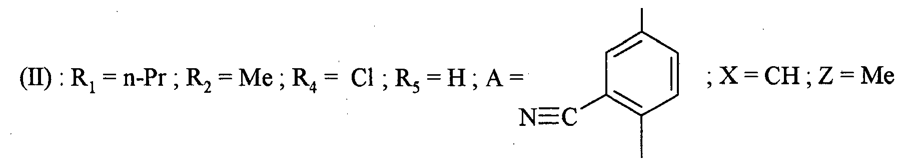

Dans un tube à vis, on dissout 0,78 g du composé de la Préparation 9.21 dans 15 ml de DMF. On dégaze le milieu réactionnel à l'argon, puis ajoute 0,21 g de cyanure de zinc (II) et 0,08 g de tétrakis(triphénylphosphine)palladium(0). On ferme le tube puis chauffe à 100°C pendant 6 heures. On verse le milieu réactionnel sur une solution dilué de bicarbonate de sodium en s'assurant qu'à la fin de l'ajout le pH de la solution est basique. On extrait à l'AcOEt, lave à l'eau, à la saumure, sèche sur Na₂SO₄ et évapore. On triture le résidu solide dans l'éther iso puis filtre. On purifie le résidu solide par chromatographie sur gel de silice en éluant avec un mélange de DCM /MeOH jusqu'à (95/5 ; v/v). On obtient 0,59 g de poudre blanche.

RMN ¹H : DMSO-d₆ (250 MHz) : δ (ppm) : 0,94 (3H, t) ; 1,64 (2H, m) ; 2,59 (3H, s) ; 2,83 (2H, t) ; 3,73 (3H, s) ; 5,37 (2H, s) ; 7,32 (1H, d) ; 7,64 (1H, d) ; 7,80 (1H, d) ; 7,91 (1H, d) ; 8,11 (1H, s) ; 8,17 (1H, s) ; 8,33 (2H, d) ; 10,40 (1H, s).

### EXEMPLES

### EXEMPLE 1 : Composé n° 1

(3-{[5-(4-butyryl-5-méthyl-1*H*-pyrazol-1-yl)pyrazin-2-yl]carbamoyl}-5-chloro-1*H-*indol-1-yl)acétate de sodium.

A 0,14 g du composé de la Préparation 9.1 dans 6 ml d'un mélange MeOH/dioxane (50/50 ; v/v), on ajoute 0,80 ml d'une solution aqueuse de NaOH 1N et agite pendant 3h. Le milieu réactionnel est évaporé à sec. On triture le résidu solide à l'eau puis essore pour obtenir 0,10 g d'une poudre blanche.

### EXEMPLE 2 : Composé n° 2

Acide (3-{[6-(4-butyryl-5-méthyl-1*H*-pyrazol-1-yl)pyridazin-3-yl]carbamoyl}-5-chloro-1*H*-indol-1-yl)acétique.

A 2,20 g du composé de la Préparation 9.2 dans 42 ml de dioxane, on ajoute 5,30 ml d'une solution aqueuse de NaOH 1N et agite pendant 2h. Le milieu réactionnel est évaporé à sec. On redissout le résidu solide dans 30 ml d'eau puis acidifie avec 7 ml d'une solution d' HCl 1N. On essore le précipité formé, le lave à l'eau pour obtenir 2,07 g d'une poudre beige.

### EXEMPLE 3 Composé n° 3 Acide (3-{[4-(4-butyryl-5-méthyl-1H-pyrazol-1-yl)phényl]carbamoyl}-5-chloro-1H-indol-1-yl)acétique.

A 16,2 g du composé de la Préparation 9.11 dans 60 ml d'un mélange MeOH/dioxane (50/50 ; v/v), on ajoute 32,9 ml d'une solution aqueuse de NaOH 2N et agite pendant 2 heures. Le milieu réactionnel est acidifié avec une solution de HCl 1N puis on extrait avec de l'AcOEt. Les phases organiques rassemblées sont lavées à la saumure, séchées sur Na₂SO₄ puis évaporées. On triture le résidu solide avec un mélange acétone/éther iso (50/50 ; v/v) puis filtre pour obtenir 11 g d'une poudre blanche. Le précipité formé pendant la concentration du filtrat permet d'obtenir 2,95 g supplémentaires soit 13,95 g au total.

### EXEMPLE 4 : Composé n° 4

### (3-{[4-(4-butyryl-5-méthyl-1H-pyrazol-1-yl)phényl]carbamoyl}-5-chloro-6-méthyl-1H-indol-1-yl)acétate de sodium.

A 0,11 g de la Préparation 9.8 dans 5 ml d'un mélange MeOH/dioxane (50/50 ; v/v), on ajoute 0,22 ml d'une solution aqueuse de NaOH 1N puis évapore à sec. On redissout le résidu solide dans un minimum d'acétone (environ 1 ml) et ajoute goutte à goutte sur 10 ml de pentane. Le précipité formé est filtré, séché à l'étuve à vide pour obtenir 0,082 g d'une poudre blanche.

En procédant selon les modes opératoires décrits dans les exemples 1 à 4, on prépare les composés de Formule (I) rassemblés dans le TABLEAU X ci-après :
Dans ce tableau :
- Me représente un radical méthyle ;
- Et représente un radical éthyle ;
- nPr représente un radical n-propyle ;
- Ph représente un radical phényle ;
- Na représente un composé sous forme de sel de sodium.

**TABLEAU X**

| | | | | | | | | |
|---|---|---|---|---|---|---|---|---|
| 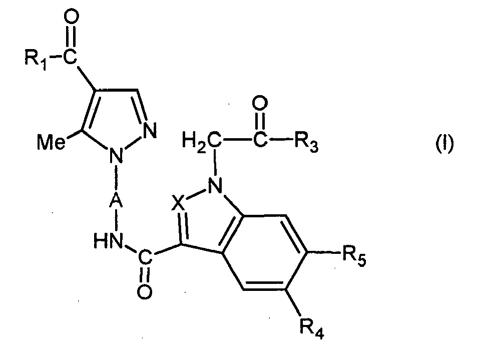 | | | | | | | | |

| Composés N° | R₁ | A | X | R₃ | R₄ | R₅ | RMN ¹H : DMSO-d₆ (250 MHz) : δ (ppm) : | Inhibition de l'agrégation plaquettaire *in vitro* (sang rat) en µM |
|---|---|---|---|---|---|---|---|---|
| 1 | nPr | 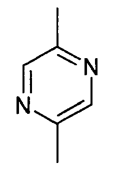 | CH | OH | Cl | H | 0,93 (3H, t) ; 1,63 (2H, m) ; 2,77 (3H, s) ; 2,85 (2H, t) ; 4,48 (2H, s) ; 7,20 (1H, d) ; 7,43 (1H, d) ; 8,22 (1H, s) ; 8,35 (1H, s) ; 8,63 (1H, s) ; 8,83 (1H, s) ; 9,43 (1H, s) ; 10,95 (1H, s). | / |
| 2 | nPr | 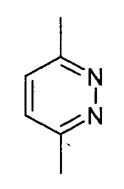 | CH | OH | Cl | H | 0,93 (3H, t) ; 1,65 (2H, m) ; 2,82 (3H, s) ; 2,86 (2H, t) ; 5,17 (2H, s) ; 7,30 (1H, d) ; 7,61 (1H, d) ; 8,13 (1H, d) ; 8,23 (1H, s) ; 8,40 (1H, s) ; 8,66 (1H, s) ; 8,68 (1H, d) ; 11,35 (1H, s) ; 13,25 (1H, br). | / |
| 3 | nPr | 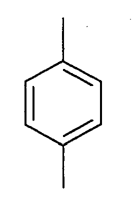 | CH | OH | Cl | H | 0,94 (3H, t) ; 1,63 (2H, m) ; 2,52 (3H, s) ; 2,81 (2H, t) ; 5,20 (2H, s) ; 7,28 (1H, d) ; 7,50 (2H, d) ; 7,59 (1H, d) ; 7,94 (2H, d) ; 8,21 (1H, s) ; 8,23 (1H, s) ; 8,36 (1H, s) ; 10,15 (1H, s) ; 13,30 (1H, br). | 2.8 |
| 4 | nPr | 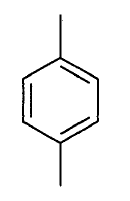 | CH | ONa | Cl | Me | 0,94 (3H, t) ; 1,64 (2H, m) ; 2,42 (3H, s) ; 2,52 (3H, s) ; 2,81 (2H, t) ; 4,48 (2H, s) ; 7,39 (1H, s) ; 7,46 (2H, d) ; 7,96 (2H, d) ; 8,18 (1H, s) ; 8,23 (1H, s) ; 8,31 (1H, s) ; 10,05 (1H, s). | 3.3 |
| 5 | OEt | 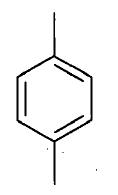 | CH | OH | Cl | H | 1,30 (3H, t) ; 2,52 (3H, s) ; 4,26 (2H, q) ; 5,21 (2H, s) ; 7,28 (1H, d) ; 7,51 (2H, d) ; 7,58 (1H, d) ; 7,95 (2H, d) ; 7,98 (1H, s) ; 8,21 (1H, s) ; 8,35 (1H, s) ; 10,15 (1H, s) ; 13,25 (1H, br). | / |
| 6 | Me | 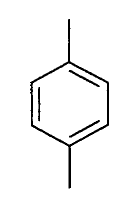 | CH | OH | Cl | H | 2,45 (3H, s) ; 2,52 (3H, s) ; 5,20 (2H, s) ; 7,27 (1H, d) ; 7,49 (2H, d) ; 7,59 (1H, d) ; 7,95 (2H, d) ; 8,21 (1H, s) ; 8,23 (1H, s) ; 8,36 (1H, s) ; 10,15 (1H, s) ; 13,25 (1H, br). | / |
| 7 | Et | 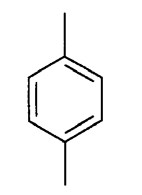 | CH | OH | Cl | H | 1,08 (3H, t) ; 2,51 (3H, s) ; 2,84 (2H, q) ; 5,19 (2H, s) ; 7,27 (1H, d) ; 7,49 (2H, d) ; 7,58 (1H, d) ; 7,95 (2H, d) ; 8,21 (1H, s) ; 8,23 (1H, s) ; 8,36 (1H, s) ; 10,15 (1H, s) ; 13,25 (1H, br). | / |
| 8 | nPr | 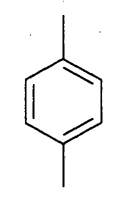 | CH | OH | H | Me | 0,94 (3H, t) ; 1,64 (2H, m) ; 2,43 (3H, s) ; 2,52 (3H, s) ; 2,81 (2H, t) ; 5,10 (2H, s) ; 7,04 (1H, d) ; 7,30 (1H, s) ; 7,47 (2H, d) ; 7,95 (2H, d) ; 8,08 (1H, d) ; 8,20 (1H, s) ; 8,23 (1H, s) ; 10,01 (1H, s). | 63 |
| 9 | nPr | 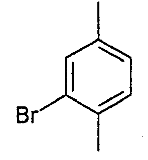 | CH | OH | Cl | H | MH⁺ = 557 ; tr = 9,17 min (Méthode G) | / |
| 10 | nPr | 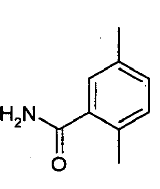 | CH | OH | Cl | H | 0,94 (3H, t) ; 1,64 (2H, m) ; 2,55 (3H, s) ; 2,83 (2H, t) ; 5,18 (2H, s) ; 7,29 (1H, d) ; 7,59 (1H, d) ; 7,72 (1H, d) ; 7,95 (1H, s) ; 8,04 (1H, s) ; 8,11 (1H, s) ; 8,25 (1H, s) ; 8,29 (1H, s) ; 8,47 (1H, s) ; 8,86 (1H, d) ; 12,60 (1H, s) ; 13,20 (1H, br). | / |
| 11 | nPr | 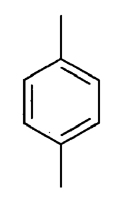 | CH | OH | Me | H | 0,94 (3H, t) ; 1,64 (2H, m) ; 2,43 (3H, s) ; 2,52 (3H, s) ; 2,81 (2H, t) ; 5,12 (2H, s) ; 7,07 (1H, d) ; 7,39 (1H, d) ; 7,48 (2H, d) ; 7,95 (2H, d) ; 8,02 (1H, s) ; 8,23 (1H, s) ; 8,24 (1H, s) ; 10,02 (1H, s) ; 13,19 (1H, br). | 9.6 |
| 12 | nPr | 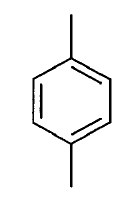 | CH | ONa | OMe | H | 0,94 (3H, t) ; 1,63 (2H, m) ; 2,52 (3H, s) ; 2,81 (2H, t) ; 3,79 (3H, s) ; 4,41 (2H, s) ; 6,80 (1H, d) ; 7,27 (1H, d) ; 7,45 (2H, d) ; 7,73 (1H, d) ; 7,98 (2H, d) ; 8,24 (1H, s) ; 8,29 (1H, s) ; 9,97 (1H, s). | 17 |
| 13 | nPr | 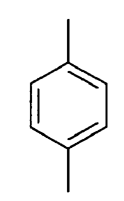 | CH | ONa | CF₃ | H | 0,95 (3H, t) ; 1,64 (2H, m) ; 2,52 (3H, s) ; 2,81 (2H, t) ; 4,57 (2H, s) ; 7,45-7,49 (3H, m) ; 7,62 (1H, d) ; 7,98 (2H, d) ; 8,24 (1H, s) ; 8,56 (1H, s) ; 8,57 (1H, s) ; 10,30 (1H, s). | 4.6 |
| 14 | nPr | 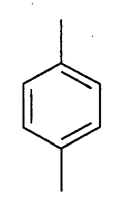 | CH | ONa | Br | H | 0,94 (3H, t) ; 1,63 (2H, m) ; 2,52 (3H, s) ; 2,82 (2H, t) ; 4,51 (2H, s) ; 7,28 (1H, d) ; 7,39 (1H, d) ; 7,46 (2H, d) ; 7,97 (2H, d) ; 8,25 (1H, s) ; 8,35 (1H, s) ; 8,42 (1H, s) ; 10,23 (1H, s). | 1.9 |
| 15 | nPr | 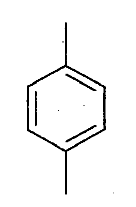 | CH | OH | Ph | H | 0,94 (3H, t) ; 1,65 (2H, m) ; 2,52 (3H, s) ; 2,82 (2H, t) ; 5,22 (2H, s) ; 7,35 (1H, t) ; 7,47-7,52 (4H, m) ; 7,56 (1H, d) ; 7,63 (1H, d) ; 7,70 (2H, d) ; 7,97 (2H, d) ; 8,25 (1H, s) ; 8,33 (1H, s) ; 8,48 (1H, s) ; 10,15 (1H, s) ; 13,30 (1H, br). | 25 |
| 16 | nPr | 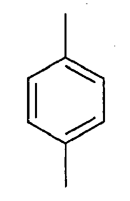 | CH | OH | Me | Me | 0,94 (3H, t) ; 1,63 (2H, m) ; 2,36 (6H, s) ; 2,52 (3H, s) ; 2,82 (2H, t) ; 5,07 (2H, s) ; 7,28 (1H, s) ; 7,48 (2H, d) ; 7,94-7,98 (3H, m) ; 8,15 (1H, s) ; 8,24 (1H, s) ; 9,97 (1H, s) ; 13,20 (1H, br). | 4.6 |
| 17 | nPr | 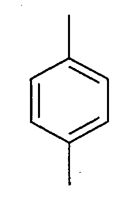 | CH | ONa | Cl | Cl | 0,96 (3H, t) ; 1,65 (2H, m) ; 2,54 (3H, s) ; 2,83 (2H, t) ; 4,51 (2H, s) ; 7,49 (2H, d) ; 7,74 (1H, s) ; 7,97 (2H, d) ; 8,25 (1H, s) ; 8,36 (1H, s) ; 8,44 (1H, s) ; 10,19 (1H, s). | 7.3 |
| 18 | nPr | 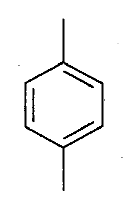 | CH | ONa | F | H | 0,94 (3H, t) ; 1,63 (2H, m) ; 2,52 (3H, s) ; 2,81 (2H, t) ; 4,49 (2H, s) ; 7,01 (1H, t) ; 7,37-7,49 (3H, m) ; 7,87 (1H, d) ; 7,98 (2H, d) ; 8,24 (1H, s) ; 8,43 (1H, s) ; 10,15 (1H, s). | 36 |
| 19 | nPr | 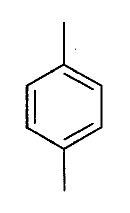 | CH | ONa | Me | Br | 0,94 (3H, t) ; 1,63 (2H, m) ; 2,48 (3H, s) ; 2,52 (3H, s) ; 2,81 (2H, t) ; 4,46 (2H, s) ; 7,45 (2H, d) ; 7,63 (1H, s) ; 7,96 (2H, d) ; 8,15 (1H, s) ; 8,23 (1H, s) ; 8,30 (1H, s) ; 10,10 (1H, s). | 42 |
| 20 | nPr | 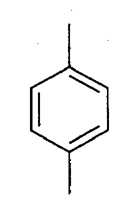 | CH | ONa | Br | Me | 0,94 (3H, t) ; 1,64 (2H, m) ; 2,43 (3H, s) ; 2,52 (3H, s) ; 2,81 (2H, t) ; 4,45 (2H, s) ; 7,40 (1H, s) ; 7,45 (2H, d) ; 7,96 (2H, d) ; 8,23 (1H, s) ; 8,32 (1H, s) ; 8,37 (1H, s) ; 10,09 (1H, s). | 3.9 |
| 21 | nPr | 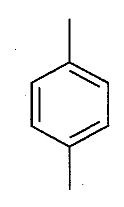 | CH | OH | Cl | F | 0,94 (3H, t) ; 1,63 (2H, m) ; 2,52 (3H, s) ; 2,82 (2H, t) ; 5,19 (2H, s) ; 7,50 (2H, d) ; 7,77 (1H, d) ; 7,95 (2H, d) ; 8,24 (1H, s) ; 8,30 (1H, d) ; 8,38 (1H, s) ; 10,20 (1H, s) ; 13,33 (1H, br). | 2.8 |
| 22 | nPr | 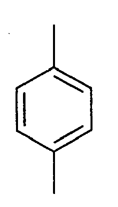 | CH | OH | NMe² | H | 0,94 (3H, t) ; 1,63 (2H, m) ; 2,52 (3H, s) ; 2,82 (2H, t) ; 3,23 (6H, s) ; 5,26 (2H, s) ; 7,50 (2H, d) ; 7,70 (1H, d) ; 7,79 (1H, d) ; 7,99 (2H, d) ; 8,25 (1H, s) ; 8,52 (1H, s) ; 8,57 (1H, s) ; 10,31 (1H, s) ; 12,72 (1H, br). | 1.2 |
| 23 | nPr | 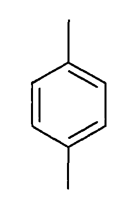 | CH | ONa | CN | H | 0,94 (3H, t) ; 1,64 (2H, m) ; 2,53 (3H, s) ; 2,81 (2H, t) ; 4,61 (2H, s) ; 7,44 (2H, d) ; 7,49 (1H, d) ; 7,61 (1H, d) ; 7,96 (2H, d) ; 8,24 (1H, s) ; 8,58 (1H, s) ; 8,60 (1H, s) ; 10,49 (1H, s). | 4.1 |
| 24 | nPr | 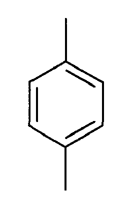 | N | ONa | Me | H | 0,95 (3H, t) ; 1,64 (2H, m) ; 2,46 (3H, s) ; 2,53 (3H, s) ; 2,82 (2H, t) ; 4,80 (2H, s) ; 7,25 (1H, d) ; 7,44-7,51 (3H, m) ; 8,00 (1H, s) ; 8,11 (2H, d) ; 8,24 (1H, s) ; 10,45 (1H, s). | 37 |

### EXEMPLE 5 : Composé n° 25

### Acide (3-{[4-(4-butyryl-5-méthyl-1H-pyrazol-1-yl)-2-(méthoxycarbonyl)phényl]carbamoyl}-5-chloro-1H-indol-1-yl)acétique.

A 0,71 g du composé de la Préparation 9.22 dans 20 ml de THF, on ajoute 0,03 g d'hydroxyde de lithium et agite pendant 2 heures. On évapore à sec, triture le résidu solide au DCM puis essore. On reprend le précipité à l'eau, acidifie avec 0,175 mg de KHSO₄, essore le précipité formé, lave à l'eau et sèche sous vide pour obtenir 0,47 g d'une poudre beige.

En procédant selon le mode opératoire décrit à l'exemple 5, on prépare les composés de formule (I) représentés dans le TABLEAU XI ci-après :

**TABLEAU XI**

| | | | | | |
|---|---|---|---|---|---|
| 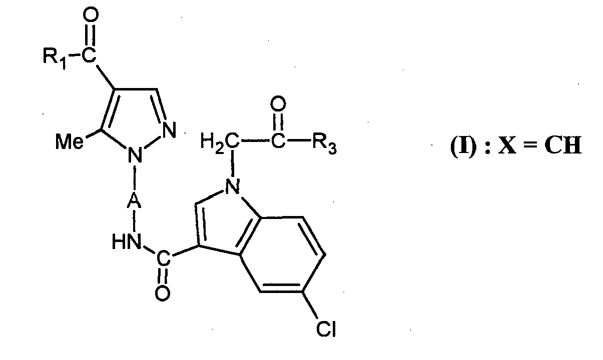 | | | | | |

| Composé N° | R₁ | A | R₃ | RMN ¹H : DMSO-d₆ (250 MHz) : δ (ppm) _{:} | Inhibition de l'agrégation plaquettaire *in vitro* (sans rat) en µM |
|---|---|---|---|---|---|
| 25 | nPr | 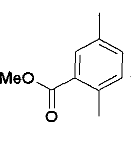 | OH | 0,94 (3H, t) ; 1,64 (2H, m) ; 2,55 (3H, s) ; 2,83 (2H, t) ; 3,93 (3H, s) ; 5,26 (2H, s) ; 7,30 (1H, d) ; 7,61 (1H, d) ; 7,86 (1H, d) ; 8,08 (1H, s) ; 8,20-8,25 (2H, m) ; 8,29 (1H, s) ; 8,73 (1H, d) ; 11,30 (1H, s) ; 13,35 (1H, br). | / |
| 26 | nPr | 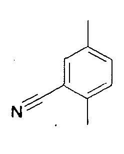 | OH | 0,95 (3H, t) ; 1,64 (2H, m) ; 2,59 (3H, s) ; 2,83 (2H, t) ; 5,17 (2H, s) ; 7,29 (1H, d) ; 7,60 (1H, d) ; 7,80 (1H, d) ; 7,91 (1H, d) ; 8,11 (1H, s) ; 8,17 (1H, s) 8,32 (1H, s) ; 8,36 (1H, s) ; 10,40 (1H, s). | / |

### EXEMPLE 6 : Composé n° 27

### Chlorhydrate de N-[5-(4-Butyryl-5-méthyl-1H-pyrazol-1-yl)pyrazin-2-yl]-5-chloro-1-[2-(4-méthylpipérazin-1-yl)-2-oxoéthyl]-1H-indole-3-carboxamide.

A 0,10 g du composé de l'Exemple 1 dans 5 ml de DCM, on ajoute successivement 0,055 g de 1-méthylpipérazine (commercial) et 0,086 g de BOP-Cl. Le milieu est hétérogène. On ajoute 2 ml de DMF et agite le milieu réactionnel homogène pendant une nuit. On évapore à sec, ajoute une solution de NaHCO₃ 1M et extrait au DCM. On lave les phases organiques rassemblées à l'eau, à la saumure, sèche sur Na₂SO₄ puis évapore sous vide. On purifie le résidu huileux ainsi obtenu par chromatographie sur gel de silice en éluant avec un mélange DCM/MeOH (gradient de 1 à 10 % de MeOH) pour obtenir 0,08 g d'une poudre blanche. On resolubilise dans 6 ml d'un mélange de DCM/acétone (50/50 ; v/v) puis ajoute 0,09 ml d'une solution d'éther chlorhydrique 2N. On évapore à sec, triture à l'acétone, essore puis sèche à l'étuve à vide à 60°C pour obtenir 0,06 g d'une poudre blanche.

### EXEMPLE 7 : Composé n° 28

### Chlorhydrate de N-[6-(4-Butyryl-5-méthyl-1H-pyrazol-1-yl)pyridazin-3-yl]-5-chloro-1-[2-(4-méthylpipérazin-1-yl)-2-oxoéthyl]-1H-indole-3-carboxamide.

A 2,65 g du composé de l'Exemple 2 dans 80 ml de DMF, on ajoute successivement 1,66 g de 1-méthylpipérazine (commercial), 2,18 g de pyridine et 4,30 g de BOP-Cl. On laisse sous agitation pendant 48 h puis verse le milieu réactionnel sur un mélange d'AcOEt et d'une solution saturée en NaHCO₃. Le précipité formé est filtré et lavé à l'éther iso. Le filtrat est transféré dans une ampoule à décanter et la phase organique est lavée à l'eau, à la saumure puis séchée sur Na₂SO₄. On concentre partiellement la phase organique et filtre le précipité formé. Les deux précipités sont rassemblés, séchés à l'étuve à vide pour obtenir 2,68 g d'une poudre blanche. On met en suspension dans 200 ml de MeOH puis on ajoute 6,6 ml d'une solution d'HCl 1N dans l'éther et agite pendant 1 h. On essore le précipité formé, le lave à l'éther iso puis sèche à l'étuve à vide à 40°C pour obtenir 2,29 g d'une poudre jaune pâle.

### EXEMPLE 8 : Composé n° 29

### Chlorhydrate de N-[6-(4-butyryl-5-méthyl-1H-pyrazol-1-yl)pyridazin-3-yl]-5-chloro-1-{2-[4-(2-méthoxyéthyl)pipérazin-1-yl]-2-oxoéthyl}-1H-indole-3-carboxamide.

A 0,11 g du composé de l'Exemple 2 dans 10 ml de DMF, on ajoute successivement 0,14 g de DMAP, 0,10 g de 1-(2-méthoxyéthyl)pipérazine, et 0,17 g de BOP-Cl. On agite pendant une nuit puis verse le milieu réactionnel sur un mélange d'AcOEt et d'une solution saturée en NaHCO₃. La phase organique est lavée à l'eau, à la saumure, séchée sur Na₂SO₄ puis évaporée à sec. On purifie le résidu solide ainsi obtenu par chromatographie sur gel de silice en éluant avec un mélange de DCM/MeOH (gradient de 2 à 5 % de MeOH). On resolubilise le produit purifié dans 5 ml d'un mélange de DCM/acétone (50/50 ; v/v) puis ajoute 0,12 ml d'une solution d'éther chlorhydrique 2N. On concentre partiellement. Un précipité se forme lentement. On essore, lave à l'acétone puis au pentane et sèche à l'étuve à vide à 60°C pour obtenir 0.11 g d'une poudre jaune pâle.

### EXEMPLE 9 : Composé n° 30

### Chlorhydrate de N-[6-(4-butyryl-5-méthyl-1H-pyrazol-1-yl)pyridazin-3-yl]-5-chloro-1-(2-oxo-2-{4-[2-(trifluorométhoxy)éthyl]pipérazin-1-yl}éthyl)-1H-indole-3-carboxamide

A 0,15 g du composé de l'Exemple 2 dans 20 ml de DMF, on ajoute successivement 0,11 g de DMAP, 0,08 g de 1-[2-(trifluorométhoxy)éthyl]pipérazine, 0,13 ml de pyridine et 0,24 g de BOP-Cl et agite à TA pendant une nuit. On ajoute de l'AcOEt, lave à l'eau, à la saumure, sèche sur Na₂SO₄ puis évapore à sec. On purifie le résidu solide ainsi obtenu par chromatographie colonne sur gel de silice en éluant avec un mélange de DCM et de méthanol (gradient de 1 à 10 % de méthanol) pour obtenir 0,08 g de poudre. On resolubilise le produit purifié dans 4 ml d'un mélange (1:1) de méthanol et d'acétone puis ajoute 0,15 ml d'une solution d'éther chlorhydrique 2N. On verse sur 30 ml d'éther iso, essore le précipité formé et sèche à l'étuve à vide à 60°C pour obtenir 0,06 g d'une poudre blanche.

### EXEMPLE 10 : Composé n° 31

### Chlorhydrate de N-[6-(4-butyryl-5-méthyl-1H-pyrazol-1-yl)pyridazin-3-yl]-5-chloro-1-{2-oxo-2-[4-(3,3,3-trifluoropropyl)pipérazin-1-yl]éthyl}-1H-indole-3-carboxamide

A 0,16 g du composé de l'Exemple 2 dans 5 ml de DCM, on ajoute successivement 0,13 g de 1-(3,3,3-trifluoropropyl)pipérazine, 0,04 g de DMAP et 0,13 g de BOP-Cl et agite à TA pendant 48 h. On évapore le milieu réactionnel à sec, triture le résidu solide avec une solution de NaHCO₃ 1M. On filtre le précipité et lave à l'eau pour obtenir 0,085 g d'une poudre blanche. On resolubilise dans 4 ml d'un mélange (1:1) de DCM et d'acétone puis ajoute 0,28 ml d'une solution d'HCl 1N dans l'éther. On essore le précipité formé, et sèche à l'étuve à vide à 60°C pour obtenir 0,08 g d'une poudre blanche.

En procédant selon le mode opératoire décrit dans les Exemples 6 à 10, on prépare les composés de formule (I) rassemblés dans le TABLEAU XII ci-après :
Dans ce tableau :
- dans la colonne "Sel", "-" représente un composé sous forme de base libre, alors que "HCl" représente un composé sous forme de chlorhydrate, "TFA" représente un composé sous forme de trifluoroacétate;
- Me représente un radical méthyle ;
- Et représente un radical éthyle ;
- nPr représente un radical n-propyle.

**TABLEAU XII**

| | | | | |
|---|---|---|---|---|
| 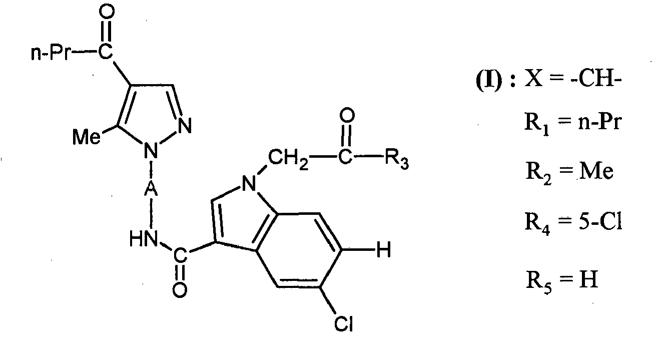 | | | | |

| Composés n° | R₃ | A | Sel F°C MH⁺; tr (Conditions) RMN | Inhibition de l'agrégation plaquettaire *in vitro* (sang rat) en µM |
|---|---|---|---|---|
| 27 | 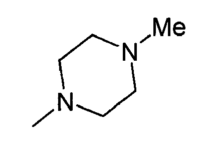 | 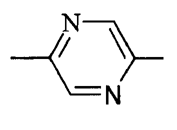 | HCl | 0.30 |
| | | | 282 | |
| | | | 563 ; 6,69 | |
| | | | (MG) | |
| | | | RMN | |
| 28 | 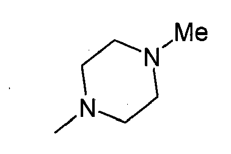 | 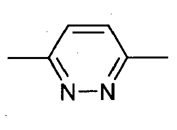 | HCl | 0.06 |
| | | | 287 | |
| | | | 563 ; 6,59 | |
| | | | (MG) | |
| | | | RMN | |
| 29 | 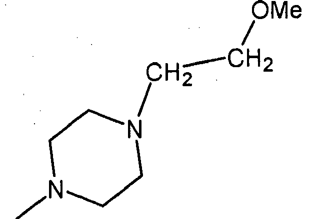 | 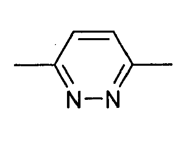 | HCl | 0.08 |
| | | | 258 | |
| | | | 607 ; 6,61 | |
| | | | (MG) | |
| | | | RMN | |
| 30 | 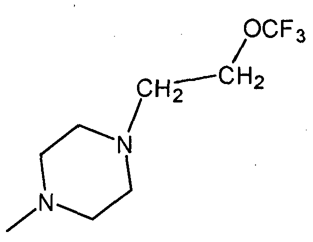 | 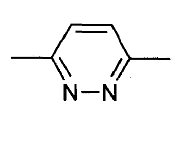 | 2 HCl | 0.10 |
| | | | 165 | |
| | | | 661 ; 9,75 | |
| | | | (MI) | |
| | | | RMN | |
| 31 | 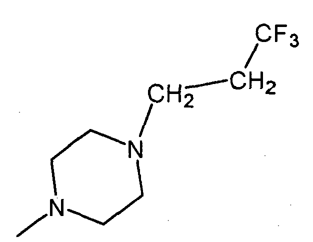 | 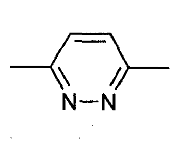 | HCl | / |
| | | | 230 | |
| | | | 645 ; 7,50 | |
| | | | (MG) | |
| | | | RMN | |
| 32 | 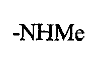 | 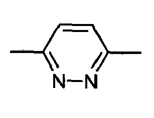 | - | 0,19 |
| | | | - | |
| | | | 494,17 ; | |
| | | | 1,14 | |
| | | | (MJ) | |
| | | | - | |
| 33 | 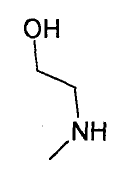 | 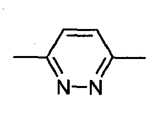 | - | 0,08 |
| | | | - | |
| | | | 524,18 ; | |
| | | | 1,1 | |
| | | | (MJ) | |
| | | | - | |
| 34 | 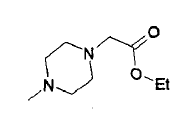 | 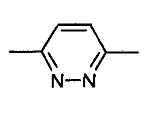 | TFA ; | 0,09 |
| | | | - | |
| | | | 635,25 | |
| | | | 1,11 | |
| | | | (MJ) | |
| | | | - | |
| 35 | 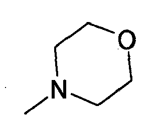 | 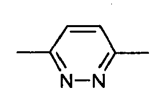 | 550,2 ; | 0,19 |
| | | | 1,15 | |
| | | | - | |
| | | | (MJ) | |
| | | | - | |
| 36 | 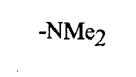 | 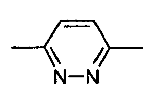 | - | 0,11 |
| | | | - | |
| | | | 508,19 ; | |
| | | | 1,16 | |
| | | | (MJ) | |
| | | | - | |
| 37 | 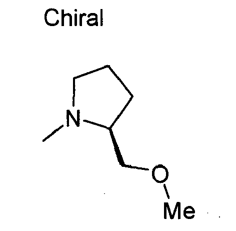 | 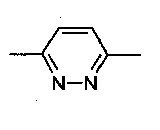 | - | 0,09 |
| | | | - | |
| | | | 578,23 ; | |
| | | | 1,2 | |
| | | | (MJ) | |
| | | | - | |
| 38 | 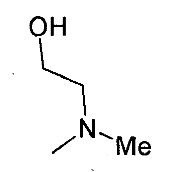 | 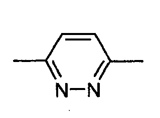 | - | 0,07 |
| | | | - | |
| | | | 538,2 ; | |
| | | | 1,12 | |
| | | | (MJ) | |
| | | | - | |
| 39 | 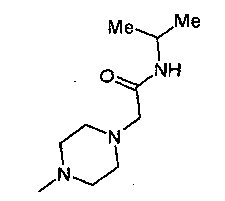 | 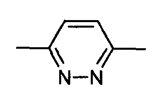 | TFA | 0,04 |
| | | | - | |
| | | | 648,28 ; | |
| | | | 1,05 | |
| | | | (MJ) | |
| | | | - | |
| 40 | 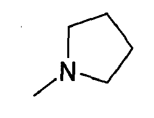 | 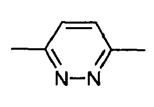 | - | 0,10 |
| | | | - | |
| | | | 534,2 ; | |
| | | | 1,18 | |
| | | | (MJ) | |
| | | | - | |
| 41 | 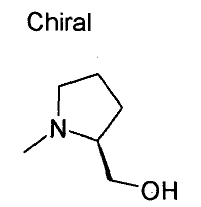 | 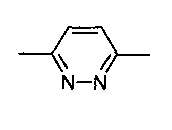 | - | 0,11 |
| | | | - | |
| | | | 564,21 ; | |
| | | | 1,14 | |
| | | | (MJ) | |
| | | | - | |
| 42 | 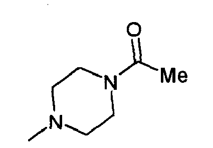 | 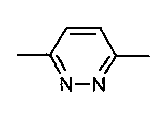 | - | 0,15 |
| | | | - | |
| | | | 591,22 ; | |
| | | | 1,12 | |
| | | | (MJ) | |
| | | | - | |
| 43 | 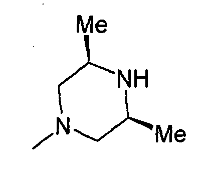 | 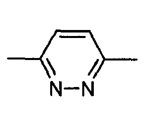 | TFA | 0,12 |
| | | | - | |
| | | | 577,24 ; 1 | |
| | | | (MJ) | |
| | | | - | |
| 44 | 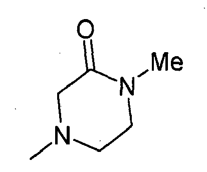 | 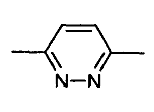 | - | 0,16 |
| | | | - | |
| | | | 577,21 ; | |
| | | | 1,12 | |
| | | | (MJ) | |
| | | | - | |
| 45 | 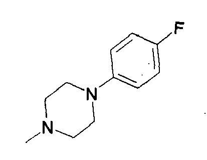 | 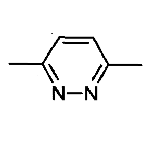 | - | 0,42 |
| | | | - | |
| | | | 643,23 ; | |
| | | | 1,24 | |
| | | | (MJ) | |
| | | | - | |
| 46 | 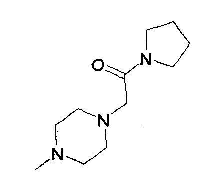 | 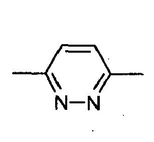 | TFA | 0,04 |
| | | | - | |
| | | | 660,28 ; | |
| | | | 1,01 | |
| | | | (MJ) | |
| | | | - | |
| 47 | 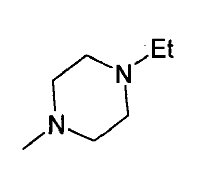 | 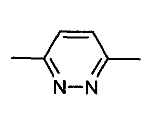 | TFA | 0.06 |
| | | | - | |
| | | | 577,24 ; | |
| | | | 0,99 | |
| | | | (MJ) | |
| | | | - | |
| 48 | 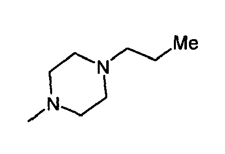 | 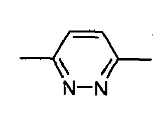 | TFA | 0,14 |
| | | | - | |
| | | | 591,26 ; 1 | |
| | | | (MJ) | |
| | | | - | |
| 49 | 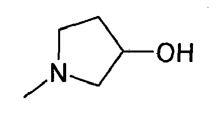 | 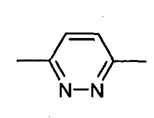 | - | 0,22 |
| | | | - | |
| | | | 550,2 ; | |
| | | | 1,11 | |
| | | | (MJ) | |
| | | | - | |
| 50 | 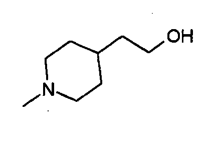 | 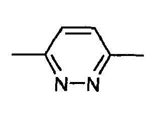 | - | 0,05 |
| | | | - | |
| | | | 592,24 ; | |
| | | | 1,15 | |
| | | | (MJ) | |
| | | | - | |
| 51 | 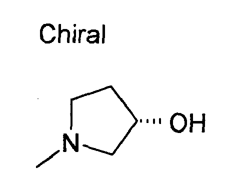 | 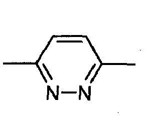 | - | 0,18 |
| | | | - | |
| | | | 550,2 ; | |
| | | | 1,11 | |
| | | | (MJ) | |
| | | | - | |
| 52 | 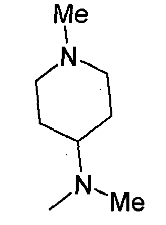 | 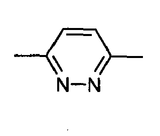 | TFA | 0,06 |
| | | | - | |
| | | | 591,26 ; | |
| | | | 0,99 | |
| | | | (MJ) | |
| | | | - | |
| 53 | 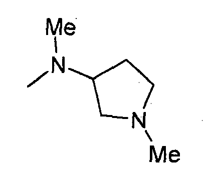 | 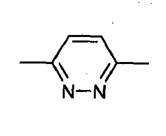 | TFA | 0,09 |
| | | | - | |
| | | | 577,24 ; | |
| | | | 0,99 | |
| | | | (MJ) | |
| | | | - | |
| 54 | 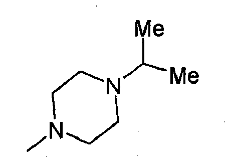 | 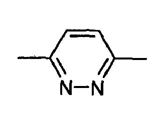 | TFA | 0,11 |
| | | | - | |
| | | | 591,26 ; 1 | |
| | | | (MJ) | |
| | | | - | |
| 55 | 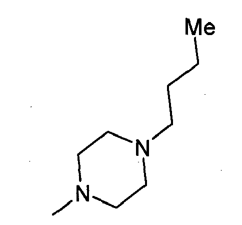 | 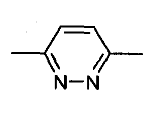 | TFA | 0,16 |
| | | | - | |
| | | | 605,28 ; | |
| | | | 1,01 | |
| | | | (MJ) | |
| | | | - | |
| 56 | 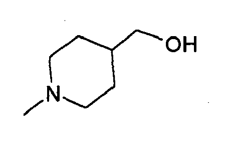 | 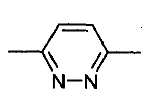 | - | 0,30 |
| | | | 578,23 ; | |
| | | | 1,14 | |
| | | | (MJ) | |
| | | | - | |
| 57 | 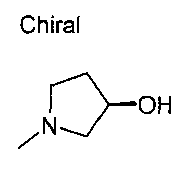 | 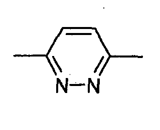 | - | 0,06 |
| | | | - | |
| | | | 550,2 ; | |
| | | | 1,11 | |
| | | | (MJ) | |
| | | | - | |
| 58 | 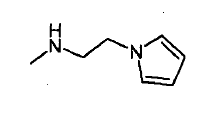 | 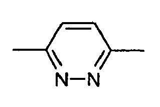 | - | 0,45 |
| | | | - | |
| | | | 573,21 ; | |
| | | | 1,19 | |
| | | | (MJ) | |
| | | | - | |
| 59 | 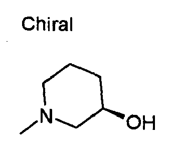 | 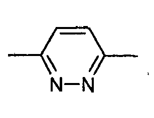 | - | 0,09 |
| | | | - | |
| | | | 564,21 ; | |
| | | | 1,13 | |
| | | | (MJ) | |
| | | | - | |
| 60 | 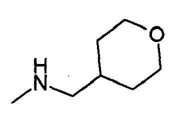 | 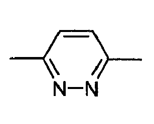 | - | 0,09 |
| | | | - | |
| | | | 578,23 ; | |
| | | | 1,16 | |
| | | | (MJ) | |
| | | | - | |
| 61 | 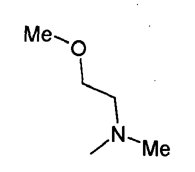 | 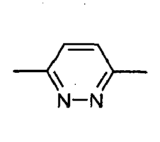 | - | 0,09 |
| | | | - | |
| | | | 552,21 ; | |
| | | | 1,18 | |
| | | | (MJ) | |
| | | | - | |
| 62 | 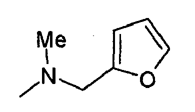 | 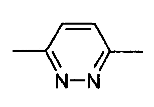 | - | 0,15 |
| | | | - | |
| | | | 574,2 ; | |
| | | | 1,21 | |
| | | | (MJ) | |
| | | | - | |
| 63 | 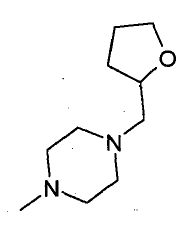 | 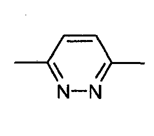 | TFA | 0,12 |
| | | | - | |
| | | | 633,27 ; | |
| | | | 1,01 | |
| | | | (MJ) | |
| | | | - | |
| 64 | 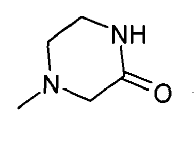 | 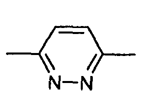 | - | 0,06 |
| | | | - | |
| | | | 563,19 ; | |
| | | | 4,23 | |
| | | | (MJ) | |
| | | | - | |
| 65 | 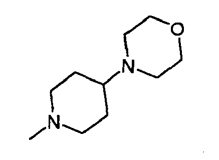 | 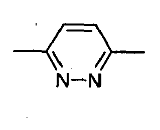 | TFA | 0,12 |
| | | | - | |
| | | | 633,27 ; | |
| | | | 0,99 | |
| | | | (MJ) | |
| | | | - | |
| 66 | 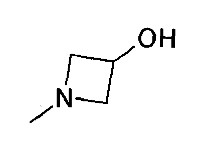 | 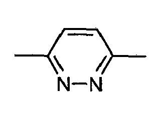 | - | 0,22 |
| | | | - | |
| | | | 536,18 ; | |
| | | | 1,11 | |
| | | | (MJ) | |
| | | | - | |
| 67 | 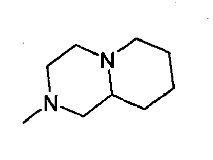 | 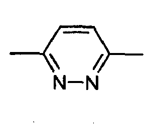 | TFA | 0,09 |
| | | | - | |
| | | | 603,26 ; | |
| | | | 1,01 | |
| | | | (MJ) | |
| | | | - | |
| 68 | 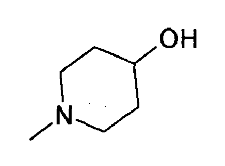 | 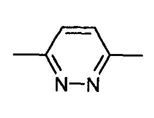 | - | 0,07 |
| | | | - | |
| | | | 564,21 ; | |
| | | | 1,13 | |
| | | | (MJ) | |
| | | | - | |
| 69 | 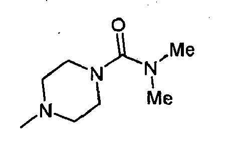 | 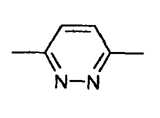 | - | 0,10 |
| | | | - | |
| | | | 620,25 ; | |
| | | | 1,15 | |
| | | | (MJ) | |
| | | | - | |
| 70 | 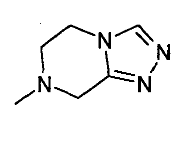 | 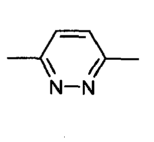 | - | 0,11 |
| | | | - | |
| | | | 587,2 ; | |
| | | | 1,09 | |
| | | | (MJ) | |
| | | | - | |
| 71 | 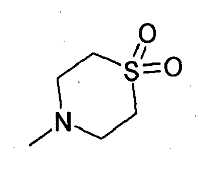 | 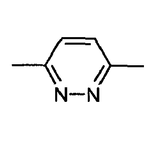 | - | 0,12 |
| | | | - | |
| | | | 598,16 ; | |
| | | | 1,14 | |
| | | | (MJ) | |
| | | | - | |
| 72 | 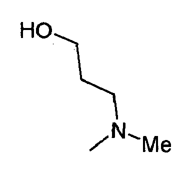 | 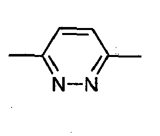 | - | 0,06 |
| | | | - | |
| | | | 552,21 ; | |
| | | | 1,13 | |
| | | | (MJ) | |
| | | | - | |
| 73 | 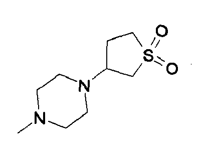 | 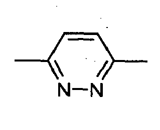 | TFA | 0,05 |
| | | | - | |
| | | | 667,22 ; | |
| | | | 1,12 | |
| | | | (MJ) | |
| | | | - | |
| 74 | 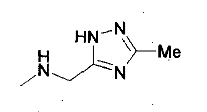 | 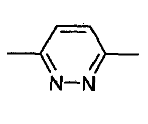 | - | 0,12 |
| | | | - | |
| | | | 575,2 ; | |
| | | | 1,08 | |
| | | | (MJ) | |
| | | | - | |
| 75 | 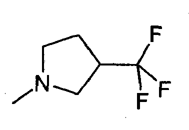 | 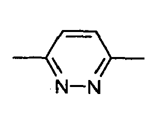 | - | 0,27 |
| | | | - | |
| | | | 602,19 ; | |
| | | | 1,22 | |
| | | | (MJ) | |
| | | | - | |
| 76 | 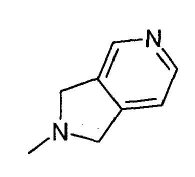 | 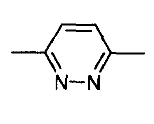 | - | 0,06 |
| | | | - | |
| | | | 583,2 ; | |
| | | | 1,08 | |
| | | | (MJ) | |
| | | | - | |
| 77 | 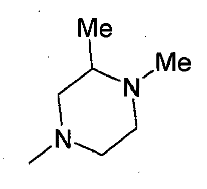 | 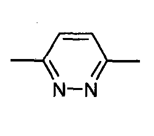 | TFA | 0,09 |
| | | | - | |
| | | | 577,24 ; 1 | |
| | | | (MJ) | |
| | | | - | |
| 78 | 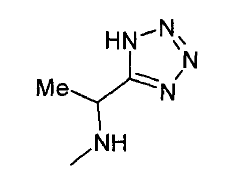 | 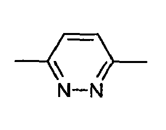 | - | 0,28 |
| | | | - | |
| | | | 576,2 ; | |
| | | | 1,12 | |
| | | | (MJ) | |
| | | | - | |
| 79 | 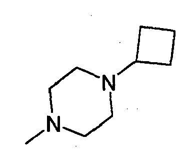 | 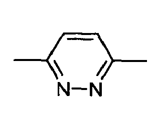 | TFA | 0,08 |
| | | | - | |
| | | | 603,26 ; | |
| | | | 1,01 | |
| | | | (MJ) | |
| | | | - | |
| 80 | 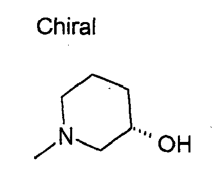 | 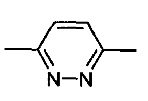 | - | 0,09 |
| | | | - | |
| | | | 564,21 ; | |
| | | | 1,14 | |
| | | | (MJ) | |
| | | | - | |
| 81 | 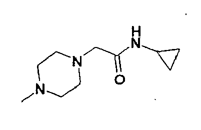 | 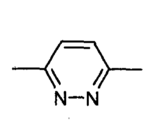 | - | 0,04 |
| | | | - | |
| | | | 646,27 ; | |
| | | | 1,05 | |
| | | | (MJ) | |
| | | | - | |
| 82 | 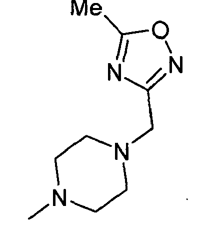 | 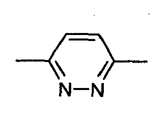 | TFA | 0,05 |
| | | | - | |
| | | | 645,25 ; | |
| | | | 1,14 | |
| | | | (MJ) | |
| | | | - | |
| 83 | 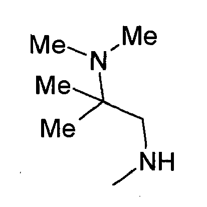 | 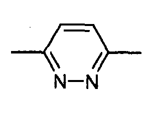 | TFA | 0,33 |
| | | | - | |
| | | | 579,26 ; 1 | |
| | | | (MJ) | |
| | | | - | |
| 84 | 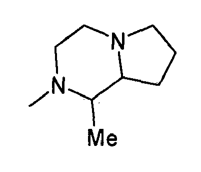 | 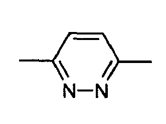 | TFA | 0,09 |
| | | | - | |
| | | | 603,26 ; | |
| | | | 1,01 | |
| | | | (MJ) | |
| | | | - | |
| 85 | 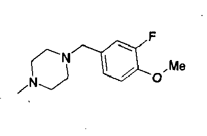 | 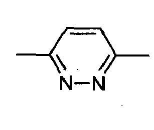 | TFA | 0,10 |
| | | | - | |
| | | | 687,26 ; | |
| | | | 1,06 | |
| | | | (MJ) | |
| | | | - | |
| 86 | 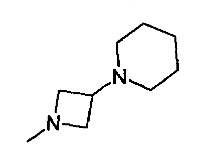 | 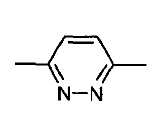 | TFA | 0,39 |
| | | | - | |
| | | | 603,26 ; 1 | |
| | | | (MJ) | |
| | | | - | |
| 87 | 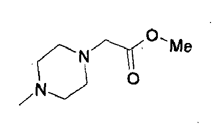 | 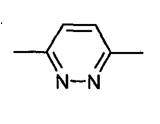 | TFA | 0,04 |
| | | | - | |
| | | | 621,23 ; | |
| | | | 1,1 | |
| | | | (MJ) | |
| | | | - | |
| 88 | 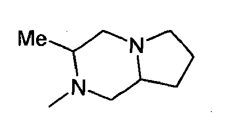 | 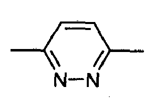 | TFA | 0,09 |
| | | | - | |
| | | | 603,26 ; | |
| | | | 1,01 | |
| | | | (MJ) | |
| | | | - | |
| 89 | 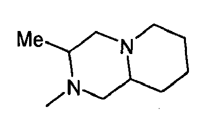 | 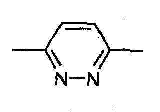 | TFA | 0,17 |
| | | | - | |
| | | | 617,28 ; | |
| | | | 1,02 | |
| | | | (MJ) | |
| | | | - | |
| 90 | 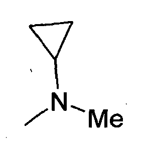 | 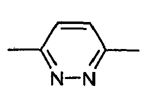 | - | 0,06 |
| | | | - | |
| | | | 534,2 ; 1,2 | |
| | | | (MJ) | |
| | | | - | |
| 91 | 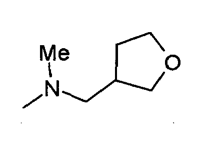 | 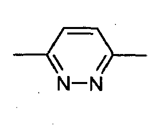 | - | 0,12 |
| | | | - | |
| | | | 578,23 ; 1,17 | |
| | | | (MJ) | |
| | | | - | |
| 92 | 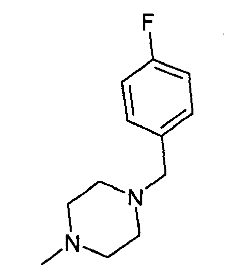 | 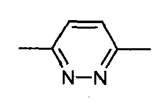 | TFA | 0,20 |
| | | | - | |
| | | | 657,25 ; | |
| | | | 1,06 | |
| | | | (MJ) | |
| | | | - | |
| 93 | 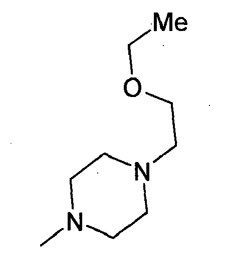 | 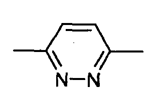 | TFA | 0,15 |
| | | | - | |
| | | | 621,27 ; | |
| | | | 1,02 | |
| | | | (MJ) | |
| | | | - | |
| 94 | 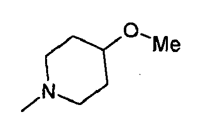 | 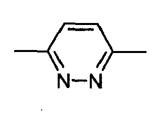 | - | 0,18 |
| | | | - | |
| | | | 578,23 ; | |
| | | | 1,18 | |
| | | | (MJ) | |
| | | | - | |
| 95 | 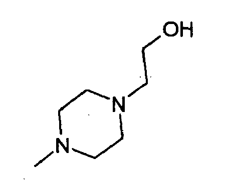 | 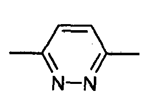 | - | 0,05 |
| | | | - | |
| | | | 593,24 ; | |
| | | | 1,13 | |
| | | | (MJ) | |
| | | | - | |
| 96 | 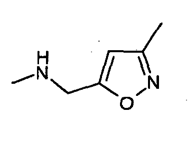 | 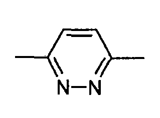 | - | 0.93 |
| | | | - | |
| | | | 575,19 ; | |
| | | | 1,17 | |
| | | | (MJ) | |
| | | | - | |
| 97 | 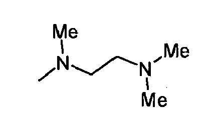 | 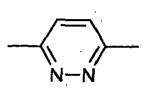 | - | 0,09 |
| | | | - | |
| | | | 565,24 ; | |
| | | | 1,14 | |
| | | | (MJ) | |
| | | | - | |
| 98 | 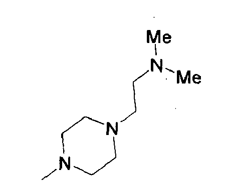 | 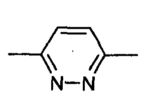 | - | 0,07 |
| | | | - | |
| | | | 620,29 ; | |
| | | | 1,14 | |
| | | | (MJ) | |
| | | | - | |
| 99 | 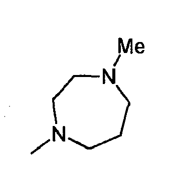 | 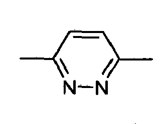 | - | 0,11 |
| | | | - | |
| | | | 577,24 ; | |
| | | | 1,02 | |
| | | | (MJ) | |
| | | | - | |
| 100 | 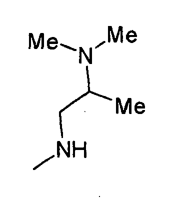 | 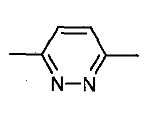 | - | 0,19 |
| | | | - | |
| | | | 565,24 ; | |
| | | | 1,03 | |
| | | | (MJ) | |
| | | | - | |
| 101 | 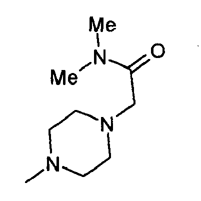 | 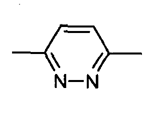 | - | 0,11 |
| | | | - | |
| | | | 634,27 ; | |
| | | | 1,03 | |
| | | | (MJ) | |
| | | | - | |
| 102 | 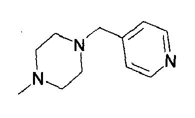 | 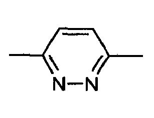 | - | 0,03 |
| | | | - | |
| | | | 640,26 ; | |
| | | | 1,05 | |
| | | | (MJ) | |
| | | | - | |
| 103 | 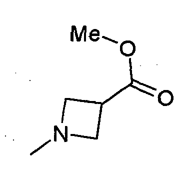 | 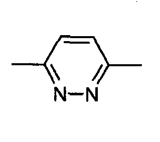 | - | 0,09 |
| | | | - | |
| | | | 578,19 ; | |
| | | | 1,16 | |
| | | | (MJ) | |
| | | | - | |
| 104 | 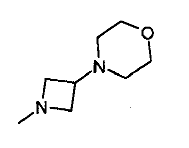 | 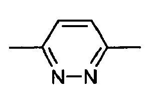 | - | 0,10 |
| | | | - | |
| | | | 605,24 ; | |
| | | | 1,07 | |
| | | | (MJ) | |
| | | | - | |

Les analyses effectuées par RMN pour certains composés sont données ci-après :
Composé 27 :
   RMN ¹H : DMSO-d₆ (400 MHz) : δ (ppm) : 0,94 (3H, t) ; 1,64 (2H, m) ; 2,70-4,50 (16H, m) ; 5,38 (2H, br) ; 7,29 (1H, d) ; 7,57 (1H, d) ; 8,25 (1H, s) ; 8,36 (1H, s) ; 8,57 (1H, s) ; 8,85 (1H, s) ; 9,42 (1H, d) ; 11,10 (1H, s) ; 11,15 (1H, br).
Composé 28 :
   RMN ¹H : DMSO-d₆ (400 MHz) : δ (ppm) : 0,95 (3H, t) ; 1,65 (2H, m) ; 2,80-2,89 (8H, m) ; 2,93-3,25 (3H, m) ; 3,41-4,08 (3H, m) ; 4,20 (1H, d) ; 4,39 (1H, d) ; 5,35 (1H, d) ; 5,50 (1H, d) ; 7,30 (1H, d) ; 7,59 (1H, d) ; 8,14 (1H, d) ; 8,24 (1H, s) ; 8,41 (1H, s) ; 8,58 (1H, s) ; 8,71 (1H, d) ; 10,97 (1H, br) ; 11,38 (1H, s).
Composé 29 :
   RMN ¹H : DMSO-d₆ (400 MHz) : δ (ppm) : 0,95 (3H, t) ; 1,65 (2H, m) ; 2,82 (3H, s) ; 2,87 (2H, t) ; 2,98-3,29 (3H, m) ; 3,35 (3H, s) ; 3,36-3,42 (2H, m) ; 3,50-3,88 (5H, m) ; 4,19 (1H, d) ; 4,37 (1H, d) ; 5,36 (1H, d) ; 5,50 (1H, d) ; 7,30 (1H, d) ; 7,58 (1H, d) ; 8,14 (1H, d) ; 8,24 (1H, s) ; 8,41 (1H, s) ; 8,58 (1H, s) ; 8,71 (1H, d) ; 10,56 (1H, br) ; 11,38 (1H, s),
Composé 30 :
   RMN ¹H : DMSO-d₆ (500 MHz) : δ (ppm) : 0,95 (3H,t) ; 1,65 (2H,m) ; 2,82 (3H,s) ; 2,86 (2H,t) ; 3,11-3,80 (9H,m) ; 3,95-4,80 (3H,m) ; 5,20-5,65 (2H,m) ; 7,30 (1H,d) ; 7,57 (1H,d) ; 8,13 (1H,d) ; 8,24 (1H,s) ; 8,41 (1H,s) ; 8,59 (1H,s) ; 8,70 (1H,d).
Composé 31 :
   RMN ¹H : DMSO-d₆ (400 MHz) : δ (ppm) : 0,95 : t : 3H ; 1,65 : m : 2H ; 2,82 : s : 3H ; 2,86 : t : 2H ; 3,00-3,90 : m : 9H ; 3,95-4,70 : m : 3H ; 5,25-5,55 : m : 2H ; 7,30 : d : 1H ; 7,57 : d : 1H ; 8,13 : d : 1H ; 8,24 : s : 1H ; 8,41 : s : 1H ; 8,59 : s : 1H ; 8,70 : d : 1H.

### EXEMPLE 11 : Composé n° 105

### Chlorhydrate de N-[4-(4-butyryl-5-méthyl-1H-pyrazol-1-yl)phényl]-5-chloro-1-{2-[4-(2-méthoxyéthyl)pipérazin-1-yl]-2-oxoéthyl}-1H-indole-3-carboxamide.

A 1,70 g du composé n°3 dans 35 ml de DCM, on ajoute successivement 1,28 g de 1-(2-méthoxyéthyl)pipérazine et 1,40 g de BOP-Cl. On agite à TA pendant 20 heures. On évapore le milieu réactionnel à sec, ajoute une solution diluée de NaHCO₃ et extrait à l'AcOEt. On lave la phase organique à l'eau, à la saumure, sèche sur Na₂SO₄ et évapore à sec. On triture dans l'éther iso puis essore. Le produit est solubilisé dans 5 ml d'un mélange DCM/acétone (50/50 ; v/v) puis on ajoute 5 ml d'une solution d'HCl 1N dans l'éther. On précipite le chlorhydrate avec 10 ml d'éther puis essore pour obtenir 1,96 g d'une poudre blanche.

RMN ¹H : DMSO-d₆ (250 MHz) : δ (ppm) : 0,94 (3H, t) ; 1,63 (2H, m) ; 2,52 (3H, s) ; 2,82 (2H, t) ; 2,97-3,29 (3H, m) ; 3,35-3,41 (5H, m) ;3,50-3,78 (5H, m) ; 4,18 (1H, d) ; 4,37 (1H, d) ; 5,36 (1H, d) ; 5,51 (1H, d) ; 7,27 (1H, d) ; 7,49 (2H, d) ; 7,57 (1H, d) ; 7,97 (2H, d) ; 8,22 (1H, s) ; 8,25 (1H, s) ; 8,34 (1H, s) ; 10,22 (1H, s) ; 10,81 (1H, br).

F = 218°C.

En procédant selon le mode opératoire décrit à l'Exemple 11 et à partir des composés de formule (IA) correspondants, on prépare les composés de formule (I) rassemblés dans le TABLEAU XIII ci-après :

Dans ce tableau :
- dans la colonne "Sel", "Base" représente un composé sous forme de base libre, alors que "HCl" représente un composé sous forme de chlorhydrate, "TFA" représente un composé sous forme de trifluoroacétate ;
- Me représente un radical méthyle ;
- Et représente un radical éthyle ;
- nPr représente un radical n-propyle.

**TABLEAU XIII**

| | | | | | | | | |
|---|---|---|---|---|---|---|---|---|
| 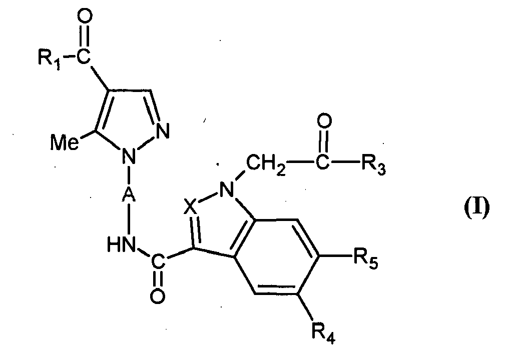 | | | | | | | | |

| Composés n° | R₁ | A | R₃ | R₄ | R₅ | X | Sel, Hydrate F°C MH+; tr (min) Méthode | Inhibition de l'agrégation plaquettaire *in vitro* (sang rat) en µM |
|---|---|---|---|---|---|---|---|---|
| 105 | nPr | 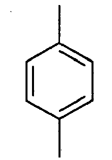 | 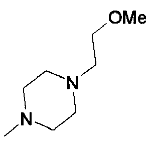 | Cl | H | CH | HCl, 0,7 H₂O | 0.32 |
| | | | | | | | 218 | |
| | | | | | | | 605 ; 6,07 | |
| | | | | | | | G | |
| 106 | nPr | 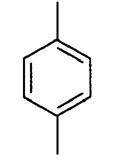 | 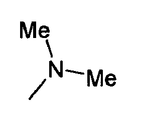 | Me | Me | CH | Base, H₂O | 2.21 |
| | | | | | | | 265 | |
| | | | | | | | 500 ; 8,48 | |
| | | | | | | | G | |
| 107 | nPr | 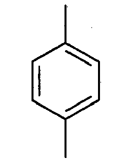 | 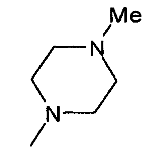 | Me | Me | CH | HCl, 3 H₂O | 0.30 |
| | | | | | | | 201 | |
| | | | | | | | 555 ; 6,46 | |
| | | | | | | | G | |
| 108 | nPr | 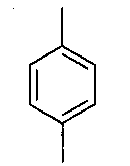 | 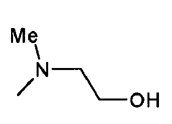 | Me | Me | CH | Base, 0,3 H₂O | 0.98 |
| | | | | | | | 177 | |
| | | | | | | | 530 ; 8,04 | |
| | | | | | | | G | |
| 109 | nPr | 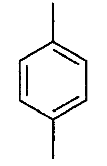 | 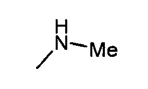 | Me | Me | CH | Base, 0,3 H₂O | 2.42 |
| | | | | | | | 300 | |
| | | | | | | | 486 ; 8,27 | |
| 110 | nPr | 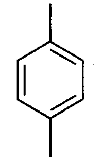 | 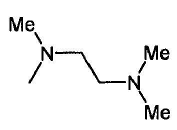 | Me | Me | CH | HCl, 2,5 H₂O | 3.99 |
| | | | | | | | 179 | |
| | | | | | | | 557 ; 6,50 | |
| | | | | | | | G | |
| 111 | nPr | 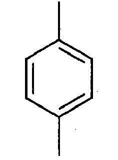 | 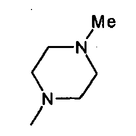 | Cl | Cl | CH | HCl, 2 H₂O | 3.70 |
| | | | | | | | 213 | |
| | | | | | | | 595 , 6,83 | |
| | | | | | | | G | |
| 112 | nPr | 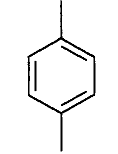 | 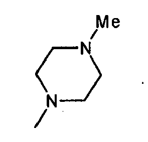 | Br | Me | CH | HCl, 2 H₂O | 0.36 |
| | | | | | | | 204 | |
| | | | | | | | 619 ; 6,78 | |
| | | | | | | | G | |
| 113 | nPr | 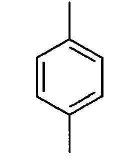 | 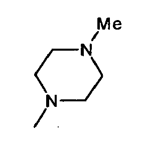 | Cl | Me | CH | HCl,2 H₂O | 0.62 |
| | | | | | | | 210 | |
| | | | | | | | 575 ; 13,09 | |
| | | | | | | | H | |
| 114 | nPr | 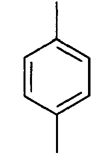 | 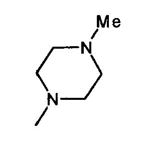 | Me | H | CH | HCl,1,7 H₂O | 0.27 |
| | | | | | | | 204 | |
| | | | | | | | 541 ; 6,31 | |
| | | | | | | | G | |
| 115 | nPr | 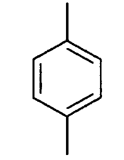 | 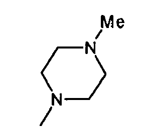 | Cl | H | CH | HCl, 1,4 H₂O | 0.32 |
| | | | | | | | 243 | |
| | | | | | | | 561; 6,51 | |
| | | | | | | | G | |
| 116 | nPr | 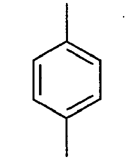 | 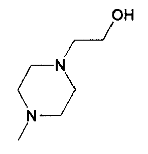 | Me | H | CH | HCl, 2,5 H₂O | 0.26 |
| | | | | | | | 184 | |
| | | | | | | | 571 ; 6,28 | |
| | | | | | | | G | |
| 117 | nPr | 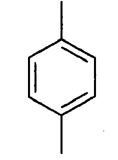 | 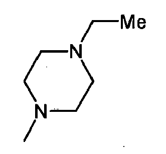 | Me | H | CH | HCl, 0,5 H₂O | 0.50 |
| | | | | | | | 212 | |
| | | | | | | | 555 ; 6,37 | |
| | | | | | | | G | |
| 118 | nPr | 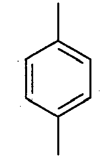 | 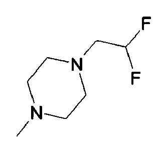 | Me | H | CH | HCl, 1 H₂O | 0.29 |
| | | | | | | | 281 | |
| | | | | | | | 591 ; 8,22 | |
| | | | | | | | G | |
| 119 | nPr | 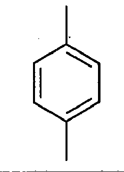 | 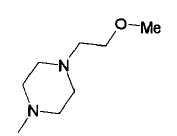 | Me | H | CH | HCl, 2 H₂O | 0.18 |
| | | | | | | | 180 | |
| | | | | | | | 585 ; 6,41 | |
| | | | | | | | G | |
| 120 | nPr | 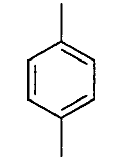 | 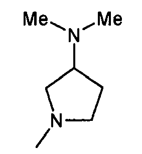 | Me | H | CH | HCl, 1 H₂O | 0.27 |
| | | | | | | | 171 | |
| | | | | | | | 555 ; 6,3 | |
| | | | | | | | G | |
| 121 | nPr | 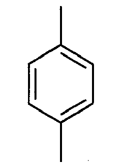 | 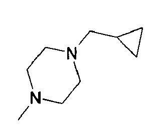 | Me | H | CH | HCl, 0,5 H₂O | 1.06 |
| | | | | | | | 296 | |
| | | | | | | | 581 ; 6,49 | |
| | | | | | | | G | |
| 122 | nPr | 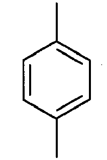 | 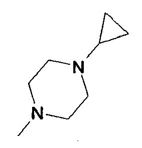 | Me | H | CH | HCl, 2,5 H₂O | 0.45 |
| | | | | | | | 199 | |
| | | | | | | | 567 ; 6,38 | |
| | | | | | | | G | |
| 123 | nPr | 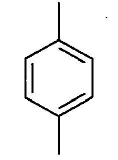 | 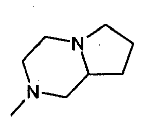 | Me | H | CH | HCl, 2,1 H₂O | 0.35 |
| | | | | | | | 140 | |
| | | | | | | | 567 ; 6,54 | |
| | | | | | | | G | |
| 124 | nPr | 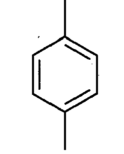 | 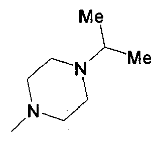 | Me | H | CH | HCl, H₂O | 0.21 |
| | | | | | | | 220 | |
| | | | | | | | 569 ; 6,42 | |
| | | | | | | | G | |
| 125 | nPr | 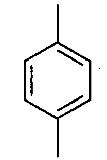 | 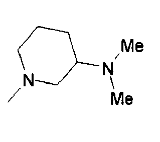 | Me | H | CH | HCl, 2 H₂O | 0.58 |
| | | | | | | | 164 | |
| | | | | | | | 569 ; 6,37 | |
| | | | | | | | G | |
| 126 | nPr | 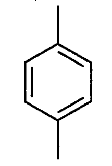 | 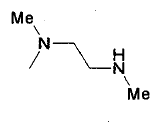 | Me | H | CH | HCl, 2,5 H₂O | 0.76 |
| | | | | | | | 164 | |
| | | | | | | | 529 ; 6,29 | |
| | | | | | | | G | |
| 127 | nPr | 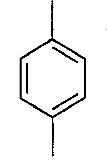 | 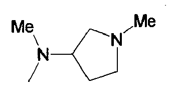 | Me | H | CH | HCl, H₂O | 0.50 |
| | | | | | | | 198 | |
| | | | | | | | 556 ; 6,37 | |
| | | | | | | | G | |
| 128 | nPr | 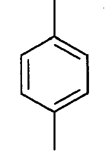 | 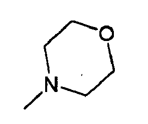 | Me | H | CH | HCl, 2,1 H₂O | 0.29 |
| | | | | | | | 189 | |
| | | | | | | | 528 ; 8,23 | |
| | | | | | | | G | |
| 129 | nPr | 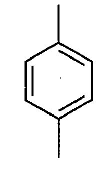 | 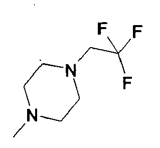 | Me | H | CH | Base, 0,2 H₂O | 0.65 |
| | | | | | | | 143 | |
| | | | | | | | 609 ; 9,17 | |
| | | | | | | | G | |
| 130 | nPr | 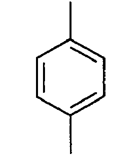 | 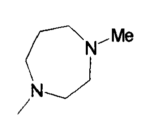 | Me | H | CH | HCl, 1,4 H₂O | 0.47 |
| | | | | | | | 200 | |
| | | | | | | | 555 ; 6,32 | |
| | | | | | | | G | |
| 131 | nPr | 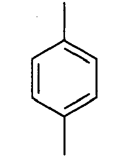 | 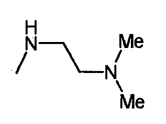 | Me | H | CH | HCl, 1,3 H₂O | 0.63 |
| | | | | | | | 192 | |
| | | | | | | | 529 ; 6,27 | |
| | | | | | | | G | |
| 132 | nPr | 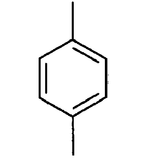 | 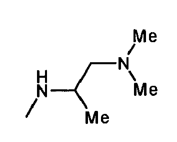 | Me | H | CH | HCl, 2 H₂O | 0.98 |
| | | | | | | | 140 | |
| | | | | | | | 543 ; 6,33 | |
| | | | | | | | G | |
| 133 | nPr | 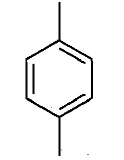 | 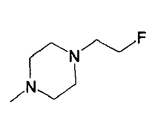 | Me | H | CH | HCl, 1,6 H₂O | 0.32 |
| | | | | | | | 192 | |
| | | | | | | | 573 ; 6,45 | |
| | | | | | | | G | |
| 134 | nPr | 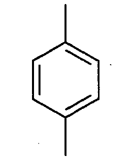 | 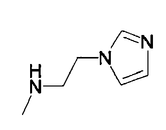 | Me | H | CH | HCl, H₂O | 0.95 |
| | | | | | | | 172 | |
| | | | | | | | 552 ; 6,22 | |
| | | | | | | | G | |
| 135 | nPr | 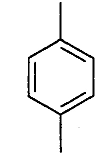 | 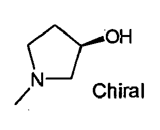 | Me | H | CH | Base, 0,5 H₂O | 0.21 |
| | | | | | | | 170 | |
| | | | | | | | 528 ; 7,26 | |
| | | | | | | | G | |
| 136 | nPr | 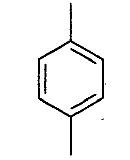 | 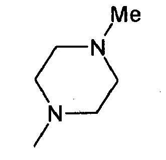 | OM e | H | CH | HCl, 1,6 H₂O | 0.40 |
| | | | | | | | 204 | |
| | | | | | | | 557 ; 5,53 | |
| | | | | | | | G | |
| 137 | nPr | 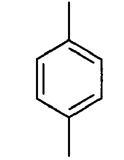 | 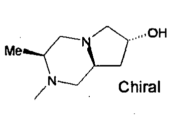 | Me | H | CH | HCl, 2.5 H₂O | 0.39 |
| | | | | | | | 215 | |
| | | | | | | | 597 ; 5,83 | |
| | | | | | | | G | |
| 138 | nPr | 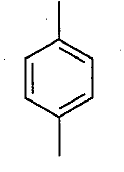 | 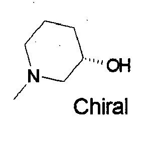 | Me | H | CH | Base, 0,3 H₂O | 0.42 |
| | | | | | | | 142 | |
| | | | | | | | 542 ; 8,00 | |
| | | | | | | | G | |
| 139 | nPr | 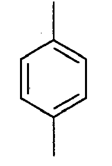 | 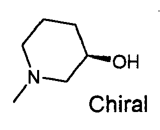 | Me | H | CH | Base, 0,65 H₂O | 0.47 |
| | | | | | | | 142 | |
| | | | | | | | 542 ; 7, 99 | |
| | | | | | | | G | |
| 140 | nPr | 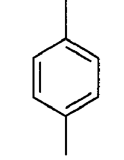 | 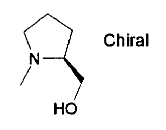 | Me | H | CH | HCl, 0,3 H₂O | 0.34 |
| | | | | | | | 152 | |
| | | | | | | | 542 ; 8,10 | |
| | | | | | | | G | |
| 141 | nPr | 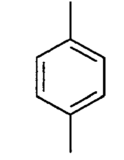 | 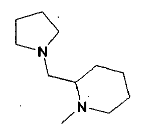 | Me | H | CH | HCl, 1,8 H₂O | 0.64 |
| | | | | | | | 202 | |
| | | | | | | | 609 ; 6,11 | |
| | | | | | | | G | |
| 142 | nPr | 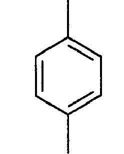 | 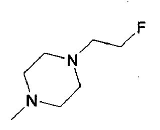 | Cl | H | CH | HCl, H₂O | 0.13 |
| | | | | | | | 192 | |
| | | | | | | | 593 ; 6,59 | |
| | | | | | | | G | |
| 143 | nPr | 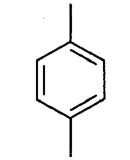 | 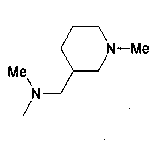 | Me | H | CH | HCl, 2,3 H₂O | 0.53 |
| | | | | | | | 202 | |
| | | | | | | | 583 ; 6,42 | |
| | | | | | | | G | |
| 144 | nPr | 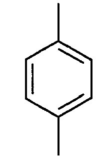 | 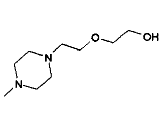 | Cl | H | CH | HCl, 3,3 H₂O | 0.19 |
| | | | | | | | 125 | |
| | | | | | | | 635; 6,51 | |
| | | | | | | | G | |
| 145 | nPr | 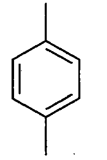 | 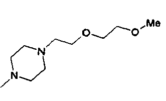 | Cl | H | CH | HCl, 1,4 H₂O | 0.25 |
| | | | | | | | 135 | |
| | | | | | | | 649 ; 6,69 | |
| | | | | | | | G | |
| 146 | nPr | 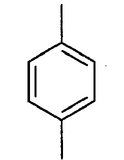 | 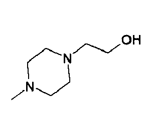 | Cl | H | CH | HCl, 2 H₂O | 0.10 |
| | | | | | | | 212 | |
| | | | | | | | 591 ; 6,50 | |
| | | | | | | | G | |
| 147 | nPr | 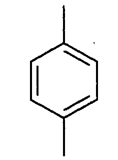 | 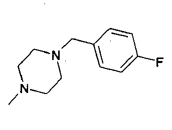 | Me | H | CH | HCl, H₂O | 0.19 |
| | | | | | | | 251 | |
| | | | | | | | 635 ; 6,80 | |
| | | | | | | | G | |
| 148 | nPr | 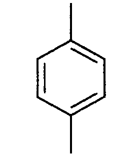 | 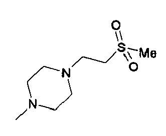 | Cl | H | CH | HCl, 0,8 H₂O | 0.15 |
| | | | | | | | 252 | |
| | | | | | | | 653 ; 6,78 | |
| | | | | | | | G | |
| 149 | nPr | 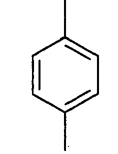 | 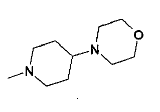 | Cl | H | CH | HCl, 1,5 H₂O | 0.41 |
| | | | | | | | 217 | |
| | | | | | | | 631 ; 6,45 | |
| | | | | | | | G | |
| 150 | nPr | 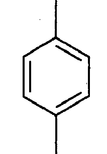 | 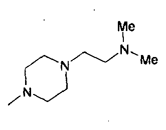 | Cl | H | CH | HCl, 1,25 H₂O G | 0.24 |
| | | | | | | | 272 | |
| | | | | | | | 618 ; 6,42 | |
| | | | | | | | G | |
| 151 | nPr | 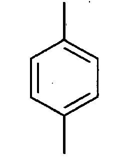 | 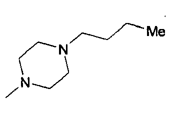 | Cl | H | CH | HCl, 0,4 H₂O G | 0.25 |
| | | | | | | | 220 | |
| | | | | | | | 603 ; 6,67 | |
| | | | | | | | G | |
| 152 | nPr | 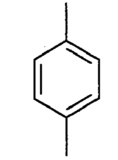 | 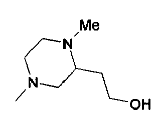 | Cl | H | CH | HCl, 2,5 H₂O G | 0.22 |
| | | | | | | | 212 | |
| | | | | | | | 605 ; 6,15 | |
| | | | | | | | G | |
| 153 | nPr | 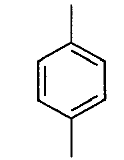 | 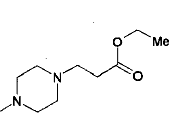 | Cl | H | CH | HCl, H₂O | 0.29 |
| | | | | | | | 190 | |
| | | | | | | | 647 ; 6,39 | |
| | | | | | | | G | |
| 154 | nPr | 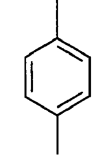 | 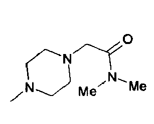 | Cl | H | CH | HCl, 1,5 H₂O | 0.15 |
| | | | | | | | 210 | |
| | | | | | | | 632 ; 6,12 | |
| | | | | | | | G | |
| 155 | nPr | 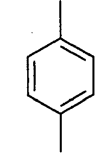 | 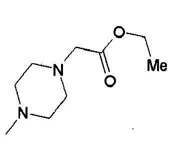 | Cl | H | CH | HCl, 0,15 H₂O | 0.26 |
| | | | | | | | 195 | |
| | | | | | | | 633 ; 7,53 | |
| | | | | | | | G | |
| 156 | nPr | 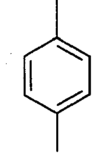 | 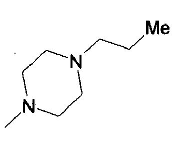 | Cl | H | CH | HCl, 0,9 H₂O | 0.30 |
| | | | | | | | 279 | |
| | | | | | | | 589 ; 6,14 | |
| | | | | | | | G | |
| 157 | nPr | 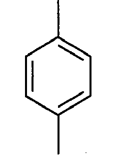 | 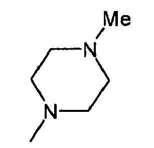 | Cl | F | CH | HCl, H₂O G | 0.18 |
| | | | | | | | 222 | |
| | | | | | | | 579 ; 6,7 | |
| | | | | | | | G | |
| 158 | nPr | 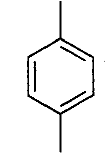 | 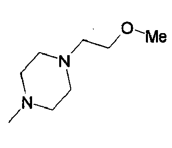 | Cl | F | CH | HCl, 0,15 H₂O | 0.34 |
| | | | | | | | 226 | |
| | | | | | | | 623 ; 6,76 | |
| | | | | | | | G | |
| 159 | nPr | 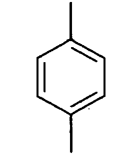 | 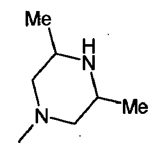 | Cl | H | CH | HCl, H₂O | 0.41 |
| | | | | | | | 230 | |
| | | | | | | | 575 ; 6,03 | |
| | | | | | | | G | |
| 160 | nPr | 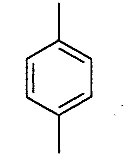 | 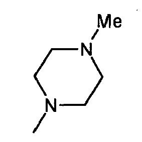 | CN | H | CH | HCl, 1,5 H₂O | 1.38 |
| | | | | | | | 209 | |
| | | | | | | | 552 ; 5,58 | |
| | | | | | | | G | |
| 161 | nPr | 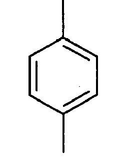 | 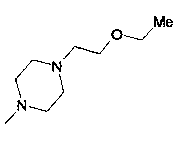 | Cl | H | CH | HCl, H₂O | 0.37 |
| | | | | | | | 196 | |
| | | | | | | | 619 ; 6,8 | |
| | | | | | | | G | |
| 162 | nPr | 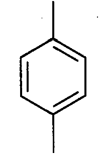 | 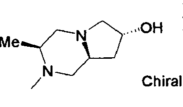 | Cl | H | CH | Base, H₂O | 0.50 |
| | | | | | | | 227 | |
| | | | | | | | 617 ; 6,53 | |
| | | | | | | | G | |
| 163 | nPr | 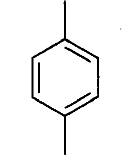 | 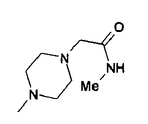 | Cl | H | CH | HCl, 0,8 H₂O | 0.29 |
| | | | | | | | 216 | |
| | | | | | | | 618 ; 6,43 | |
| | | | | | | | G | |
| 164 | nPr | 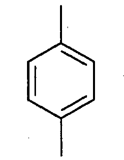 | 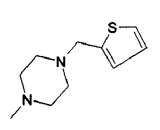 | Me | H | CH | HCl, 0,3 H₂O | 1.01 |
| | | | | | | | 240 | |
| | | | | | | | 623 ; 6,42 | |
| | | | | | | | G | |
| 165 | nPr | 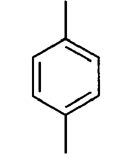 | 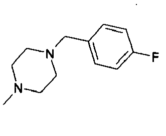 | Cl | H | CH | HCl, 1,2 H₂O | 0.31 |
| | | | | | | | 195 | |
| | | | | | | | 655 ; 7,14 | |
| | | | | | | | G | |
| 166 | nPr | 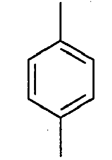 | 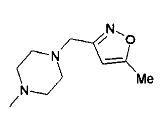 | Cl | H | CH | HCl, 0,6 H₂O | 0.14 |
| | | | | | | | 219 | |
| | | | | | | | 642 ; 7,52 | |
| | | | | | | | G | |
| 167 | nPr | 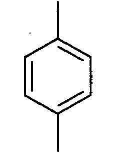 | 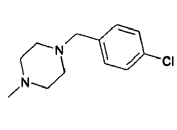 | Cl | H | CH | - | 0.47 |
| | | | | | | | - | |
| | | | | | | | 671; 3,74 | |
| | | | | | | | A | |
| 168 | nPr | 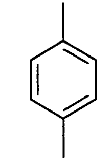 | 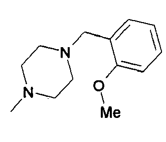 | Cl | H | CH | - | 0.37 |
| | | | | | | | - | |
| | | | | | | | 667 ; 3,44 | |
| | | | | | | | A | |
| 169 | nPr | 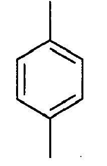 | 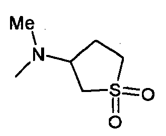 | Cl | H | CH | - | 0.40 |
| | | | | | | | - | |
| | | | | | | | 610 ; 3,24 | |
| | | | | | | | A | |
| 170 | nPr | 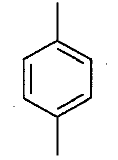 | 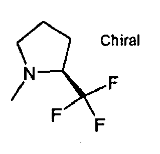 | Cl | H | CH | - | 0.12 |
| | | | | | | | - | |
| | | | | | | | 600 ; 3,62 | |
| | | | | | | | A | |
| 171 | nPr | 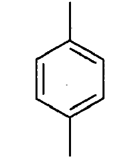 | 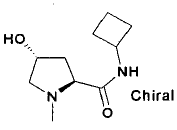 | Cl | H | CH | - | 0.57 |
| | | | | | | | - | |
| | | | | | | | 645 ; 3,14 | |
| | | | | | | | A | |
| 172 | nPr | 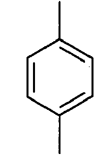 | 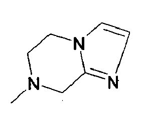 | Cl | H | CH | - | 0.33 |
| | | | | | | | - | |
| | | | | | | | 584 ; 3,17 | |
| | | | | | | | A | |
| 173 | nPr | 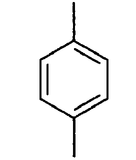 | 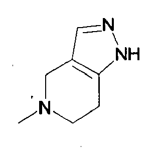 | Cl | H | CH | - | 0.17 |
| | | | | | | | - | |
| | | | | | | | 584 ; 4,50 | |
| | | | | | | | B | |
| 174 | nPr | 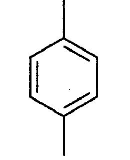 | 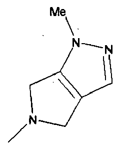 | Cl | H | CH | - | 0.49 |
| | | | | | | | - | |
| | | | | | | | 584 ; 4,63 | |
| | | | | | | | B | |
| 175 | nPr | 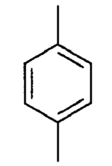 | 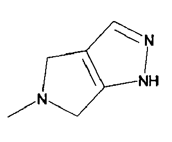 | Cl | H | CH | - | 0.29 |
| | | | | | | | - | |
| | | | | | | | 570 ; 4,49 | |
| | | | | | | | B | |
| 176 | nPr | 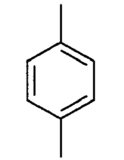 | 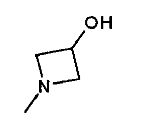 | Cl | H | CH | - | 0.39 |
| | | | | | | | - | |
| | | | | | | | 534 ; 3,05 | |
| | | | | | | | B | |
| 177 | nPr | 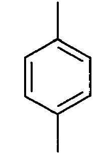 | 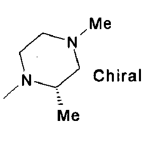 | Cl | H | CH | HCl, H₂O | 0.37 |
| | | | | | | | 247 | |
| | | | | | | | 575 ; 6,66 | |
| | | | | | | | G | |
| 178 | nPr | 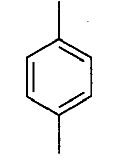 | 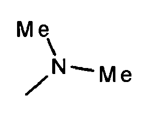 | NMe₂ | H | CH | HCl, 2 H₂O | 0.50 |
| | | | | | | | 185 | |
| | | | | | | | 515 ; 5,60 | |
| | | | | | | | G | |
| 179 | nPr | 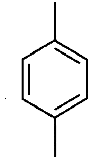 | 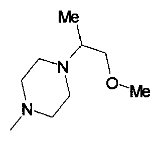 | Cl | H | CH | HCl, H₂O | 0.17 |
| | | | | | | | 198 | |
| | | | | | | | 619 ; 6,61 | |
| | | | | | | | G | |
| 180 | nPr | 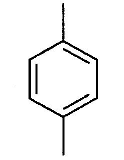 | 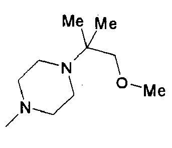 | Cl | H | CH | HCl, 1,5 H₂O | 0.24 |
| | | | | | | | 196 | |
| | | | | | | | 633 ; 6,66 | |
| | | | | | | | G | |
| 181 | nPr | 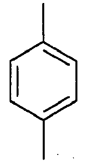 | 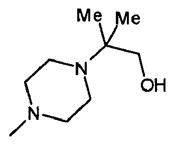 | Cl | H | CH | HCl, 2,2 H₂O | 0.18 |
| | | | | | | | 212 | |
| | | | | | | | 619 ; 6,51 | |
| | | | | | | | G | |
| 182 | nPr | 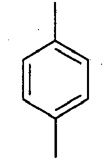 | 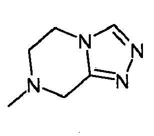 | Cl | H | CH | - | 0.27 |
| | | | | | | | - | |
| | | | | | | | 585 ; 2,96 | |
| | | | | | | | A | |
| 183 | nPr | 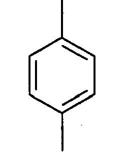 | 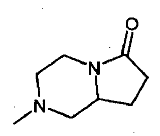 | Cl | H | CH | - | 0.23 |
| | | | | | | | - | |
| | | | | | | | 601 ; 3,12 | |
| | | | | | | | A | |
| 184 | nPr | 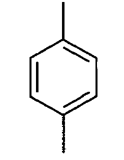 | 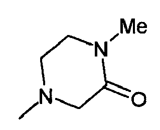 | Cl | H | CH | - | 0.17 |
| | | | | | | | - | |
| | | | | | | | 575 ; 3,10 | |
| | | | | | | | A | |
| 185 | nPr | 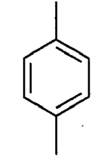 | 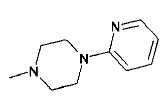 | Cl | H | CH | HCl, 1,3 H₂O | |
| | | | | | | | 232 | |
| | | | | | | | 624 ; 9,42 | |
| | | | | | | | I | |
| 186 | nPr | 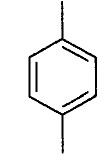 | 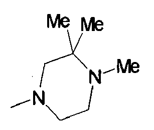 | Cl | H | CH | - | 0.25 |
| | | | | | | | - | |
| | | | | | | | 589 ; 2,98 | |
| | | | | | | | A | |
| 187 | nPr | 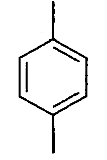 | 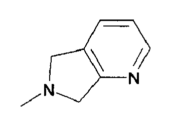 | Cl | H | CH | HCl, 0,3 H₂O | 0.61 |
| | | | | | | | 270 | |
| | | | | | | | 581 ; 8,79 | |
| | | | | | | | I | |
| 188 | nPr | 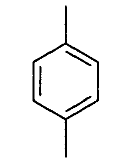 | 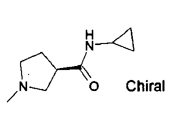 | Cl | H | CH | - | 0.55 |
| | | | | | | | - | |
| | | | | | | | 615 ; 3,17 | |
| | | | | | | | A | |
| 189 | nPr | 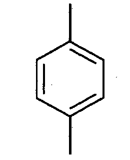 | 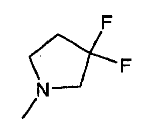 | Cl | H | CH | - | 0.41 |
| | | | | | | | - | |
| | | | | | | | 568 ; 4,70 | |
| | | | | | | | E | |
| 190 | nPr | 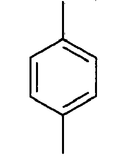 | 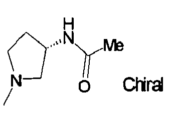 | Cl | H | CH | - | 0.36 |
| | | | | | | | - | |
| | | | | | | | 589 ; 3,25 | |
| | | | | | | | A | |
| 191 | nPr | 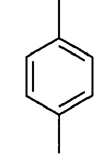 | 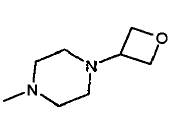 | Cl | H | CH | - | 0.16 |
| | | | | | | | - | |
| | | | | | | | 603 ; 3,12 | |
| | | | | | | | A | |
| 192 | nPr | 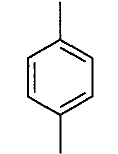 | 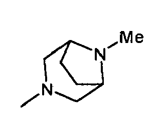 | Cl | H | CH | HCl | 0.27 |
| | | | | | | | 295 | |
| | | | | | | | 587 ; 6,54 | |
| | | | | | | | G | |
| 193 | nPr | 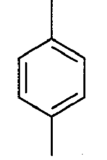 | 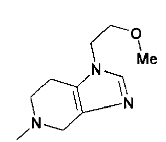 | Cl | H | CH | - | 0.12 |
| | | | | | | | - | |
| | | | | | | | 642 ; 2,98 | |
| | | | | | | | A | |
| 194 | nPr | 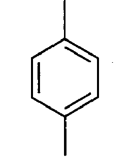 | 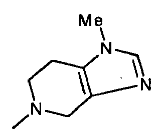 | Cl | H | CH | - | 0.20 |
| | | | | | | | - | |
| | | | | | | | 598 ; 2,91 | |
| | | | | | | | A | |
| 195 | nPr | 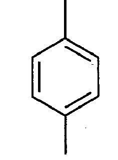 | 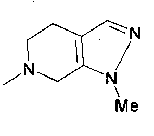 | Cl | H | CH | - | 0.28 |
| | | | | | | | - | |
| | | | | | | | 598 ; 3,27 | |
| | | | | | | | A | |
| 196 | nPr | 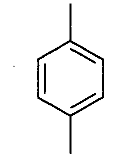 | 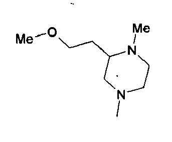 | Cl | H | CH | - | 0.23 |
| | | | | | | | - | |
| | | | | | | | 619 ; 2,96 | |
| | | | | | | | A | |
| 197 | nPr | 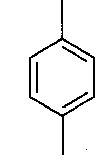 | 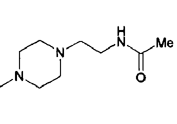 | Cl | H | CH | - | 0.25 |
| | | | | | | | - | |
| | | | | | | | 632 ; 2,94 | |
| | | | | | | | A | |
| 198 | nPr | 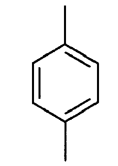 | 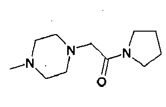 | Cl | H | CH | - | 0.41 |
| | | | | | | | - | |
| | | | | | | | 658 ; 3,15 | |
| | | | | | | | A | |
| 199 | nPr | 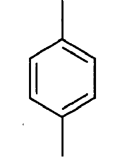 | 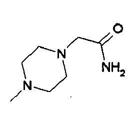 | Cl | H | CH | HCl, 3 H₂O | 0.27 |
| | | | | | | | 221 | |
| | | | | | | | 604 ; 6,71 | |
| | | | | | | | G | |
| 200 | nPr | 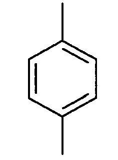 | 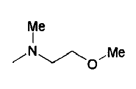 | Cl | H | CH | Base | 0.48 |
| | | | | | | | 218 | |
| | | | | | | | 550 ; 8,79 | |
| | | | | | | | G | |
| 201 | nPr | 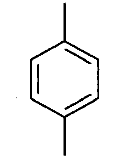 | 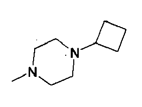 | Cl | H | CH | - | 0.65 |
| | | | | | | | - | |
| | | | | | | | 601 ; 3,25 | |
| | | | | | | | A | |
| 202 | nPr | 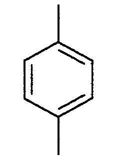 | 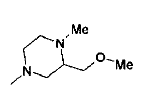 | Cl | H | CH | - | 0.43 |
| | | | | | | | - | |
| | | | | | | | 605 ; 3,04 | |
| | | | | | | | C | |
| 203 | nPr | 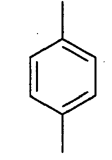 | 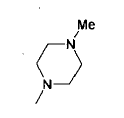 | CF₃ | H | CH | HCl, H₂O | 0.36 |
| | | | | | | | 293 | |
| | | | | | | | 595 ; 6,75 | |
| | | | | | | | G | |
| 204 | nPr | 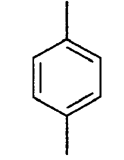 | 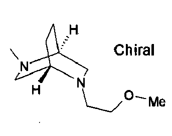 | Cl | H | CH | - | 0.44 |
| | | | | | | | - | |
| | | | | | | | 631 ; 3,26 | |
| | | | | | | | A | |
| 205 | nPr | 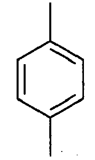 | 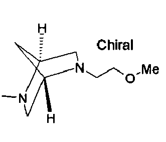 | Cl | H | CH | - | 0.44 |
| | | | | | | | - | |
| | | | | | | | 617 ; 3,22 | |
| | | | | | | | A | |
| 206 | nPr | 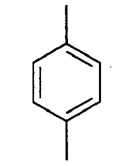 | 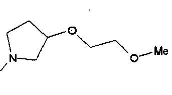 | Cl | H | CH | - | 0.83 |
| | | | | | | | - | |
| | | | | | | | 606 ; 3,27 | |
| | | | | | | | A | |
| 207 | nPr | 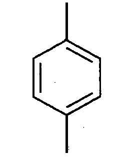 | 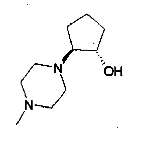 | Cl | H | CH | - | 0.31 |
| | | | | | | | - | |
| | | | | | | | 631 ; 3,10 | |
| | | | | | | | A | |
| 208 | nPr | 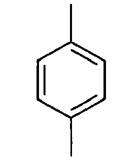 | 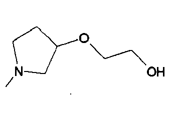 | Cl | H | CH | - | 0.37 |
| | | | | | | | - | |
| | | | | | | | 592 ; 3,07 | |
| | | | | | | | A | |
| 209 | nPr | 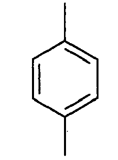 | 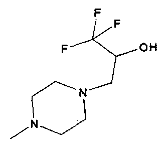 | Cl | H | CH | - | 0.42 |
| | | | | | | | - | |
| | | | | | | | 659 ; 3,14 | |
| | | | | | | | A | |
| 210 | nPr | 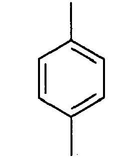 | 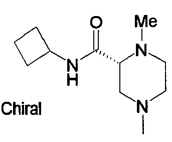 | Cl | H | CH | - | 0.70 |
| | | | | | | | - | |
| | | | | | | | 658 ; 3,19 | |
| | | | | | | | A | |
| 211 | nPr | 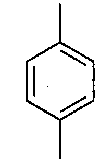 | 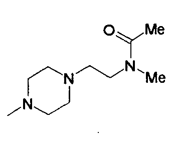 | Cl | H | CH | - | 0.28 |
| | | | | | | | - | |
| | | | | | | | 646 ; 2,84; | |
| | | | | | | | A | |
| 212 | nPr | 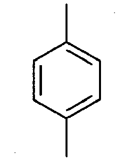 | 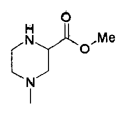 | Cl | H | CH | RMN | / |
| 213 | nPr | 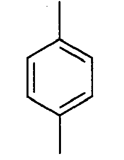 | 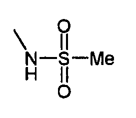 | Me | H | CH | HCl, 1,6 H₂O | 4.86 |
| | | | | | | | 175 | |
| | | | | | | | 536 ; 9,02 | |
| | | | | | | | G | |
| 214 | nPr | 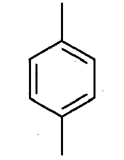 | 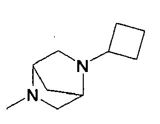 | Cl | H | CH | - | 0.66 |
| | | | | | | | - | |
| | | | | | | | 613 ; 3,33 | |
| | | | | | | | A | |
| 215 | nPr | 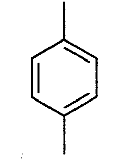 | 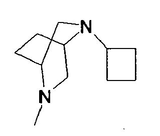 | Cl | H | CH | - | 0.76 |
| | | | | | | | - | |
| | | | | | | | 627 ; 3,44 | |
| | | | | | | | A | |
| 216 | Me | 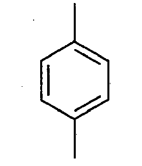 | 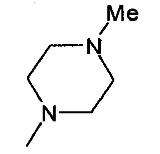 | Cl | H | CH | HCl, 1,9 H₂O | 0.73 |
| | | | | | | | 228 | |
| | | | | | | | 533 ; 5,89 | |
| | | | | | | | J | |
| 217 | Et | 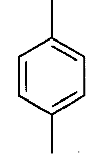 | 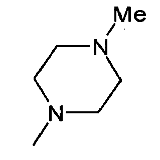 | Cl | H | CH | HCl, 0,3 H₂O | 0.60 |
| | | | | | | | 241 | |
| | | | | | | | 547 ; 6,23 | |
| | | | | | | | G | |
| 218 | EtO | 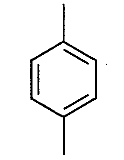 | 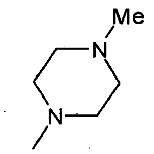 | Cl | H | CH | HCl, 1,2 H₂O | 0.56 SAR196584 |
| | | | | | | | 261 | |
| | | | | | | | 563 ; 11,81 | |
| | | | | | | | H | |
| 219 | nPr | 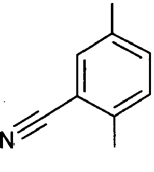 | 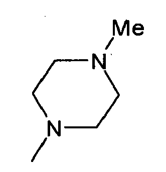 | Cl | H | CH | Base | 0.54 |
| | | | | | | | 126 | |
| | | | | | | | 586 ; 6,67 | |
| | | | | | | | G | |
| 220 | nPr | 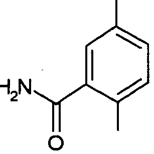 | 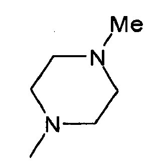 | Cl | H | CH | HCl, 2 H₂O | 0.22 |
| | | | | | | | 250 | |
| | | | | | | | 604 ; 12,37 | |
| | | | | | | | H | |
| 221 | nPr | 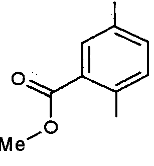 | 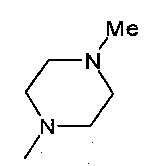 | Cl | H | CH | - | / |
| | | | | | | | - | |
| | | | | | | | 619 ; 6,85 | |
| | | | | | | | G | |
| 222 | nPr | 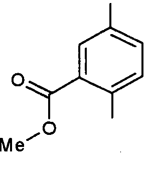 | 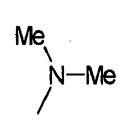 | Cl | H | CH | - | / |
| | | | | | | | - | |
| | | | | | | | 564 ; 9,38 | |
| | | | | | | | G | |
| 223 | nPr | 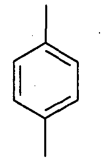 | 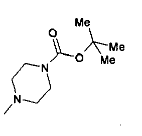 | Me | H | CH | - | / |
| | | | | | | | - | |
| | | | | | | | 627 ; 9,26 | |
| | | | | | | | G | |
| 224 | nPr | 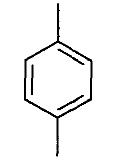 | 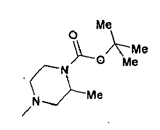 | Me | H | CH | - | / |
| | | | | | | | - | |
| | | | | | | | 641 ; 9,45 | |
| | | | | | | | G | |
| 225 | nPr | 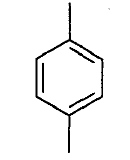 | 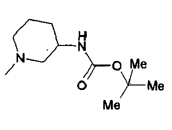 | Me | H | CH | - | / |
| | | | | | | | - | |
| | | | | | | | 641 ; 9,31 | |
| | | | | | | | G | |
| 226 | nPr | 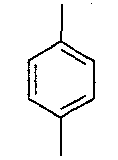 | 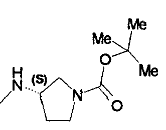 | Me | H | CH | - | / |
| | | | | | | | - | |
| | | | | | | | 627 ; 8,58 | |
| | | | | | | | G | |
| 227 | nPr | 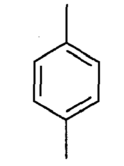 | 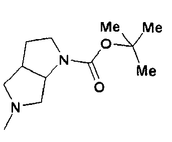 | Me | H | CH | - | / |
| | | | | | | | - | |
| | | | | | | | ND | |
| | | | | | | | - | |
| 228 | nPr | 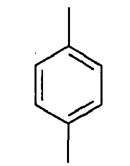 | 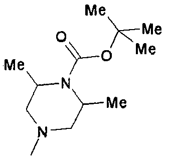 | Me | H | CH | - | / |
| | | | | | | | - | |
| | | | | | | | 655 ; 9,26 | |
| | | | | | | | G | |
| 229 | nPr | 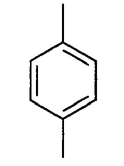 | 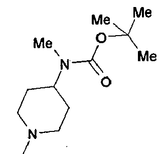 | Me | H | CH | - | / |
| | | | | | | | - | |
| | | | | | | | 657 ; 9,09 | |
| | | | | | | | G | |
| 230 | nPr | 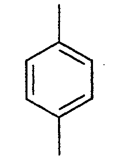 | 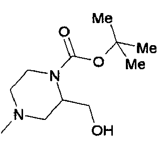 | Me | H | CH | - | / |
| | | | | | | | - | |
| | | | | | | | 657 ; 8,25 | |
| | | | | | | | G | |
| 231 | nPr | 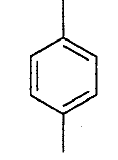 | 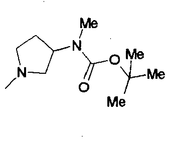 | Me | H | CH | - | / |
| | | | | | | | - | |
| | | | | | | | ND | |
| | | | | | | | - | |
| 232 | nPr | 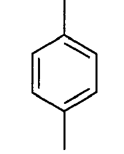 | 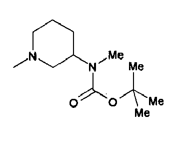 | Me | H | CH | - | / |
| | | | | | | | - | |
| | | | | | | | ND | |
| | | | | | | | - | |
| 233 | nPr | 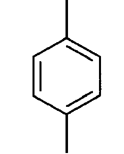 | 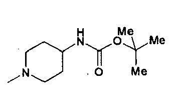 | Me | H | CH | - | / |
| | | | | | | | - | |
| | | | | | | | ND | |
| | | | | | | | - | |
| 234 | nPr | 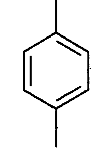 | 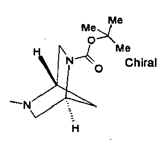 | Me | H | CH | - | / |
| | | | | | | | - | |
| | | | | | | | 639 ; 9,11 | |
| | | | | | | | G | |
| 235 | nPr | 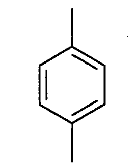 | 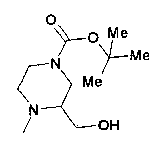 | Me | H | CH | - | / |
| | | | | | | | - | |
| | | | | | | | 657 ; 8,83 | |
| | | | | | | | G | |
| 236 | nPr | 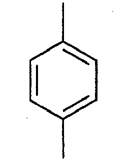 | 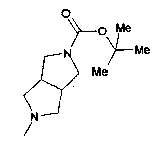 | Me | H | CH | - | / |
| | | | | | | | - | |
| | | | | | | | 653 ; 8,70 | |
| | | | | | | | G | |
| 237 | nPr | 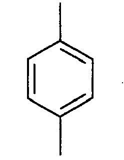 | 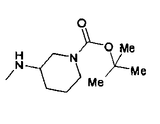 | Me | H | CH | - | / |
| | | | | | | | - | |
| | | | | | | | 641 ; 9.39 | |
| | | | | | | | G | |
| 238 | nPr | 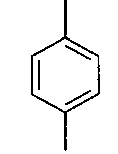 | 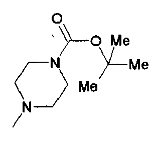 | Cl | H | CH | - | / |
| | | | | | | | - | |
| | | | | | | | 647 ; 9,50 | |
| | | | | | | | G | |
| 239 | nPr | 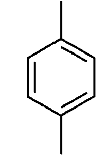 | 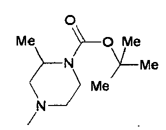 | Cl | H | CH | - | / |
| | | | | | | | - | |
| | | | | | | | ND | |
| | | | | | | | - | |
| 240 | nPr | 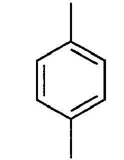 | 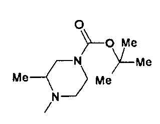 | Cl | H | CH | - | / |
| | | | | | | | - | |
| | | | | | | | ND | |
| | | | | | | | - | |
| 241 | nPr | 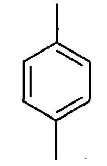 | 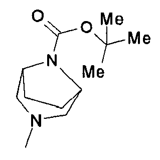 | Cl | H | CH | - | / |
| | | | | | | | - | |
| | | | | | | | 673 ; 9,77 | |
| | | | | | | | G | |

Les composés N° 223 à 241 de formule (I) portant un groupe protecteur *tert-*butyloxycarbonyle sur un des atomes d'azote ne présentent pas l'activité pharmacologique recherchée. Ceux sont des composés intermédiaires utiles pour la préparation des composés de formule (I) N° 242 à 260.

Les analyses effectuées par RMN pour certains composés sont données ci-après :
Composé 106 :
   RMN ¹H : DMSO-d₆ (250 MHz) : δ (ppm) : 0,94 (3H, t) ; 1,63 (2H, m) ; 2,33 (6H, s) ; 2,52 (3H, s) ; 2,81 (2H, t) ; 2,89 (3H, s) ; 3,14 (3H, s) ; 5,22 (2H, s) ; 7,25 (1H, s) ; 7,47 (2H, d) ; 7,93-7,99 (3H, m) ; 8,09 (1H, s) ; 8,24 (1H, s) ; 9,97 (1H, s).
Composé 107 :
   RMN ¹H : DMSO-d₆ (250 MHz) : δ (ppm) : 0,94 (3H, t) ; 1,63 (2H, m) ; 2,33 (6H, s) ; 2,52 (3H, s) ; 2,78-2,84 (5H, m) ; 2,91-3,24 (3H, m) ; 3,39-3,75 (3H, m) ; 4,11-4,45 (2H, m) ; 5,20-5,45 (2H, m) ; 7,27 (1H, s) ; 7,47 (2H, d) ; 7,95-8,01 (3H, m) ; 8,12 (1H, s) ; 8,25 (1H, s) ; 10,06 (1H, s) ; 11,18 (1H, br).
Composé 114 :
   RMN ¹H : DMSO-d₆ (250 MHz) : δ (ppm) : 0,94 (3H, t) ; 1,64 (2H, m) ; 2,43 (3H, s) ; 2,52 (3H, s) ; 2,78-2,81 (5H, m) ; 2,93-3,24 (3H, m) ; 3,41-3,64 (3H, m) ; 4,15-4,46 (2H, m) ; 5,29 (1H, d) ; 5,43 (1H, d) ; 7,06 (1H, d) ; 7,37 (1H, d) ; 7,48 (2H, d) ; 7,97 (2H, d) ; 8,02 (1H, s) ; 8,17 (1H, s) ; 8,24 (1H, s) ; 10,06 (1H, s) ; 10,60 (1H, br).
Composé 115 :
   RMN ¹H : DMSO-d₆ (250 MHz) : δ (ppm) : 0,94 (3H, t) ; 1,64 (2H, m) ; 2,52 (3H, s) ; 2,78-2,89 (5H, m) ; 2,93-3,24 (3H, m) ; 3,39-3,69 (3H, m) ; 4,20 (1H, s) ; 4,39 (1H, s) ; 5,35 (1H, d) ; 5,50 (1H, d) ; 7,28 (1H, d) ; 7,49 (2H, d) ; 7,56 (1H, d) ; 7,96 (2H, d) ; 8,22 (1H, s) ; 8,25 (1H, s) ; 8,31 (1H, s) ; 10,21 (1H, s) ; 10,95 (1H, br).
Composé 119 :
   RMN ¹H : DMSO-d₆ (250 MHz) : δ (ppm) : 0,94 (3H, t) ; 1,64 (2H, m) ; 2,43 (3H, s) ; 2,52 (3H, s) ; 2,82 (2H, t) ; 2,97-3,28 (3H, m) ; 3,34 (3H, s) ; 3,35-3,41 (2H, m) ; 3,50-3,77 (6H, m) ; 4,20 (1H, d) ; 4,38 (1H, d) ; 5,29 (1H, d) ; 5,42 (1H, d) ; 7,06 (1H, d) ; 7,39 (1H, d) ; 7,48 (2H, d) ; 7,98 (2H, d) ; 8,03 (1H, s) ; 8,20 (1H, s) ; 8,24 (1H, s) ; 10,08 (1H, s) ; 10,80 (1H, br).
Composé 128 :
   RMN ¹H : DMSO-d₆ (250 MHz) : δ (ppm) : 0,94 (3H, t) ; 1,64 (2H, m) ; 2,43 (3H, s) ; 2,52 (3H, s) ; 2,82 (2H, t) ; 3,48-3,70 (8H, m) ; 5,31 (2H, s) ; 7,06 (1H, d) ; 7,37 (1H, d) ; 7,48 (2H, d) ; 7,97 (2H, d) ; 8,02 (1H, s) ; 8,20 (1H, s) ; 8,24 (1H, s) ; 10,01 (1H, s).
Composé 136 :
   RMN ¹H : DMSO-d₆ (250 MHz) : δ (ppm) : 0,94 (3H, t) ; 1,64 (2H, m) ; 2,52 (3H, s) ; 2,78-3,25 (8H, m) ; 3,40-3,68 (3H, m) ; 3,80 (3H, s) ; 4,13-4,49 (2H, m) ; 5,28 (1H, d) ; 5,43 (1H, d) ; 6,87 (1H, d) ; 7,40 (1H, d) ; 7,48 (2H, d) ; 7,74 (1H, s) ; 7,97 (2H, d) ; 8,20 (1H, s) ; 8,25 (1H, s) ; 10,07 (1H, s) ; 10,70 (1H, br).
Composé 137 :
   RMN ¹H : DMSO-d₆ (250 MHz) : δ (ppm) : 0,94 (3H, t) ; 1,11 (2H, d) ; 1,34 (1H, d) ; 1,63 (2H, m) ; 1,90-2,20 (2H, m) ; 2,44 (3H, s) ; 2,52 (3H, s) ; 2,82 (2H, t) ; 3,10-4,55 (10H, m) ; 5,15-5,60 (2H, m) ; 7,07 (1H, d) ; 7,37-7,48 (3H, m) ; 7,98-8,03 (3H, m) ; 8,18-8,25 (2H, m) ; 10,05 (1H, s) ; 10,30-11,50 (1H, m).
Composé 142 :
   RMN ¹H : DMSO-d₆ (250 MHz) : δ (ppm) : 0,94 (3H,t) ; 1,64 (2H,m) ; 2,52 (3H,s) ; 2,82 (2H,t) ;3,01-3,77 (8H,m) ;4,13-4,48 (2H,m) ; 4,81-5,03 (2H,m) ; 5,36 (1H,d) ; 5,51 (1H,d) ; 7,28 (1H,d) ; 7,50 (2H,d) ; 7,56 (1H,d) ; 7,96 (2H,d) ; 8,21 (1H,s) ; 8,25 (1H,s) ; 8,30 (1H,s) ; 10,20 (1H, s) ; 10,90 (1H, br).
Composé 145 :
   RMN ¹H : DMSO-d₆ (250 MHz) : δ (ppm) : 0,94 (3H, t) ; 1,64 (2H, m) ; 2,52 (3H, s) ; 2,82 (2H, t) ; 3,06 (1H, m) ; 3,10-3,78 (14H, m) ; 3,86 (2H, m) ; 4,19 (1H, d) ; 4,37 (1H, d) ; 5,36 (1H, d) ; 5,51 (1H, d) ; 7,27 (1H, d) ; 7,49 (2H, d) ; 7,58 (1H, d) ; 7,98 (2H, d) ; 8,22 (1H, s) ; 8,25 (1H, s) ; 8,36 (1H, s) ; 10,24 (1H, s) ; 11,03 (1H, br).
Composé 146 :
   RMN ¹H : DMSO-d₆ (250 MHz) : δ (ppm) : 0,94 (3H, t) ; 1,63 (2H, m) ; 2,52 (3H, s) ; 2,82 (2H, t) ; 2,98-3,31 (5H, m) ; 3,52-3,86 (5H, m) ; 4,15-4,42 (2H, m) ; 5,37 (1H, d) ; 5,50 (1H, d) ; 7,28 (1H, d) ; 7,49 (2H, d) ; 7,57 (1H, d) ; 7,97 (2H, d) ; 8,22 (1H, s) ; 8,25 (1H, s) ; 8,34 (1H, s) ; 10,22 (1H, s) ; 10,53 (1H, br).
Composé 147 :
   RMN ¹H : DMSO-d₆ (250 MHz) : δ (ppm) : 0,94 (3H, t) ; 1,64 (2H, m) ; 2,43 (3H, s) ; 2,52 (3H,s) ; 2,81 (2H, t) ; 2,90-3,70 (6H, m) ; 4,21 (2H, d) ; 4,40 (2H, s) ; 5,27 (1H, d) ; 5,41 (1H, d) ; 7,05 (1H, d) ; 7,31-7,39 (3H, m) ; 7,47 (2H, d) ; 7,66 (1H, d) ; 7,96 (2H, d) ; 8,02 (1H, s) ; 8,15 (1H, s) ; 8,24 (1H, s) ; 10,04 (1H, s) ; 10,90 (1H, br).
Composé 148 :
   RMN ¹H : DMSO-d₆ (250 MHz) : δ (ppm) : 0,94 (3H, t) ; 1,63 (2H, m) ; 2,52 (3H, s) ; 2,82 (2H, t) ; 2,88-4,70 (15H, m) ; 5,39 (1H, br) ; 5,49 (1H, br) ; 7,28 (1H, d) ; 7,49 (2H, d) ; 7,57 (1H, d) ; 7,97 (2H, d) ; 8,22 (1H, s) ; 8,25 (1H, s) ; 8,36 (1H, s) ; 10,20 (1H, s) ; 11,64 (1H, br).
Composé 154 :
   RMN ¹H : DMSO-d₆ (250 MHz) : δ (ppm) : 0,94 (3H, t) ; 1,64 (2H, m) ; 2,52 (3H, s) ; 2,82 (2H, t) ; 2,95 (6H, d) ; 3,01-3,29 (3H, m) ; 3,47-3,80 (3H, m) ; 4,10-4,46 (4H, m) ; 5,38 (1H, s) ; 5,49 (1H, s) ; 7,28 (1H, d) ; 7,50 (2H, d) ; 7,56 (1H, d) ; 7,96 (2H, d) ; 8,22 (1H, s) ; 8,25 (1H, s) ; 8,31 (1H, s) ; 10,09-10,24 (2H, m).
Composé 157 :
   RMN ¹H : DMSO-d₆ (250 MHz) : δ (ppm) : 0,94 (3H, t) ; 1,63 (2H, m) ; 2,52 (3H, s) ; 2,80-3,70 (11H, m) ; 4,15 (1H, br) ; 4,39 (1H, br) ; 5,33 (1H, br) ; 5,48 (1H, br) ; 7,50 (2H, d) ; 7,73 (1H, d) ; 7,96 (2H, d) ; 8,25 (1H, s) ; 8,30 (1H, d) ; 8,34 (1H, s) ; 10,25 (1H, s) ; 10,95 (1H, br).
Composé 158 :
   RMN ¹H : DMSO-d₆ (250 MHz) : δ (ppm) : 0,94 (3H, t) ; 1,63 (2H, m) ; 2,52 (3H, s) ; 2,82 (2H, t) ; 2,91-3,84 (13H, m) ; 4,15 (1H, d) ; 4,37 (1H, d) ; 5,33 (1H, d) ; 5,49 (1H, d) ; 7,49 (2H, d) ; 7,74 (1H, d) ; 7,98 (2H, d) ; 8,25 (1H, s) ; 8,31 (1H, d) ; 8,34 (1H, s) ; 10,29 (1H, s) ; 11,02 (1H, br).
Composé 159 :
   RMN ¹H : DMSO-d₆ (250 MHz) : δ (ppm) : 0,94 (3H, t) ; 1,29 (3H, d) ; 1,35 (3H, d) ; 1,64 (2H, m) ; 2,52 (3H, s) ; 2,69-2,85 (3H, m) ; 3,11-3,68 (3H, m) ; 4,15 (1H, d) ; 4,42 (1H, d) ; 5,35 (1H, d) ; 5,54 (1H, d) ; 7,27 (1H, d) ; 7,49 (2H, d) ; 7,58 (1H, d) ; 7,98 (2H, d) ; 8,21 (1H, s) ; 8,24 (1H, s) ; 8,34 (1H, s) ; 9,22-9,46 (1H, m) ; 9,60-9,79 (1H, m) ; 10,22 (1H, br).
Composé 165 :
   RMN ¹H : DMSO-d₆ (250 MHz) : δ (ppm) : 0,94 (3H, t) ; 1,64 (2H, m) ; 2,52 (3H, s) ; 2,82 (2H, t) ; 2,90-3,23 (3H, m) ; 3,44-3,73 (2H, m) ; 4,19 (1H, d) ; 4,41 (3H, s) ; 5,34 (1H, d) ; 5,49 (1H, d) ; 7,27 (1H, d) ; 7,36 (2H, t) ; 7,49 (2H, d) ; 7,55 (1H,d) ; 7,68 (2H,s) ; 7,96 (2H, d) ; 8,21 (1H, s) ; 8,25 (1H, s) ; 8,30 (1H, s) ; 10,19 (1H, s) ; 11,08 (1H, br).
Composé 166 :
   RMN ¹H : DMSO-d₆ (250 MHz) : δ (ppm) : 0,94 (3H, t) ; 1,64 (2H, m) ; 2,48 (3H, s) ; 2,52 (3H, s) ; 2,82 (2H, t) ; 2,88-3,35 (4H, m) ; 3,63-4,78 (6H, m) ; 5,39 (1H,s) ; 5,44 (1H, s) ; 6,61 (1H, s) ; 7,27 (1H, d) ; 7,49 (2H, d) ; 7,56 (1H, d) ; 7,97 (2H, d) ; 8,21 (1H, s) ; 10,20 (1H, s) ; 11,83 (1H, br).
Composé 177 :
   RMN ¹H : DMSO-d₆ (250 MHz) : δ (ppm) : 0,94 (3H, t) ; 1,34 (2H, d) ; 1,56 (1H, d) ; 1,63 (2H, m) ; 2,52 (3H, s) ; 2,77-2,87 (6H, m) ; 3,13 (1H, br) ; 3,35-3,74 (3H, m) ; 4,11 (0,6H, d) ; 4,41 (0,39H, d) ; 4,59 (0,39H, br) ; 4,74 (0,61H, br) ; 5,22-5,65 (2H, m) ; 7,28 (1H, d) ; 7,45-7,58 (3H, m) ; 7,97 (2H, d) ; 8,20-8,35 (3H, m) ; 10,21 (1H, s) ; 10,68 (1H, br).
Composé 179 :
   RMN ¹H : DMSO-d₆ (250 MHz) : δ (ppm) : 0,94 (3H, t) ; 1,33 (3H, d) ; 1,64 (2H, m) ; 2,52 (3H, s) ; 2,82 (2H, t) ; 3,00-3,79 (12H, m) ; 4,18 (1H, d) ; 4,42 (1H, d) ; 5,36 (1H, d) ; 5,49 (1H, d) ; 7,28 (1H, d) ; 7,49 (2H, d) ; 7,58 (1H, d) ; 7,97 (2H, d) ; 8,21 (1H, s) ; 8,24 (1H, s) ; 8,38 (1H, s) ; 10,21 (1H, s) ; 10,62 (1H, br).
Composé 180 :
   RMN ¹H : DMSO-d₆ (250 MHz) : δ (ppm) : 0,94 (3H, t) ; 1,38 (6H, s) ; 1,63 (2H, m) ; 2,52 (3H, s) ; 2,81 (2H, t) ; 2,96-3,09 (1H, m) ; 3,19-3,30 (2H, m) ; 3,37 (3H, s) ; 3,47-3,61 (4H, m) ; 3,72-3,84 (1H, m) ; 4,16 (1H, d) ; 4,42 (1H, d) ; 5,37 (1H, d) ; 5,47 (1H, d) ; 7,27 (1H, d) ; 7,49 (2H, d) ; 7,59 (1H, d) ; 7,98 (2H, d) ; 8,22 (1H, s) ; 8,24 (1H, s) ; 8,35 (1H, s) ; 10,22 (1H, s) ; 10,44 (1H, br).
Composé 181 :
   RMN ¹H : DMSO-d₆ (250 MHz) : δ (ppm) : 0,94 (3H, t) ; 1,33 (6H, s) ; 1,63 (2H, m) ; 2,52 (3H, s) ; 2,81 (2H, t) ; 2,97-3,10 (1H, m) ; 3,20-3,30 (2H, m) ; 3,50-3,64 (4H, m) ; 3,73-3,84 (1H, m) ; 4,18 (1H, d) ; 4,43 (1H, d) ; 5,38 (1H, d) ; 5,48 (1H, d) ; 5,80 (1H, br) ; 7,27 (1H, d) ; 7,49 (2H, d) ; 7,59 (1H, d) ; 7,98 (2H, d) ; 8,22 (1H, s) ; 8,24 (1H, s) ; 8,36 (1H, s) ; 10,13 (1H, br) ; 10,24 (1H, s).
Composé 192 :
   RMN ¹H : DMSO-d₆ (250 MHz) : δ (ppm) : 0,94 (3H, t) ; 1,64 (2H, m) ; 1,88-2,30 (4H, m) ; 2,52 (3H,s) ; 2,76-2,85 (5H, m) ; 3,08 (1H, t) ; 3,36-3,57 (3H, m) ; 4,65 (1H, s) ; 4,72 (1H, s) ; 5,31 (1H, d) ; 5,42 (1H, d) ; 7,29 (1H, d) ; 7,49 (2H, d) ; 7,60 (1H, d) ; 7,97 (2H, d) ; 8,22 (1H, s) ; 8,25 (1H, s) ; 8,36 (1H, s) ; 10,15 (1H, br) ; 10,19 (1H, s).
Composé 196 :
   RMN ¹H : DMSO-d₆ (250 MHz) : δ (ppm) : 0,95 (3H, t) ; 1,64 (2H, m) ; 1,76 (1H, m) ; 1,91 (1H, m) ; 2,24 (1H, m) ; 2,55 (3H, s) ; 2,85 (2H, t) ; 2,88 (1H, m) ; 2,95 (2H, m) ; 3,27 (3H, s) ; 3,42-3,72 (5H, m) ; 3,89-4,07 (1H, br) ; 4,23 (1H, br) ; 4,48 (1H, br) ; 5,50 (2H, br) ; 7,34 (1H, br) ; 7,56 (2H, d) ; 7,63 (1H, d) ; 8,02 (2H, d) ; 8,28 (1H, d) ; 8,32 (1H, s) ; 8,35 (1H, s) ; 10,23 (2H, s br).
Composé 199 :
   RMN ¹H : DMSO-d₆ (250 MHz) : δ (ppm) : 0,94 (3H, t) ; 1,64 (2H, m) ; 2,52 (3H, s) ; 2,82 (2H, t) ; 2,98-4,48 (10H, m) ; 5,38 (1H, s) ; 5,46 (1H, s) ; 7,28 (1H, d) ; 7,49 (2H, d) ; 7,56 (1H, d) ; 7,74 (1H, s) ; 7,97 (2H, d) ; 8,02 (1H, s) ; 8,21 (1H, s) ; 8,24 (1H, s) ; 8,31 (1H, s) ; 10,20 (1H, s) ; 10,31 (1H, br).
Composé 200 :
   RMN ¹H : DMSO-d₆ (250 MHz) : δ (ppm) : 0,94 (3H, t) ; 1,64 (2H, m) ; 2,53 (3H, s) ; 2,82 (2H, t) ; 2,89 (1,65H, s) ; 3,18 (1,35H, s) ; 3,26 (1,35H, s) ; 3,41 (1,65H, s) ; 3,47 (2H, m) ; 3,64 (2H, m) ; 5,34 (2H, s) ; 7,27 (1H, m) ; 7,43 (0,55H, d) ; 7,49 (2H, d) ; 7,54 (0,45H, d) ; 7,96 (2H, d) ; 8,21 (1H, s) ; 8,24 (1H, s) ; 8,30 (1H, s) ; 10,11 (0,55H, s) ; 10,13 (0,45H, s).
Composé 203 :
   RMN ¹H : DMSO-d₆ (250 MHz) : δ (ppm) : 0,94 (3H, t) ; 1,64 (2H, m) ; 2,53 (3H, s) ; 2,79-3,25 (8H, m) ; 3,40-3,69 (3H, m) ; 4,10-4,50 (2H, m) ; 5,43 (1H, d) ; 5,58 (1H, d) ; 7,50 (2H, d) ; 7,57 (1H, d) ; 7,75 (1H, d) ; 7,98 (2H, d) ; 8,25 (1H, s) ; 8,41 (1H, s) ; 8,59 (1H, s) ; 10,29 (1H, s) ; 10,49 (1H, br).
Composé 209 :
   RMN ¹H : DMSO-d₆ (250 MHz) : δ (ppm) : 0,93 (3H, t) ; 1,63 (2H, m) ; 2,52 (3H, s) ; 2,82 (2H, t) ; 3,05-3,75 (11H, m) ; 4,32 (1H, br) ; 4,83 (1H, br) ; 5,49 (2H, br) ; 7,33 (1H, d) ; 7,55 (2H, d) ; 7,61 (1H, d) ; 8,01 (2H, d) ; 8,27 (1H, d) ; 8,31 (1H, s) ; 8,36 (1H, s) ; 10,23 (1H, s) ; 10,68 (1H, br).
Composé 212 :TLC.
   RMN ¹H : DMSO-d₆ (250 MHz) : δ (ppm) : 0,94 (3H, t) ; 1,64 (2H, m) ; 2,53 (3H, s) ; 2,55-4,20 (13H, m) ; 5,20-5,60 (2H, m) ; 7,26 (1H, d) ; 7,45-7,55 (3H, m) ; 7,96 (2H, d) ; 8,22 (1H, s) ; 8,24 (1H, s) ; 8,32 (1H, s) ; 10,15 (1H, s).
Composé 219 :
   RMN ¹H : DMSO-d₆ (250 MHz) : δ (ppm) : 0,94 (3H, t) ; 1,64 (2H, m) ; 2,23 (3H, s) ; 2,30 (2H, m) ; 2,44 (2H, m) ; 2,58 (3H, s) ; 2,84 (2H, t) ; 3,48 (2H, m) ; 3,59 (2H, m) ; 5,40 (2H, s) ; 7,28 (1H, d) ; 7,56 (1H, d) ; 7,81 (1H, d) ; 7,90 (1H, d) ; 8,11 (1H, s) ; 8,16 (1H, s) ; 8,28 (1H, s) ; 8,32 (1H, s) ; 10,37 (1H, s).
Composé 221 :
   RMN ¹H : DMSO-d₆ (250 MHz) : δ (ppm) : 0,95 (3H, t) ; 1,64 (2H, m) ; 2,24 (3H, s) ; 2,32 (2H, m) ; 2,45 (2H, m) ; 2,55 (3H, s) ; 2,83 (2H, t) ; 3,48 (2H, m) ; 3,58 (2H, m) ; 5,43 (2H, s) ; 7,30 (1H, d) ; 7,56 (1H, d) ; 7,85 (1H, d) ; 8,08 (1H, s) ; 8,12 (1H, s) ; 8,22 (1H, s) ; 8,29 (1H, s) ; 8,76 (1H, d) ; 11,25 (1H, s).

### EXEMPLE 12 : Composé n° 242 :

### Chlorhydrate de N-[4-(4-butyryl-5-méthyl-1H-pyrazol-1-yl)phényl]-5-méthyl-1-[2-(3-méthylpipérazin-1-yl)-2-oxoéthyl]-1H-indole-3-carboxamide.

A 1,05 g du composé n°224 dans 20 ml d'un mélange AcOEt/MeOH (50/50 ; v/v), on ajoute 8,19 ml d'une solution d'HCl 4N dans le dioxane. Après 20 heures à TA, on filtre le précipité, le triture à l'acétone puis filtre pour obtenir 0,77 g du composé attendu.

RMN ¹H : DMSO-d₆ (250 MHz) : δ (ppm) : 0,94 (3H, t) ; 1,22-1,39 (3H, m) ; 1,64 (2H, m) ; 2,43 (3H ,s) ; 2,52 (3H, s) ; 2,81 (2H, t) ; 2,85-3,75 (5H, m) ; 4,02-4,39 (2H, m) ; 5,22-5,50 (2H, m) ; 7,06 (1H, d) ; 7,40 (1H, d) ; 7,48 (2H, d) ; 7,99 (2H, d) ; 8,03 (1H, s) ; 8,22 (1H, s) ; 8,24 (1H, s) ; 9,44-9,74 (2H, m) ; 10,09 (1H, s).

MH⁺ = 541 ; tr = 6,33 min (Méthode G).

F = 223°C.

En procédant selon le mode opératoire décrit à l'Exemple 12 et à partir des composés n° 224 à 241 N-protégés, on prépare les composés de formule (I) rassemblés dans le TABLEAU XIV ci-après :

Dans ce tableau :
- dans la colonne "Sel", "-" représente un composé sous forme de base libre, alors que "HCl" représente un composé sous forme de chlorhydrate, "TFA" représente un composé sous forme de trifluoroacétate ;
- Me représente un radical méthyle ;

**TABLEAU XIV**

| | | | | |
|---|---|---|---|---|
| 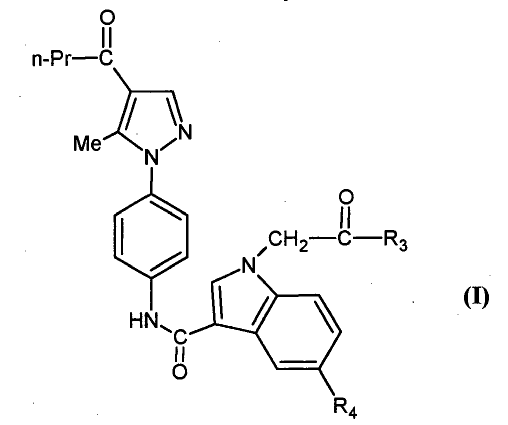 | | | | |

| Composés n° | R₄ | R₃ | Sel, Hydrate F°C MH⁺; tr (min) Méthode | Inhibition de l'agrégation plaquettaire *in vitro* (sang rat) en µM |
|---|---|---|---|---|
| 242 | Me | 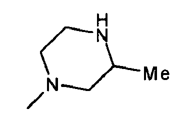 | HCl, | 0.51 |
| | | | 223 | |
| | | | 541 ; 6,33 | |
| | | | G | |
| 243 | Me | 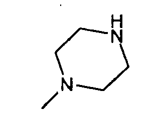 | HCl, 1,7 H₂O | 0.54 |
| | | | 195 | |
| | | | 527 ; 6,27 | |
| | | | G | |
| 244 | Me | 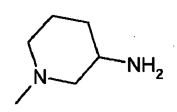 | HCl, 3,7 H₂O G | 0.96 |
| | | | 208 | |
| | | | 541 ; 5.85 | |
| | | | G | |
| 245 | Me | 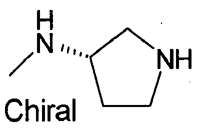 | HCl, 1,3 H₂O | 0.53 |
| | | | 180 | |
| | | | 527 ; 6.25 | |
| | | | G | |
| 246 | Me | 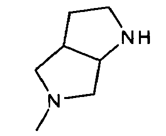 | HCl, 1,4 H₂O | 0.85 |
| | | | 186 | |
| | | | 553 ; 6.27 | |
| | | | G | |
| 247 | Me | 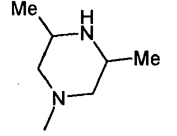 | HCl, 2,5 H₂O | 0.26 |
| | | | 196 | |
| | | | 555 ; 6.41 | |
| | | | G | |
| 248 | Me | 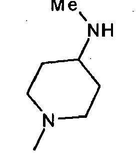 | HCl,1 H₂O | 0.32 |
| | | | 199 | |
| | | | 555 ; 6.30 | |
| | | | G | |
| 249 | Me | 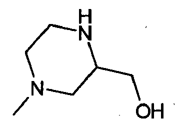 | HCl, 2 H₂O | 0.24 |
| | | | 213 | |
| | | | 557 ; 6.27 | |
| | | | G | |
| 250 | Me | 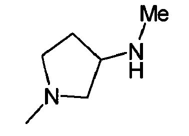 | HCl | 0.61 |
| | | | 175 | |
| | | | 541; 6.27 | |
| | | | G | |
| 251 | Me | 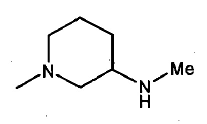 | HCl, H₂O | 0.54 |
| | | | 196 | |
| | | | 555 ; 6.36 | |
| | | | G | |
| 252 | Me | 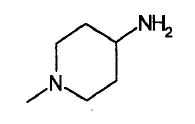 | HCl, 1,5 H₂O | 0.40 |
| | | | 222 | |
| | | | 541 ; 6.28 | |
| | | | G | |
| 253 | Me | 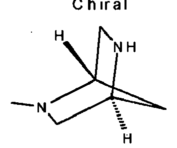 | HCl, 2,3 H₂O | 0.33 |
| | | | 223 | |
| | | | 539 ; 6.25 | |
| | | | G | |
| 254 | Me | 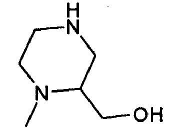 | HCl, 3 H₂O | 0.19 |
| | | | 225 | |
| | | | 557 ; 6.23 | |
| | | | G | |
| 255 | Me | 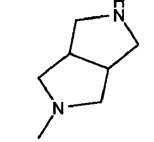 | HCl, 1,8 H₂O H | 0.18 |
| | | | 230 | |
| | | | 553 ; 11.44 | |
| | | | H | |
| 256 | Cl | 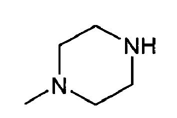 | HCl, 2,6 H₂O | 0.23 |
| | | | 258 | |
| | | | 547 ; 5.86 | |
| | | | G | |
| 257 | Cl | 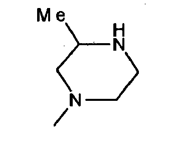 | - | 0.46 |
| | | | - | |
| | | | 561 ; 3,24 | |
| | | | A | |
| 258 | Cl | 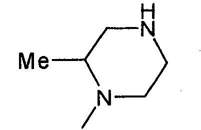 | - | 0.30 |
| | | | - | |
| | | | 561 ; 3,20 | |
| | | | A | |
| 259 | Cl | 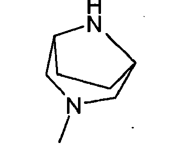 | HCl, H₂O | 0.45 |
| | | | 219 | |
| | | | 573 ; 6,52 | |
| | | | G | |
| 260 | Me | 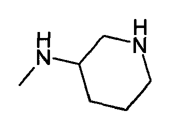 | - | / |
| | | | - | |
| | | | 541 ; 6,33 | |
| | | | G | |

Les analyses effectuées par RMN pour certains composés sont données ci-après :
Composé 256 :
   RMN ¹H : DMSO-d₆ (250 MHz) : δ (ppm) : 0,94 (3H, t) ; 1,63 (2H, m) ; 2,52 (3H, s) ; 2,82 (2H, t) ; 3,11 (2H, br) ; 3,27 (2H, br) ; 3,64-3,84 (4H, m) ; 5,42 (2H, s) ; 7,27 (1H, d) ; 7,49 (2H, d) ; 7,58 (1H, d) ; 7,97 (2H, d) ; 8,21 (1H, s) ; 8,25 (1H, s) ; 8,33 (1H, s) ; 9,32 (2H, br) ; 10,21 (1H, s).
Composé 259 :
   RMN ¹H : DMSO-d₆ (250 MHz) : δ (ppm) : 0,94 (3H, t) ; 1,64 (2H, m) ; 1,86-2,17 (4H, m) ; 2,52 (3H, s) ; 2,82 (2H, t) ; 3,10 (1H, d) ; 3,67 (1H, d) ; 3,92 (1H, d) ; 4,02-4,20 (3H, m) ; 5,30 (2H, d) ; 5,58 (2H, d) ; 7,28 (1H, d) ; 7,49 (2H, d) ; 7,56 (1H, d) ; 7,96 (2H, d) ; 8,21 (1H, s) ; 8,25 (1H, s) ; 8,33 (1H, s) ; 9,18 (2H, br) ; 10,16 (1H, s).

### EXEMPLE 13 : Composé n° 261 Chlorhydrate de N-[4-(4-butyryl-5-méthyl-1H-pyrazol-1-yl)phényl]-5-chloro-1-{2-[4-(2-méthoxyéthyl)-3,5-diméthylpipérazin-1-yl]-2-oxoéthyl}-1H-indole-3-carboxamide

A 0,4 g du composé n° 159 dans 5 ml de DMF, on ajoute 0,12 g de carbonate de potassium, 0,13 g d'iodure de sodium puis 0,080 ml de 1-bromo-2-méthoxyéthane. Le milieu réactionnel est placé dans un appareil à microondes à 160°C pendant 40 min (300 W). Après retour à TA, on verse sur 50 ml d'eau et extrait à l'AcOEt. Les phases organiques rassemblées sont lavées à l'eau, séchées sur Na₂SO₄ et évaporées à sec. On purifie le résidu solide par chromatographie sur gel de silice en éluant avec un mélange de DCM/MeOH (gradient de 3 à 5 % de MeOH) pour obtenir 0,12 g de poudre. Le produit est solubilisé dans 4 ml d'un mélange DCM/MeOH (1 /1 ; v/v) puis on ajoute 0,38 ml d'une solution d'HCl 1M dans l'éther. On précipite le chlorhydrate avec 10 ml d'éther puis filtre pour obtenir 0,103 g d'une poudre blanche.

RMN ¹H : DMSO-d₆ (250 MHz) : δ (ppm) : 0,94 (3H, t) ; 1,36 (3H, d) ; 1,43 (3H, d) ; 1,63 (2H, m) ; 2,52 (3H, s) ; 2,81 (2H, t) ; 3,05 (1H, t) ; 3,35 (3H, s) ; 3,38-3,77 (7H, m) ; 4,19 (1H, d) ; 4,41 (1H, d) ; 5,33 (1H, d) ; 5,55 (1H, d) ; 7,28 (1H, d) ; 7,49 (2H, d) ; 7,58 (1H, d) ; 7,97 (2H, d) ; 8,22 (1H, s) ; 8,24 (1H, s) ; 8,34 (1H, s) ; 10,21 (1H, s) ; 10,97 (1H, br),

MH⁺ = 633 ; tr = 6,25 min (Méthode G).

F = 187°C.

### EXEMPLE 14 : Composé n° 262

### Chlorhydrate de N-[4-(4-butyryl-5-méthyl-1H-pyrazol-1-yl)phényl]-5-chloro-1-[2-oxo-2-(3,4,5-triméthylpipérazin-1-yl)éthyl]-1H-indole-3-carboxamide.

A 0,4 g du composé n° 159 dans 5 ml de DMF, on ajoute 0,12 g de carbonate de potassium, puis 0,053 ml d'iodométhane. Après 20 h à TA, on ajoute de l'eau et extrait à l'AcOEt. La phase organique est lavée à l'eau, séchée sur Na₂SO₄ puis évaporée à sec. On purifie le résidu solide par chromatographie sur gel de silice en éluant avec un mélange de DCM/MeOH (95/5 ; v/v) pour obtenir 0,17 g de poudre. Le produit est solubilisé dans 5 ml d'acétone puis on ajoute 1 ml d'une solution d'HCl 1M dans l'éther. On précipite le chlorhydrate avec 10 ml de diéthyléther puis filtre pour obtenir 0,095 g d'une poudre blanche.

RMN ¹H : DMSO-d₆ (250 MHz) : δ (ppm) : 0,94 (3H, t) ; 1,38 (3H, d) ; 1,45 (3H, d) ; 1,63 (2H, m) ; 2,52 (3H, s) ; 2,79-2,85 (5H, m) ; 2,97 (1H, t) ; 3,22-3,52 (3H, m) ; 4,21 (1H, d) ; 4,41 (1H, d) ; 5,33 (1H, d) ; 5,55 (1H, d) ; 7,28 (1H, d) ; 7,49 (2H, d) ; 7,57 (1H, d) ; 7,97 (2H, d) ; 8,22 (1H, s) ; 8,25 (1H, s) ; 8,33 (1H, s) ; 10,21 (1H, s) ; 10,85 (1H, br),

MH⁺ = 589 ; tr = 6,12 min (Méthode G).

F = 217°C.

En procédant selon le mode opératoire décrit à l'Exemple 14, on prépare les composés de formule (I) rassemblés dans le TABLEAU XV ci-après :

Dans ce tableau :
- dans la colonne "Sel", "Base" représente un composé sous forme de base libre, alors que "HCl" représente un composé sous forme de chlorhydrate, "TFA" représente un composé sous forme de trifluoroacétate ;
- Me représente un radical méthyle ;

**TABLEAU XV**

| | | | | |
|---|---|---|---|---|
| 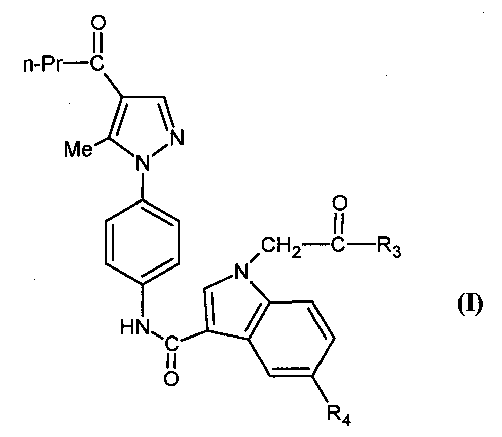 | | | | |

| Composés n° | R₄ | R₃ | Sel, Hydrate F°C + MH ; tr (min) Méthode | Inhibition de l'agrégation plaquettaire *in vitro* (sang rat) en µM |
|---|---|---|---|---|
| 263 | Me | 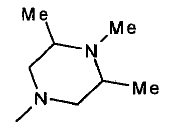 | HCl, 2 H₂O | 0.42 |
| | | | 195 | |
| | | | 569 ; 6.44 | |
| | | | G | |
| 264 | Me | 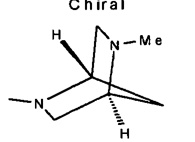 | HCl, 1.7 H₂O | 0.56 |
| | | | 217 | |
| | | | 553 ; 5.71 | |
| | | | G | |
| 265 | Me | 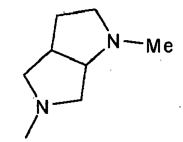 | HCl, 1.1 H₂O | 0.79 |
| | | | 205 | |
| | | | 567 ; 6.29 | |
| | | | G | |
| 266 | Me | 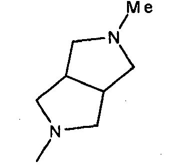 | HCl, 2.7 H₂O G | 0.40 |
| | | | 260 | |
| | | | 567 ; 6.18 | |
| | | | G | |
| 267 | Me | 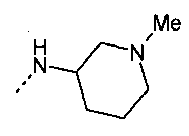 | HCl, 1.5 H₂O | 0.45 |
| | | | 197 | |
| | | | 555 ; 5.79 | |
| | | | G | |
| 268 | Me | 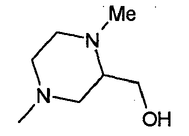 | HCl, 2 H₂O G | 0.24 |
| | | | 234 | |
| | | | 571 ; 6.27 | |
| | | | G | |
| 269 | Me | 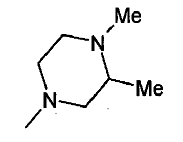 | HCl, 1.7 H₂O | 0.35 |
| | | | 198 | |
| | | | 555 ; 6.37 | |
| | | | G | |

Les analyses effectuées par RMN pour certains composés sont données ci-après :
Composé 263 :
   RMN ¹H : DMSO-d₆ (250 MHz) : δ (ppm) : 0,94 (3H, t) ; 1,36 (3H, d) ; 1,58-1,68 (5H, m) ; 2,43 (3H, s) ; 2,52 (3H, s) ; 2,82 (2H, t) ; 2,88 (3H, s) ; 3,06-3,30 (2H, m) ; 3,42-3,59 (2H, m) ; 4,56-4,75 (2H, m) ; 5,18 (1H, d) ; 5,56 (1H, d) ; 7,07 (1H, d) ; 7,31 (1H, d) ; 7,48 (2H, d) ; 7,98 (2H, d) ; 8,03 (1H, s) ; 8,25 (2H, s) ; 10,07 (1H, s) ; 10,56 (1H, br).
Composé 268 :
   RMN ¹H : DMSO-d₆ (250 MHz) : δ (ppm) : 0,94 (3H, t) ; 1,64 (2H, m) ; 2,43 (3H, s) ; 2,52 (3H, s) ; 2,82 (2H, t) ; 2,89 (3H, s) ; 3,00-4,50 (9H, m) ; 5,20-5,52 (2H, m) ; 5,70 (1H, br) ; 7,06 (1H, d) ; 7,38 (1H, d) ; 7,48 (2H, d) ; 7,97 (2H, d) ; 7,99 (1H, s) ;
   8,03 (1H, s) ; 8,21 (1H, s) ; 10,00-10,30 (2H, m).
Composé 269 :
   RMN ¹H : DMSO-d₆ (250 MHz) : δ (ppm) : 0,94 (3H, t) ; 1,37 (3H, d) ; 1,63 (2H, m) ; 2,43 (3H, s) ; 2,52 (3H, s) ; 2,74-2,85 (5H, m) ; 2,89-4,46 (7H, m) ; 5,22-5,53 (2H, m) ; 7,06 (1H, d) ; 7,38 (1H, d) ; 7,48 (2H, d) ; 7,98 (2H, d) ; 8,03 (1H, s) ; 8,19 (1H, s) ; 8,25 (1H, s) ; 10,08 (1H, s) ; 11,01 (0,8H, br) ; 11,19 (0,2H, br).

### EXEMPLE 15: Composé n° 270

### 4-[(3-{[4-(4-butyryl-5-méthyl-1H-pyrazol-1-yl)phényl]carbamoyl}-5-chloro-1H-indol-1-yl)acétyl]-1-méthylpipérazine-2-carboxylate de méthyle.

A 0,5 g du composé n° 212 dans 1 ml d'acide formique à 50°C, on ajoute 0,62 ml de formaldéhyde en solution à 37% dans l'eau et chauffe à 70°C pendant 1h30. On ajoute le mélange réactionnel à 15 ml d'une solution saturée de bicarbonate de sodium. On filtre le précipité formé, lave à l'eau puis sèche à l'étuve à vide à 50°C pour obtenir 0,49 g d'une poudre blanche.

RMN ¹H : DMSO-d₆ (250 MHz) : δ (ppm) : 0,94 (3H, t) ; 1,65 (2H, m) ; 2,20-2,45 (4H, m) ; 2,52 (3H, s) ; 2,82 (2H, t) ; 3,06-3,35 (2H, m) ; 3,40-3,85 (7H, m) ; 5,10-5,50 (2H, m) ; 7,27 (1H, d) ; 7,48-7,52 (3H, m) ; 7,95 (2H, d) ; 8,21 (1H, s) ; 8,24 (1H, s) ; 8,29 (1H, s) ; 10,15 (1H, s).

MH⁺ = 619 ; tr = 7,41 min (Méthode G)

### EXEMPLE 16: Composé n° 271 1

Acide 4-[(3-{[4-(4-butyryl-5-méthyl-1*H*-pyrazol-1-yl)phényl]carbamoyl}-5-chloro-1H-indol-1-yl)acétyl]-1-méthylpipérazine-2-carboxylique.

A 0,49 g du composé n° 270 dans 5 ml de méthanol, on ajoute 0,89 ml d'une solution de soude 1N et agite pendant 18 h. On évapore à sec, reprend le résidu solide à l'eau puis ajoute, goutte à goutte, 2 ml de HCl 1N. On filtre le précipité formé, lave à l'eau puis sèche à l'étuve à vide à 50°C pour obtenir 0,40 g d'une poudre blanche. RMN ¹H : DMSO-d₆ + acide trifluoroacétique (250 MHz) : δ (ppm) : 0.93 (3H, t) ; 1.64 (2H, m) ; 2.51 (3H, s) ; 2.80 (2H, t) ; 2.99 (3H, s) ; 3.10-4.60 (7H, m) ; 5.30-5.60 (2H, m) ; 7.26 (1H, d) ; 7.48 (2H, d) ; 7.52 (1H, d) ; 7.96 (2H, d) ; 8.21 (1H, s) ; 8.22 (1H, s) ; 8.27 (1H, s).

MH⁺ = 605 ; tr = 6,79 min (Méthode G)

### EXEMPLE 17 : Composé n° 272

N-[4-(4-butyryl-5-méthyl-1H-pyrazol-1-yl)phényl]-5-chloro-1-{2-[4-méthyl-3-(méthylcarbamoyl)pipérazin-1-yl]-2-oxoéthyl}-1H-indole-3-carboxamide

A 0,20 g du composé n° 271 dans 2 ml de THF à -10°C, on ajoute 29 µL de N-méthylmorpholine puis 25 µL de chloroformate d'éthyle et agite pendant 2 h en maintenant la température à -10°C. Après ajout de 159 µL d'une solution de méthylamine 2M dans le THF, on laisse doucement remonter à TA puis agite pendant 15 h. On évapore le milieu réactionnel puis on purifie le résidu solide par chromatographie sur gel de silice en éluant avec un mélange de DCM/MeOH (gradient de 0 à 10 % de MeOH). Le produit est solubilisé dans 5 ml d'un mélange d'acétone/MeOH (1/1 : v/v) puis on ajoute 0,4 ml d'une solution d'HCl 1M dans l'éther. On évapore à sec, triture le résidu solide à l'acétone, lave au MeOH et sèche sous vide pour obtenir 0,090 g d'une poudre blanche.

RMN ¹H : DMSO-d₆ + acide trifluoroacétique (250 MHz) : δ (ppm) : 0,94 (3H, t) ; 1,64 (2H, m) ; 2,52 (3H, s) ; 2,69 (2H, s) ; 2,77-2,87 (6H, m) ; 2,89-4,70 (7H, m) ; 5,25-5,67 (2H, m) ; 7,28 (1H, d) ; 7,45-7,59 (3H, m) ; 7,96 (2H, d) ; 8,23 (2H, s) ; 8,30 (1H, d).

MH⁺ = 618 ; tr = 6,63 min (Méthode G)

F = 274°C.

Inhibition de l'agrégation plaquettaire *in vitro* (sang rat) : 0.21 µM

### EXEMPLE 18: Composé n° 273

### Chlorhydrate de N-[4-(4-butyryl-5-méthyl-1H-pyrazol-1-yl)phényl]-5-chloro-1-[2-oxo-2-(9-oxooctahydro-2H-pyrazino[1,2-a]pyrazin-2-yl)éthyl]-1H-indole-3-carboxamide

A 1 g du composé n° 212 en suspension dans 15 ml de MeOH, on ajoute 0,34 g de *tert*-butyl (2-oxoethyl)carbamate, 0,177 g de cyanoborohydrure de sodium puis 0,5 ml d'acide acétique. Après agitation à TA pendant 20 h, on évapore à sec puis purifie le brut réactionnel par chromatographie sur gel de silice en éluant avec un mélange de DCM/MeOH (gradient de 0 à 5 % de méthanol). La poudre obtenue est solubilisée dans 2 ml d'acide trifluoroacétique puis on agite à TA pendant 4 h. On verse, le milieu réactionnel sur 30 ml d'une solution de soude 2N. Le milieu hétérogène est agité pendant 15 min puis extrait à l'AcOEt. La phase organique est lavée à l'eau, à la saumure puis évaporée à sec. On purifie le résidu solide par chromatographie sur gel de silice en éluant avec un mélange de DCM/MeOH (gradient de 2 à 10 % de méthanol). Le produit est solubilisé dans 5 ml d'un mélange d'acétone/MeOH (1/1 : v/v) puis on ajoute 1 ml d'une solution d'HCl 2N dans l'éther. On évapore à sec, recristallise dans l'acétone à chaud et filtre pour obtenir 0,38 g d'une poudre blanche.

RMN ¹H : DMSO-d₆ (250 MHz) : δ (ppm) : 0,94 (3H, t) ; 1,64 (2H, m) ; 2,52 (3H, s) ; 2,80 (2H, t) ; 3,20-4,90 (11H, m) ; 5,20-5,65 (2H, m) ; 7,20-7,35 (1H, m) ; 7,49 (2H, d) ; 7,56 (1H, d) ; 7,96 (2H, d) ; 8,22 (1H, s) ; 8,23 (1H, s) ; 8,28(1H, s).

MH⁺ = 616 ; tr = 7,74 min (Méthode G)

F = 230°C.

Inhibition de l'agrégation plaquettaire *in vitro* (sang rat) : 0.29 µM

### EXEMPLE 19 : Composé n° 274 SAR164737

### Chlorhydrate de N-[4-(4-butyryl-5-méthyl-1H-pyrazol-1-yl)phényl]-5-chloro-1-[2-(8-méthyl-9-oxooctahydro-2H-pyrazino[1,2-a]pyrazin-2-yl)-2-oxoéthyl]-1H-indole-3-carboxamide.

A 0,79 g du composé n° 212 en suspension dans 15 ml de MeOH, on ajoute 0,23 g de *tert*-butyl méthyl(2-oxoéthyl)carbamate, 0,082 g de cyanoborohydrure de sodium puis 0,4 ml d'acide acétique. Après agitation à TA pendant 20 h, on évapore à sec puis purifie le brut réactionnel par chromatographie sur gel de silice en éluant avec un mélange de DCM/MeOH (gradient de 0 à 5 % de méthanol). La poudre obtenue est solubilisée dans 2 ml d'acide trifluoroacétique puis on agite à TA pendant 4 h. On verse, le milieu réactionnel sur 30 ml d'une solution de soude 2N. Le milieu hétérogène est agité pendant 15 min puis extrait à l'AcOEt. La phase organique est lavée à l'eau, à la saumure puis évaporée à sec. On purifie le résidu solide par chromatographie colonne sur gel de silice en éluant avec un mélange de DCM/MeOH (gradient de 2 à 10 % de méthanol). Le produit est trituré à l'éther iso puis filtré pour obtenir 0,48 g d'une poudre blanche.

RMN ¹H : DMSO-d₆ (250 MHz) : δ (ppm) : 0,94 (3H, t) ; 1,64 (2H, m) ; 2,07-3,54 (18H, m) ; 3,97 (0,5H, d) ; 4,16 (0,5H, d) ; 4,25 (0,5H, d) ; 4,72 (0,5H, d) ; 5,27-5,56 (2H, m) ; 7,22-7,29 (1H, m) ; 7,49 (2H, d) ; 7,52-7,61 (1H, m) ; 7,95 (2H, d) ; 8,19-8,22 (1H, m) ; 8,24 (1H, s) ; 8,27-8,31 (1H, m).

MH⁺ = 630 ; tr = 7,96 min (Méthode G)

F = 220°C.

Inhibition de l'agrégation plaquettaire *in vitro* (sang rat) : 0.10 µM

### EXEMPLE 20 : Composé n° 275

### Chlorhydrate de 2-{4-[(3-{[4-(4-butyryl-5-méthyl-1H-pyrazol-1-yl)phényl] carbamoyl}-5-chloro-1H-indol-1-yl)acétyl]pipérazin-1-yl}éthyl acétate

A 0,40 g du composé n° 146 dans 5 ml de DCM à 0°C, on ajoute 0,27 ml de triéthylamine puis 0,14 ml de chlorure d'acétyle. Après agitation à TA pendant 1 h, on lave le milieu réactionnel à l'eau, à la saumure puis sèche sur Na₂SO₄ et évapore à sec. On purifie le résidu solide par chromatographie colonne sur gel de silice en éluant avec un mélange de DCM/MeOH (gradient de 4 à 10 % de MeOH). On solubilise le produit dans 2 ml d'un mélange d'acétone/MeOH (1/1 : v/v), ajoute 1,5 ml d'une solution d'HCl 1N dans l'éther puis verse sur 15 ml d'éther. Le précipité formé est filtré pour obtenir 0,32 g d'une poudre blanche.

RMN ¹H : DMSO-d₆ (250 MHz) : δ (ppm) : 0,94 (3H, t) ; 1,63 (2H, m) ; 2,10 (3H, s) ; 2,52 (3H, s) ; 2,82 (2H, t) ; 3,02-3,32 (3H, m) ; 3,45-3,75 (5H, m) ; 4,15-4,45 (4H, m) ; 5,36 (1H, d) ; 5,50 (1H, d) ; 7,28 (1H, d) ; 7,49 (2H, d) ; 7,57 (1H, d) ; 7,98 (2H, d) ; 8,22 (1H, s) ; 8,25 (1H, s) ; 8,32 (1H, s) ; 10,15 (1H, s) ; 11,10 (1H, br).

MH⁺ = 633 ; tr = 6,26 min (Méthode G)

F = 160°C.

Inhibition de l'agrégation plaquettaire *in vitro* (sang rat) : 0.20 µM

### EXEMPLE 21: Composé n° 276

### Chlorhydrate de N-[4-(4-butyryl-5-méthyl-1H-pyrazol-1-yl)phényl]-5-chloro-1-{2-oxo-2-[4-(2-oxopropyl)pipérazin-1-yl]éthyl}-1H-indole-3-carboxamide

A 0,25 g de composé n° 256 dans 11 ml de DMF, on ajoute 0,21 g de K₂CO₃ puis 0,057 ml de chloroacétone. Après agitation à TA pendant 48 h, on ajoute 100 ml d'AcOEt, lave à l'eau, à la saumure, sèche sur Na₂SO₄ puis évapore à sec. On purifie le résidu solide par chromatographie colonne sur gel de silice en éluant avec un mélange de DCM/MeOH (gradient de 0 à 5 % de MeOH). On solubilise le produit dans 1 ml de DCM, ajoute 0,5 ml d'une solution d'HCl 1N dans l'éther puis verse sur 15 ml d'éther. Le précipité formé est filtré pour obtenir 0,073 g d'une poudre beige.

RMN ¹H : DMSO-d₆ (250 MHz) : δ (ppm) : 0,94 (3H, t) ; 1,63 (2H, m) ; 2,23 (3H, s) ; 2,52 (3H, s) ; 2,82 (2H, t) ; 2,93-4,64 (10H, m) ; 5,38 (1H, br) ; 5,47 (1H, br) ; 7,28 (1H, d) ; 7,50 (2H, d) ; 7,57 (1H, d) ; 7,97 (2H, d) ; 8,22 (1H, s) ; 8,25 (1H, s) ; 8,33 (1H, s) ; 10,21 (1H, s) ; 10,50 (1H, br).

MH⁺ = 603 ; tr = 6,11 min (Méthode G)

F = 248°C.

Inhibition de l'agrégation plaquettaire *in vitro* (sang rat) : 0.22 µM

Les composés selon l'invention ont fait l'objet d'essais pharmacologiques.

### Inhibition de l'agrégation plaquettaire in vitro (sang humain)

Le sang est prélevé chez des volontaires sains, en utilisant des seringues de 20ml contenant 2ml de citrate de sodium tamponné. Le sang est transféré dans des tubes en polypropylène, et centrifugé pendant 5 minutes (100g) à température ambiante (sans utiliser le frein de la centrifugeuse). Le plasma riche en plaquettes (PRP) surnageant est alors prélevé, dilué, et numéré en plaquettes avant d'être utilisé dans les mesures d'agrégation.

Les mesures d'agrégation plaquettaire sont réalisées à 37°C dans des tubes en verre (agrégomètre Chrono-Log - Kordia). 4µl du composé à tester (solution 100 fois plus concentrée que la concentration finale désirée, en DMSO) sont mélangés à 392µl de PRP frais, et incubés pendant 1 minute sous agitation. Puis, 4µl d'une solution d'ADP à 250µM sont ajoutés au mélange. Les mesures d'agrégation sont suivies pendant 6 à 8 minutes, sous agitation permanente, par enregistrement des variations de la densité optique selon la méthode de G.V.R. BORN (Born Nature (1962) 194, 927).

Les résultats sont calculés en utilisant l'amplitude de l'agrégation exprimée en hauteur, et exprimés par le pourcentage d'inhibition.

Les composés selon l'invention présentent des CI₅₀ (d'inhibition de l'agrégation plaquettaire) comprises entre 0,1 et 2 µM.

### Inhibition de l'agrégation plaquettaire in vitro (sang rat)

Le sang est prélevé sur des rats mâles de souche Sprague-Dawley, pesant 250-300g. Le prélèvement sur citrate de sodium à 3.8% (1 volume pour 9 volumes de sang) est effectué par ponction à l'aorte abdominale après anesthésie de l'animal au pentobarbital sodique.

Le plasma riche en plaquettes (PRP) est obtenu par centrifugation du sang à 300g pendant 5 minutes, et les mesures d'agrégation plaquettaire sont réalisées comme décrites ci-dessus.

Les résultats sont calculés en utilisant l'aire sous la courbe d'absorbance mesurée pendant 6 minutes, et exprimés par le pourcentage d'inhibition.

Les composés selon l'invention pour lesquels R3 = NR7R8 présentent des CI50 (d'inhibition de l'agrégation plaquettaire) comprises entre 0,02 et 1.5 µM.

Les composés selon l'invention pour lesquels R3 = OH présentent des CI50 (d'inhibition de l'agrégation plaquettaire) > 1.5 µM.

Les résultats obtenus pour chaque composé sont indiqués dans les tableaux X à XV

### Inhibition de l'agrégation plaquettaire ex vivo (sang rat)

Des rats mâles de souche Sprague-Dawley, pesant 250-300g, sont utilisés à raison de 6 animaux par lot. Chaque composé à tester est dilué dans une solution d'eau glucosée (glucose 5%) contenant 5% de crémophore et 3% de glycofurol.

Les composés selon l'invention sont administrés par gavage (10ml/kg à 1mg/ml) deux heures avant le prélèvement ou par infusion (**1ml/kg à 10mg/ml**) deux heures avant le prélèvement.

Le prélèvement sur citrate de sodium à 3,8% (1 volume pour 9 volumes de sang) est effectué par ponction à l'aorte abdominale après anesthésie de l'animal au pentobarbital sodique.

Le plasma riche en plaquettes (PRP) est obtenu par centrifugation du sang à 300g pendant 5 minutes, et les mesures d'agrégation plaquettaire sont réalisées comme décrites ci-dessus.

Les résultats sont calculés en utilisant l'aire sous la courbe d'absorbance mesurée pendant 6 minutes, et exprimés par le pourcentage (%) d'inhibition.

Le tableau XVI ci-après indique les résultats obtenus pour les composés 11, 28, 29, 105, 107, 115, 119, 133, 142, 145, 147, 166, 170, 177, 179, 180, 219, 262, 274 et 276 :

**TABLEAU XVI**

| Composés n° | **Inhibition de l'agrégation plaquettaire *ex vivo* (sang rat) en %** | |
|---|---|---|
| | Infusion (Intravenous) 10 mg/kg | Gavage (oral) 10 mg/kg |
| 11 | 40.7 | / |
| 28 | 79.0 (3mg/kg) | 73.5 |
| 29 | 76.0 (1mg/kg) | 86.3 |
| 105 | / | 67.9 |
| 107 | 57.8 | 36.8 |
| 115 | 78.2 | 50.6 |
| 119 | / | 61.1 |
| 133 | / | 74.2 |
| 142 | / | 76.5 |
| 145 | / | 53.7 |
| 147 | / | 74.9 |
| 166 | / | 72.0 |
| 170 | / | 71.5 |
| 177 | / | 63.9 |
| 179 | / | 76.1 |
| 180 | / | 65.1 |
| 219 | / | 65.1 |
| 262 | / | 58.8 |
| 274 | / | 82.7 |
| 276 | / | 49.9 |

Les composés de la présente invention sont notamment des principes actifs compatibles avec leur utilisation en tant que médicaments et/ou compositions pharmaceutiques.

Selon un de ces aspects, la présente invention concerne l'utilisation d'un composé de formule (I) ou d'un de ses sels, pharmaceutiquement acceptable pour la préparation de médicaments destinés à prévenir ou à traiter toute pathologie humaine et/ou à usage vétérinaire. Ainsi les composés selon l'invention peuvent être utilisés chez l'homme ou chez l'animal (notamment chez les mammifères incluant de façon non limitative les chiens, les chats, les chevaux, les bovins, les moutons) pour la prévention ou le traitement de maladies impliquant le récepteur P2Y12.

Ils sont donc indiqués en tant qu'inhibiteurs de l'activation plaquettaire, de l'agrégation et de la dégranulation plaquettaire, en tant que promoteurs de la désagrégation plaquettaire, et comme agents anti-thrombotiques. Ils sont également indiqués dans le traitement ou la prophylaxie d'angor (angine de poitrine) instable, d'angioplastie coronarienne percutanée (PTCA), d'infarctus du myocarde, de péri-thrombolyse, des complications artérielles thrombotiques de l'athérosclérose tels que les accidents vasculaires cérébraux emboliques ou thrombotiques, les accidents ischémiques transitoires, la maladie vasculaire périphérique, l'infarctus du myocarde avec ou sans thrombolyse, les complications artérielles de l'athérosclérose dues à des interventions chirurgicales comme l'angioplastie, l'endartériectomie, la pose de stent, les greffes vasculaires coronaires et autres, les complications thrombotiques de la chirurgie ou de dommages mécaniques comme la récupération de tissus après un traumatisme accidentel ou chirurgical, la chirurgie reconstructive (y compris la peau et les lambeaux musculaires), la coagulation intravasculaire disséminée, le purpura thrombotique thrombocytopénique, le syndrome hémolytique et urémique, les complications thrombotiques de la septicémie, le syndrome de détresse respiratoire, le syndrome des anti-phospholipides, la thrombocytopénie induite par l'héparine et la pré-éclampsie/éclampsie ; ou les thromboses veineuses telles que la thrombose veineuse profonde, la maladie veino-occlusive, les conditions hématologiques telles que la maladie myélo-proliférative (y compris la thrombocytémie), la drépanocytose; ou dans la prévention de l'activation plaquettaire induite mécaniquement *in vivo,* comme lors de la dérivation cardio-pulmonaire et de l'oxygénation extracorporelle (prévention des micro-thrombo-embolismes), dans la prévention de l'activation plaquettaire induite mécaniquement *in vitro* (utilisation dans la conservation des produits sanguins - par exemple, les concentrés plaquettaires - utilisation lors de dérivations telles que la dialyse rénale et la plasmaphérèse), la thrombose secondaire à une lésion vasculaire / inflammation tels que l'angéite, l'artérite, la glomérulonéphrite, la maladie inflammatoire de l'intestin, et le rejet de greffe d'organes, les conditions telles que la migraine, le phénomène de Raynaud, les conditions dans lesquelles les plaquettes peuvent contribuer au processus de la maladie inflammatoire sous-jacente dans la paroi vasculaire tel que la formation / progression de plaques athéromateuses, la sténose / resténose, et dans d'autres pathologies inflammatoires comme l'asthme, dans lequel les plaquettes et les facteurs dérivés des plaquettes sont impliqués dans le processus de la maladie immunologique.

Les composés selon l'invention pour l'utilisation dans la prévention et/ou le traitement des maladies ci-dessus mentionnées, ainsi que pour la préparation de médicaments destinés à traiter ces maladies fait partie intégrante de l'invention.

Les composés de formule (I) ci-dessus, ou l'un de leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif

Ainsi, selon un autre de ses aspects, la présente invention concerne des compositions pharmaceutiques renfermant, en tant que principe actif, un composé de formule (I), ou l'un de ses sels pharmaceutiquement acceptables ainsi qu'un ou plusieurs excipients pharmaceutiquement acceptables.

Dans les compositions pharmaceutiques de la présente invention pour l'administration par voie orale, sublinguale, inhalée, sous-cutanée, intramusculaire, intraveineuse, topique, locale, intratrachéale, intranasale, transdermique, locale ou rectale, les principes actifs peuvent être administrés sous formes unitaires d'administration, en mélange avec des supports pharmaceutiques classiques, aux animaux et aux êtres humains.

Les formes unitaires d'administration appropriées comprennent les formes par voie orale telles que les comprimés, les gélules molles ou dures, les poudres, les granules, les solutions ou suspensions orales, les formes d'administration sublinguale, buccale, intratrachéale, intraoculaire, intranasale, par inhalation, les aérosols, les formes d'administration topique, transdermique, les implants, les formes d'administration sous-cutanée, intramusculaire, intraveineuse et les formes d'administration rectale.

Pour l'administration topique on peut utiliser les composés selon l'invention dans des crèmes, des pommades, des gels, ou des lotions.

A titre d'exemple, une forme unitaire d'administration d'un composé selon l'invention sous forme de comprimé peut comprendre les composants suivants :

| | |
|---|---|
| Composé selon l'invention : | 50,0 mg |
| Mannitol : | 223,75 mg |
| Croscarmellose sodique : | 6,0 mg |
| Amidon de maïs : | 15,0 mg |
| Hydroxypropyl-méthylcellulose : | 2,25 mg |
| Stéarate de magnésium : | 3,0 mg |

Par voie orale, la dose de principe actif administrée par jour peut atteindre 0,01 à 100 mg/kg, en une ou plusieurs prises, préférentiellement 0,02 à 50 mgHkg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également les composés de formule (I) pour utilisation dans une méthode de traitement des pathologies ci-dessus indiquées qui comprend l'administration, à un patient, d'une dose efficace d'un composé selon l'invention, ou d'un de ses sels pharmaceutiquement acceptables.

Les composés selon l'invention pourront également être utilisés pour la préparation de compositions à usage vétérinaire.

## Revendications

1. Composé répondant à la formule (I) : 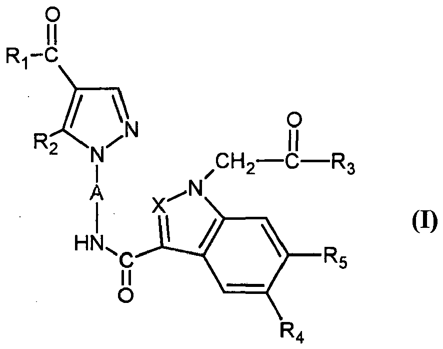 dans laquelle :
- A représente un radical aromatique bivalent choisi parmi : 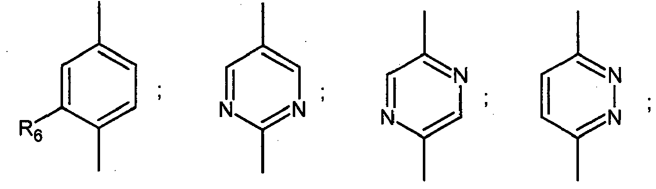
- X représente un groupe -CH- ou un atome d'azote ;
- R₁ représente un (C₁-C₄)alkyle ou un (C₁-C₄)alcoxy ;
- R₂ représente un groupe Alk ;
- R₃ représente un hydroxy ou un groupe -NR₇R₈ ;
- R₄ représente un atome d'hydrogène, un atome d'halogène, un cyano, un phényle, un groupe Alk, un groupe OAlk ou un groupe -NR₉R₁₀ ;
- R₅ représente un atome d'hydrogène, un atome d'halogène ou un groupe Alk ;
- R₆ représente un atome d'hydrogène, un atome d'halogène, un cyano, un groupe
- COOAlk ou un groupe -CONH₂;
- R₇ représente un atome d'hydrogène ou un (C₁-C₄)alkyle ;
- R₈ représente :
a) un atome d'hydrogène ;
b) un (C₁-C₄)alkyle non substitué ou substitué par :
(i) un hydroxy ;
(ii) un groupe OAlk ;
(iii) un groupe -NR₉R₁₀ ;
(iv) un (C₃-C₆)hétérocycloalkyle non substitué ou substitué par un (C₁-C₄)alkyle ou par un groupe -COOAlk ;
(v) un hétéroaryle non substitué ou substitué par un (C₁-C₄)alkyle ;
c) un (C₃-C₇)cycloalkyle ;
d) un (C₃-C₆)hétérocycloalkyle non substitué ou substitué par un (C₁-C₄)alkyle, un groupe -COOAlk ou par un ou deux oxo ;
e) un groupe -SO₂Alk ;
- ou bien R₇ et R₈ ensemble avec l'atome d'azote auquel ils sont liés constituent un hétérocycle, saturé ou insaturé, mono- ou polycyclique, condensé ou ponté, comprenant de 4 à 10 chainons et pouvant contenir un, deux ou trois autres atomes d'azote ou un autre hétéroatome choisi parmi l'atome d'oxygène ou de soufre ; ledit hétérocycle étant non substitué ou substitué une, deux ou trois fois par des substituants choisis indépendamment parmi :
a) un atome d'halogène ;
b) un hydroxy ;
c) un groupe -OR₁₁ ;
d) un oxo ;
e) un groupe -NR₉R₁₀ ;
f) un groupe -NR₁₂COR₁₃ ;
g) un groupe -NR₁₂COOR₁₃ ;
h) un groupe -COR₁₃ ;
i) un groupe -COOR₁₃ ;
j) un groupe -CONR₁₄R₁₅ ;
k) un (C₃-C₇)cycloalkyle non substitué ou substitué par un hydroxy ou par un (C₁-C₄)alkyle ;
l) un (C₃-C₆)hétérocycloalkyle non substitué ou substitué par un ou deux oxo ;
m) un phényle non substitué ou substitué une ou plusieurs fois par des substituants choisis indépendamment parmi un atome d'halogène, un groupe Alk ou un groupe OAlk ;
n) un pyridinyle ;
o) un (C₁-C₄)alkyle non substitué ou substitué une ou plusieurs fois par des substituants choisis indépendamment parmi :
(i) un atome d'halogène ;
(ii) un hydroxy ;
(iii) un groupe -OR₁₁ ;
(iv) un groupe -NR₉R₁₀ ;
(v) un groupe -NR₁₂COR₁₃ ;
(vi) un groupe -COOR₁₃ ;
(vii) un groupe -CONR₁₄R₁₅ ;
(viii) un groupe -SO₂Alk ;
(ix) un (C₃-C₇)cycloalkyle ;
(x) un (C₃-C₆)hétérocycloalkyle ;
(xi) un phényle non substitué ou substitué une ou plusieurs fois par des substituants choisis indépendamment parmi un atome d'halogène, un groupe Alk ou un groupe Alk ;
(xii) un hétéroaryle non substitué ou substitué par un (C₁-C₄)alkyle ;
- R₉ et R₁₀ représentent chacun indépendamment un atome d'hydrogène ou un (C₁-C₄)alkyle ;
- R₁₁ représente un groupe Alk, un -(C₁-C₄)alkylène-OH ou un -(C₁-C₄)alkylène-OAlk ;
- R₁₂ représente un atome d'hydrogène ou un (C₁-C₄)alkyle ;
- R₁₃ représente un (C₁-C₄)alkyle ;
- R₁₄ et R₁₅ représentent chacun indépendamment un atome d'hydrogène, un (C₁-C₄)alkyle ou un (C₃-C₇)cycloalkyle ;
- ou bien R₁₄ et R₁₅ ensemble avec l'atome d'azote auquel ils sont liés constituent un radical hétérocyclique choisi parmi : l'azétidin-1-yle, la pyrrolidin-1-yle, la pipéridin-1-yle, la morpholin-4-yle ou la pipérazin-1-yle ;
- Alk représente un (C₁-C₄)alkyle non substitué ou substitué une ou plusieurs fois par un atome de fluor ;
à l'état de base ou de sel d'addition à un acide ou à une base.

2. Composé de formule (I) selon la revendication 1, dans laquelle R₃ représente un hydroxy (référencé IA) et les autres substituants A, X, R₁, R₂, R₄ et R₅ sont tels que définis pour un composé de formule (I) à la revendication 1 ;
à l'état de base ou de sel d'addition à un acide ou à une base.

3. Composé de formule (I) selon la revendication 1, dans laquelle R₃ représente un groupe -NR₇R₈ (référencé IB) et les autres substituants A, X, R₁, R₂, R₄, R₅, R₇ et R₈ sont tels que définis pour un composé de formule (I) à la revendication 1 ;
à l'état de base ou de sel d'addition à un acide ou à une base.

4. Composé de formule (I) selon la revendication 1, dans laquelle :
- A représente un radical aromatique bivalent choisi parmi : 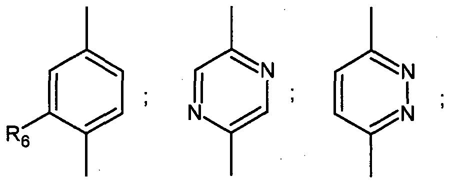
- X représente un groupe -CH- ou un atome d'azote ;
- R₁ représente un radical méthyle, éthyle, n-propyle ou un radical éthoxy;
- R₂ représente un radical méthyle;
- R₃ représente un hydroxy ou un groupe -NR₇R₈ ;
- R₄ représente un atome d'hydrogène, un atome de brome, de chlore ou de fluor, un cyano, un phényle, un radical méthyle, un radical trifluorométhyle, un radical méthoxy ou un groupe diméthylamino ;
- R₅ représente un atome d'hydrogène, un atome de brome, de chlore ou de fluor ou un radical méthyle ;
- R₆ représente un atome d'hydrogène, un atome de brome, un cyano, un groupe méthoxycarbonyle ou un groupe -CONH₂ ;
- R₇ représente un atome d'hydrogène ou un radical méthyle ;
- R₈ représente :
a) un atome d'hydrogène ;
b) un radical méthyle, un 2-hydroxyéthyle, un 3-hydroxypropyle, un 2-méthoxyéthyle, un 2-(méthylamino)éthyle, un 2-(diméthylamino)éthyle, un 2-(diméthylamino)propyle, un 2-(diméthylamino)-1-méthyléthyle, un 2-(diméthylamino)-2-méthylpropyl, un 3-(diméthylamino)propyle, un (1-méthylpipéridin-3-yl)méthyle, un tétrahydrofuran-3-ylméthyle, un tétrahydro-2*H-*pyran-4-ylméthyle, un 2-furylméthyle, un (3-méthyl-1*H*-1,2,4-triazol-5-yl)méthyle, 1-(1*H*-tétrazol-5-yl)éthyle, un 2-(1*H*-pyrrol-1-yl)éthyle, un 2-(1*H*-imidazol-1-yl)éthyle ;
c) un cyclopropyle ;
d) un 1,1-dioxidotétrahydro-3-thiényle, un pyrrolidin-3-yle, un 1-méthylpyrrolidin-3-yle, un 1-(*tert*-butoxycarbonyl)pyrrolidin-3-yle, un pipéridin-3-yle, un 1-méthylpipéridin-3-yle, un 1-(*tert*-butoxycarbonyl)pipéridin-3-yle, un 1-méthylpipéridin-4-yle ;
e) un groupe méthylsulfonyle ;
- ou bien R₇ et R₈ ensemble avec l'atome d'azote auquel ils sont liés constituent un hétérocycle choisi parmi : une azétidine, une pyrrolidine, une pipéridine, une pipérazine, une morpholine, une thiomorpholine, un octahydro-2*H*-pyrido[1,2-*a*]pyrazine, un 5,6,7,8-tétrahydro[1,2,4]triazolo[4,3-*a*]pyrazine, un 2,3-dihydro-1*H-*pyrrolo[3,4-*c*]pyridine, un octahydropyrrolo[1,2-*a*]pyrazine, un 1,4-diazépane, un 5,6,7,8-tétrahydroimidazo[1,2-*a*]pyrazine, un 4,5,6,7-tétrahydro-1*H*-pyrazolo[4,3-*c*]pyridine, un 1,4,5,6-tétrahydropyrrolo[3,4-*c*]pyrazole, un 6,7-dihydro-5*H-*pyrrolo[3,4-*b*]pyridine, un 3,8-diazabicyclo[3.2.1]octane, un 4,5,6,7-tétrahydro-1*H-*imidazo[4,5-*c*]pyridine, un 4,5,6,7-tétrahydro-1*H*-pyrazolo[3,4-c]pyridine, un 2,5-diazabicyclo[2.2.2]octane, un 2,5-diazabicyclo[2.2.1]heptane, un octahydropyrrolo[3,4-*b*]pyrrole, un octahydropyrrolo[3,4-*c*]pyrrole, un octahydro-2*H*-pyrazino[1,2-a]pyrazine; ledit hétérocycle étant non substitué ou substitué une, deux ou trois fois par des substituants choisis indépendamment parmi :
a) un atome de fluor ;
b) un hydroxy ;
c) un radical méthoxy, un 2-hydroxyéthoxy, un 2-méthoxyéthoxy ;
d) un oxo ;
e) un groupe amino, un méthylamino, un diméthylamino ;
f) un groupe acétamido ;
g) un groupe (*tert*-butoxycarbonyl)amino, un (*tert*-butoxycarbonyl)(méthyl)amino ;
h) un groupe acétyle ;
i) un groupe méthoxycarbonyle, un *tert*-butoxycarbonyle ;
j) un groupe diméthylcarbamoyle, un cyclopropylcarbamoyle, un cyclobutylcarbamoyle ;
k) un radical cyclopropyle, un cyclobutyle, un 2-hydroxycyclopentyle ;
l) un oxétan-3-yle, un 1,1-dioxidotétrahydro-3-thiényle, une pipéridin-1-yle, une morpholin-4-yle ;
m) un 4-fluorophényle;
n) un pyridin-2-yle ;
o) un radical méthyle, un éthyle, un n-propyle, un isopropyle, un n-butyle, un trifluorométhyle, un 2-fluoroéthyle, un 2,2-difluoroéthyle, un 2,2,2-trifluoroéthyle, un 3,3,3-trifluoropropyle, un 3,3,3-trifluoro-2-hydroxypropyle, un hydroxyméthyle, un 2-hydroxyéthyle, un 2-hydroxy-1,1-diméthyléthyle, un méthoxyméthyle, un 2-méthoxyéthyle, un 2-méthoxy-1-méthyléthyle, un 2-méthoxy-1,1-diméthyléthyle, un 2-éthoxyéthyle, un 2-(trifluorométhoxy)éthyle, un 2-(2-hydroxyéthoxy)éthyle, un 2-(2-méthoxyéthoxy)éthyle, un 2-(diméthylamino)éthyle, un 2-acétamidoéthyle, un 2-[acétyl(méthyl)amino]éthyle, un 2-méthoxy-2-oxoéthyle, un 2-éthoxy-2-oxoéthyle, un 3-éthoxy-3-oxopropyle, un 2-amino-2-oxoéthyle, un 2-(méthylamino)-2-oxoéthyle, un 2-(isopropylamino)-2-oxoéthyle, un 2-(diméthylamino)-2-oxoéthyle, un 2-(cyclopropylamino)-2-oxoéthyle, un 2-oxo-2-pyrrolidin-1-yléthyle, un 2-(méthylsulfonyl)éthyle, un cyclopropylméthyle, un pyrrolidin-1-ylméthyle, un tétrahydrofuran-2-ylméthyle, un 2-thiénylméthyle, un 4-chlorobenzyle, un 4-fluorobenzyle, un 2-méthoxybenzyle, un 3-fluoro-4-méthoxybenzyle, un pyridin-4-ylméthyle, un (5-méthyl-1,2,4-oxadiazol-3-yl)méthyle, un (5-méthylisoxazol-3-yl)méthyle ;
à l'état de base ou de sel d'addition à un acide ou à une base.

5. Composé de formule (I) selon la revendication 1, dans laquelle :
- A représente un radical aromatique bivalent choisi parmi : 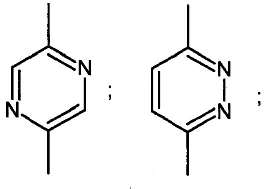
- X représente un groupe -CH-;
- R₁ représente un radical n-propyle;
- R₂ représente un radical méthyle;
- R₃ représente un hydroxy ou un groupe -NR₇R₈ ;
- R₄ représente un atome de chlore;
- R₅ représente un atome d'hydrogène ;
- R₇ représente un atome d'hydrogène ou un radical méthyle ;
- R₈ représente :
b) un radical méthyle, un 2-hydroxyéthyle, un 3-hydroxypropyle, un 2-méthoxyéthyle, un 2-(diméthylamino)éthyle, un 2-(diméthylamino)propyle, , un 2-(diméthylamino)-2-méthylpropyl, un 3-(diméthylamino)propyle, , un tétrahydrofuran-3-ylméthyle, un tétrahydro-2*H*-pyran-4-ylméthyle, un 2-furylméthyle, un (3-méthyl-1*H*-1,2,4-triazol-5-yl)méthyle, 1-(1*H*-tétrazol-5-yl)éthyle, un 2-(1*H*-pyrrol-1-yl)éthyle;
c) un cyclopropyle ;
d) un 1-méthylpyrrolidin-3-yle, un 1-(*tert*-butoxycarbonyl)pipéridin-3-yle, un 1-méthylpipéridin-4-yle ;
- ou bien R₇ et R₈ ensemble avec l'atome d'azote auquel ils sont liés constituent un hétérocycle choisi parmi : une azétidine, une pyrrolidine, une pipéridine, une pipérazine, une morpholine, une thiomorpholine, un octahydro-2*H*-pyrido[1,2-a]pyrazine, un 5,6,7,8-tétrahydro[1,2,4]triazolo[4,3-*a*]pyrazine, un 2,3-dihydro-1*H-*pyrrolo[3,4-*c*]pyridine, un octahydropyrrolo[1,2-*a*]pyrazine, un 1,4-diazépane; ledit hétérocycle étant non substitué ou substitué une, deux ou trois fois par des substituants choisis indépendamment parmi :
b) un hydroxy ;
c) un radical méthoxy;
d) un oxo ;
h) un groupe acétyle ;
i) un groupe méthoxycarbonyle;
j) un groupe diméthylcarbamoyle;
k) un radical cyclobutyle;
l) un 1,1-dioxidotétrahydro-3-thiényle, une pipéridin-1-yle, une morpholin-4-yle ;
m) un 4-fluorophényle;
o) un radical méthyle, un éthyle, un n-propyle, un isopropyle, un n-butyle, un trifluorométhyle, un 3,3,3-trifluoropropyle, un hydroxyméthyle, un 2-hydroxyéthyle, un méthoxyméthyle, un 2-méthoxyéthyle, un 2-éthoxyéthyle, un 2-(trifluorométhoxy)éthyle, un 2-(diméthylamino)éthyle, un 2-méthoxy-2-oxoéthyle, un 2-éthoxy-2-oxoéthyle, un 2-(isopropylamino)-2-oxoéthyle, un 2-(diméthylamino)-2-oxoéthyle, un 2-(cyclopropylamino)-2-oxoéthyle, un 2-oxo-2-pyrrolidin-1-yléthyle, un tétrahydrofuran-2-ylméthyle, un 4-fluorobenzyle, un 3-fluoro-4-méthoxybenzyle, un pyridin-4-ylméthyle, un (5-méthyl-1,2,4-oxadiazol-3-yl)méthyle,;
à l'état de base ou de sel d'addition à un acide ou à une base.

6. Composé de formule (I) selon la revendication 1, dans laquelle :
- A représente un radical aromatique bivalent : 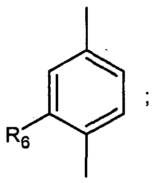
- X représente un groupe -CH- ou un atome d'azote ;
- R₁ représente un radical méthyle, éthyle, n-propyle ou un radical éthoxy;
- R₂ représente un radical méthyle;
- R₃ représente un hydroxy ou un groupe -NR₇R₈ ;
- R₄ représente un atome d'hydrogène, un atome de brome, de chlore ou de fluor, un cyano, un phényle, un radical méthyle, un radical trifluorométhyle, un radical méthoxy ou un groupe diméthylamino ;
- R₅ représente un atome d'hydrogène, un atome de brome, de chlore ou de fluor ou un radical méthyle ;
- R₆ représente un atome d'hydrogène, un atome de brome, un cyano, un groupe méthoxycarbonyle ou un groupe -CONH₂ ;
- R₇ représente un atome d'hydrogène ou un radical méthyle ;
- R₈ représente :
b) un radical méthyle, un 2-hydroxyéthyle, un 2-méthoxyéthyle, un 2-(méthylamino)éthyle, un 2-(diméthylamino)éthyle, un 2-(diméthylamino)-1-méthyléthyle, un (1-méthylpipéridin-3-yl)méthyle, un 2-(1H-imidazol-1-yl)éthyle;
d) un 1,1-dioxidotétrahydro-3-thiényle, un pyrrolidin-3-yle, un 1-méthylpyrrolidin-3-yle, un 1-(*tert*-butoxycarbonyl)pyrrolidin-3-yle, un pipéridin-3-yle, un 1-méthylpipéridin-3-yle, un 1-(*tert*-butoxycarbonyl)pipéridin-3-yle ;
e) un groupe méthylsulfonyle ;
- ou bien R₇ et R₈ ensemble avec l'atome d'azote auquel ils sont liés constituent un hétérocycle choisi parmi : une azétidine, une pyrrolidine, une pipéridine, une pipérazine, une morpholine, un 5,6,7,8-tétrahydro[1,2,4]triazolo[4,3-*a*]pyrazine, un octahydropyrrolo[1,2-*a*]pyrazine, un 1,4-diazepane, un 5,6,7,8-tétrahydroimidazo[1,2-a]pyrazine, un 4,5,6,7-tétrahydro-1*H*-pyrazolo[4,3-c]pyridine, un 1,4,5,6-tétrahydropyrrolo[3,4-c]pyrazole, un 6,7-dihydro-5*H-*pyrrolo[3,4-*b*]pyridine, un 3,8-diazabicyclo[3.2.1]octane, un 4,5,6,7-tétrahydro-1*H-*imidazo[4,5-*c*]pyridine, un 4,5,6,7-tétrahydro-1*H*-pyrazolo[3,4-c]pyridine, un 2,5-diazabicyclo[2.2.2]octane, un 2,5-diazabicyclo[2.2.1]heptane, un octahydropyrrolo[3,4-*b*]pyrrole, un octahydropyrrolo[3,4-*c*]pyrrole, un octahydro-2*H*-pyrazino[1,2-a]pyrazine; ledit hétérocycle étant non substitué ou substitué une, deux ou trois fois par des substituants choisis indépendamment parmi :
a) un atome de fluor ;
b) un hydroxy ;
c) un 2-hydroxyéthoxy, un 2-méthoxyéthoxy ;
d) un oxo ;
e) un groupe amino, un méthylamino, un diméthylamino ;
f) un groupe acétamido ;
g) un groupe (*tert*-butoxycarbonyl)amino, un (*tert*-butoxycarbonyl)(méthyl)amino ;
i) un groupe méthoxycarbonyle, un *tert*-butoxycarbonyle ;
j) un groupe, un cyclopropylcarbamoyle, un cyclobutylcarbamoyle ;
k) un radical cyclopropyle, un cyclobutyle, un 2-hydroxycyclopentyle ;
l) un oxétan-3-yle, une morpholin-4-yle ;
n) un pyridin-2-yle ;
o) un radical méthyle, un éthyle, un n-propyle, un isopropyle, un n-butyle, un trifluorométhyle, un 2-fluoroéthyle, un 2,2-difluoroéthyle, un 2,2,2-trifluoroéthyle, un 3,3,3-trifluoro-2-hydroxypropyle, un hydroxyméthyle, un 2-hydroxyéthyle, un 2-hydroxy-1,1-diméthyléthyle, un méthoxyméthyle, un 2-méthoxyéthyle, un 2-méthoxy-1-méthyléthyle, un 2-méthoxy-1,1-diméthyléthyle, un 2-éthoxyéthyle, un 2-(trifluorométhoxy)éthyle, un 2-(2-hydroxyéthoxy)éthyle, un 2-(2-méthoxyéthoxy)éthyle, un 2-(diméthylamino)éthyle, un 2-acétamidoéthyle, un 2-[acétyl(méthyl)amino]éthyle, un 2-éthoxy-2-oxoéthyle, un 3-éthoxy-3-oxopropyle, un 2-amino-2-oxoéthyle, un 2-(méthylamino)-2-oxoéthyle, un 2-(diméthylamino)-2-oxoéthyle, un 2-oxo-2-pyrrolidin-1-yléthyle, un 2-(méthylsulfonyl)éthyle, un cyclopropylméthyle, un pyrrolidin-1-ylméthyle, un 2-thiénylméthyle, un 4-chlorobenzyle, un 4-fluorobenzyle, un 2-méthoxybenzyle, un (5-méthylisoxazol-3-yl)méthyle ;
à l'état de base ou de sel d'addition à un acide ou à une base.

7. Composé de formule (I) selon la revendication 1, dans laquelle :
- A représente un radical aromatique bivalent choisi parmi : 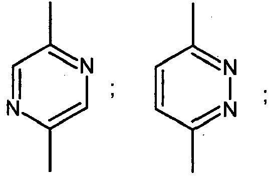
- X représente un groupe -CH-;
- R₁ représente un radical n-propyle;
- R₂ représente un radical méthyle;
- R₃ représente un hydroxy ou un groupe -NR₇R₈ choisi parmi : 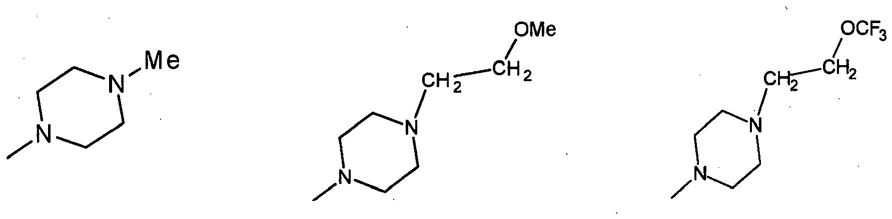 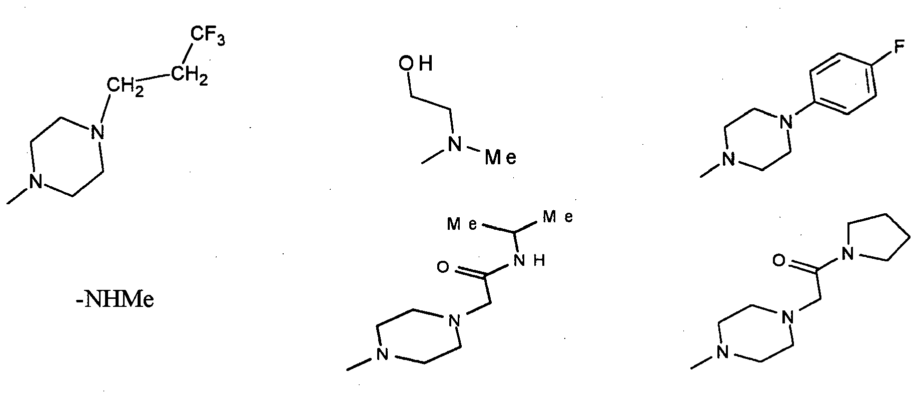 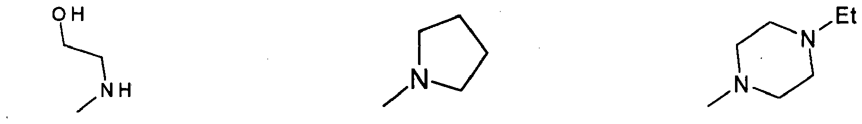 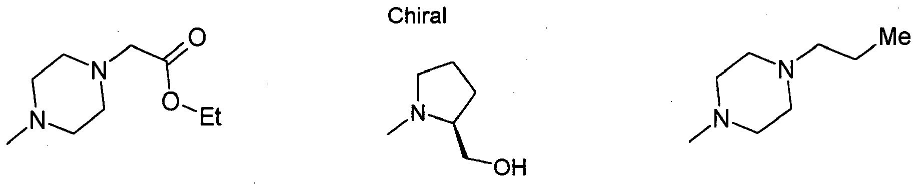 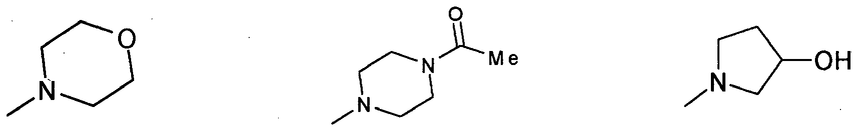 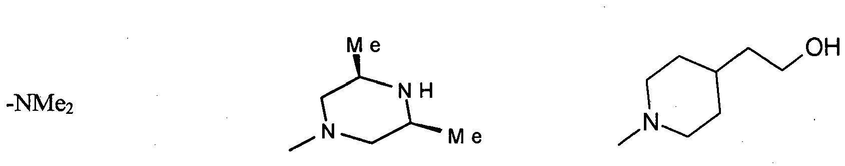 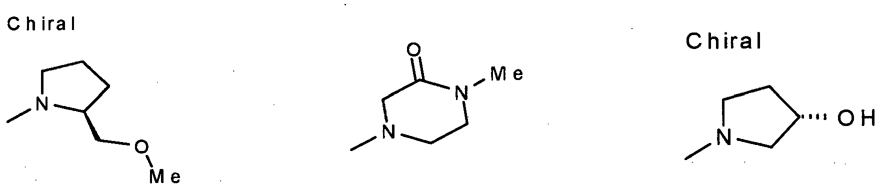 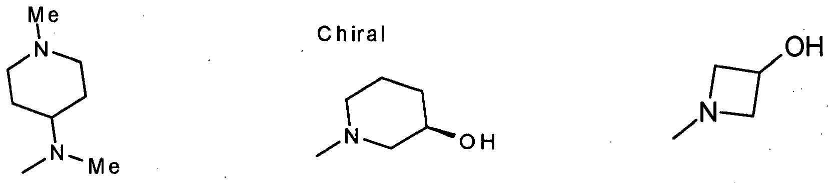 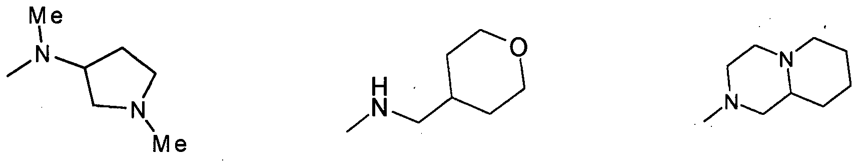 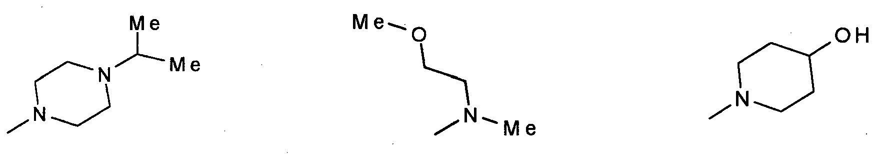 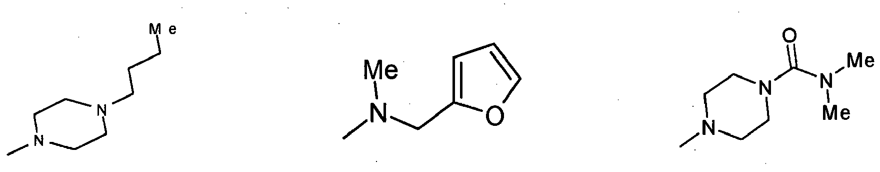 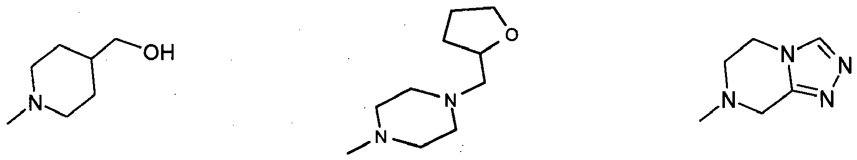 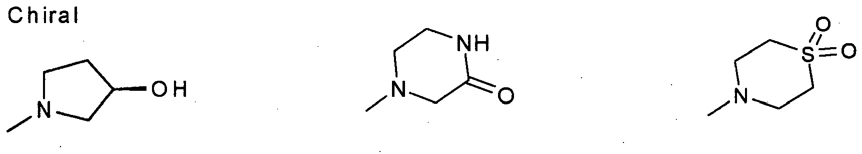 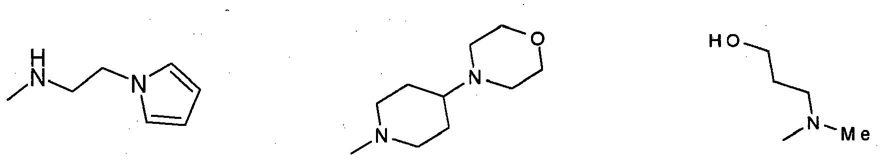 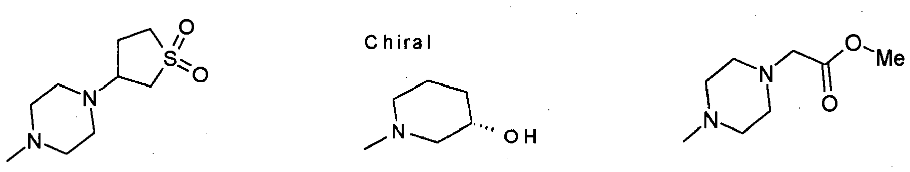 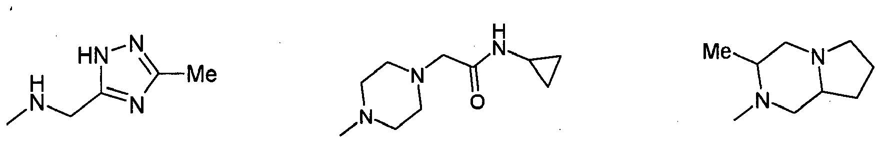 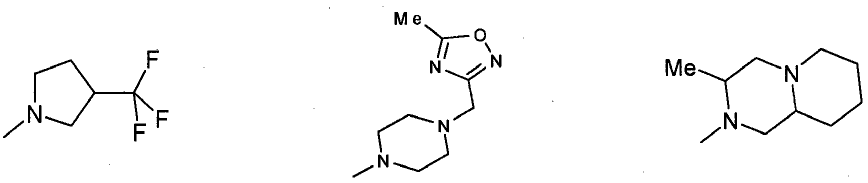 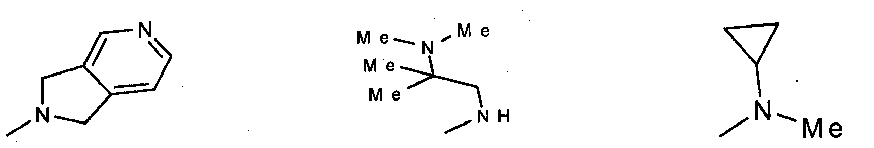 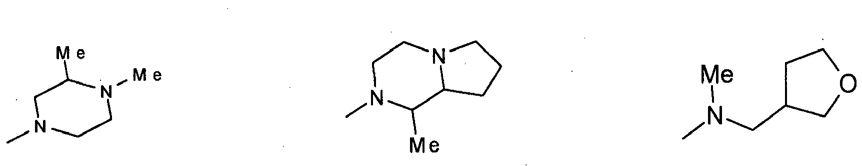 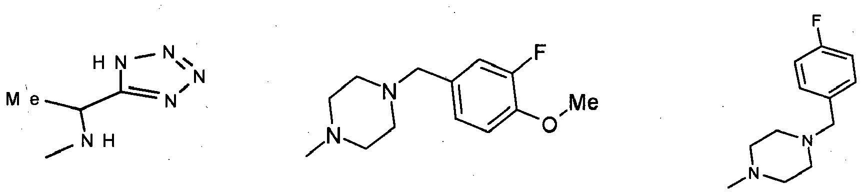 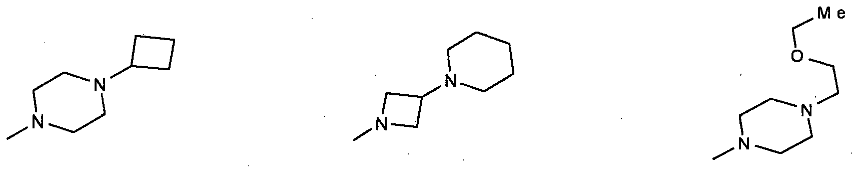 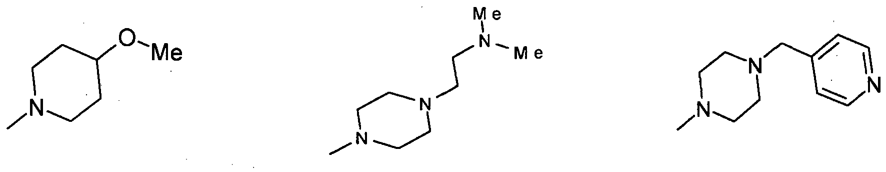 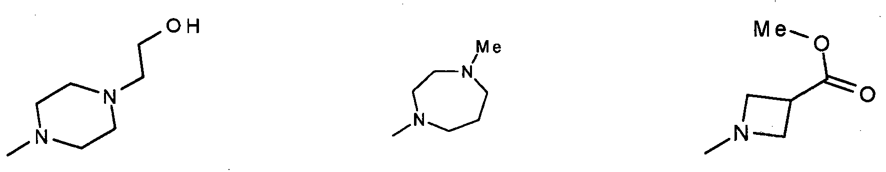 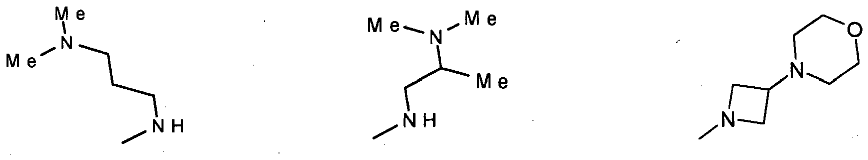 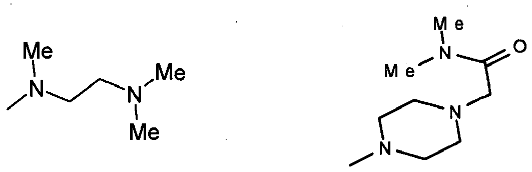
- R₄ représente un atome de chlore ;
- R₅ représente un atome d'hydrogène ;
à l'état de base ou de sel d'addition à un acide ou à une base.

8. Composé de formule (I) selon la revendication 1, dans laquelle :
- A représente un radical aromatique bivalent : 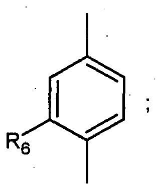
- X représente un groupe -CH- ou un atome d' azote ;
- R₁ représente un radical méthyle, éthyle, n-propyle ou un radical éthoxy ;
- R₂ représente un radical méthyle ;
- R₃ représente un hydroxy ou un groupe -NR₇R₈ choisi parmi : 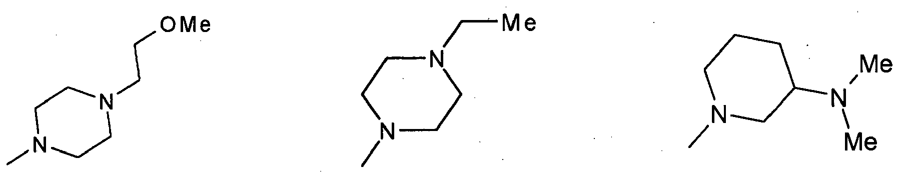 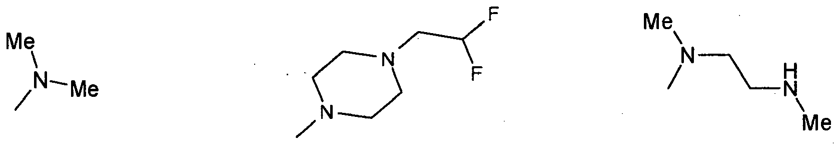 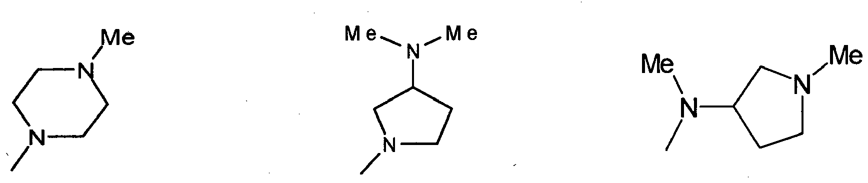 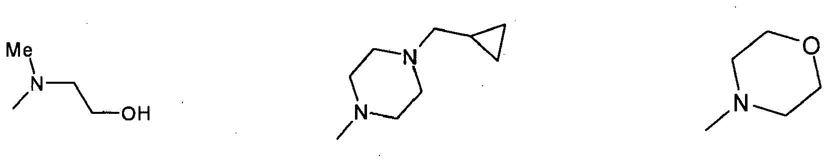 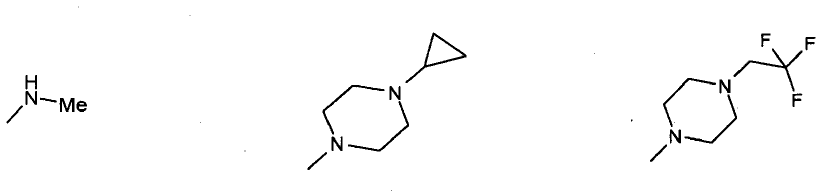 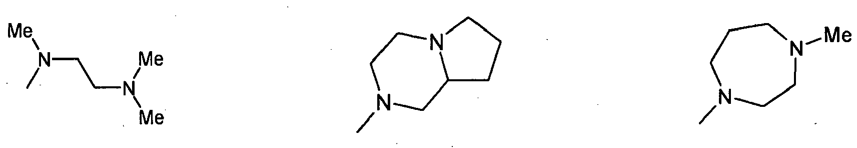 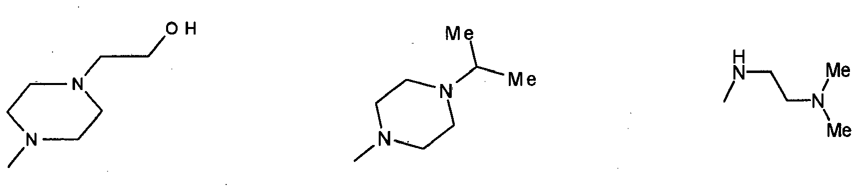 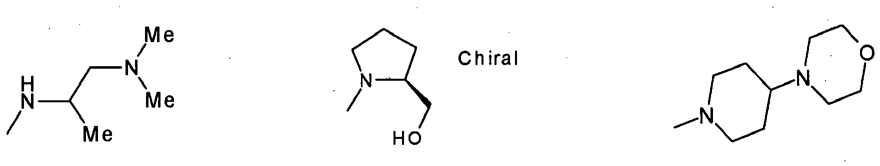 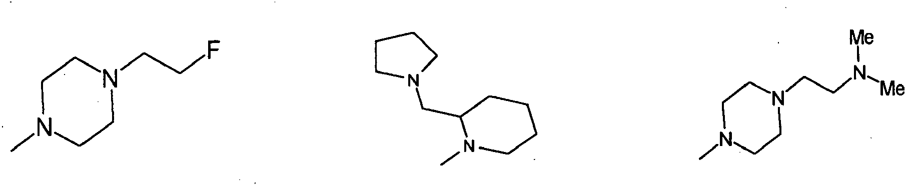 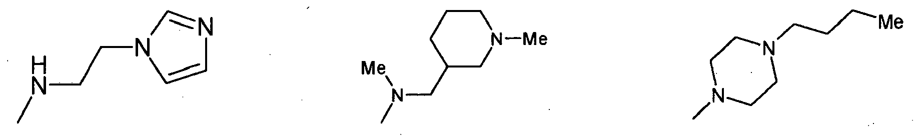 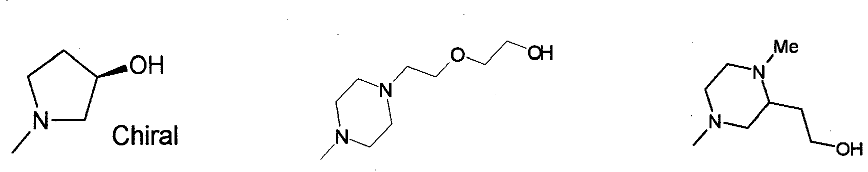 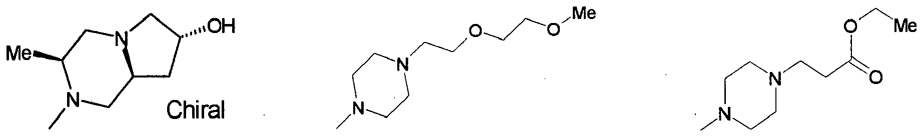 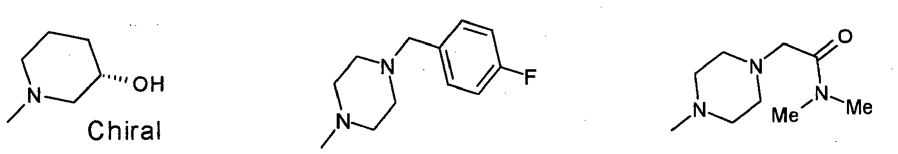 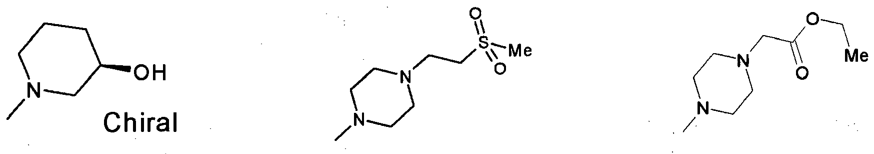 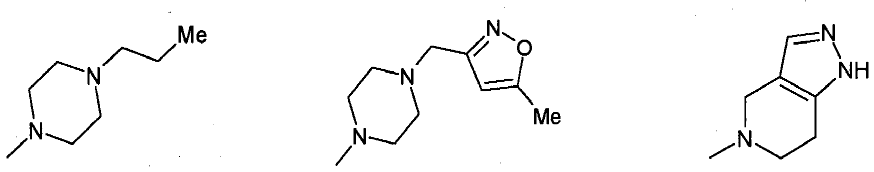 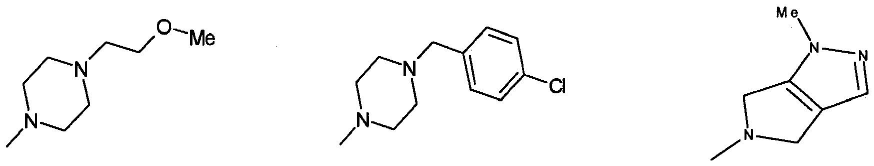 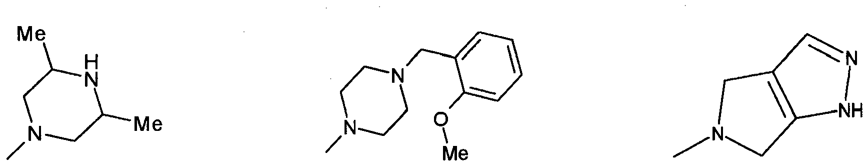 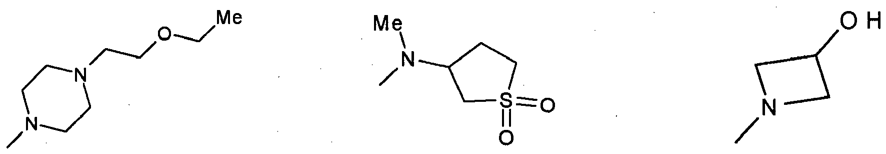 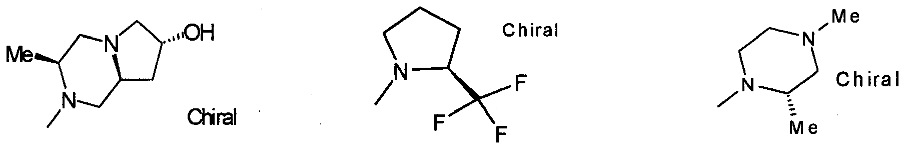 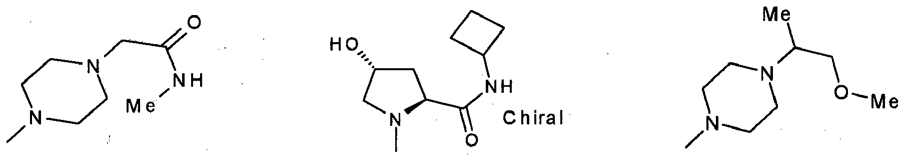 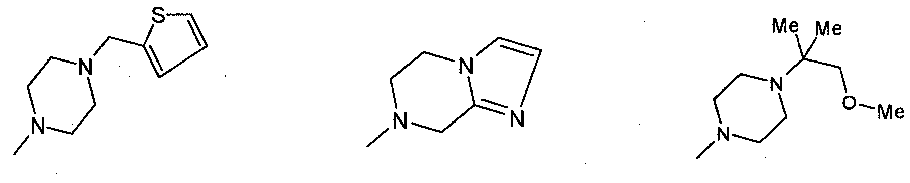 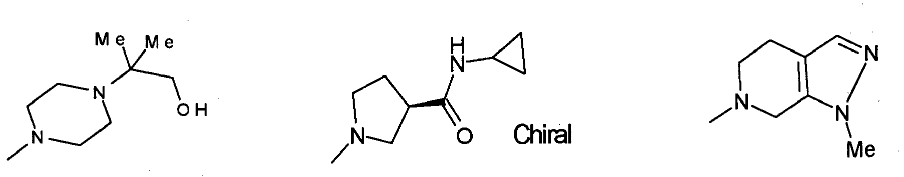 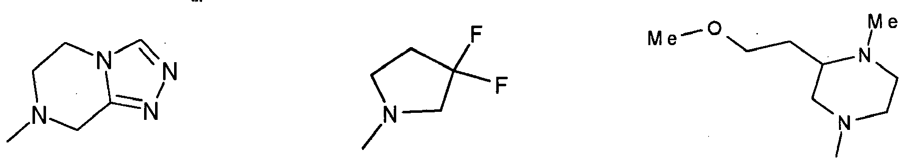 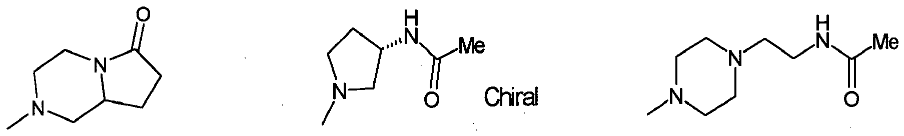 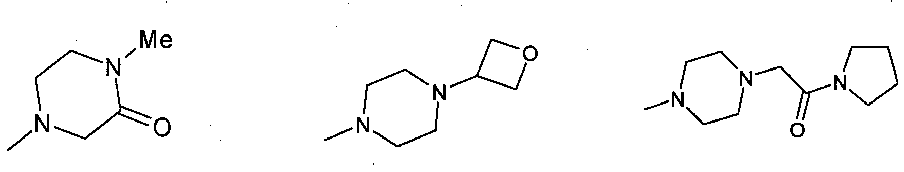 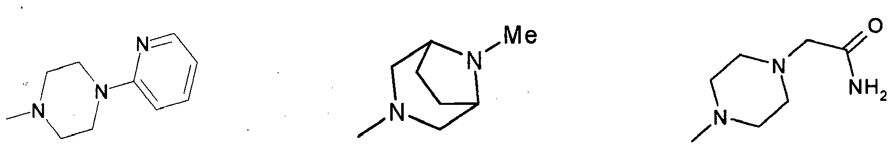 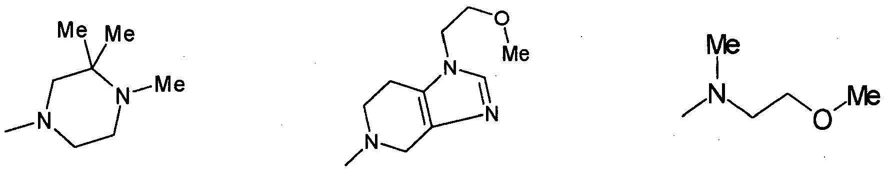 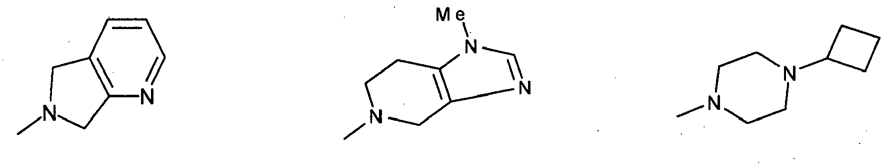 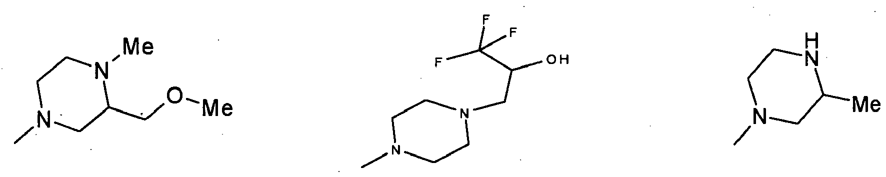 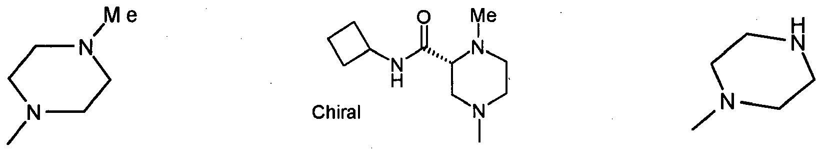 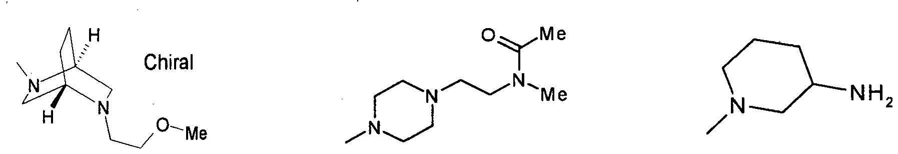 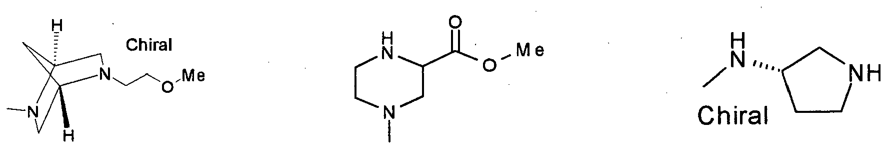 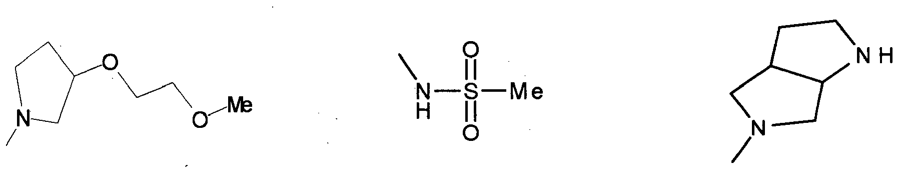 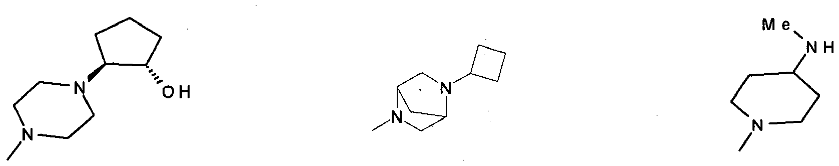 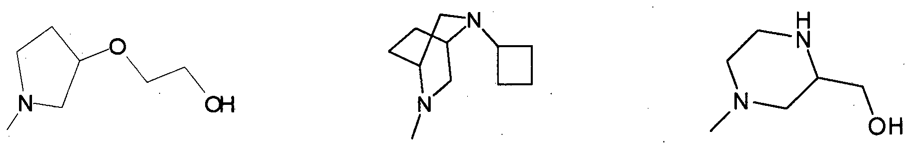 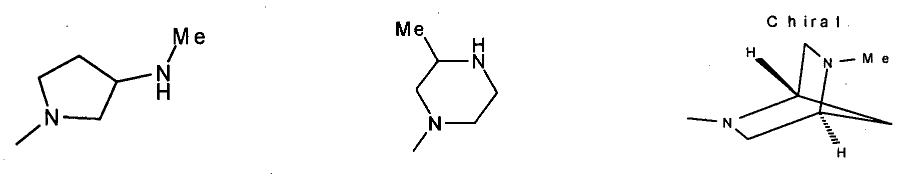 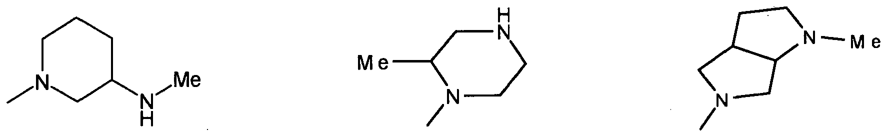 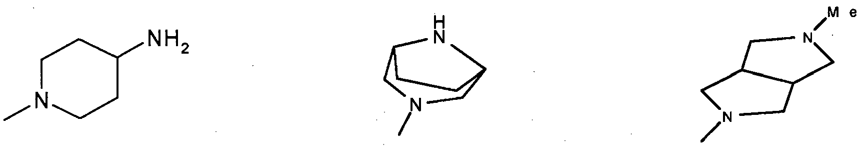 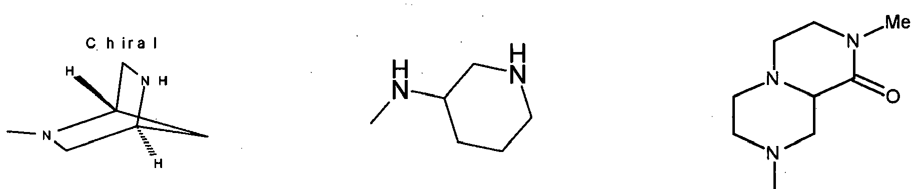 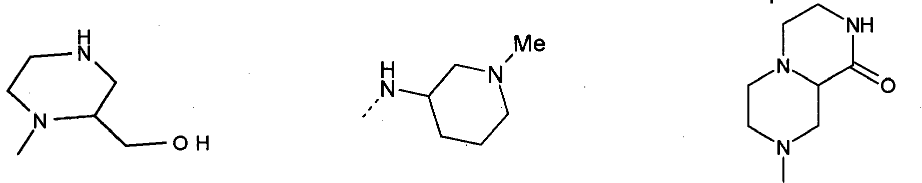 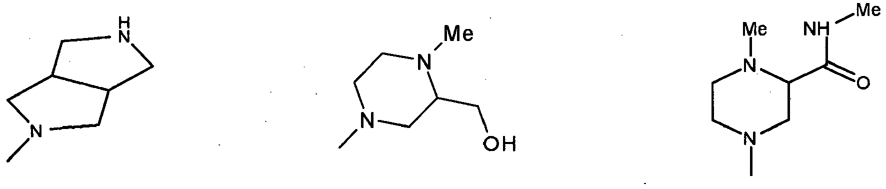 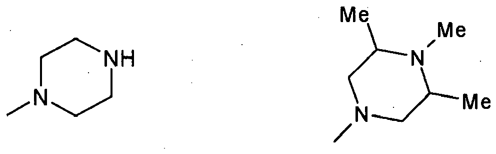
- R₄ représente un atome d'hydrogène, un atome de brome, de chlore ou de fluor, un cyano, un phényle, un radical méthyle, un radical trifluorométhyle, un radical méthoxy ou un groupe diméthylamino ;
- R₅ représente un atome d'hydrogène, un atome de brome, de chlore ou de fluor ou un radical méthyle ;
- R₆ représente un atome d'hydrogène, un atome de brome, un cyano, un groupe méthoxycarbonyle ou un groupe -CONH₂ ;
à l'état de base ou de sel d'addition à un acide ou à une base.

9. Composé de formule (I) selon la revendication 1, dans laquelle :
- A représente un radical aromatique bivalent choisi parmi : 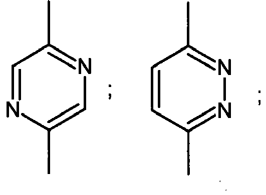
- X représente un groupe -CH- ;
- R₁ représente un radical n-propyle ;
- R₂ représente un radical méthyle ;
- R₃ représente un hydroxy ou un groupe -NR₇R₈ choisi parmi : 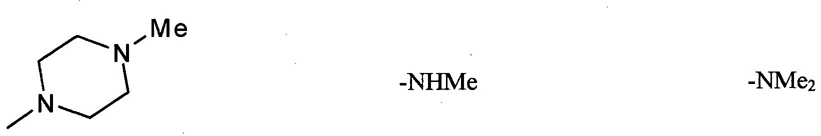 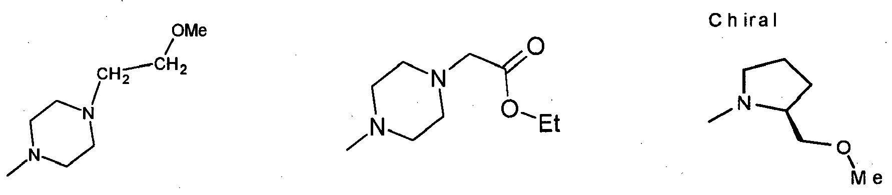 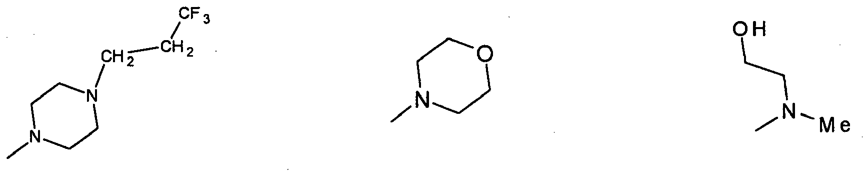 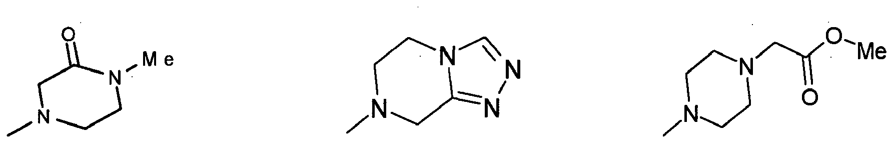 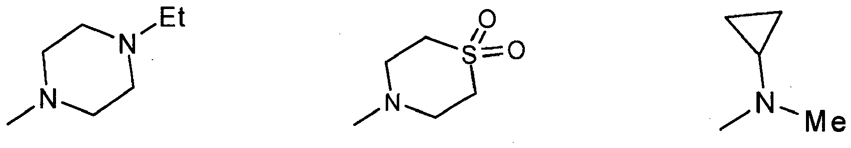 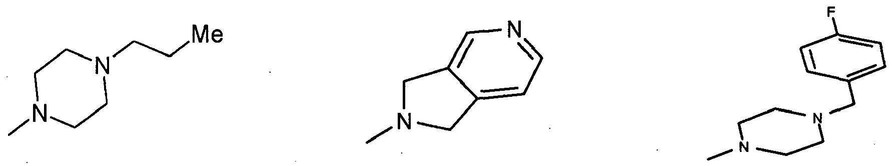 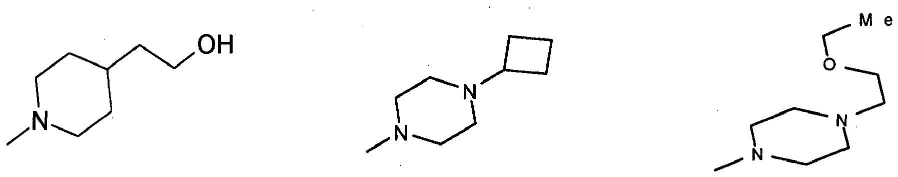 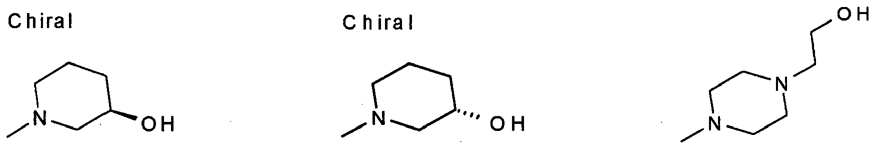 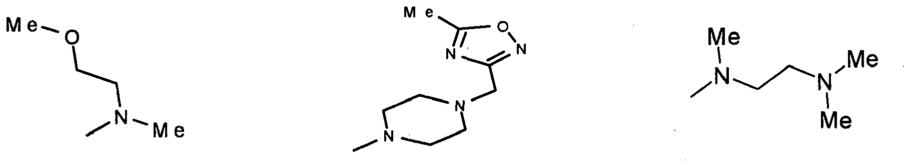 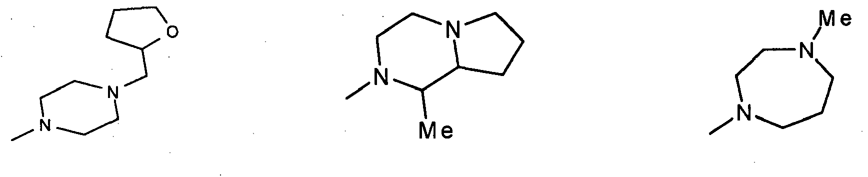
- R₄ représente un atome de chlore ;
- R₅ représente un atome d'hydrogène ;
à l'état de base ou de sel d'addition à un acide ou à une base.

10. Composé de formule (I) selon la revendication 1, dans laquelle :
- A représente un radical aromatique bivalent : 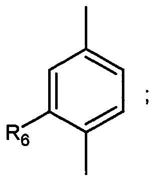
- X représente un groupe -CH- ou un atome d'azote ;
- R₁ représente un radical méthyle, éthyle, n-propyle ou un radical éthoxy ;
- R₂ représente un radical méthyle ;
- R₃ représente un hydroxy ou un groupe -NR₇R₈ choisi parmi : 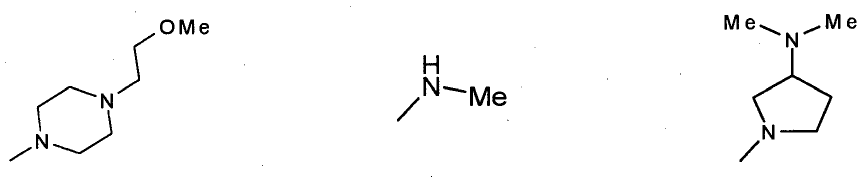 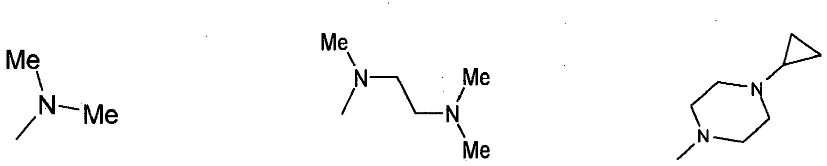 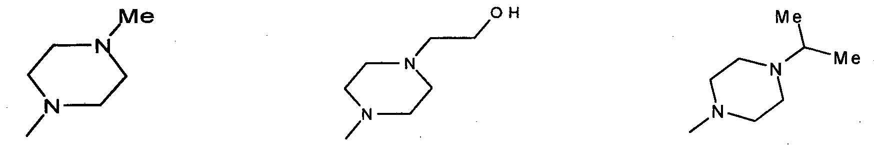 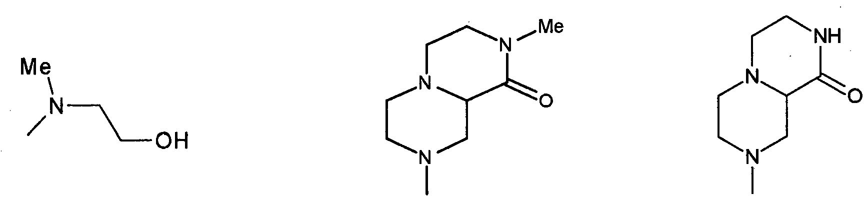 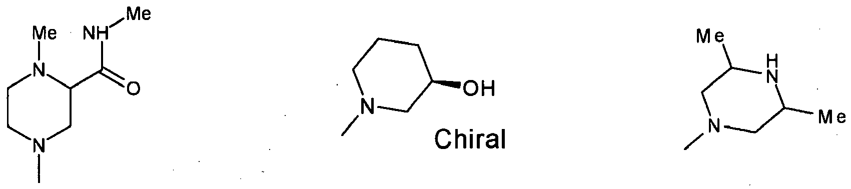 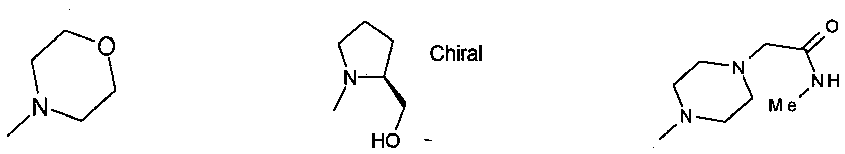 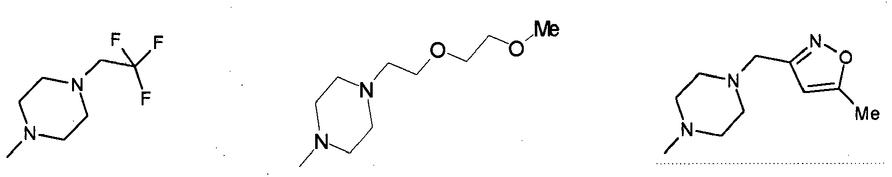 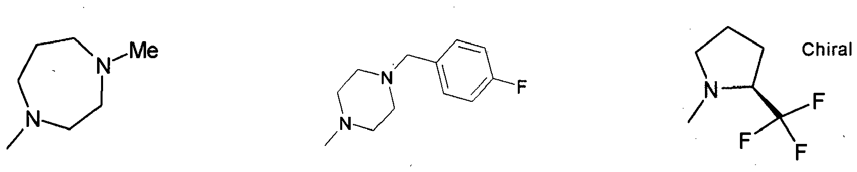 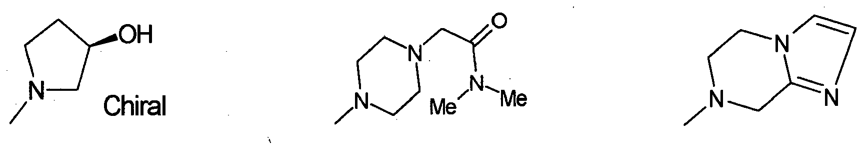 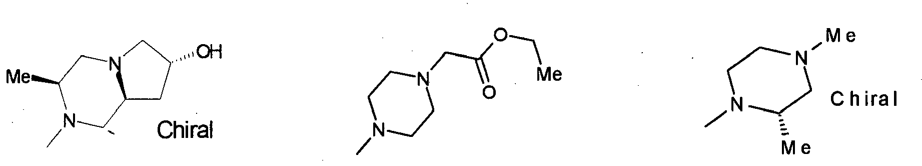 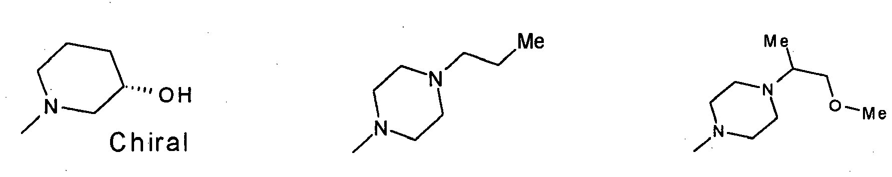 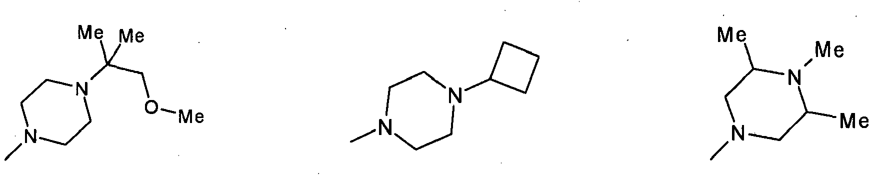 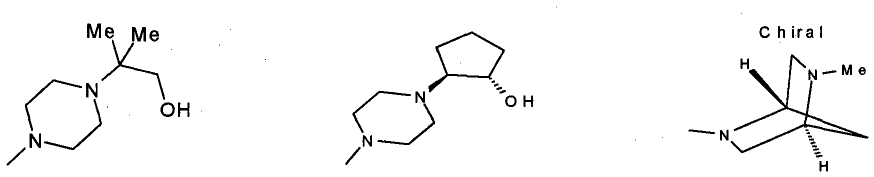 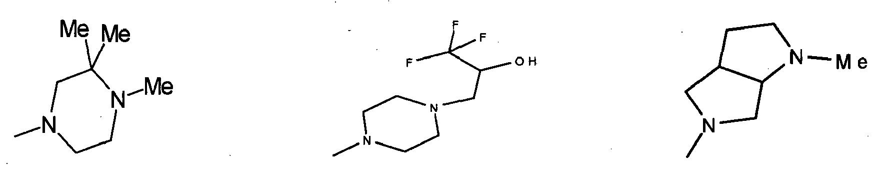 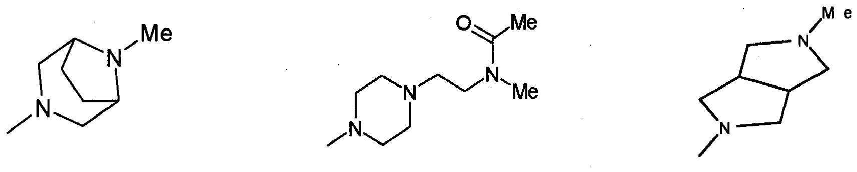 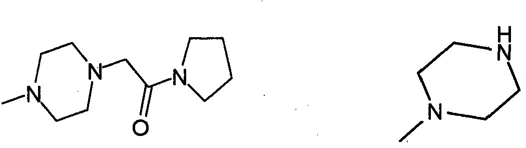 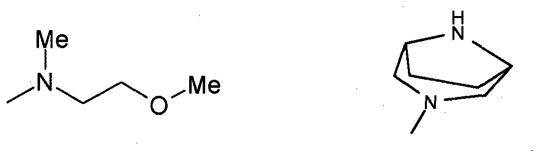
- R₄ représente un atome d'hydrogène, un atome de brome, de chlore ou de fluor, un cyano, un phényle, un radical méthyle, un radical trifluorométhyle, un radical méthoxy ou un groupe diméthylamino ;
- R₅ représente un atome d'hydrogène, un atome de brome, de chlore ou de fluor ou un radical méthyle ;
- R₆ représente un atome d'hydrogène, un atome de brome, un cyano, un groupe méthoxycarbonyle ou un groupe -CONH₂ ;
à l'état de base ou de sel d'addition à un acide ou à une base.

11. Composé de formule (I) selon la revendication 1 choisi parmi :
- Acide (3-{[4-(4-butyryl-5-méthyl-1*H*-pyrazol-1-yl)phényl]carbamoyl}-5-méthyl - 1*H*-indol-1-yl)acétique ;
- Acide (3-{[4-(4-butyryl-5-méthyl-1*H*-pyrazol-1-yl)phényl]carbamoyl}-5,6-diméthyl -1*H*-indol-1-yl)acétique ;
- Acide (3-{[6-(4-butyryl-5-méthyl-1H-pyrazol-1-yl)pyridazin-3-yl]carbamoyl}-5-chloro-1H-indol-1-yl)acétique ;
- N-[5-(4-butyryl-5-méthyl-1*H*-pyrazol-1-yl)pyrazin-2-yl]-5-chloro-1-[2-(4-méthylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-[2-(4-méthylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-{2-[4-(2-méthoxyéthyl)pipérazin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-oxo-2-[4-(3,3,3-trifluoropropyl)pipérazin-1-yl]éthyl}-1*H*-indole-3-carboxamide ;
- N-[6-(4-butyryl-5-méthyl-1*H*-pyrazol-1-yl)pyridazin-3-yl]-5-chloro-1-[2-(méthylamino)-2-oxoéthyl]-1*H*-indole-3-carboxamide ;
- N-[6-(4-butyryl-5-méthyl-1*H*-pyrazol-1-yl)pyridazin-3-yl]-5-chloro-1-(2-morpholin-4-yl-2-oxoéthyl)-1*H*-indole-3-carboxamide ;
- N-[6-(4-butyryl-5-méthyl-1*H*-pyrazol-1-yl)pyridazin-3-yl]-5-chloro-1-[2-(diméthylamino)-2-oxoéthyl]-1*H*-indole-3-carboxamide ;
- N-[6-(4-butyryl-5-méthyl-1*H*-pyrazol-1-yl)pyridazin-3-yl]-5-chloro-1-{2-[(2S)-2-(méthoxyméthyl)pyrrolidin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-[2-(4-éthylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-[2-(4-propylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-{2-[(2-méthoxyéthyl)(méthyl)amino]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-oxo-2-[4-(teétrahydrofuran-2-ylméthyl)pipérazin-1-yl]éthyl}-1*H*-indole-3-carboxamide ;
- N-[6-(4-butyryl-5-méthyl-1*H*-pyrazol-1-yl)pyridazin-3-yl]-5-chloro-1-[2-(5,6-dihydro[1,2,4]triazolo[4,3-a]pyrazin-7(8*H*)-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-[2-(4-cyclobutylpipérazin-1-yl)-2-oxoéthyl]-1*H*-indole-3-carboxamide ;
- N-[6-(4-butyryl-5-méthyl-1*H*-pyrazol-1-yl)pyridazin-3-yl]-5-chloro-1-(2-{4-[(5-méthyl-1,2,4-oxadiazol-3-yl)méthyl]pipérazin-1-yl }-2-oxoéthyl)-1H-indole-3-carboxamide ;
- {4-[(3-{[6-(4-butyryl-5-méthyl-1*H*-pyrazol-1-yl)pyridazin-3-yl]carbamoyl}-5-chloro-1*H*-indol-1-yl)acétyl]pipérazin-1-yl}acétate de méthyle ;
- N-[6-(4-butyryl-5-méthyl-1*H*-pyrazol-1-yl)pyridazin-3-yl]-5-chloro-1-{2-[cyclopropyl(méthyl)amino]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-[4-(4-fluorobenzyl)pipérazin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-[4-(2-éthoxyéthyl)pipérazin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-{2-[4-(2-hydroxyéthyl)pipérazin-1-yl]-2-oxoéthyl}-1*H*-indole-3-carboxamide ;
- N-[6-(4-butyryl-5-méthyl-1*H*-pyrazol-1-yl)pyridazin-3-yl]-5-chloro-1-(2-{[2-(diméthylamino)éthyl](méthyl)amino}-2-oxoéthyl)-1*H*-indole-3-carboxamide ;
- N-[6-(4-butyryl-5-méthyl-1*H*-pyrazol-1-yl)pyridazin-3-yl]-5-chloro-1-[2-(4-méthyl-1,4-diazépan-1-yl)-2-oxoéthyl]-1*H*-indole-3-carboxamide ;
- N-[4-(4-butyryl-5-méthyl-1*H*-pyrazol-1-yl)phényl]-5-chloro-1-[2-(8-méthyl-9-oxooctahydro-2H-pyrazino[1,2-a]pyrazin-2-yl)-2-oxoéthyl]-1*H*-indole-3-carboxamide ;
- N-[4-(4-butyryl-5-méthyl-1*H*-pyrazol-1-yl)-2-cyanophényl]-5-chloro-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-1-{2-[4-(4-fluorobenzyl)pipérazin-1-yl]-2-oxoéthyl}-1*H*-indole-3-carboxamide ;
- N-[4-(4-butyryl-5-méthyl-1*H*-pyrazol-1-yl)phényl]-5-chloro-1-{2-[4-(2-méthoxyéthyl)pipérazin-1-yl]-2-oxoéthyl}-1*H*-indole-3-carboxamide ;
- N-[4-(4-butyryl-5-méthyl-1*H*-pyrazol-1-yl)phényl]-1-{2-[4-(2-methoxyéthyl)pipérazin-1-yl]-2-oxoéthyl}-5-méthyl-1*H*-indole-3-carboxamide ;
- N-[4-(4-butyryl-5-méthyl-1*H*-pyrazol-1-yl)phényl]-5-chloro-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-1-{2-[4-(2-méthoxy-1-méthyléthyl)pipérazin-1-yl]-2-oxoéthyl}-1*H*-indole-3-carboxamide ;
- N-[4-(4-butyryl-5-méthyl-1*H*-pyrazol-1-yl)phényl]-5-méthyl-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-6-fluoro-1-{2-[4-(2-methoxyéthyl)pipérazin-1-yl]-2-oxoéthyl}-1*H*-indole-3-carboxamide ;
- N-[4-(4-butyryl-5-méthyl-1*H*-pyrazol-1-yl)phényl]-5-méthoxy-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-1-{2-[4-méthyl-3-(méthylcarbamoyl)pipérazin-1-yl]-2-oxoéthyl}-1*H*-indole-3-carboxamide ;
- N-[4-(4-butyryl-5-méthyl-1*H*-pyrazol-1-yl)phényl]-5,6-diméthyl-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-méthyl-1-[2-(4-méthylpipérazin-1-yl)-2-oxoéthyl]-1*H*-indazole-3-carboxamide ;
- N-[4-(4-butyryl-5-méthyl-1*H*-pyrazol-1-yl)phényl]-5-méthyl-1-(2-oxo-2-pipérazin-1-yléthyl)-1*H*-indole-3-carboxamide ;
- N-[4-(4-butyryl-5-méthyl-1*H*-pyrazol-1-yl)phényl]-5-chloro-6-fluoro-1-[2-(4-méthylpipérazin-1-yl)-2-oxoéthyl]-1*H*-indole-3-carboxamide ;
- N-[4-(4-butyryl-5-méthyl-1*H*-pyrazol-1-yl)phényl]-5-chloro-1-(2-{4-[(5-méthylisoxazol-3-yl)méthyl]pipérazin-1-yl}-2-oxoéthyl)-1*H*-indole-3-carboxamide ;
- N-[4-(4-butyryl-5-méthyl-1*H*-pyrazol-1-yl)phényl]-5-méthyl-1-(2-morpholin-4-yl-2-oxoéthyl)-1*H*-indole-3-carboxamide;
- N-[4-(4-butyryl-5-méthyl-1*H*-pyrazol-1-yl)phényl]-5-chloro-1-(2-oxo-2-pipérazin-1-yléthyl)-1*H*-indole-3-carboxamide ;
- N-[4-(4-butyryl-5-méthyl-1*H*-pyrazol-1-yl)phényl]-5-chloro-1-{2-[4-(2-méthoxy-1,1-diméthyléthyl)pipérazin-1-yl]-2-oxoéthyl}-1*H*-indole-3-carboxamide ;
- N-[4-(4-butyryl-5-méthyl-1*H*-pyrazol-1yl)phényl]-5-chloro-1-{2-[4-(2-hydroxy-1,1-diméthyléthyl)pipérazin-1-yl]-2-oxoéthyl}-1*H*-indole-3-carboxamide ;
- N-[4-(4-butyryl-5-méthyl-1*H*-pyrazol-1-yl)phényl]-5-chloro-1-[2-(8-méthyl-3,8-diazabicyclo[3.2.1]oct-3-yl)-2-oxoéthyl]-1*H*-indole-3-carboxamide ;
- N-[4-(4-butyryl-5-méthyl-1*H*-pyrazol-1-yl)phényl]-1-[2-(4-méthylpipérazin-1-yl)-2-oxoéthyl]-5-(trifluorométhyl)-1*H*-indole-3-carboxamide ;
- N-[4-(4-butyryl-5-méthyl-1*H*-pyrazol-1-yl)phényl]-5-chloro-1-[2-oxo-2-(9-oxooctahydro-2H-pyrazino[1,2-a]pyrazin-2-yl)éthyl]-1H-indole-3-carboxamide ;
à l'état de base ou de sel d'addition à un acide ou à une base.

12. Procédé de préparation des composés de formule (I) selon la revendication 1 dans laquelle R₃ = -OH (composé IA), **caractérisé en ce que** :
on hydrolyse, en milieu acide ou basique, un composé de formule (II) : 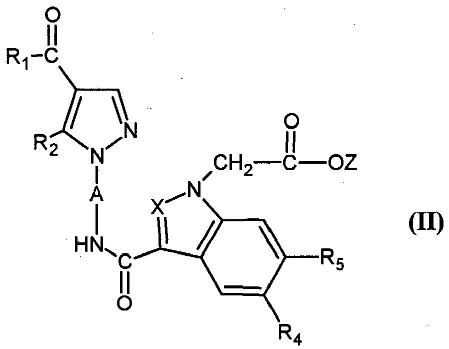 dans laquelle A, X, R₁, R₂, R₄ et R₅ sont tels que définis pour un composé de formule (I) à la revendication 1 et Z représente un (C₁-C₄) alkyle.

13. Procédé de préparation des composés de formule (I) selon la revendication 1 dans laquelle R₃ = -NR₇R₈ (composé IB), **caractérisé en ce que** :
on fait réagir un composé de formule (IA) : 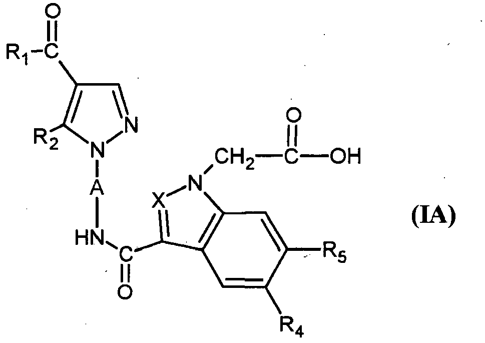 dans laquelle A, X, R₁, R₂, R₄ et R₅ sont tels que définis pour un composé de formule (I) à la revendication 1, avec une amine de formule (III) :
HNR₇R₈ **(III)**
dans laquelle R₇ et R₈ sont tels que définis pour un composé de formule (I) à la revendication 1

14. Composé de formule : 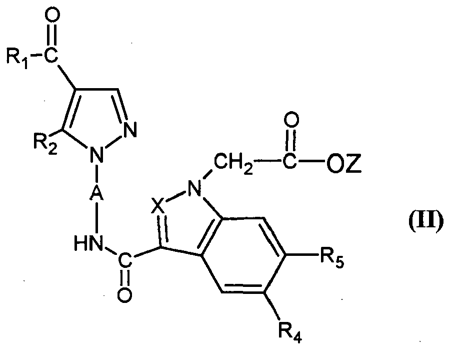 dans laquelle :
- A représente un radical aromatique bivalent choisi parmi : 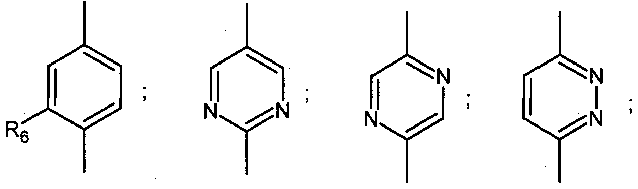
- X représente un groupe -CH- ou un atome d'azote ;
- R₁ représente un (C₁-C₄)alkyle ou un (C₁-C₄)alcoxy ;
- R₂ représente un groupe Alk ;
- R₄ représente un atome d'hydrogène, un atome d'halogène, un cyano, un phényle, un groupe Alk, un groupe OAlk ou un groupe -NR₉R₁₀ ;
- R₅ représente un atome d'hydrogène, un atome d'halogène ou un groupe Alk ;
- R₆ représente un atome d'hydrogène, un atome d'halogène, un cyano, un groupe
- COOAlk ou un groupe -CONH₂ ;
- R₉ et R₁₀ représentent chacun indépendamment un atome d'hydrogène ou un (C₁-C₄)alkyle ;
- Z représente un (C₁-C₄)alkyle ;
- Alk représente un (C₁-C₄)alkyle non substitué ou substitué une ou plusieurs fois par un atome de fluor.

15. Médicament, **caractérisé en ce qu'**il contient un composé de formule (I) selon l'une quelconque des revendications 1 à 11, ou encore un sel pharmaceutiquement acceptable du composé de formule (I).

16. Composition pharmaceutique, **caractérisé en ce qu'**elle comprend un composé de formule (I) selon l'une quelconque des revendications 1 à 11, un sel pharmaceutiquement acceptable de ce composé, ainsi qu'au moins un excipient pharmaceutiquement acceptable.

17. Composé de formule (I) selon l'une quelconque des revendications 1 à 11 pour le traitement et la prévention d'angor (angine de poitrine) instable, d'angioplastie coronarienne percutanée (PTCA), d'infarctus du myocarde, des complications artérielles thrombotiques de l'athérosclérose tels que les accidents vasculaires cérébraux emboliques ou thrombotiques, les accidents ischémiques transitoires, la maladie vasculaire périphérique, les complications artérielles de l'athérosclérose dues à des interventions chirurgicales comme l'angioplastie, l'endartériectomie, la pose de stent, les greffes vasculaires coronaires , les complications thrombotiques de la chirurgie ou de dommages mécaniques comme la récupération de tissus après un traumatisme accidentel ou chirurgical, la chirurgie reconstructive (y compris la peau et les lambeaux musculaires), le purpura thrombotique thrombocytopénique, le syndrome des anti-phospholipides, la thrombocytopénie induite par l'héparine et la pré-éclampsie/éclampsie; ou les thromboses veineuses telles que la thrombose veineuse profonde, la maladie veino-occlusive; ou dans la prévention de l'activation plaquettaire induite mécaniquement *in vivo,* comme lors de la dérivation cardiopulmonaire et de l'oxygénation extracorporelle (prévention des micro-thrombo-embolismes), dans la prévention de l'activation plaquettaire induite mécaniquement *in vitro* (utilisation dans la conservation des produits sanguins (les concentrés plaquettaires ), utilisation lors de dérivations telles que la dialyse rénale et la plasmaphérèse), la thrombose secondaire à une lésion vasculaire / inflammation tels que l'angéite, l'artérite, la glomérulonéphrite, et le rejet de greffe d'organes, les conditions dans lesquelles les plaquettes peuvent contribuer au processus de la maladie inflammatoire sous-jacente dans la paroi vasculaire tel que la formation / progression de plaques athéromateuses, la sténose / resténose.

## Patentansprüche

1. Verbindung der Formel (I): 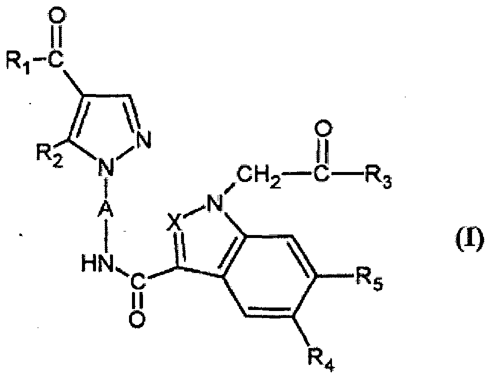 worin:
- A für einen zweiwertigen aromatischen Rest, der aus: 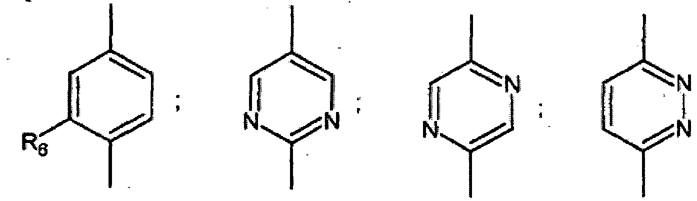
ausgewählt ist, steht;
- X für eine -CH-Gruppe oder ein Stickstoffatom steht;
- R₁ für ein (C₁-C₄)-Alkyl oder ein (C₁-C₄)-Alkoxy steht;
- R₂ für eine Alk-Gruppe steht;
- R₃ für ein Hydroxy oder eine -NR₇R₈-Gruppe steht;
- R₄ für ein Wasserstoffatom, ein Halogenatom, ein Cyano, ein Phenyl, eine Alk-Gruppe, eine OAlk-Gruppe oder eine -NR₉R₁₀-Gruppe steht;
- R₅ für ein Wasserstoffatom, ein Halogenatom oder eine Alk-Gruppe steht;
- R₆ für ein Wasserstoffatom, ein Halogenatom, ein Cyano, eine -COOAlk-Gruppe oder eine -CONH₂-Gruppe steht;
- R₇ für ein Wasserstoffatom oder ein (C₁-C₄)-Alkyl steht;
- R₈ für:
a) ein Wasserstoffatom;
b) ein (C₁-C₄) -Alkyl, das unsubstituiert oder durch:
(i) ein Hydroxy;
(ii) eine OAlk-Gruppe;
(iii) eine -NR₉R₁₀-Gruppe;
(iv) ein (C₃-C₆)-Heterocycloalkyl, das unsubstituiert oder durch ein (C₁-C₄)-Alkyl oder durch eine -COOAlk-Gruppe substituiert ist;
(v) ein Heteroaryl, das unsubstituiert oder durch ein (C₁-C₄)-Alkyl substituiert ist;
substituiert ist;
c) ein (C₃-C₇)-Cycloalkyl;
d) ein (C₃-C₆)-Heterocycloalkyl, das unsubstituiert oder durch ein (C₁-C₄)-Alkyl, eine -COOAlk-Gruppe oder durch ein oder zwei Oxo substituiert ist;
e) eine -SO₂-Alk-Gruppe;
steht
- oder R₇ und R₈ zusammen mit dem Stickstoffatom, an das sie gebunden sind, einen gesättigten oder ungesättigten, mono- oder polyzyklischen, kondensierten oder verbrückten 4- bis 10-gliedrigen Heterocyclus bilden, der ein, zwei oder drei weitere Stickstoffatome oder ein weiteres Heteroatom, das aus einem Sauerstoff- oder Schwefelatom ausgewählt ist, enthalten kann; wobei der Heterocyclus unsubstituiert oder ein-, zwei- oder dreifach durch Substituenten substituiert ist, die unabhängig voneinander ausgewählt sind aus:
a) einem Halogenatom;
b) einem Hydroxy;
c) einer -OR₁₁-Gruppe;
d) einem Oxo;
e) einer -NR₉R₁₀-Gruppe;
f) einer -NR₁₂COR₁₃-Gruppe;
g) einer -NR₁₂COOR₁₃-Gruppe;
h) einer -COR₁₃-Gruppe;
i) einer -COOR₁₃-Gruppe;
j) einer -CONR₁₄R₁₅-Gruppe;
k) einem (C₃-C₇)-Cycloalkyl, das unsubstituiert oder durch ein Hydroxy oder durch ein (C₁-C₄)-Alkyl substituiert ist;
l) einem (C₃-C₆)-Heterocycloalkyl, das unsubstituiert oder durch ein oder zwei Oxo substituiert ist;
m) einem Phenyl, das unsubstituiert oder ein- oder mehrfach durch Substituenten, die unabhängig voneinander aus einem Halogenatom, einer Alk-Gruppe oder einer OAlk-Gruppe ausgewählt sind, substituiert ist;
n) einem Pyridinyl;
o) einem (C₁-C₄)-Alkyl, das unsubstituiert oder ein- oder mehrfach durch Substituenten substituiert ist, die unabhängig voneinander ausgewählt sind aus:
(i) einem Halogenatom;
(ii) einem Hydroxy;
(iii) einer -OR₁₁-Gruppe;
(iv) einer -NR₉R₁₀-Gruppe;
(v) einer -NR₁₂COR₁₃-Gruppe;
(vi) einer -COOR₁₃-Gruppe;
(vii) einer -CONR₁₄R₁₅-Gruppe;
(viii) einer -SO₂Alk-Gruppe;
(ix) einem (C₃-C₇)-Cycloalkyl;
(x) einem (C₃-C₆)-Heterocycloalkyl;
(xi) einem Phenyl, das unsubstituiert oder ein- oder mehrfach durch Substituenten substituiert ist, die unabhängig voneinander aus einem Halogenatom, einer Alk-Gruppe oder einer OAlk-Gruppe ausgewählt sind;
(xii) einem Heteroaryl, das unsubstituiert oder durch ein (C₁-C₄)-Alkyl substituiert ist;
- R₉ und R₁₀ jeweils unabhängig voneinander für ein Wasserstoffatom oder ein (C₁-C₄)-Alkyl stehen;
- R₁₁ für eine Alk-Gruppe, ein -(C₁-C₄)-Alkylen-OH oder ein -(C₁-C₄)-Alkylen-OAlk stehen;
- R₁₂ für ein Wasserstoffatom oder ein (C₁-C₄)-Alkyl steht;
- R₁₃ für (C₁-C₄)-Alkyl steht;
- R₁₄ und R₁₅ jeweils unabhängig voneinander für ein Wasserstoffatom, ein (C₁-C₄)-Alkyl oder ein (C₃-C₇)-Cycloalkyl stehen;
- oder R₁₄ und R₁₅ zusammen mit dem Stickstoffatom, an das sie gebunden sind, einen heterocyclischen Rest bilden, der ausgewählt ist aus: Azetidin-1-yl, Pyrrolidin-1-yl, Piperidin-1-yl, Morpholin-4-yl oder Piperazin-1-yl
- Alk für ein (C₁-C₄)-Alkyl, das unsubstituiert oder ein- oder mehrfach durch ein Fluoratom substituiert ist, steht;
in Basen- oder Säure- oder Basenadditionssalzform.

2. Verbindung der Formel (I) nach Anspruch 1, worin R₃ für ein Hydroxy steht (Referenz IA) und die anderen Substituenten A, X, R₁, R₂, R₄ und R₅ wie für eine Verbindung der Formel (I) in Anspruch 1 definiert sind;
in Basen- oder Säure- oder Basenadditionssalzform.

3. Verbindung der Formel (I) nach Anspruch 1, worin R₃ für eine Gruppe -NR₇R₈ steht (Referenz IB) und die anderen Substituenten A, X, R₁, R₂, R₄, R₅, R₇ und R₈ wie für eine Verbindung der Formel (I) in Anspruch 1 definiert sind;
in Basen- oder Säure- oder Basenadditionssalzform.

4. Verbindung der Formel (I) nach Anspruch 1, worin:
- A für einen zweiwertigen aromatischen Rest, der unter : 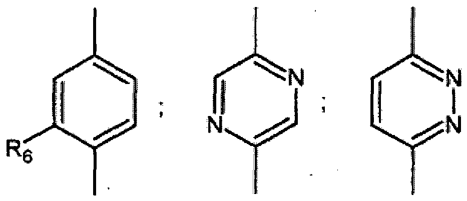 ausgewählt ist, steht;
- X für eine -CH-Gruppe oder ein Stickstoffatom steht;
- R₁ für einen Methyl-, Ethyl- oder n-Propylrest oder einen Ethoxyrest steht;
- R₂ für einen Methylrest steht;
- R₃ für ein Hydroxy oder eine -NR₇R₈-Gruppe steht;
- R₄ für ein Wasserstoffatom, ein Brom-, Chlor- oder Fluoratom, ein Cyano, ein Phenyl, einen Methylrest, einen Trifluormethylrest, einen Methoxyrest oder eine Dimethylaminogruppe steht;
- R₅ für ein Wasserstoffatom, ein Brom-, Chlor- oder Fluoratom oder einen Methylrest steht;
- R₆ für ein Wasserstoffatom, ein Bromatom, ein Cyano, eine Methoxycarbonylgruppe oder eine -CONH₂-Gruppe steht;
- R₇ für ein Wasserstoffatom oder einen Methylrest steht;
- R₈ für:
a) ein Wasserstoffatom;
b) einen Methylrest, ein 2-Hydroxyethyl, ein 3-Hydroxypropyl, ein 2-Methoxyethyl, ein 2-(Methyl-amino)ethyl, ein 2-(Dimethylamino)ethyl, ein 2-(Dimethylamino)propyl, ein 2-(Dimethylamino)-1-methylethyl, ein 2-(Dimethylamino)-2-methylpropyl, ein 3-(Dimethylamino)propyl, ein (1-Methylpiperidin-3-yl)methyl, ein Tetrahydrofuran-3-ylmethyl, ein Tetrahydro-2*H*-pyran-4-ylmethyl, ein 2-Furylmethyl, ein (3-Methyl-1*H*-1,2,4-triazol-5-yl)methyl, 1-(1*H*-Tetrazol-5-yl)ethyl, ein 2-(1*H-*Pyrrol-1-yl)ethyl oder ein 2-(1*H*-Imidazol-1-yl)ethyl;
c) ein Cyclopropyl;
d) ein 1,1-Dioxidotetrahydro-3-thienyl, ein Pyrrolidin-3-yl, ein 1-Methylpyrrolidin-3-yl, ein (1-(*tert*.-Butoxycarbonyl)pyrrolidin-3-yl, ein Piperidin-3-yl, ein 1-Methylpiperidin-3-yl, ein 1-(*tert*.-Butoxycarbonyl)piperidin-3-yl oder ein 1-Methylpiperidin-4-yl;
e) eine Methylsulfonylgruppe
steht;
- oder R₇ und R₈ zusammen mit dem Stickstoffatom, an das sie gebunden sind, einen Heterocyclus bilden, der ausgewählt ist aus: einem Azetidin, einem Pyrrolidin, einem Piperidin, einem Piperazin, einem Morpholin, einem Thiomorpholin, einem Octahydro-2*H*-pyrido[1,2-*a*]pyrazin, einem 5,6,7,8-Tetrahydro[1,2,4]triazolo[4,3-a]pyrazin, einem 2,3-Dihydro-1*H*-pyrrolo[3,4-*c*]pyridin, einem Octahydropyrrolo[1,2-*a*]pyrazin, einem 1,4-Diazepan, einem 5,6,7,8-Tetrahydroimidazo[1,2-a]pyrazin, einem 4,5,6,7-Tetrahydro-1*H-*pyrazolo[4,3-*c*]pyridin, einem 1,4,5,6-Tetrahydropyrrolo[3,4-*c*]pyrazol, einem 6,7-Dihydro-5*H-*pyrrolo[3,4-*b*]pyridin, einem 3,8-Diazabicyclo[3.2.1]octan, einem 4,5,6,7-Tetrahydro-1*H-*imidazo[4,5-*c*]pyridin, einem 4,5,6,7-Tetrahydro-1*H*-pyrazolo[3,4-*c*]pyridin, einem 2,5-Diazabicyclo[2.2.2]octan, einem 2,5-Diazabicyclo[2.2.1]heptan, einem Octahydropyrrolo[3,4-*b*]pyrrol, einem Octahydropyrrolo[3,4-*c*]pyrrol, einem Octahydro-2H-pyrazino[1,2-*a*]pyrazin; wobei der Heterocyclus unsubstituiert oder ein-, zwei-oder dreifach durch Substituenten substituiert ist, die unabhängig voneinander ausgewählt sind aus:
a) einem Fluoratom;
b) einem Hydroxy;
c) einem Methoxyrest, einem 2-Hydroxyethoxy, einem 2-Methoxyethoxy;
d) einem Oxo;
e) einer Aminogruppe, einem Methylamino, einem Dimethylamino;
f) einer Acetamidogruppe;
g) einer (*tert*.-Butoxycarbonyl)aminogruppe, einem (*tert*.-Butoxycarbonyl)(methyl)amino;
h) einer Acetylgruppe;
i) einer Methoxycarbonylgruppe, einem *tert*.-Butoxycarbonyl;
j) einer Dimethylcarbamoylgruppe, einem Cyclopropylcarbamoyl, einem Cyclobutylcarbamoyl;
k) einem Cyclopropylrest, einem Cyclobutyl, einem 2-Hydroxycylopentyl;
l) einem Oxetan-3-yl, einem 1,1-Dioxidotetrahydro-3-thienyl, einem Piperidin-1-yl, einem Morpholin-4-yl;
m) einem 4-Fluorphenyl;
n) einem Pyridin-2-yl;
o) einem Methylrest, einem Ethyl, einem n-Propyl, einem Isopropyl, einem n-Butyl, einem Trifluormethyl, einem 2-Fluorethyl, einem 2,2-Difluorethyl, einem 2,2,2-Trifluorethyl, einem 3,3,3-Trifluorpropyl, einem 3,3,3-Trifluor-2-hydroxypropyl, einem Hydroxymethyl, einem 2-Hydroxyethyl, einem 2-Hydroxy-1,1-Dimethylethyl, einem Methoxymethyl, einem 2-Methoxyethyl, einem 2-Methoxy-1-methylethyl, einem 2-Methoxy-1,1-dimethylethyl, einem 2-Ethoxyethyl, einem 2-(Trifluormethoxy)ethyl, einem 2-(2-Hydroxyethoxy)ethyl, einem 2-(2-Methoxyethoxy)ethyl, einem 2-(Dimethylamino)ethyl, einem 2-Acetamidoethyl, einem 2-[Acetyl(methyl)amino]ethyl, einem 2-Methoxy-2-oxoethyl, einem 2-Ethoxy-2-Oxoethyl, einem 3-Ethoxy-3-oxopropyl, einem 2-Amino-2-oxoethyl, einem 2-(Methylamino)2-oxoethyl, einem 2-(Isopropylamino)-2-oxoethyl, einem 2-(Dimethylamino)-2-oxoethyl, einem 2-(Cyclopropylamino)-2-oxoethyl, einem 2-Oxo-2-pyrrolidin-1-ylethyl, einem 2-(Methylsulfonyl)ethyl, einem Cyclopropylmethyl, einem Pyrrolidin-1-ylmethyl, einem Tetrahydrofuran-2-ylmethyl, einem 2-Thienylmethyl, einem 4-Chlorbenzyl, einem 4-Fluorbenzyl, einem 2-Methoxybenzyl, einem 3-Fluor-4-methoxybenzyl, einem Pyridin-4-ylmethyl, einem (5-Methyl-1,2,4-oxadiazol-3-yl)methyl, einem (5-Methylisoxazol-3-yl)methyl;
in Basen- oder Säure- oder Basenadditionssalzform.

5. Verbindung der Formel (I) nach Anspruch 1, worin:
- A für einen zweiwertigen aromatischen Rest, der aus: 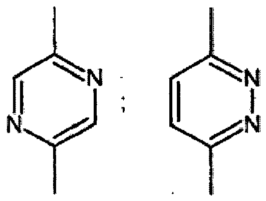 ausgewählt ist, steht;
- X für eine -CH-Gruppe steht;
- R₁ für einen n-Propylrest steht;
- R₂ für einen Methylrest steht;
- R₃ für ein Hydroxy oder eine -NR₇R₈-Gruppe steht;
- R₄ für ein Chloratom steht;
- R₅ für ein Wasserstoffatom steht;
- R₇ für ein Wasserstoffatom oder einen Methylrest steht;
- R₈ für:
b) einen Methylrest, ein 2-Hydroxyethyl, ein 3-Hydroxypropyl, ein 2-Methoxyethyl, ein 2-(Dimethylamino)ethyl, ein 2-(Dimethylamino)propyl, ein 2-(Dimethylamino)-2-methylpropyl, ein 3-(Dimethylamino)propyl, ein Tetrahydrofuran-3-ylmethyl, ein Tetrahydro-2*H*-pyran-4-ylmethyl, ein 2-Furylmethyl, ein (3-Methyl-1*H*-1,2,4-triazol-5-yl)methyl, 1-(1*H*-Tetrazol-5-yl)ethyl, ein 2-(1*H-*Pyrrol-1-yl)ethyl;
c) ein Cyclopropyl;
d) ein 1-Methylpyrrolidin-3-yl, ein 1-(*tert*.-Butoxycarbonyl)piperidin-3-yl, ein 1-Methylpiperidin-4-yl,
steht;
- oder R₇ und R₈ zusammen mit dem Stickstoffatom, an das sie gebunden sind, einen Heterocyclus bilden, der ausgewählt ist aus: einem Azetidin, einem Pyrrolidin, einem Piperidin, einem Piperazin, einem Morpholin, einem Thiomorpholin, einem Octahydro-2*H*-pyrido[1,2-*a*]pyrazin, einem 5,6,7,8-Tetrahydro[1,2,4]triazolo[4,3-*a*]pyrazin, einem 2,3-Dihydro-1*H*-pyrrolo[3,4-*c*]pyridin, einem Octahydropyrrolo[1,2-*a*]pyrazin, einem 1,4-Diazepan; wobei der Heterocyclus unsubstituiert oder ein-, zwei- oder dreifach durch Substituenten substituiert ist, die unabhängig voneinander ausgewählt sind aus:
b) einem Hydroxy;
c) einem Methoxyrest;
d) einem Oxo;
h) einer Acetylgruppe;
i) einer Methoxycarbonylgruppe;
j) einer Dimethylcarbamoylgruppe;
k) einem Cyclobutylrest;
l) einem 1,1-Dioxidotetrahydro-3-thienyl, einem Piperidin-1-yl, einem Morpholin-4-yl;
m) einem 4-Fluorphenyl;
o) einem Methylrest, einem Ethyl, einem n-Propyl, einem Isopropyl, einem n-Butyl, einem Trifluormethyl, einem 3,3,3-Trifluorpropyl, einem Hydroxymethyl, einem 2-Hydroxyethyl, einem Methoxymethyl, einem 2-Methoxyethyl, einem 2-Ethoxyethyl, einem 2-(Trifluormethoxy)ethyl, einem 2-(Dimethylamino)ethyl, einem 2-Methoxy-2-oxoethyl, einem 2-Ethoxy-2-Oxoethyl, einem 2-(Isopropylamino)-2-oxoethyl, einem 2-(Dimethylamino)-2-oxoethyl, einem 2-(Cyclopropylamino)-2-oxoethyl, einem 2-Oxo-2-pyrrolidin-1-ylethyl, einem Tetrahydrofuran-2-ylmethyl, einem 4-Fluorbenzyl, einem 3-Fluor-4-methoxybenzyl, einem Pyridin-4-ylmethyl, einem (5-Methyl-1,2,4-oxadiazol-3-yl)methyl;
in Basen- oder Säure- oder Basenadditionssalzform.

6. Verbindung der Formel (I) nach Anspruch 1, worin:
- A für einen zweiwertigen aromatischen Rest: 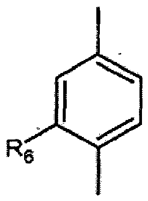 steht;
- X für eine -CH-Gruppe oder ein Stickstoffatom steht;
- R₁ für einen Methyl-, Ethyl- oder n-Propylrest oder einen Ethoxyrest steht;
- R₂ für einen Methylrest steht;
- R₃ für ein Hydroxy oder eine -NR₇R₈-Gruppe steht;
- R₄ für ein Wasserstoffatom, ein Brom-, Chlor-oder Fluoratom, ein Cyano, ein Phenyl, einen Methylrest, einen Trifluormethylrest, einen Methoxyrest oder eine Dimethylaminogruppe steht;
- R₅ für ein Wasserstoffatom, ein Brom-, Chlor-oder Fluoratom oder einen Methylrest steht;
- R₆ für ein Wasserstoffatom, ein Bromatom, ein Cyano, eine Methoxycarbonylgruppe oder eine -CONH₂-Gruppe steht;
- R₇ für ein Wasserstoffatom oder einen Methylrest steht;
- R₈ für:
b) einen Methylrest, ein 2-Hydroxyethyl, ein 2-Methoxyethyl, ein 2-(Methylamino)ethyl, ein 2-(Dimethylamino)ethyl, ein 2-(Dimethylamino)-1-methylethyl, ein (1-Methylpiperidin-3-yl)methyl, ein 2-(1H-Imidazol-1-yl)ethyl;
d) ein 1,1-Dioxidotetrahydro-3-thienyl, ein Pyrrolidin-3-yl, ein 1-Methylpyrrolidin-3-yl, ein (1-*tert*.-Butoxycarbonyl)pyrrolidin-3-yl, ein Piperidin-3-yl, ein 1-Methylpiperidin-3-yl, ein 1-(*tert*.-Butoxycarbonyl)piperidin-3-yl;
e) eine Methylsulfonylgruppe,
steht;
- oder R₇ und R₈ zusammen mit dem Stickstoffatom, an das sie gebunden sind, einen Heterocyclus bilden, der ausgewählt ist aus: einem Azetidin, einem Pyrrolidin, einem Piperidin, einem Piperazin, einem Morpholin, einem 5,6,7,8-Tetrahydro[1,2,4]triazolo[4,3-*a*]pyrazin, einem Octahydropyrrolo[1,2-*a*]pyrazin, einem 1,4-Diazepan, einem 5,6,7,8-Tetrahydroimidazo[1,2-a]pyrazin, einem 4,5,6,7-Tetrahydro-1*H-*pyrazolo[4,3-*c*]pyridin, einem 1,4,5,6-Tetrahydropyrrolo[3,4-*c*]pyrazol, einem 6,7-Dihydro-5*H-*pyrrolo[3,4-*b*]pyridin, einem 3,8-Diazabicy-clo[3.2.1]octan, einem 4,5,6,7-Tetrahydro-1*H-*imidazo[4,5-*c*]pyridin, einem 4,5,6,7-Tetrahydro-1H-pyrazolo[3,4-*c*]pyridin, einem 2,5-Diazabicyclo[2.2.2]octan, einem 2,5-Diazabicyclo[2.2.1]heptan, einem Octahydropyrrolo[3,4-*b*]pyrrol, einem Octahydropyrrolo[3,4-*c*]pyrrol, einem Octahydro-2*H*-pyrazino[1,2-*a*]pyrazin; wobei der Heterocyclus unsubstituiert oder ein-, zwei-oder dreifach durch Substituenten substituiert ist, die unabhängig voneinander ausgewählt sind aus:
a) einem Fluoratom;
b) einem Hydroxy;
c) einem 2-Hydroxyethoxy, einem 2-Methoxyethoxy;
d) einem Oxo;
e) einer Aminogruppe, einem Methylamino, einem Dimethylamino;
f) einer Acetamidogruppe;
g) einer (tert.-Butoxycarbonyl)aminogruppe, einem (tert.-Butoxycarbonyl)(methyl)amino;
i) einer Methoxycarbonylgruppe, einem tert.-Butoxycarbonyl;
j) einer Gruppe, einem Cyclopropylcarbamoyl, einem Cyclobutylcarbamoyl;
k) einem Cyclopropylrest, einem Cyclobutyl, einem 2-Hydroxycyclopentyl;
l) einem Oxetan-3-yl, einem Morpholin-4-yl;
n) einem Pyridin-2-yl;
o) einem Methylrest, einem Ethyl, einem n-Propyl, einem Isopropyl, einem n-Butyl, einem Trifluormethyl, einem 2-Fluorethyl, einem 2,2-Difluorethyl, einem 2,2,2-Trifluorethyl, einem 3,3,3-Trifluor-2-hydroxypropyl, einem Hydroxymethyl, einem 2-Hydroxyethyl, einem 2-Hydroxy-1,1-Dimethylethyl, einem Methoxymethyl, einem 2-Methoxyethyl, einem 2-Methoxy-1-methylethyl, einem 2-Methoxy-1,1-dimethylethyl, einem 2-Ethoxyethyl, einem 2-(Trifluormethoxy)ethyl, einem 2-(2-Hydroxyethoxy)ethyl, einem 2-(2-Methoxyethoxy)ethyl, einem 2-(Dimethylamino)ethyl, einem 2-Acetamidoethyl, einem 2-[Acetyl(methyl)amino]ethyl, einem 2-Ethoxy-2-oxoethyl, einem 3-Ethoxy-3-oxopropyl, einem 2-Amino-2-oxoethyl, einem 2-(Methylamino)2-oxoethyl, einem 2-(Dimethylamino)-2-oxoethyl, einem 2-Oxo-2-pyrrolidin-1-ylethyl, einem 2-(Methylsulfonyl)ethyl, einem Cyclopropylmethyl, einem Pyrrolidin-1-ylmethyl, einem 2-Thienylmethyl, einem 4-Chlorbenzyl, einem 4-Fluorbenzyl, einem 2-Methoxybenzyl, einem (5-Methylisoxazol-3-yl)methyl;
in Basen- oder Säure- oder Basenadditionssalzform.

7. Verbindung der Formel (I) nach Anspruch 1, worin:
- A für einen zweiwertigen aromatischen Rest, der aus: 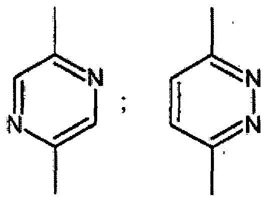 ausgewählt ist, steht;
- X für eine -CH-Gruppe steht;
- R₁ für einen n-Propylrest steht;
- R₂ für einen Methylrest steht;
- R₃ für ein Hydroxy oder eine -NR₇R₈-Gruppe, die aus: 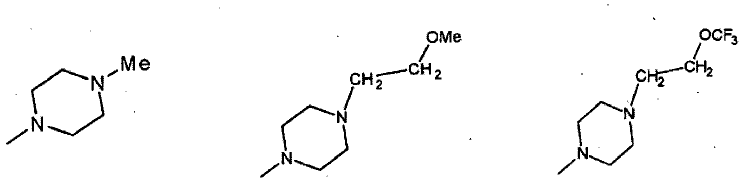 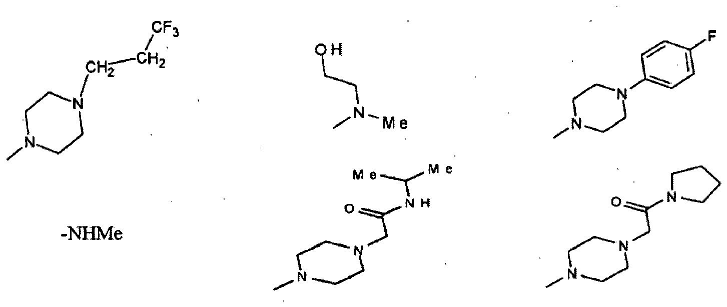 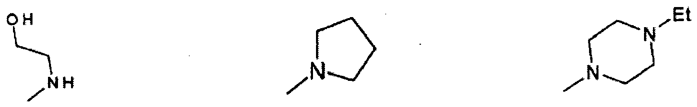 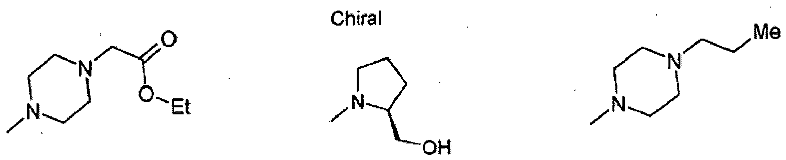 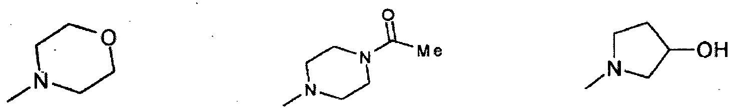 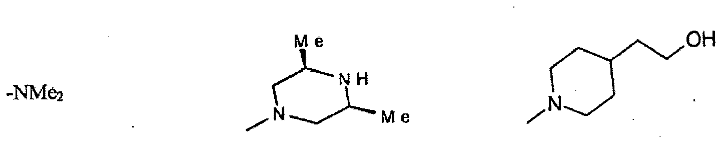 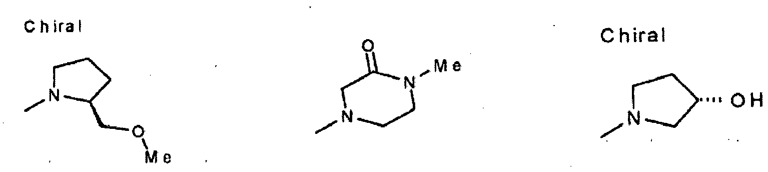 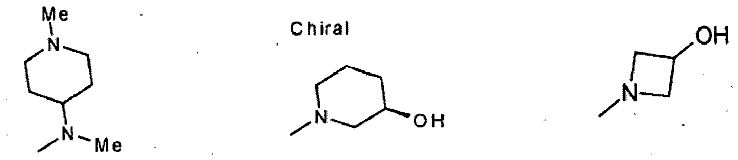 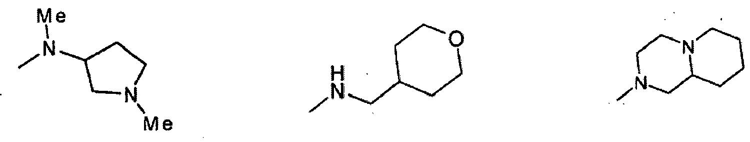 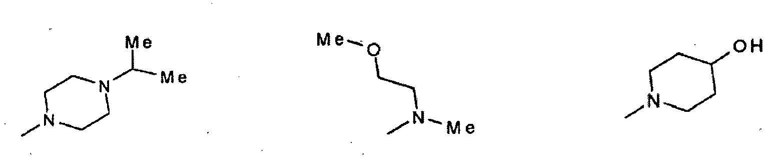 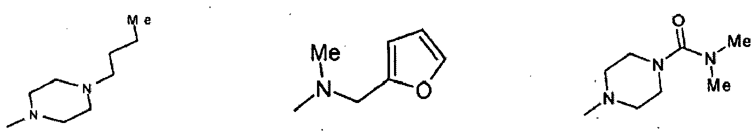 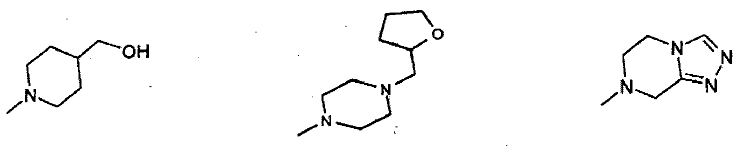 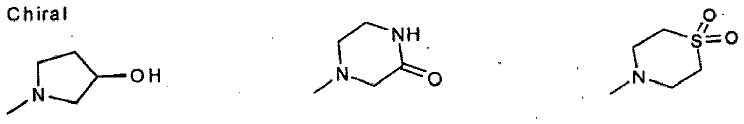 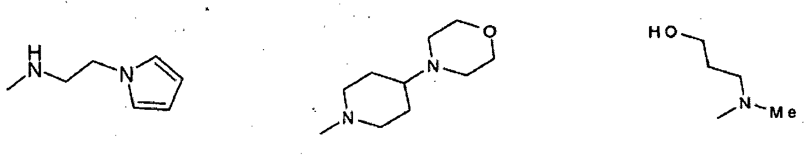 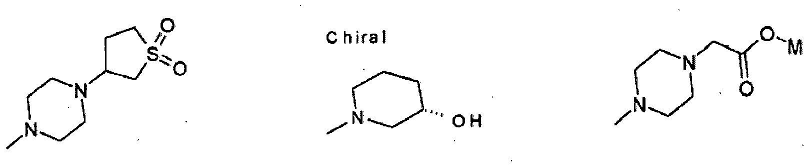 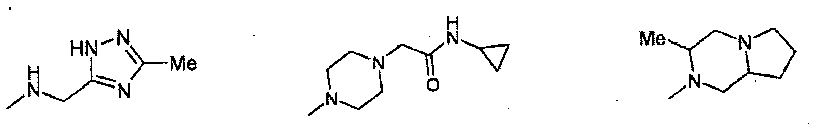 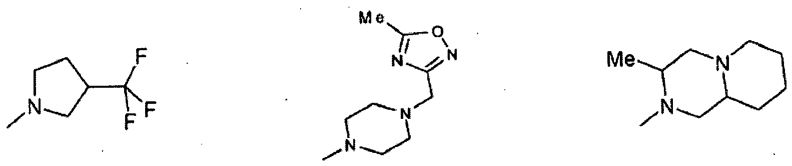 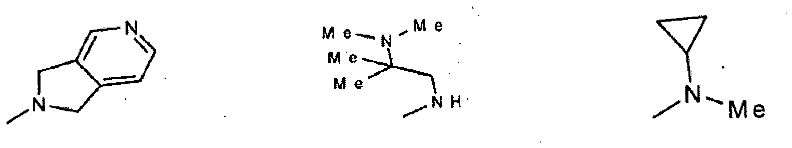 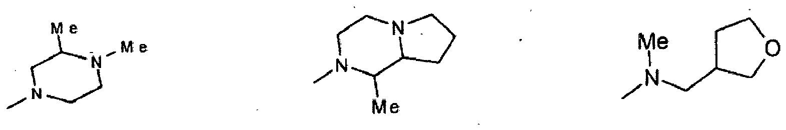 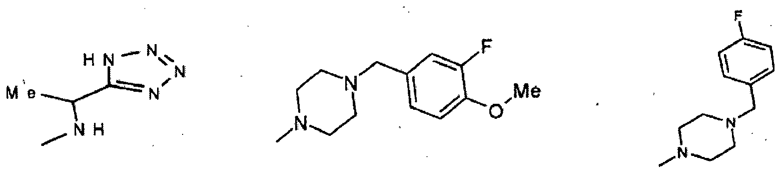 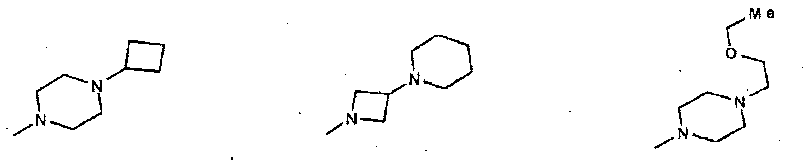 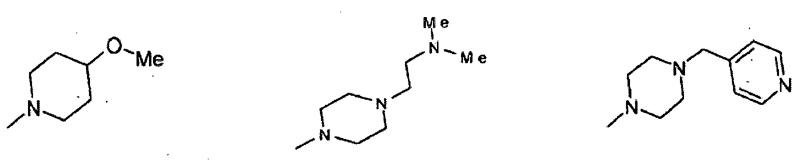 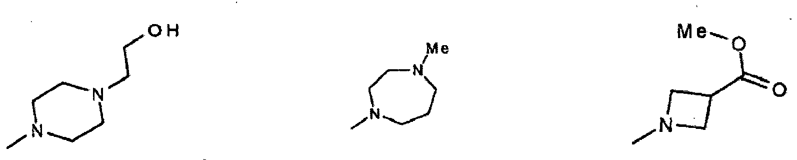 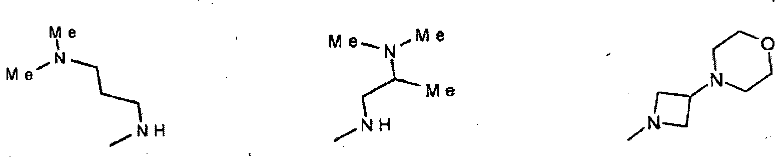 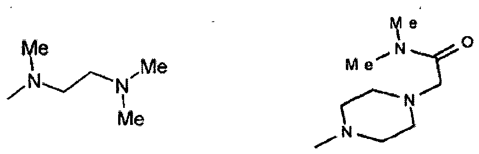 ausgewählt ist, steht;
- R₄ für ein Chloratom steht;
- R₅ für ein Wasserstoffatom steht;
in Basen- oder Säure- oder Basenadditionssalzform.

8. Verbindung der Formel (I) nach Anspruch 1, worin:
- A für einen zweiwertigen aromatischen Rest: 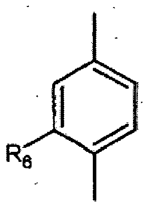 steht;
- X für eine -CH-Gruppe oder ein Stickstoffatom steht;
R₁ für einen Methyl-, Ethyl- oder n-Propylrest oder einen Ethoxyrest steht;
R₂ für einen Methylrest steht;
R₃ für ein Hydroxy oder eine -NR₇-R₈-Gruppe, die aus: 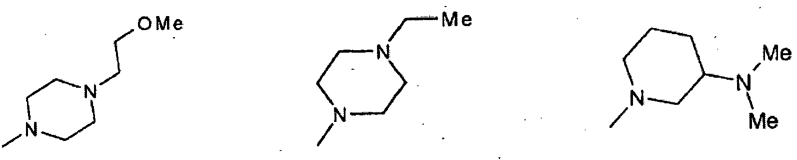 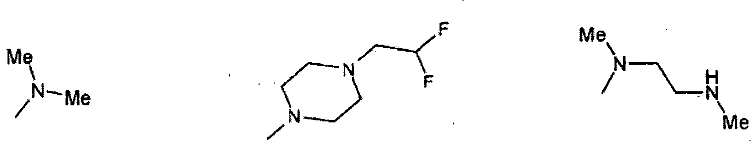 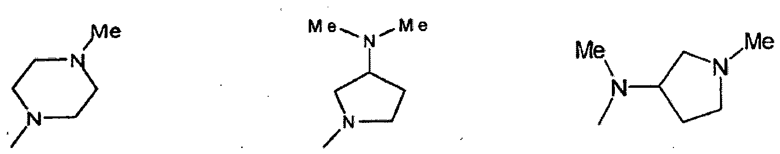 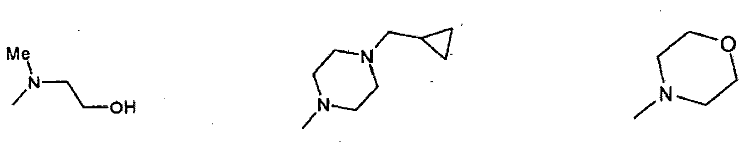 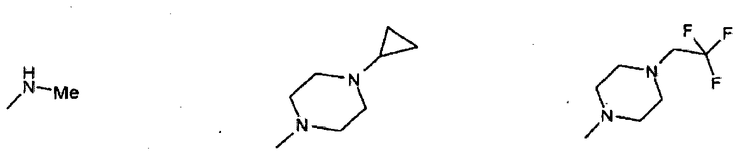 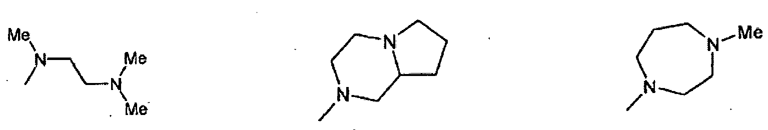 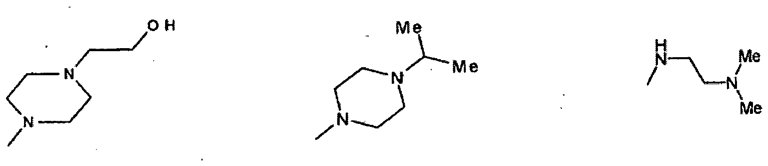 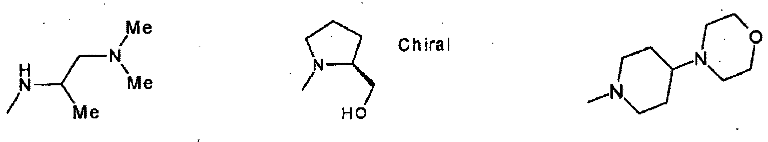 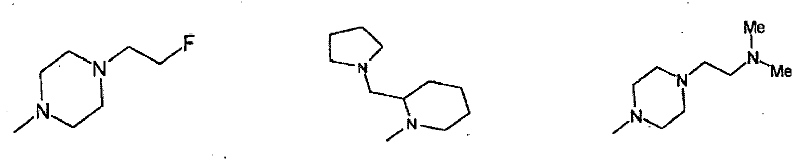 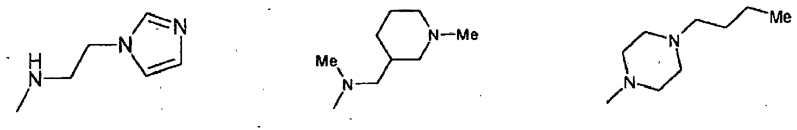 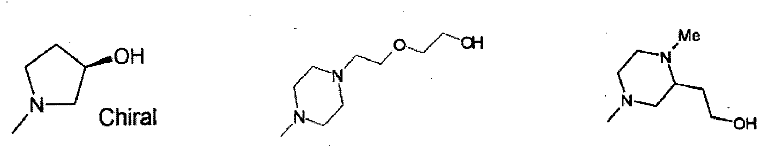 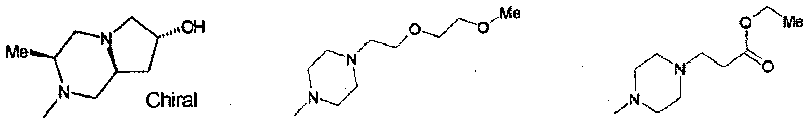 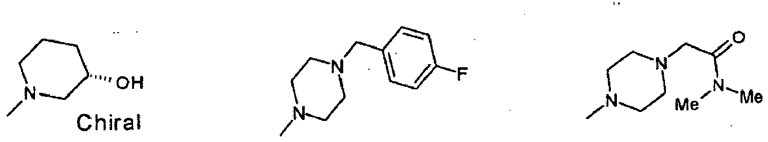 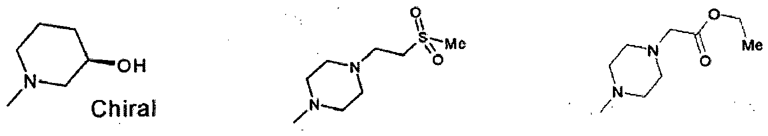 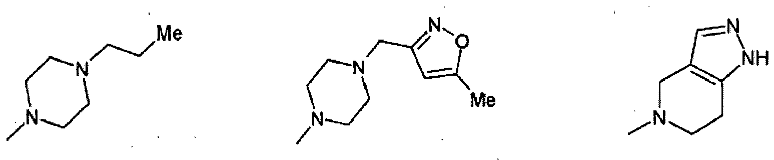 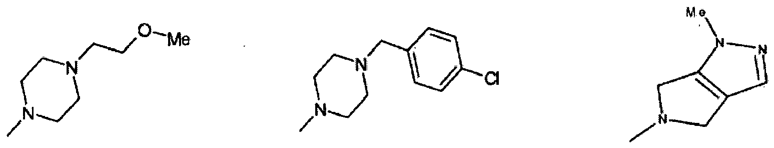 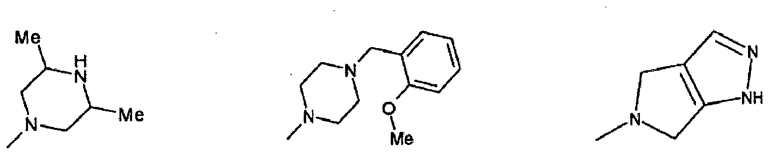 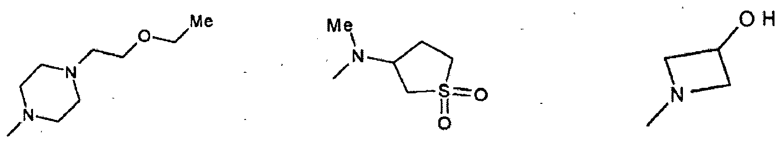 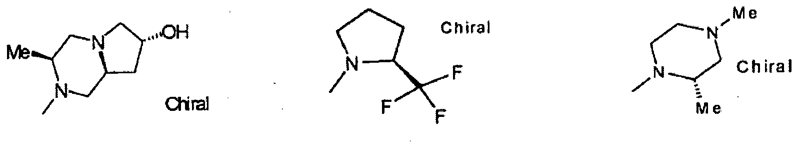 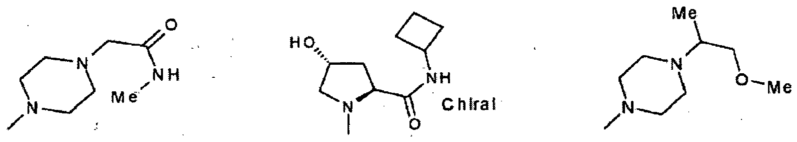 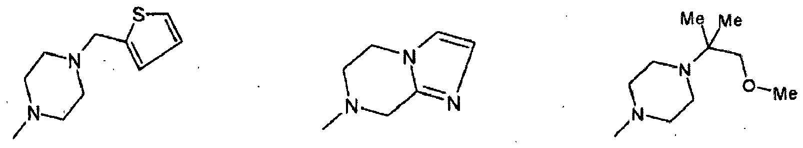 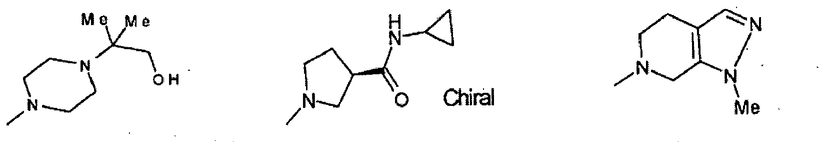 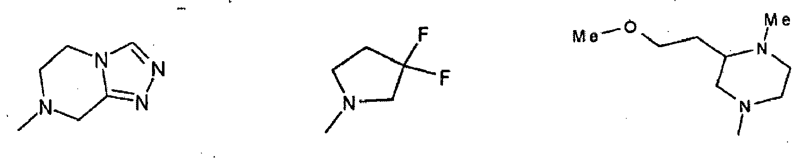 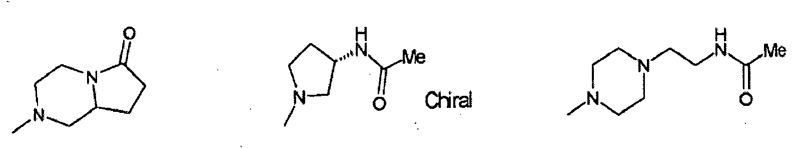 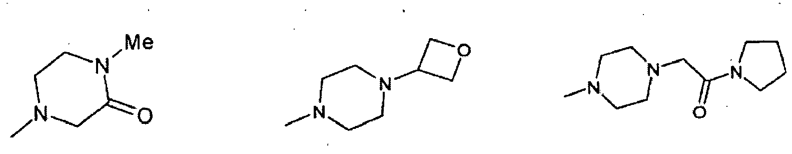 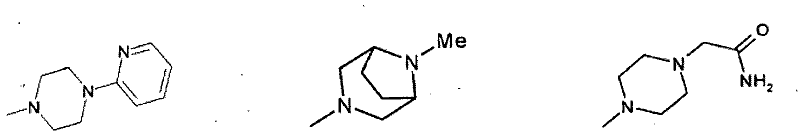 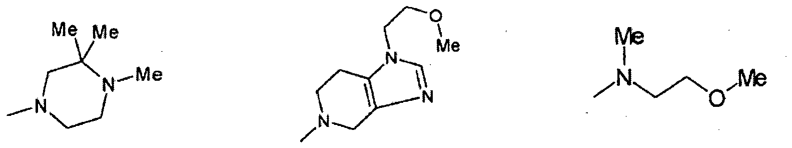 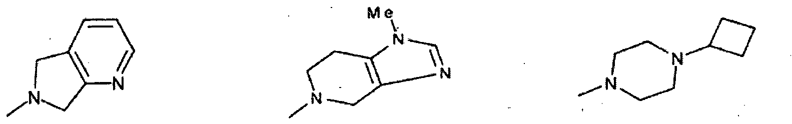 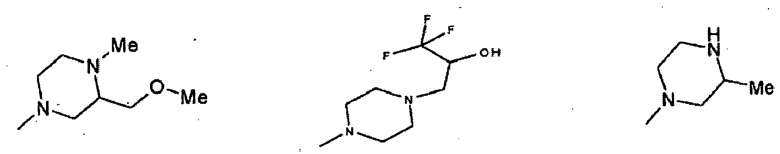 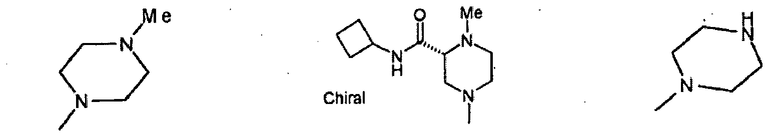 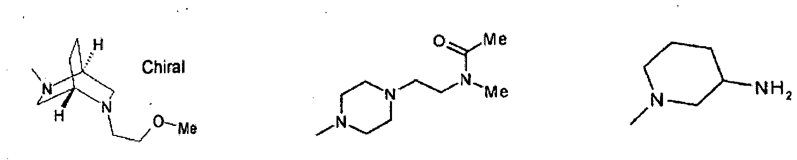 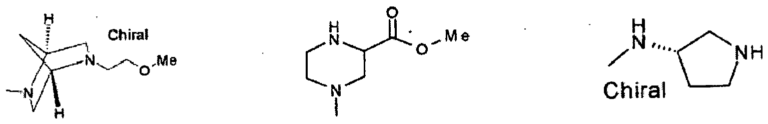 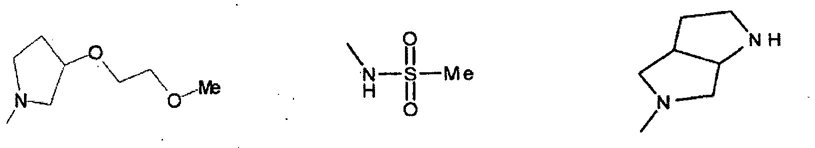 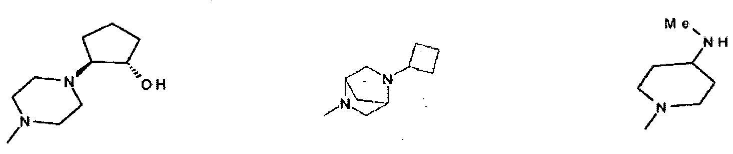 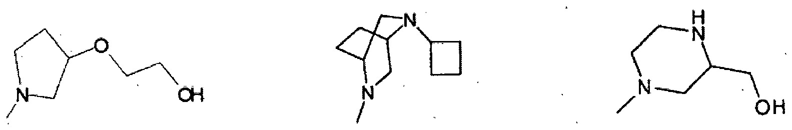 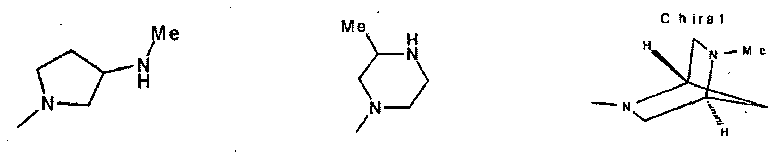 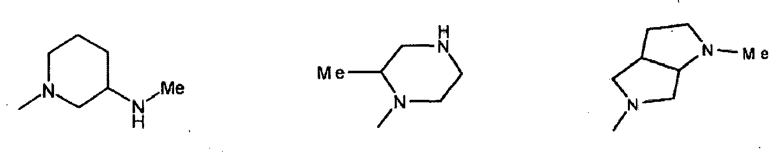 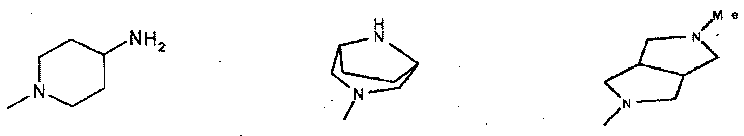 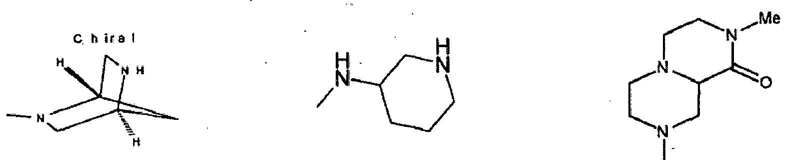 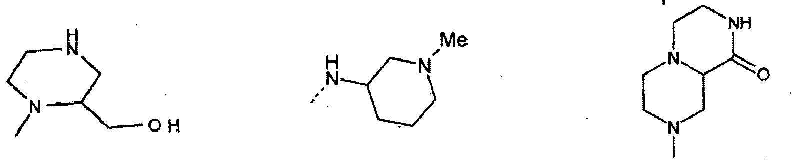 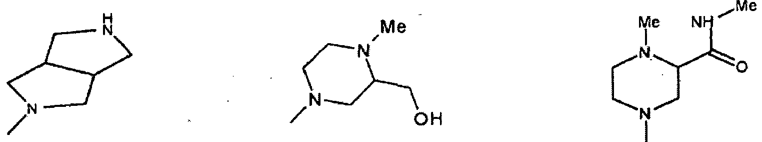 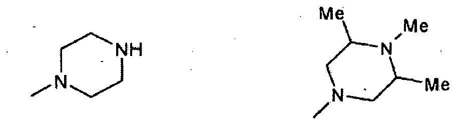 ausgewählt ist, steht;
- R₄ für ein Wasserstoffatom, ein Brom-, Chlor-oder Fluoratom, ein Cyano, ein Phenyl, einen Methylrest, einen Trifluormethylrest, einen Methoxyrest oder eine Dimethylaminogruppe steht;
- R₅ für ein Wasserstoffatom, ein Brom-, Chlor-oder Fluoratom oder einen Methylrest steht;
- R₆ für ein Wasserstoffatom, ein Bromatom, ein Cyano, eine Methoxycarbonylgruppe oder eine -CONH₂-Gruppe steht;
in Basen- oder Säure- oder Basenadditionssalzform.

9. Verbindung der Formel (I) nach Anspruch 1, worin:
- A für einen zweiwertigen aromatischen Rest, der aus: 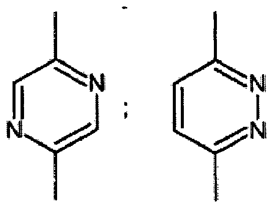 ausgewählt ist, steht;
- X für eine -CH-Gruppe steht;
- R₁ für einen n-Propylrest steht;
- R₂ für einen Methylrest steht;
- R₃ für ein Hydroxy oder eine -NR₇R₈-Gruppe, die aus: 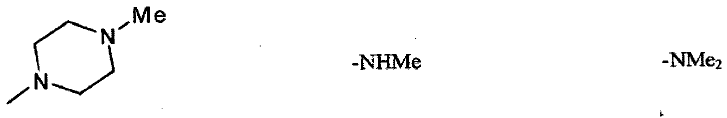 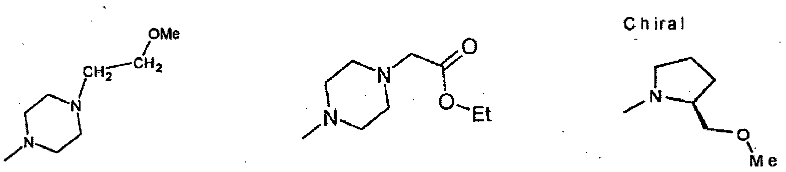 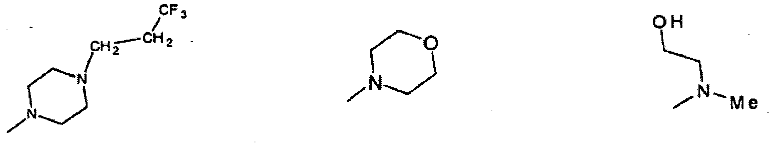 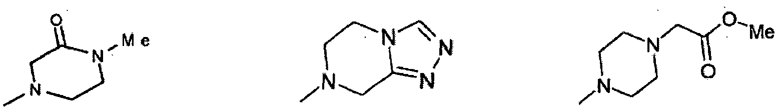 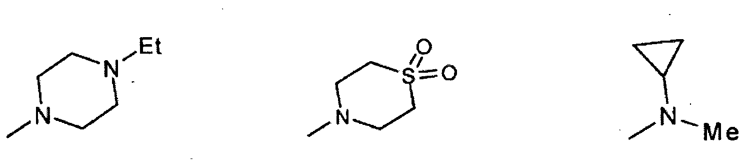 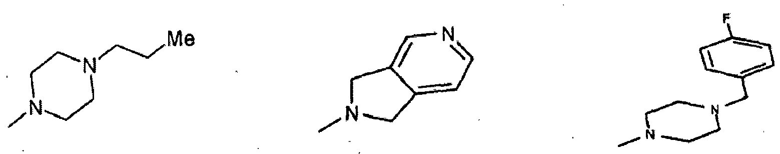 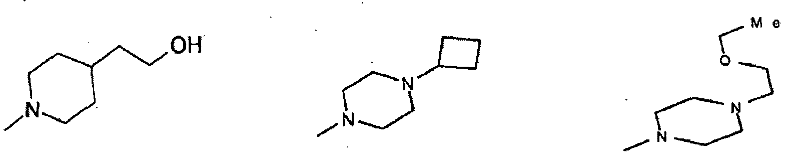 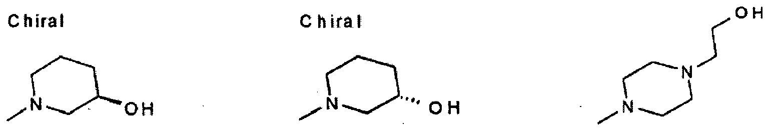 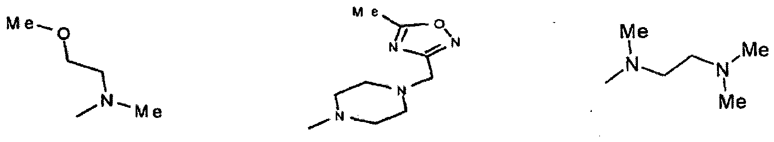 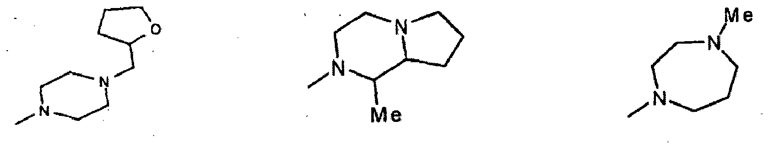 ausgewählt ist, steht;
- R₄ für ein Chloratom steht;
- R₅ für ein Wasserstoffatom steht;
in Basen- oder Säure- oder Basenadditionssalzform.

10. Verbindung der Formel (I) nach Anspruch 1, worin:
- A für einen zweiwertigen aromatischen Rest: 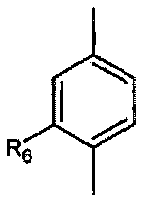 steht;
- X für eine -CH-Gruppe oder ein Stickstoffatom steht;
- R₁ für einen Methyl-, Ethyl- oder n-Propylrest oder einen Ethoxyrest steht;
- R₂ für einen Methylrest steht;
- R₃ für ein Hydroxy oder eine -NR₇R₈-Gruppe, die aus: 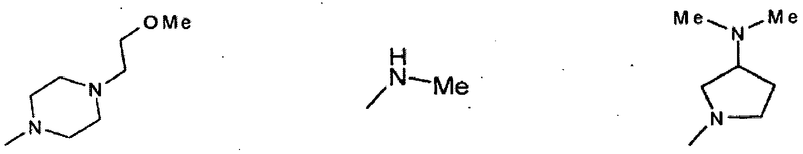 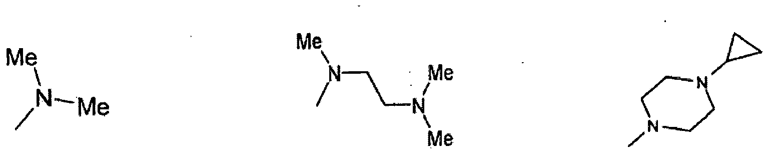 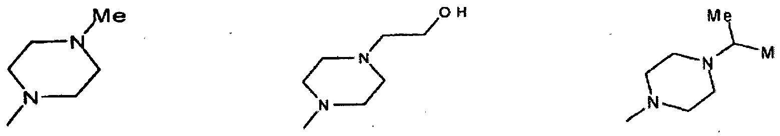 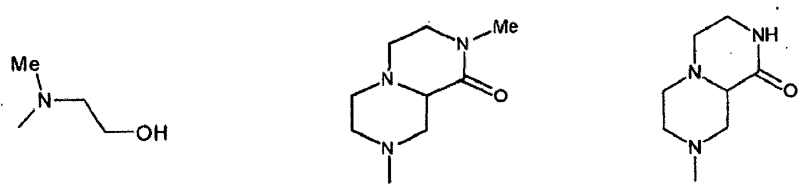 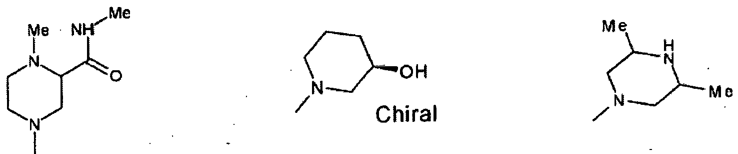 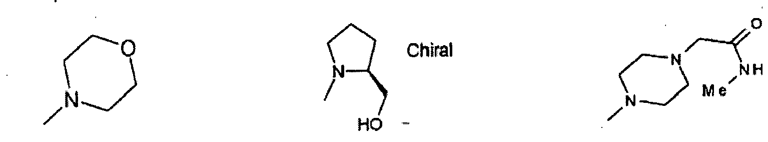 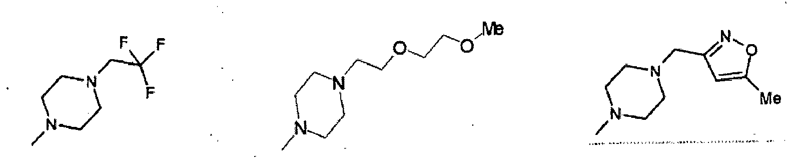 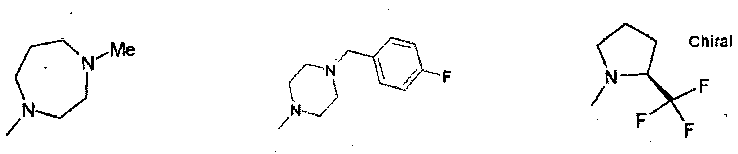 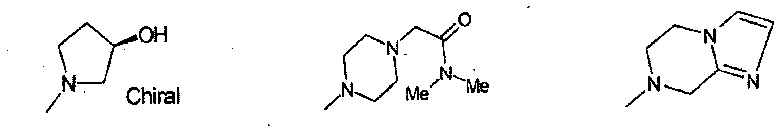 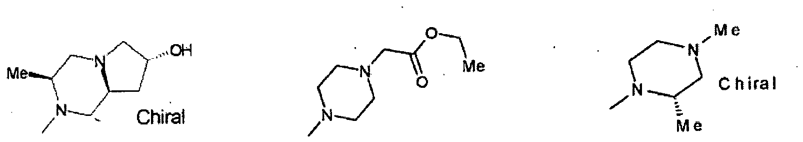 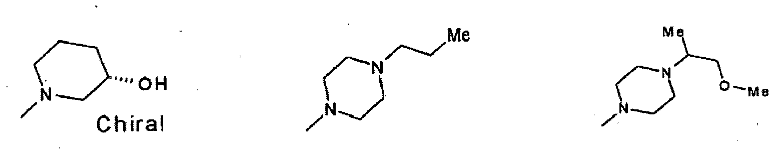 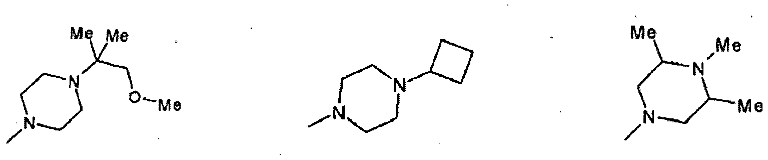 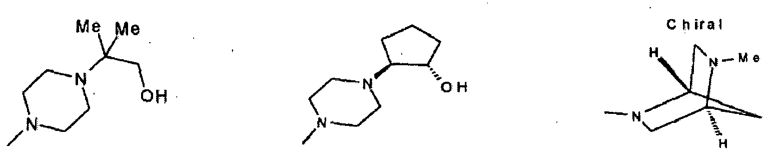 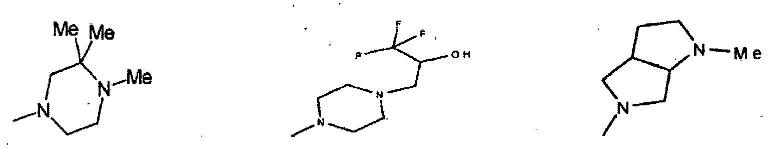 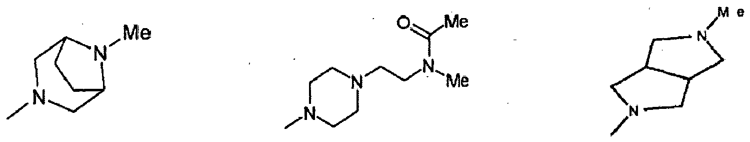 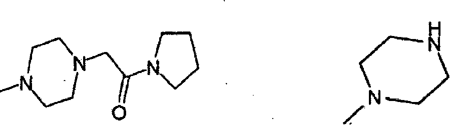 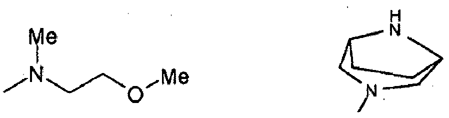 ausgewählt ist, steht;
- R₄ für ein Wasserstoffatom, ein Brom-, Chlor-oder Fluoratom, ein Cyano, ein Phenyl, einen Methylrest, einen Trifluormethylrest, einen Methoxyrest oder eine Dimethylaminogruppe steht;
- R₅ für ein Wasserstoffatom, ein Brom-, Chlor-oder Fluoratom oder einen Methylrest steht;
- R₆ für ein Wasserstoffatom, ein Bromatom, ein Cyano, eine Methoxycarbonylgruppe oder eine -CONH₂-Gruppe steht;
in Basen- oder Säure- oder Basenadditionssalzform.

11. Verbindung der Formel (I) nach Anspruch 1, ausgewählt aus:
- (3-{[4-(4-Butyryl-5-methyl-1*H*-pyrazol-1-yl)phenyl]carbamoyl}-5-methyl-1*H*-indol-1-yl)essigsäure;
- (3-{[4-(4-Butyryl-5-methyl-1*H*-pyrazol-1-yl)phenyl)carbamoyl}-5,6-dimethyl-1H-indol-1-yl)essigsäure;
- (3-{[6-(4-Butyryl-5-methyl-1*H*-pyrazol-1-yl)pyridazin-3-yl]carbamoyl}-5-chlor-1H-indol-1-yl)essigsäure;
- -N-[5-(4-Butyryl-5-methyl-1*H*-pyrazol-1-yl)pyrazin-2-yl]-5-chlor-1-[2-(4-methylpiperazin-1-yl)-2-oxoethyl]-1*H*-indol-3-carboxamid;
- N-[6-(4-Butyryl-5-methyl-1*H*-pyrazol-1-yl)pyridazin-3-yl]-5-chlor-1-[2-(4-methylpiperazin-1-yl)-2-oxoethyl]-1H-indol-3-carboxamid;
- N-[6-(4-Butyryl-5-methyl-1*H*-pyrazol-1-yl)pyridazin-3-yl]-5-chlor-1-{2-[4-(2-methoxy-ethyl)piperazin-1-yl]-2-oxoethyl}-1*H*-indol-3-carboxamid;
- N-[6-(4-Butyryl-5-methyl-1*H*-pyrazol-1-yl)pyridazin-3-yl]-5-chlor-1-{2-oxo-2-[4-(3,3,3-trifluorpropyl)piperazin-1-yl]ethyl}-1*H*-indol-3-carboxamid;
- N-[6-(4-Butyryl-5-methyl-1*H*-pyrazol-1-yl)pyridazin-3-yl]-5-chlor-1-[2-(methylamino)-2-oxoethyl]-1*H*-indol-3-carboxamid;
- N-[6-(4-Butyryl-5-methyl-1*H*-pyrazol-1-yl)pyridazin-3-yl]-5-chlor-1-(2-morpholin-4-yl-2-oxoethyl)-1*H-*indol-3-carboxamid;
- N-[6-(4-Butyryl-5-methyl)-1*H*-pyrazol-1-yl)pyridazin-3-yl]-5-chlor-1-[2-(dimethylamino)-2-oxoethyl]-1*H*-indol-3-carboxamid;
- N-[6-(4-Butyryl-5-methyl-1*H*-pyrazol-1-yl)pyridazin-3-yl]-5-chlor-1-{2-[(2S)-2-(methoxymethyl)pyrrolidin-1-yl]-2-oxoethyl}-1*H-*indol-3-carboxamid;
- N-[6-(4-Butyryl-5-methyl-1*H*-pyrazol-1-yl)pyridazin-3-yl]-5-chlor-1-[2-(4-ethylpiperazin-1-yl)-2-oxoethyl]-1*H*-indol-3-carboxamid;
- N-[6-(4-Butyryl-5-methyl-1*H*-pyrazol-1-yl)pyridazin-3-yl]-5-chlor-1-[2-(4-propylpiperazin-l-yl)-2-oxoethyl]-1*H*-indol-3-carboxamid;
- N-[6-(4-Butyryl-5-methyl-1*H*-pyrazol-1-yl)pyridazin-3-yl]-5-chlor-1-{2-[(2-methoxyethyl)(methyl)amino]-2-oxoethyl}-1*H*-indol-3-carboxamid;
- N-[6-(4-Butyryl-5-methyl-1*H*-pyrazol-1-yl)pyridazin-3-yl]-5-chlor-1-{2-oxo-2-[4-(tetrahydrofuran-2-ylmethyl)piperazin-1-yl]ethyl}-1*H*-indol-3-carboxamid;
- N-[6-(4-Butyryl-5-methyl-1*H*-pyrazol-1-yl)pyridazin-3-yl]-5-chlor-1-[2-(5,6-dihydro[1,2,4]triazolo[4,3-a]pyrazin-7(8*H*)-yl)-2-oxoethyl]-1*H*-indol-3-carboxamid;
- N-[6-(4-Butyryl-5-methyl-1*H*-pyrazol-1-yl)pyridazin-3-yl]-5-chlor-1-[2-(4-cyclobutylpiperazin-1-yl)-2-oxoethyl]-1*H*-indol-3-carboxamid;
- N-[6-(4-Butyryl-5-methyl-1*H*-pyrazol-1-yl)pyridazin-3-yl]-5-chlor-1-(2-{4-[(5-methyl-1,2,4-oxadiazol-3-yl)methyl]piperazin-1-yl}-2-oxoethyl)-1*H*-indol-3-carboxamid;
- {4-[(3-{[6-(4-Butyryl-5-methyl-1*H*-pyrazol-1-yl)pyridazin-3-yl]carbamoyl}-5-chlor-1*H*-indol-1-yl)acetyl]piperazin-1-yl}methylacetat;
- N-[6-(4-Butyryl-5-methyl-1*H*-pyrazol-1-yl)pyridazin-3-yl]-5-chlor-1-{2-[cyclopropyl(methyl)amino]-2-oxoethyl}-1*H*-indol-3-carboxamid;
- N-[6-(4-Butyryl-5-methyl-1*H*-pyrazol-1-yl)pyridazin-3-yl]-5-chlor-1-{2-[4-(4-fluorbenzyl)piperazin-1-yl]-2-oxoethyl}-1*H*-indol-3-carboxamid;
- N-[6-(4-Butyryl-5-methyl-1*H*-pyrazol-1-yl)pyridazin-3-yl]-5-chlor-1-{2-[4-(2-ethoxyethyl)piperazin-1-yl]-2-oxoethyl}-1*H*-indol-3-carboxamid;
- N-[6-(4-Butyryl-5-methyl-1*H*-pyrazol-1-yl)pyridazin-3-yl]-5-chlor-1-{2-[4-(2-hydroxyethyl)piperazin-1-yl]-2-oxoethyl}-1*H*-indol-3-carboxamid;
- N-[6-(4-Butyryl-5-methyl-1*H*-pyrazol-1-yl)pyridazin-3-yl]-5-chlor-1-(2-{[2-(dimethylamino)ethyl](methyl)amino}-2-oxoethyl)-1H-indol-3-carboxamid;
- N-[6-(4-Butyryl-5-methyl-1*H*-pyrazol-1-yl)pyridazin-3-yl]-5-chlor-1-[2-(4-methyl-1,4-diazepan-1-yl)-2-oxoethyl]-1*H*-indol-3-carboxamid;
- N-[4-(4-Butyryl-5-methyl-1*H*-pyrazol-1-yl)phenyl]-5-chlor-1-[2-(8-methyl-9-oxooctahydro-2*H*-pyrazino[1,2-a]pyrazin-2-yl)-2-oxoethyl]-1*H-*indol-3-carboxamid,
- N-[4-(4-Butyryl-5-methyl-1*H*-pyrazol-1-yl)-2-cyanophenyl]-5-chlor-1-[2-(4-methylpiperazin-1-yl)-2-oxoethyl]-1*H*-indol-3-carboxamid;
- N-[4-(4-Butyryl-5-methyl-1*H*-pyrazol-1-yl)phenyl]-5-chlor-1-{2-[4-(4-fluorbenzyl)piperazin-1-yl]-2-oxoethyl}-1*H*-indol-3-carboxamid;
- N-[4-(4-Butyryl-5-methyl-1*H*-pyrazol-1-yl)phenyl]-5-chlor-1-{2-[4-(2-methoxyethyl)piperazin-1-yl]-2-oxoethyl}-1*H*-indol-3-carboxamid;
- N-[4-(4-Butyryl-5-methyl-1*H*-pyrazol-1-yl)phenyl]-1-{2-[4-(2-methoxyethyl)piperazin-1-yl]-2-oxoethyl}-5-methyl-1*H*-indol-3-carboxamid;
- N-[4-(4-Butyryl-5-methyl-1*H*-pyrazol-1-yl)phenyl]-5-chlor-1-[2-(4-methylpiperazin-1-yl)-2-oxoethyl]-1*H*-indol-3-carboxamid;
- N-[4-(4-Butyryl-5-methyl-1*H*-pyrazol-1-yl)phenyl]-5-chlor-1-{2-[4-(2-methoxy-1-methylethyl)piperazin-1-yl]-2-oxoethyl}-1*H*-indol-3-carboxamid;
- N-[4-(4-Butyryl-5-methyl-1*H*-pyrazol-1-yl)phenyl]-5-methyl-1-[2-(4-methylpiperazin-1-yl)-2-oxoethyl]-1*H*-indol-3-carboxamid;
- N-[4-(4-Butyryl-5-methyl-1*H*-pyrazol-1-yl)phenyl]-5-chlor-6-fluor-1-{2-[4-(2-methoxyethyl)piperazin-1-yl]-2-oxoethyl}-1*H*-indol-3-carboxamid;
- N-[4-(4-Butyryl-5-methyl-1*H*-pyrazol-1-yl)phenyl]-5-methoxy-1-[2-(4-methylpiperazin-1-yl)-2-oxoethyl]-1*H*-indol-3-carboxamid;
- N-[4-(4-Butyryl-5-methyl-1*H*-pyrazol-1-yl)phenyl]-5-chlor-1-{2-[4-methyl-3-(methylcarbamoyl)piperazin-1-yl]-2-oxoethyl}-1*H-*indol-3-carboxamid;
- N-[4-(4-Butyryl-5-methyl-1*H*-pyrazol-1-yl)phenyl]-5,6-dimethyl-1-[2-(4-methylpiperazin-1-yl)-2-oxoethyl]-1H-indol-3-carboxamid;
- N-[4-(4-Butyryl-5-methyl-1H-pyrazol-1-yl)phenyl]-5-methyl-1-[2-(4-methylpiperazin-1-yl)-2-oxoethyl]-1*H*-indazol-3-carboxamid;
- N-[4-(4-Butyryl-5-methyl-1*H*-pyrazol-1-yl)phenyl]-5-methyl-1-(2-oxo-2-piperazin-1-ylethyl)-1*H*-indol-3-carboxamid;
- N-[4-(4-Butyryl-5-methyl-1*H*-pyrazol-1-yl)phenyl]-5-chlor-6-fluor-1-[2-(4-methylpiperazin-1-yl)-2-oxoethyl]-1*H*-indol-3-carboxamid;
- N-[4-(4-Butyryl-5-methyl-1*H*-pyrazol-1-yl)phenyl]-5-chlor-1-(2-{4-[(5-methylisoxazol-3-yl)methyl]piperazin-1-yl}-2-oxoethyl)-1*H*-indol-3-carboxamid;
- N-[4-(4-Butyryl-5-methyl-1*H*-pyrazol-1-yl)phenyl]-5-methyl-1-(2-morpholin-4-yl-2-oxoethyl)-1*H*-indol-3-carboxamid;
- N-[4-(4-Butyryl-5-methyl-1*H*-pyrazol-1-yl)phenyl]-5-chlor-1-(2-oxo-2-piperazin-1-ylethyl)-1*H*-indol-3-carboxamid;
- N-[4-(4-Butyryl-5-methyl-1*H*-pyrazol-1-yl)phenyl]-5-chlor-1-{2-[4-(2-methoxy-1,1-dimethylethyl)piperazin-1-yl]-2-oxoethyl}-1*H-*indol-3-carboxamid;
- N-[4-(4-Butyryl-5-methyl-1H-pyrazol-1-yl)phenyl]-5-chlor-1-{2-[4-(2-hydroxy-1,1-dimethylethyl)piperazin-1-yl]-2-oxoethyl}-1H-indol-3-carboxamid;
- N-[4-(4-Butyryl-5-methyl-1H-pyrazol-1-yl)phenyl]-5-chlor-1-[2-(8-methyl-3,8-diazabicyclo[3.2.1]oct-3-yl)-2-oxoethyl]-1H-indol-3-carboxamid;
- N-[4-(4-Butyryl-5-methyl-1H-pyrazol-1-yl)phenyl]-1-[2-(4-methylpiperazin-1-yl)-2-oxoethyl]-5-(trifluormethyl)1*H*-indol-3-carboxamid;
- N-[4-(4-Butyryl-5-methyl-1*H*-pyrazol-1-yl)phenyl]-5-chlor-1-[2-oxo-2-(9-oxooctahydro-2H-pyrazino[1,2-a]pyrazin-2-yl)ethyl]-1H-indol-3-carboxamid;
in Basen- oder Säure- oder Basenadditionssalzform.

12. Verfahren zur Herstellung von Verbindungen der Formel (I) nach Anspruch 1, worin R₃ = -OH (Verbindung IA), **dadurch gekennzeichnet, dass** man:
eine Verbindung der Formel (II) : 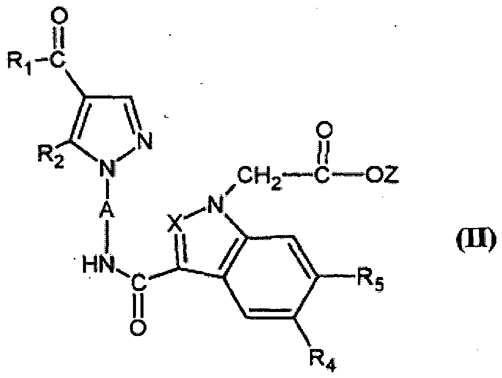 worin A, X, R₁, R₂, R₄ und R₅ wie für eine Verbindung der Formel (I) in Anspruch 1 definiert sind und Z für ein (C₁-C₄)-Alkyl steht, in saurem oder basischem Milieu hydrolysiert.

13. Verfahren zur Herstellung von Verbindungen der Formel (I) nach Anspruch 1, worin R₃ = -NR₇R₈ (Verbindung IB), **dadurch gekennzeichnet, dass** man:
eine Verbindung der Formel (IA): 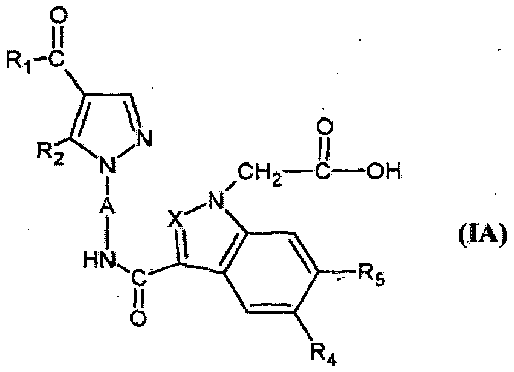 worin A, X, R₁, R₂, R₄ und R₅ wie für eine Verbindung der Formel (I) in Anspruch 1 definiert sind, mit einem Amin der Formel (III) :
**HNR₇R₈** **(III)**
worin R₇ und R₈ wie für eine Verbindung der Formel (I) in Anspruch 1 definiert sind, umsetzt.

14. Verbindung der Formel: 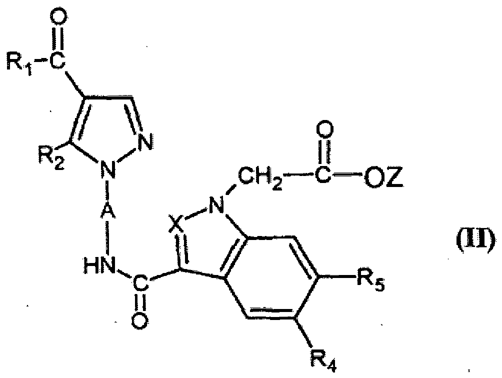 worin:
- A für einen zweiwertigen aromatischen Rest, der aus: 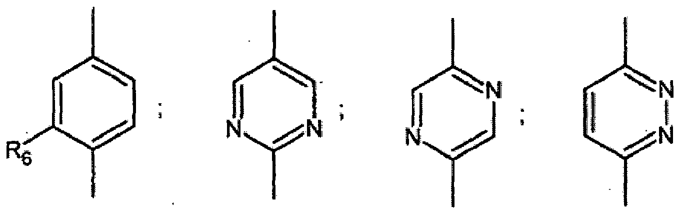
ausgewählt ist, steht;
- X für eine -CH-Gruppe oder ein Stickstoffatom steht;
- R₁ für ein (C₁-C₄)-Alkyl oder ein (C₁-C₄) -Alkoxy steht;
- R₂ für eine Alk-Gruppe steht;
- R₄ für ein Wasserstoffatom, ein Halogenatom, ein Cyano, ein Phenyl, eine Alk-Gruppe, eine OAlk-Gruppe oder eine -NR₉R₁₀-Gruppe steht;
- R₅ für ein Wasserstoffatom, ein Halogenatom oder eine Alk-Gruppe steht;
- R₆ für ein Wasserstoffatom, ein Halogenatom, ein Cyano, eine -COOAlk-Gruppe oder eine -CONH₂-Gruppe steht;
- R₉ und R₁₀ unabhängig voneinander für ein Wasserstoffatom oder ein (C₁-C₄)-Alkyl stehen;
- Z für ein (C₁-C₄)-Alkyl steht;
- Alk für ein (C₁-C₄) -Alkyl, das unsubstituiert oder ein- oder mehrfach durch ein Fluoratom substituiert ist, steht.

15. Medikament, **dadurch gekennzeichnet, dass** es eine Verbindung der Formel (I) nach einem der Ansprüche 1 bis 11 oder ein pharmazeutisch unbedenkliches Salz der Verbindung der Formel (I) enthält.

16. Pharmazeutische Zusammensetzung, **dadurch gekennzeichnet, dass** sie eine Verbindung der Formel (I) nach einem der Ansprüche 1 bis 11, ein pharmazeutisch unbedenkliches Salz dieser Verbindung sowie mindestens einen pharmazeutisch unbedenklichen Hilfsstoff umfasst.

17. Verbindung der Formel (I) nach einem der Ansprüche 1 bis 11 zur Behandlung oder Prävention von instabiler Angina (Angina pectoris), perkutaner transluminaler Koronarangioplastie (PTCA), Myokardinfarkt, thrombotischen arteriellen Komplikationen von Arterosklerose wie embolischen oder thrombotischen Schlaganfällen, transitorischen ischämischen Attacken, peripherer Verschlusskrankheit, arteriellen Komplikationen von Arterosklerose infolge von chirurgischen Eingriffen wie Angioplastie, Endarteriektomie, Stent-Platzierung, Herzgefäßstransplantationen, thrombotischen Komplikationen von Chirurgie oder mechanischen Schädigungen wie Gewebebergung nach Unfall- oder Chirurgietrauma, rekonstruktiver Chirurgie (einschließlich Haut und Muskellappen), thrombotisch-thrombozytopenischer Purpura, Antiphospholipidsyndrom, heparininduzierter Thrombozytopenie und Präeklampsie/Eklampsie; oder Venenthrombose wie tiefer Venenthrombose, Venenverschlusskrankheit; oder bei der Prävention von mechanisch induzierter Thrombozytenaktivierung *in vivo*, wie bei kardiopulmonalem Bypass und extrakorporaler Oxygenierung (Prävention von Mikrothromboembolismus), bei der Prävention der mechanisch induzierten Thrombozytenaktivierung *in vitro* (Verwendung bei der Konservierung von Blutprodukten (Thrombozytenkonzentraten), Verwendung bei Shunts wie Nierendialyse und Plasmapherese) Thrombose als Sekundärerscheinung nach Gefäßläsion/Entzündung wie Angiitis, Arteriitis, Glomerulosenephritis, Organtransplantatabstoßung, Zuständen, bei denen Thrombozyten zum Prozess der zugrundeliegenden Entzündungskrankheit in der Gefäßwand beitragen können, wie Bildung/Progression von von arteromatösen Plaques, Stenose/Restenose.

## Claims

1. Compound corresponding to formula (I): 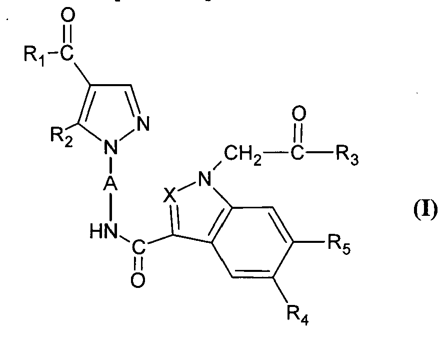 in which:
- A represents a divalent aromatic radical selected from: 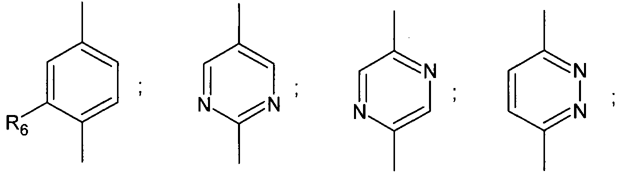
- X represents a -CH- group or a nitrogen atom;
- R₁ represents a (C₁-C₄) alkyl or a (C₁-C₄) alkoxy;
- R₂ represents a group Alk;
- R₃ represents a hydroxyl or a group -NR₇R₈;
- R₄ represents a hydrogen atom, a halogen atom, a cyano, a phenyl, a group Alk, a group OAlk or a group - NR₉R₁₀;
- R₅ represents a hydrogen atom, a halogen atom or a group Alk;
- R₆ represents a hydrogen atom, a halogen atom, a cyano, a group COOAlk or a -CONH₂ group;
- R₇ represents a hydrogen atom or a (C₁-C₄) alkyl;
- R₈ represents:
a) a hydrogen atom;
b) a (C₁-C₄)alkyl, unsubstituted or substituted with:
(i) a hydroxyl;
(ii) a group OAlk;
(iii) a group -NR₉R₁₀;
(iv) a (C₃-C₆)heterocycloalkyl, unsubstituted or substituted with a (C₁-C₄)alkyl or with a group -COOAlk;
(v) a heteroaryl, unsubstituted or substituted with a (C₁-C₄) alkyl;
c) a (C₃-C₇) cycloalkyl;
d) a (C₃-C₆)heterocycloalkyl, unsubstituted or substituted with a (C₁-C₄)alkyl, a group -COOAlk or with one or two oxo groups;
e) a group -SO₂Alk;
- or else R₇ and R₈, together with the nitrogen atom to which they are attached, constitute a heterocycle, saturated or unsaturated, mono- or polycyclic, condensed or bridged, comprising 4 to 10 ring members and that can contain one, two or three other nitrogen atoms or another heteroatom selected from an oxygen atom or a sulfur atom; said heterocycle being unsubstituted or substituted once, twice or three times with substituents selected independently from:
a) a halogen atom;
b) a hydroxyl;
c) a group -OR₁₁;
d) an oxo;
e) a group -NR₉R₁₀;
f) a group -NR₁₂COR₁₃;
g) a group -NR₁₂COOR₁₃;
h) a group -COR₁₃;
i) a group -COOR₁₃;
j) a group -CONR₁₄R₁₅;
k) a (C₃-C₇)cycloalkyl, unsubstituted or substituted with a hydroxyl or with a (C₁-C₄) alkyl;
l) a (C₃-C₆)heterocycloalkyl, unsubstituted or substituted with one or two oxo groups;
m) a phenyl, unsubstituted or substituted one or more times with substituents selected independently from a halogen atom, a group Alk or a group OAlk;
n) a pyridinyl;
o) a (C₁-C₄)alkyl, unsubstituted or substituted one or more times with substituents selected independently from:
(i) a halogen atom;
(ii) a hydroxyl;
(iii) a group -OR₁₁:
(iv) a group -NR₉R₁₀;
(v) a group -NR₁₂COR₁₃;
(vi) a group -COOR₁₃;
(vii) a group -CONR₁₄R₁₅;
(viii) a group -SO₂Alk;
(ix) a (C₃-C₇) cycloalkyl;
(x) a (C₃-C₆)heterocycloalkyl;
(xi) a phenyl, unsubstituted or substituted one or more times with substituents selected independently from a halogen atom, a group Alk or a group OAlk;
(xii) a heteroaryl, unsubstituted or substituted with a (C₁-C₄)alkyl;
- R₉ and R₁₀ represent, each independently, a hydrogen atom or a (C₁-C₄) alkyl;
- R₁₁ represents a group Alk, a - (C₁-C₄) alkylene-OH or a
- (C₁-C₄) alkylene-OAlk;
- R₁₂ represents a hydrogen atom or a (C₁-C₄) alkyl;
- R₁₃ represents a (C₁-C₄) alkyl;
- R₁₄ and R₁₅ represent, each independently, a hydrogen atom, a (C₁-C₄) alkyl or a (C₃-C₇) cycloalkyl;
- or else R₁₄ and R₁₅, together with the nitrogen atom to which they are attached, constitute a heterocyclic radical selected from: azetidin-1-yl, pyrrolidin-1-yl, piperidin-1-yl, morpholin-4-yl or piperazin-1-yl;
- Alk represents a (C₁-C₄)alkyl, unsubstituted or substituted one or more,times with a fluorine atom;
in the form of a base or salt of addition with an acid or a base.

2. Compound of formula (I) according to Claim 1, **characterized in that** R₃ represents a hydroxyl (reference IA) and the other substituents A, X, R₁, R₂, R₄ and R₅ are as defined for a compound of formula (I) in Claim 1;
in the form of a base or salt of addition with an acid or a base.

3. Compound of formula (I) according to Claim 1, **characterized in that** R₃ represents a group -NR₇R₈ (reference IB) and the other substituents A, X, R₁, R₂, R₄, R₅, R₇ and R₈ are as defined for a compound of formula (I) in Claim 1;
in the form of a base or salt of addition with an acid or a base.

4. Compound of formula (I) according to Claim 1, **characterized in that**:
- A represents a divalent aromatic radical selected from: 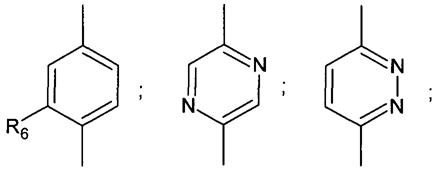
- X represents a -CH- group or a nitrogen atom;
- R₁ represents a methyl, ethyl, n-propyl radical or an ethoxy radical;
- R₂ represents a methyl radical;
- R₃ represents a hydroxyl or a group -NR₇R₈;
- R₄ represents a hydrogen atom, a bromine, chlorine or fluorine atom, a cyano, a phenyl, a methyl radical, a trifluoromethyl radical, a methoxy radical or a dimethylamino group;
- R₅ represents a hydrogen atom, a bromine, chlorine or fluorine atom or a methyl radical;
- R₆ represents a hydrogen atom, a bromine atom, a cyano, a methoxycarbonyl group or a -CONH₂ group;
- R₇ represents a hydrogen atom or a methyl radical;
- R₈ represents:
a) a hydrogen atom;
b) a methyl radical, a 2-hydroxyethyl, a 3-hydroxypropyl, a 2-methoxyethyl, a 2-(methylamino)ethyl, a 2-(dimethylamino)ethyl, a 2-(dimethylamino)propyl, a 2-(dimethylamino)-1-methylethyl, a 2-(dimethylamino)-2-methylpropyl, a 3-(dimethylamino)propyl, a (1-methylpiperidin-3-yl)methyl, a tetrahydrofuran-3-ylmethyl, a tetrahydro-2*H*-pyran-4-ylmethyl, a 2-furylmethyl, a (3-methyl-1*H-*1,2,4-triazol-5-yl)methyl, 1-(1*H*-tetrazol-5-yl)ethyl, a 2-(1*H*-pyrrol-1-yl)ethyl, a 2-(1*H*-imiddzol-1-yl)ethyl;
c) a cyclopropyl;
d) a 1,1-dioxidotetrahydro-3-thienyl, a pyrrolidin-3-yl, a 1-methylpyrrolidin-3-yl, a 1-(*tert-*butoxycarbonyl)pyrrolidin-3-yl, a piperidin-3-yl, a 1-methylpiperidin-3-yl, a 1-(*tert-*butoxycarbonyl)piperidin-3-yl, a 1-methylpiperidin-4-yl;
e) a methylsulfonyl group;
- or else R₇ and R₈, together with the nitrogen atom to which they are attached, constitute a heterocycle selected from: an azetidine, a pyrrolidine, a piperidine, a piperazine, a morpholine, a thiomorpholine, an octahydro-2*H*-pyrido[1,2-a]pyrazine, a 5,6,7,8-tetrahydro[1,2,4]triazolo[4,3-*a*]pyrazine, a 2,3-dihydro-1*H*-pyrrolo[3,4-*c*]pyridine, an octahydropyrrolo[1,2-*a*]pyrazine, a 1,4-diazepane, a 5,6,7,8-tetrahydroimidazo[1,2-*a*]pyrazine, a 4,5,6,7-tetrahydro-1*H*-pyrazolo[4,3-*c*]pyridine, a 1,4,5,6-tetrahydropyrrolo[3,4-c]pyrazole, a 6,7-dihydro-5*H-*pyrrolo[3,4-b]pyridine, a 3,8-diazabicyclo[3.2.1]octane, a 4,5,6,7-tetrahydro-1*H-*imidazo[4,5-c]pyridine, a 4,5,6,7-tetrahydro-1*H-*pyrazolo[3,4-c]pyridine, a 2,5-diazabicyclo[2.2.2]octane, a 2,5-diazabicyclo[2.2.1]heptane, an octahydropyrrolo[3,4-b]pyrrole, an octahydropyrrolo[3,4-c]pyrrole, an octahydro-2*H*-pyrazino[1,2-*a*]pyrazine; said heterocycle being unsubstituted or substituted once, twice or three times with substituents selected independently from:
a) a fluorine atom;
b) a hydroxyl;
c) a methoxy radical, a 2-hydroxyethoxy, a 2-methoxyethoxy;
d) an oxo;
e) an amino group, a methylamino, a dimethylamino;
f) an acetamido group;
g) a (*tert*-butoxycarbonyl)amino group, a (*tert-*butoxycarbonyl)(methyl)amino group;
h) an acetyl group;
i) a methoxycarbonyl group, a *tert*-butoxycarbonyl group;
j) a dimethylcarbamoyl group, a cyclopropylcarbamoyl, a cyclobutylcarbamoyl;
k) a cyclopropyl, a cyclobutyl, a 2-hydroxycyclopentyl radical;
l) an oxetan-3-yl, a 1,1-dioxidotetrahydro-3-thienyl, a piperidin-1-yl, a morpholin-4-yl;
m) a 4-fluorophenyl;
n) a pyridin-2-yl;
o) a methyl radical, an ethyl, an n-propyl, an isopropyl, an n-butyl, a trifluoromethyl, a 2-fluoroethyl, a 2,2-difluoroethyl, a 2,2,2-trifluoroethyl, a 3,3,3-trifluoropropyl, a 3,3,3-trifluoro-2-hydroxypropyl, a hydroxymethyl, a 2-hydroxyethyl, a 2-hydroxy-1,1-dimethylethyl, a methoxymethyl, a 2-methoxyethyl, a 2-methoxy-1-methylethyl, a 2-methoxy-1,1-dimethylethyl, a 2-ethoxyethyl, a 2-(trifluoromethoxy)ethyl, a 2-(2-hydroxyethoxy) ethyl, a 2-(2-methoxyethoxy)ethyl, a 2-(dimethylamino)ethyl, a 2-acetamidoethyl, a 2-[acetyl(methyl)amino]ethyl, a 2-methoxy-2-oxoethyl, a 2-ethoxy-2-oxoethyl, a 3-ethoxy-3-oxopropyl, a 2-amino-2-oxoethyl, a 2-(methylamino)-2-oxoethyl, a 2-(isopropylamino)-2-oxoethyl, a 2-(dimethylamino)-2-oxoethyl, a 2-(cyclopropylamino)-2-oxoethyl, a 2-oxo-2-pyrrolidin-1-ylethyl, a 2-(methylsulfonyl)ethyl, a cyclopropylmethyl, a pyrrolidin-1-ylmethyl, a tetrahydrofuran-2-ylmethyl, a 2-thienylmethyl, a 4-chlorobenzyl, a 4-fluorobenzyl, a 2-methoxybenzyl, a 3-fluoro-4-methoxybenzyl, a pyridin-4-ylmethyl, a (5-methyl-1,2,4-oxadiazol-3-yl)methyl, a (5-methylisoxazol-3-yl)methyl;
in the form of a base or salt of addition with an acid or a base.

5. Compound of formula (I) according to Claim 1, **characterized in that**:
- A represents a divalent aromatic radical selected from: 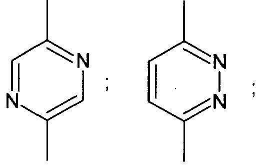
- X represents a -CH- group;
- R₁ represents an n-propyl radical;
- R₂ represents a methyl radical;
- R₃ represents a hydroxyl or a group -NR₇R₈;
- R₄ represents a chlorine atom;
- R₅ represents a hydrogen atom;
- R₇ represents a hydrogen atom or a methyl radical;
- R₈ represents:
b) a methyl radical, a 2-hydroxyethyl, a 3-hydroxypropyl, a 2-methoxyethyl, a 2-(dimethylamino)ethyl, a 2-(dimethylamino)propyl, a 2-(dimethylamino)-2-methylpropyl, a 3-(dimethylamino)propyl, a tetrahydrofuran-3-ylmethyl, a tetrahydro-2*H*-pyran-4-ylmethyl, a 2-furylmethyl, a (3-methyl-1*H*-1,2,4-triazol-5-yl)methyl, 1-(1*H*-tetrazol-5-yl)ethyl, a 2-(1*H*-pyrrol-1-yl)ethyl;
c) a cyclopropyl;
d) a 1-methylpyrrolidin-3-yl, a 1-(*tert-*butoxycarbonyl)piperidin-3-yl, a 1-methylpiperidin-4-yl;
- or else R₇ and R₈, together with the nitrogen atom to which they are attached, constitute a heterocycle selected from: an azetidine, a pyrrolidine, a piperidine, a piperazine, a morpholine, a thiomorpholine, an octahydro-2*H*-pyrido[1,2-*a*]pyrazine, a 5,6,7,8-tetrahydro[1,2,4]triazolo[4,3-*a*]pyrazine, a 2,3-dihydro-1*H*-pyrrolo[3,4-*c*]pyridine, an octahydropyrrolo[1,2-*a*]pyrazine, a 1,4-diazepane; said heterocycle being unsubstituted or substituted once, twice or three times with substituents selected independently from:
b) a hydroxyl;
c) a methoxy radical;
d) an oxo;
h) an acetyl group;
i) a methoxycarbonyl group;
j) a dimethylcarbamoyl group;
k) a cyclobutyl radical;
1) a 1,1-dioxidotetrahydro-3-thienyl, a piperidin-1-yl, a morpholin-4-yl;
m) a 4-fluorophenyl;
o) a methyl radical, an ethyl, an n-propyl, an isopropyl, an n-butyl, a trifluoromethyl, a 3,3,3-trifluoropropyl, a hydroxymethyl, a 2-hydroxyethyl, a methoxymethyl, a 2-methoxyethyl, a 2-ethoxyethyl, a 2-(trifluoromethoxy)ethyl, a 2-(dimethylamino)ethyl, a 2-methoxy-2-oxoethyl, a 2-ethoxy-2-oxoethyl, a 2-(isopropylamino)-2-oxoethyl, a 2-(dimethylamino)-2-oxoethyl, a 2-(cyclopropylamino)-2-oxoethyl, a 2-oxo-2-pyrrolidin-1-ylethyl, a tetrahydrofuran-2-ylmethyl, a 4-fluorobenzyl, a 3-fluoro-4-methoxybenzyl, a pyridin-4-ylmethyl, a (5-methyl-1,2,4-oxadiazol-3-yl)methyl,; in the form of a base or salt of addition with an acid or a base.

6. Compound of formula (I) according to Claim 1, **characterized in that**:
- A represents a divalent aromatic radical: 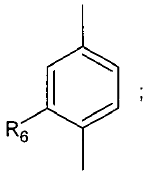
- X represents a -CH- group or a nitrogen atom;
- R₁ represents a methyl, ethyl, n-propyl radical or an ethoxy radical;
- R₂ represents a methyl radical;
- R₃ represents a hydroxyl or a group -NR₇R₈;
- R₄ represents a hydrogen atom, a bromine, chlorine or fluorine atom, a cyano, a phenyl, a methyl radical, a trifluoromethyl radical, a methoxy radical or a dimethylamino group;
- R₅ represents a hydrogen atom, a bromine, chlorine or fluorine atom or a methyl radical;
- R₆ represents a hydrogen atom, a bromine atom, a cyano, a methoxycarbonyl group or a -CONH₂ group;
- R₇ represents a hydrogen atom or a methyl radical;
- R₈ represents:
b) a methyl radical, a 2-hydroxyethyl, a 2-methoxyethyl, a 2-(methylamino)ethyl, a 2-(dimethylamino)ethyl, a 2-(dimethylamino)-1-methylethyl, a (1-methylpiperidin-3-yl)methyl, a 2-(1H-imidazol-1-yl)ethyl;
d) a 1,1-dioxidotetrahydro-3-thienyl, a pyrrolidin-3-yl, a 1-methylpyrrolidin-3-yl, a 1-(*tert-*butoxycarbonyl)pyrrolidin-3-yl, a piperidin-3-yl, a 1-methylpiperidin-3-yl, a 1-(*tert-*butoxycarbonyl)piperidin-3-yl;
e) a methylsulfonyl group;
- or else R₇ and R₈, together with the nitrogen atom to which they are attached, constitute a heterocycle selected from: an azetidine, a pyrrolidine, a piperidine, a piperazine, a morpholine, a 5,6,7,8-tetrahydro[1,2,4]triazolo[4,3-a]pyrazine, an octahydropyrrolo[1,2-*a*]pyrazine, a 1,4-diazepane, a 5,6,7,8-tetrahydroimidazo[1,2-*a*]pyrazine, a 4,5,6,7-tetrahydro-1*H*-pyrazolo[4,3-*c*]pyridine, a 1,4,5,6-tetrahydropyrrolo[3,4-*c*]pyrazole, a 6,7-dihydro-5*H-*pyrrolo[3,4-*b*]pyridine, a 3,8-diazabicyclo[3.2.1]octane, a 4,5,6,7-tetrahydro-1*H-*imidazo[4,5-*c*]pyridine, a 4,5,6,7-tetrahydro-1*H-*pyrazolo[3,4-*c*]pyridine, a 2,5-diazabicyclo[2.2.2]octane, a 2,5-diazabicyclo[2.2.1]heptane, an octahydropyrrolo[3,4-b]pyrrole, an octahydropyrrolo[3,4-c]pyrrole, an octahydro-2*H*-pyrazino[1,2-*a*]pyrazine; said heterocycle being unsubstituted or substituted once, twice or three times with substituents selected independently from:
a) a fluorine atom;
b) a hydroxyl;
c) a 2-hydroxyethoxy, a 2-methoxyethoxy;
d) an oxo;
e) an amino group, a methylamino, a dimethylamino;
f) an acetamido group;
g) a (*tert*-butoxycarbonyl)amino group, a (*tert-*butoxycarbonyl)(methyl)amino group;
i) a methoxycarbonyl group, a *tert*-butoxycarbonyl;
j) a group, a cyclopropylcarbamoyl, a cyclobutylcarbamoyl;
k) a cyclopropyl, a cyclobutyl, a 2-hydroxycyclopentyl radical;
l) an oxetan-3-yl, a morpholin-4-yl;
n) a pyridin-2-yl;
o) a methyl radical, an ethyl, an n-propyl, an isopropyl, an n-butyl, a trifluoromethyl, a 2-fluoroethyl, a 2,2-difluoroethyl, a 2,2,2-trifluoroethyl, a 3,3,3-trifluoro-2-hydroxypropyl, a hydroxymethyl, a 2-hydroxyethyl, a 2-hydroxy-1,1-dimethylethyl, a methoxymethyl, a 2-methoxyethyl, a 2-methoxy-1-methylethyl, a 2-methoxy-1,1-dimethylethyl, a 2-ethoxyethyl, a 2-(trifluoromethoxy)ethyl, a 2-(2-hydroxyethoxy) ethyl, a 2-(2-methoxyethoxy)ethyl, a 2-(dimethylamino)ethyl, a 2-acetamidoethyl, a 2-[acetyl(methyl)amino]ethyl, a 2-ethoxy-2-oxoethyl, a 3-ethoxy-3-oxopropyl, a 2-amino-2-oxoethyl, a 2-(methylamino)-2-oxoethyl, a 2-(dimethylamino)-2-oxoethyl, a 2-oxo-2-pyrrolidin-1-ylethyl, a 2-(methylsulfonyl)ethyl, a cyclopropylmethyl, a pyrrolidin-1-ylmethyl, a 2-thienylmethyl, a 4-chlorobenzyl, a 4-fluorobenzyl, a 2-methoxybenzyl, a (5-methylisoxazol-3-yl)methyl;
in the form of a base or salt of addition with an acid or a base.

7. Compound of formula (I) according to Claim 1, **characterized in that**:
- A represents a divalent aromatic radical selected from: 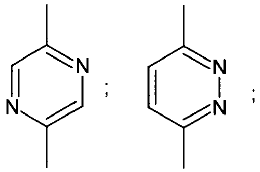
- X represents a -CH- group;
- R₁ represents an n-propyl radical;
- R₂ represents a methyl radical;
- R₃ represents a hydroxyl or a group -NR₇R₈ selected from: 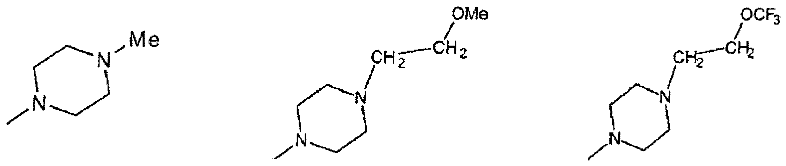 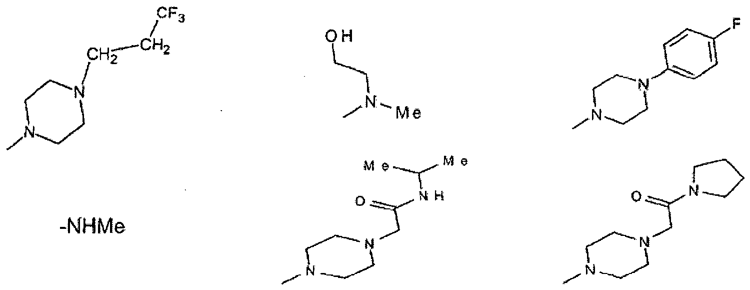 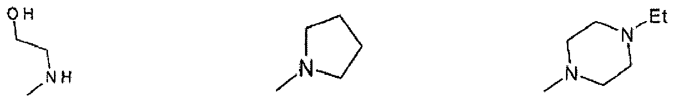 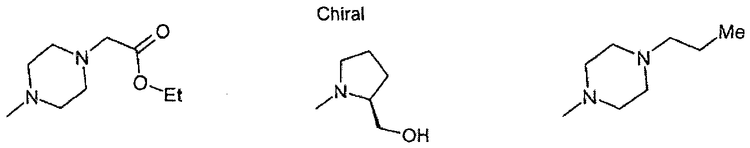 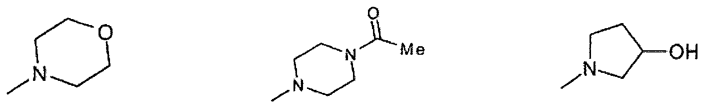 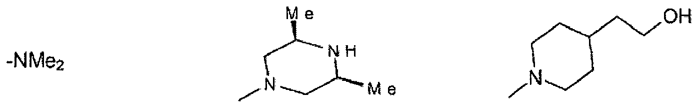 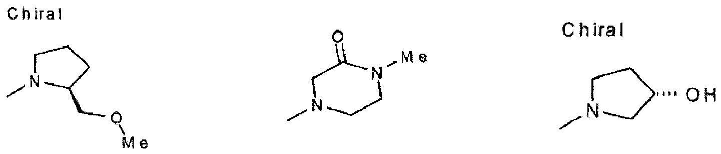 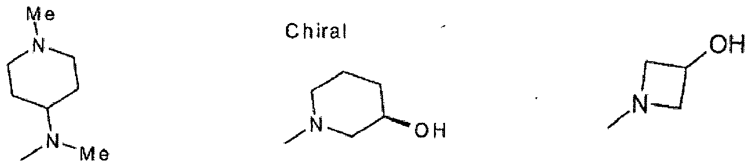 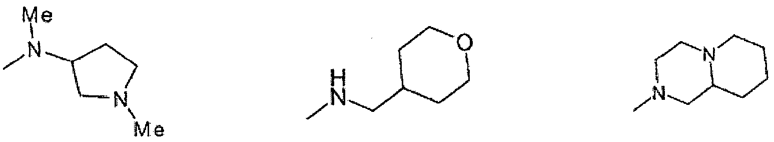 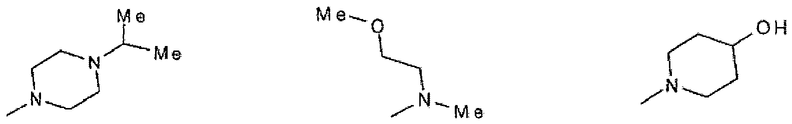 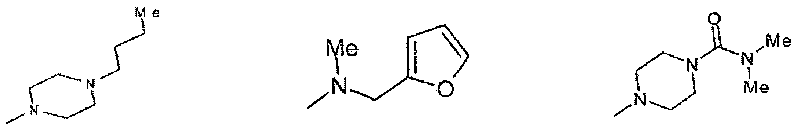 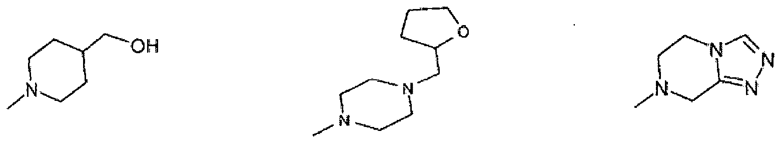 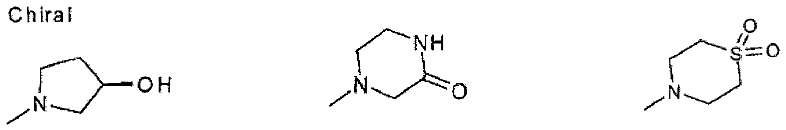 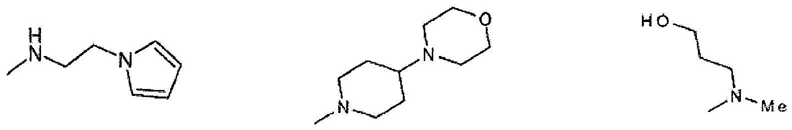 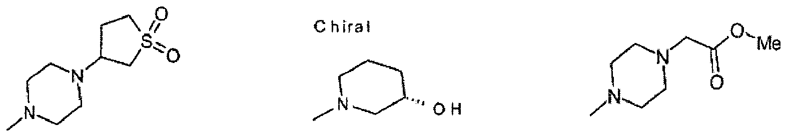 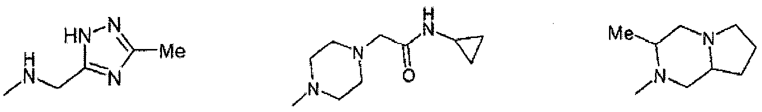 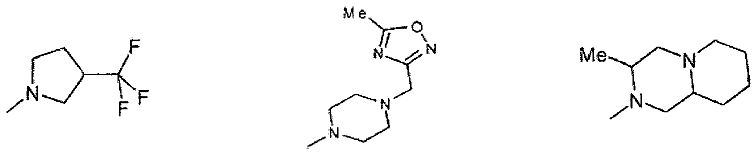 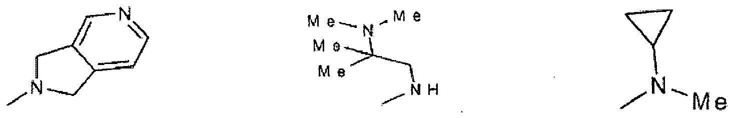 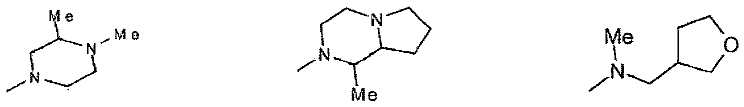 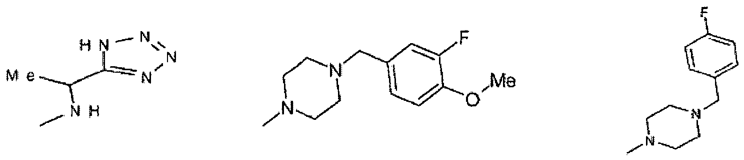 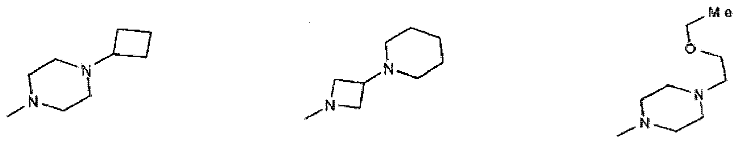 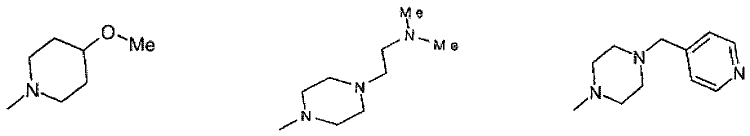 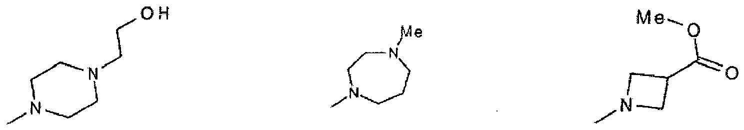 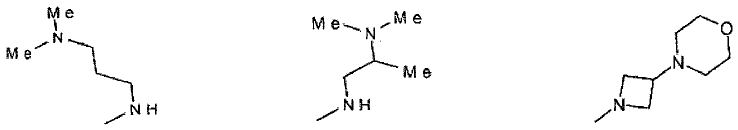 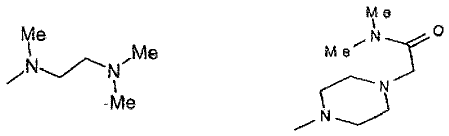
- R₄ represents a chlorine atom;
- R₅ represents a hydrogen atom;
in the form of a base or salt of addition with an acid or a base.

8. Compound of formula (I) according to Claim 1, **characterized in that**:
- A represents a divalent aromatic radical: 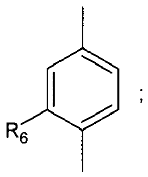
- X represents a -CH- group or a nitrogen atom;
- R₁ represents a methyl, ethyl, n-propyl radical or an ethoxy radical;
- R₂ represents a methyl radical;
- R₃ represents a hydroxyl or a group -NR₇R₈ selected from: 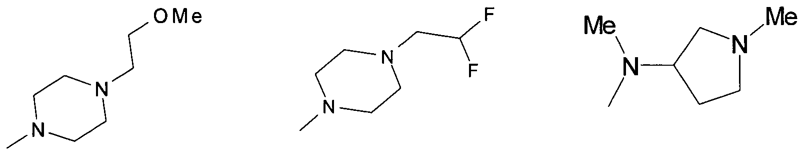 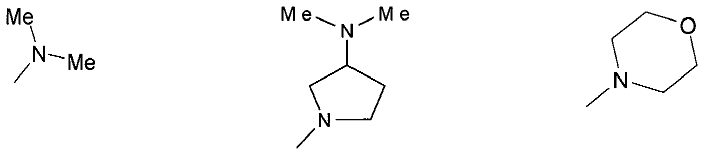 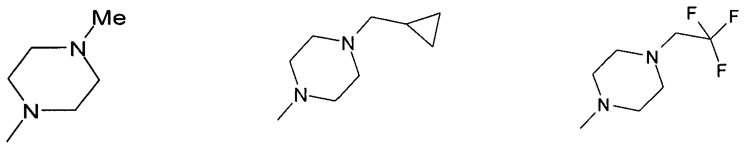 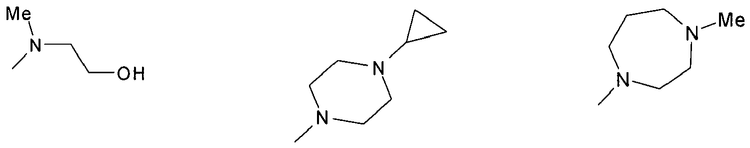 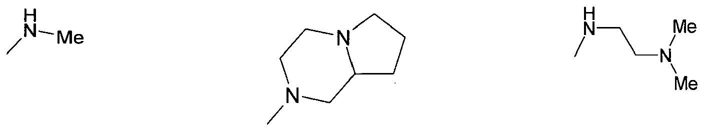 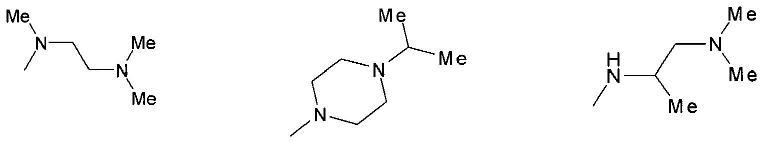 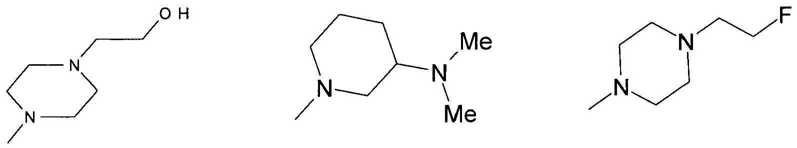 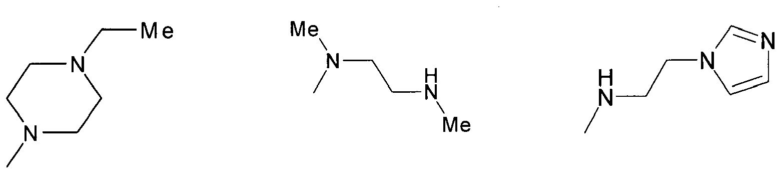 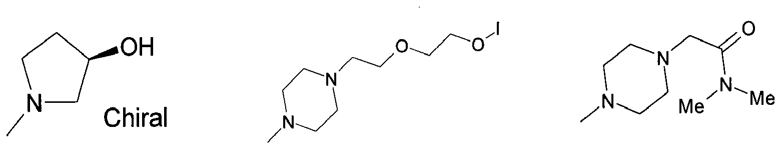 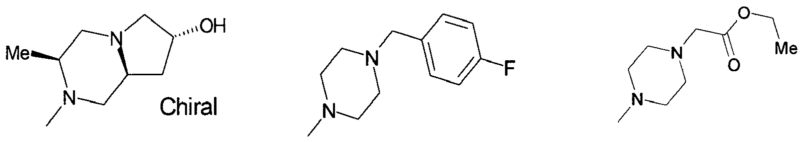 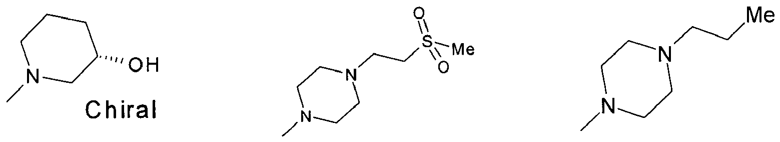 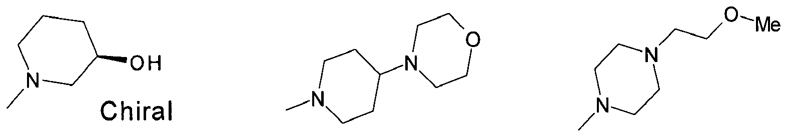 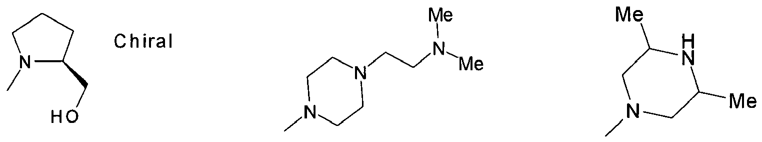 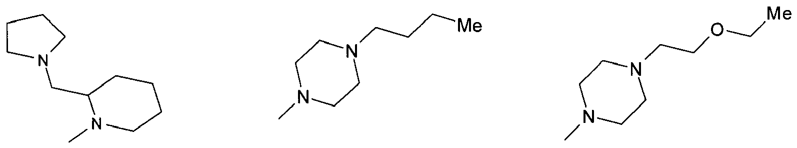 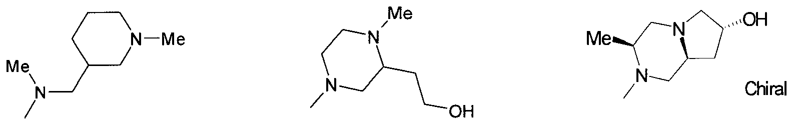 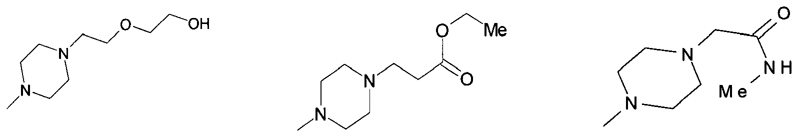 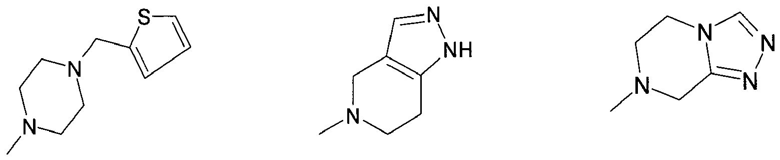 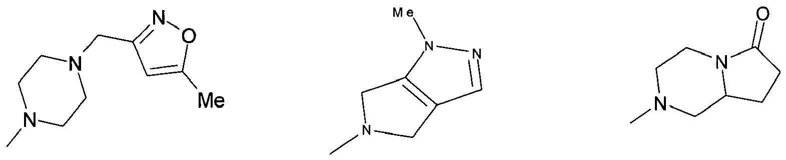 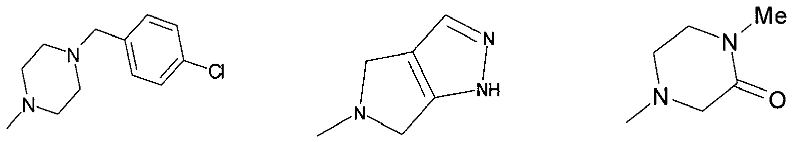 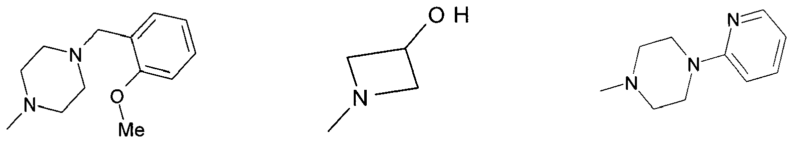 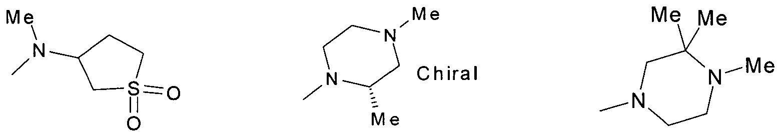 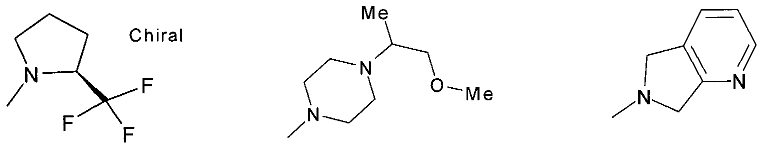 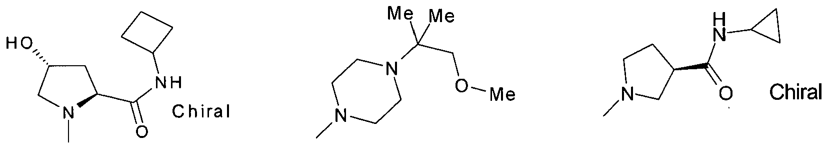 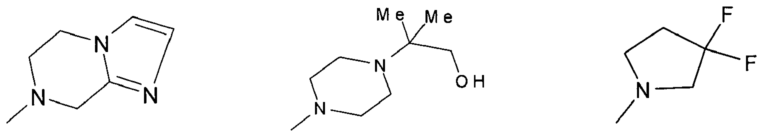 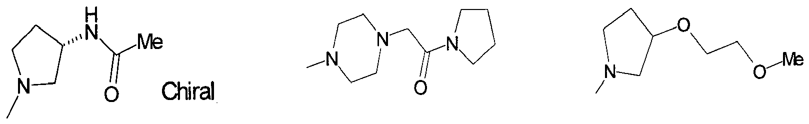 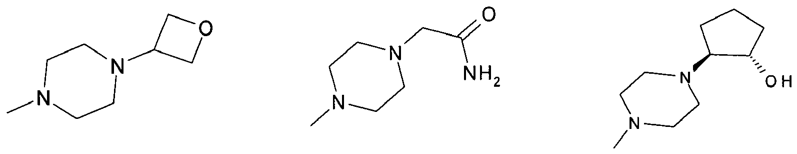 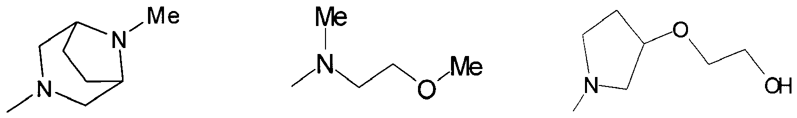 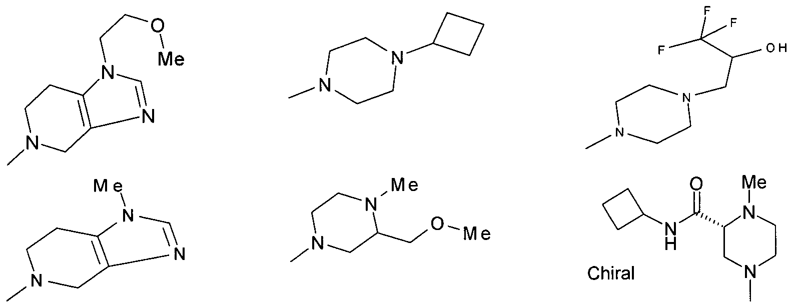 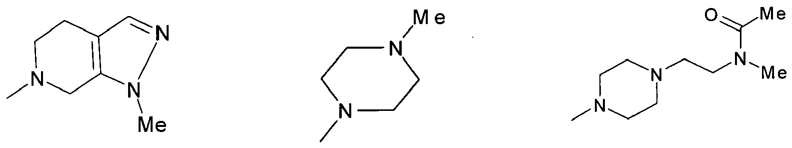 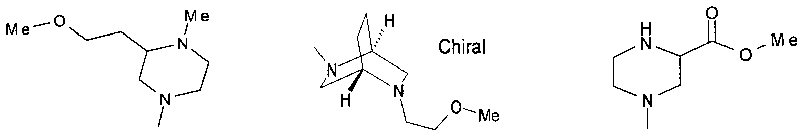 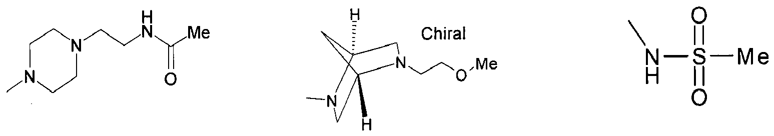 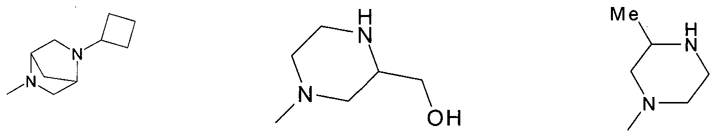 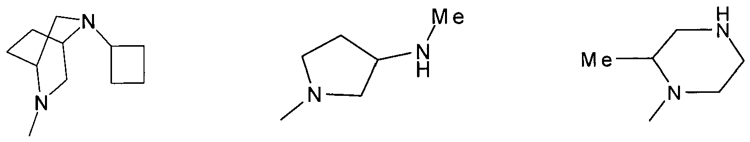 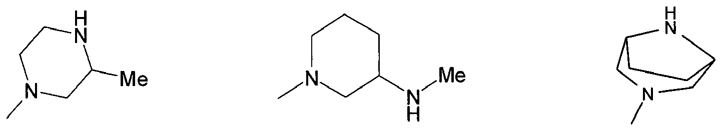 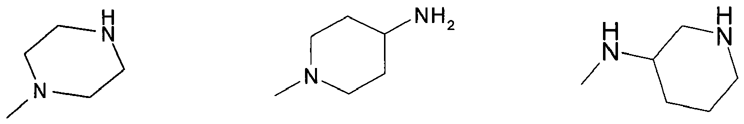 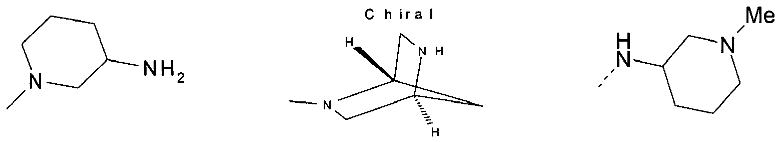 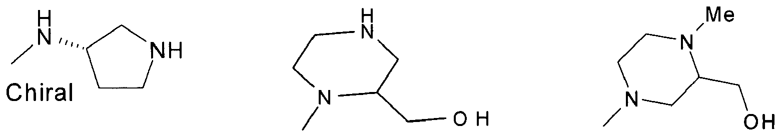 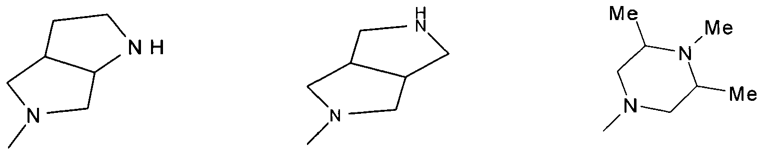 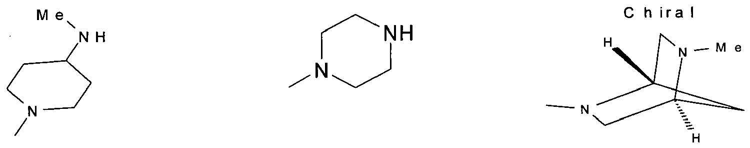 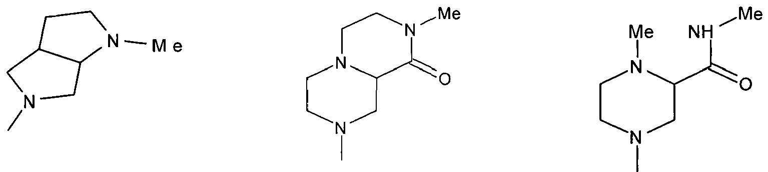 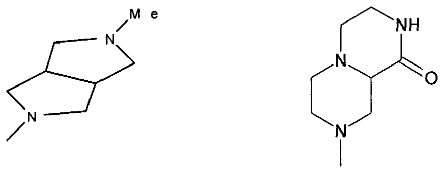
- R₄ represents a hydrogen atom, a bromine, chlorine or fluorine atom, a cyano, a phenyl, a methyl radical, a trifluoromethyl radical, a methoxy radical or a dimethylamino group;
- R₅ represents a hydrogen atom, a bromine, chlorine or fluorine atom or a methyl radical;
- R₆ represents a hydrogen atom, a bromine atom, a cyano, a methoxycarbonyl group or a -CONH₂ group;
in the form of a base or salt of addition with an acid or a base.

9. Compound of formula (I) according to Claim 1, **characterized in that**:
- A represents a divalent aromatic radical selected from: 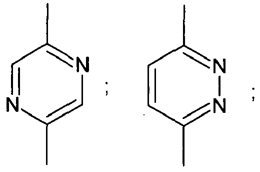
- X represents a -CH- group;
- R₁ represents an n-propyl radical;
- R₂ represents a methyl radical;
- R₃ represents a hydroxyl or a group -NR₇R₈ selected from: 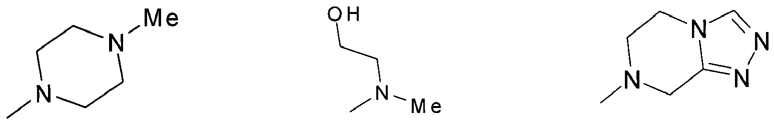 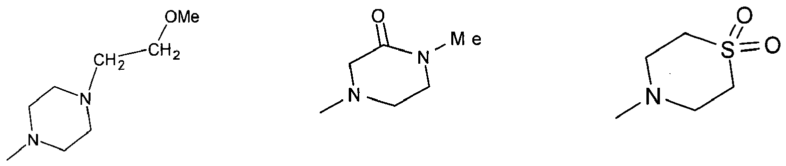 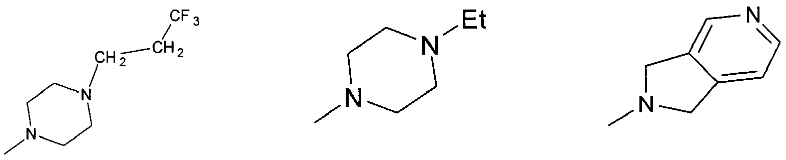 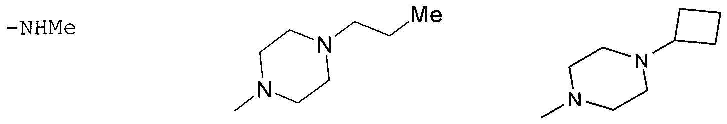 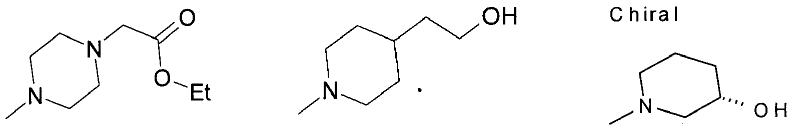 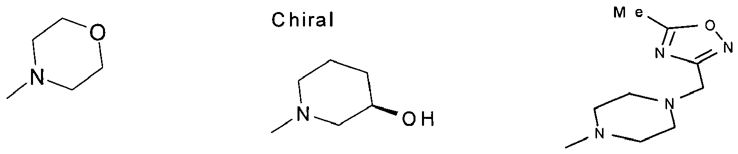 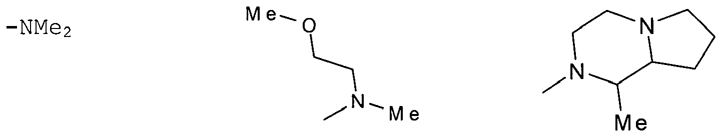 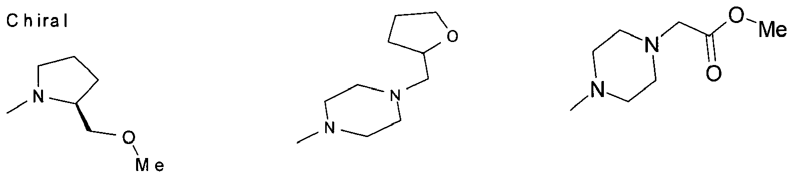 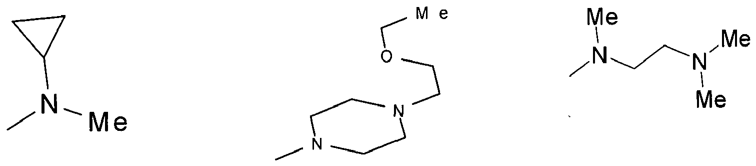 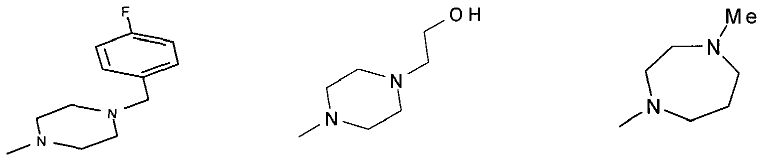
- R₄ represents a chlorine atom;
- R₅ represents a hydrogen atom;
in the form of a base or salt of addition with an acid or a base.

10. Compound of formula (I) according to Claim 1, **characterized in that**:
- A represents a divalent aromatic radical: 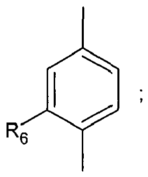
- X represents a -CH- group or a nitrogen atom;
- R₁ represents a methyl, ethyl, n-propyl radical or an ethoxy radical;
- R₂ represents a methyl radical;
- R₃ represents a hydroxyl or a group -NR₇R₈ selected from: 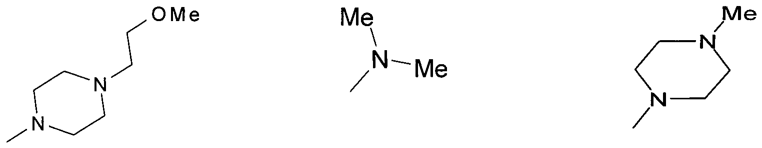 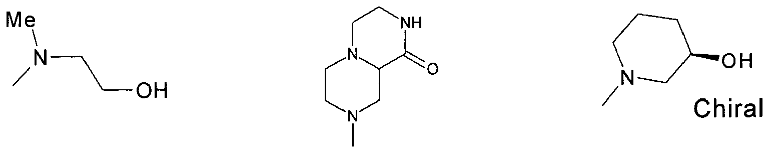 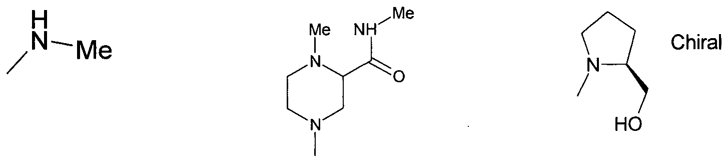 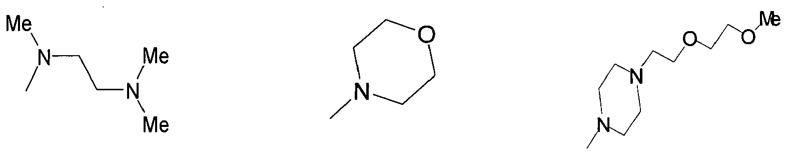 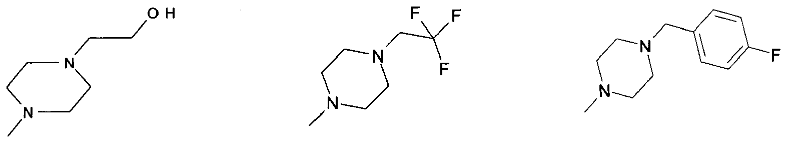 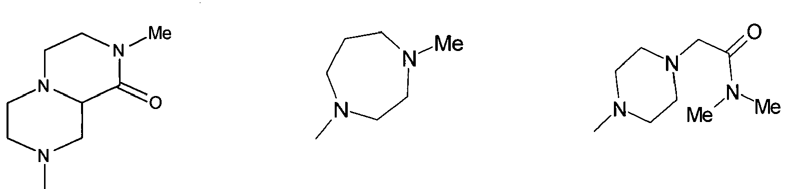 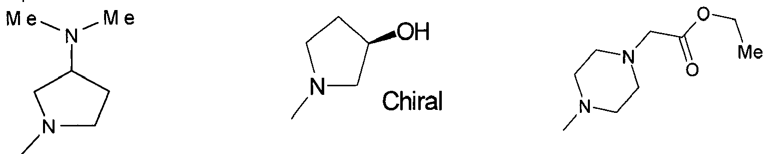 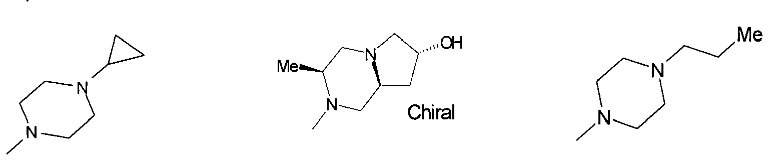 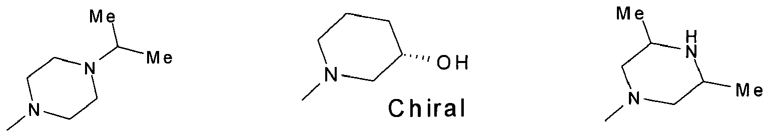 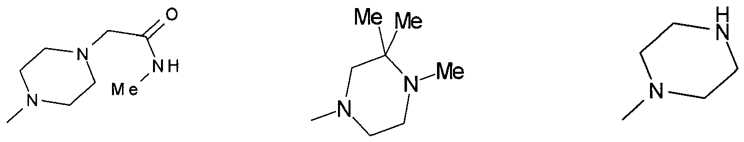 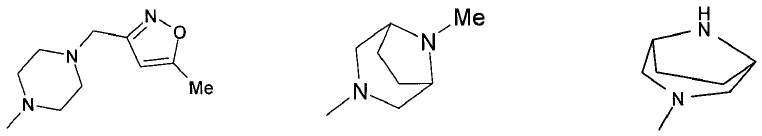 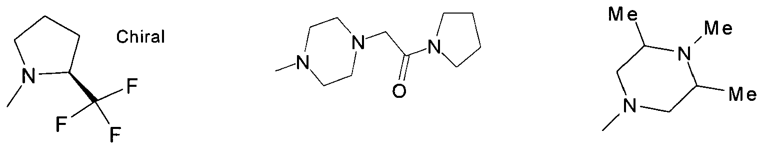 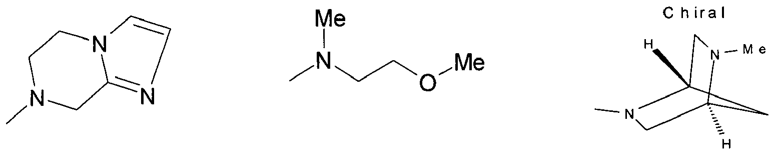 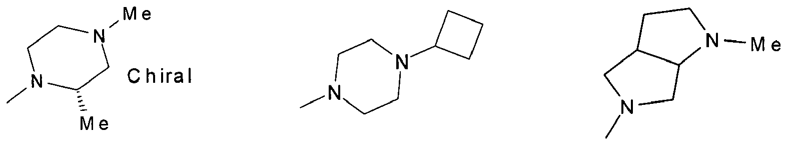 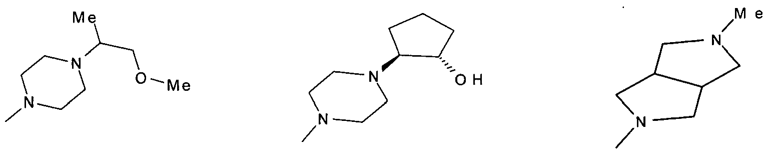 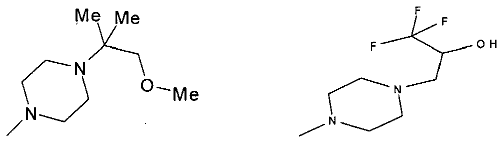 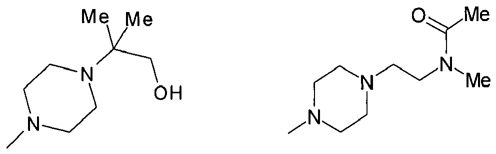
- R₄ represents a hydrogen atom, a bromine, chlorine or fluorine atom, a cyano, a phenyl, a methyl radical, a trifluoromethyl radical, a methoxy radical or a dimethylamino group;
- R₅ represents a hydrogen atom, a bromine, chlorine or fluorine atom or a methyl radical;
- R₆ represents a hydrogen atom, a bromine atom, a cyano, a methoxycarbonyl group or a -CONH₂ group;
in the form of a base or salt of addition with an acid or a base.

11. Compound of formula (I) according to Claim 1 selected from:
- (3-{[4-(4-butyryl-5-methyl-1*H*-pyrazol-1-yl)phenyl]carbamoyl}-5-methyl-1*H*-indol-1-yl)acetic acid;
- (3-{[4-(4-butyryl-5-methyl-1*H*-pyrazol-1-yl)phenyl]carbamoyl}-5,6-dimethyl -1*H*-indol-1-yl)acetic acid;
- (3-{[6-(4-butyryl-5-methyl-1*H*-pyrazol-1-yl)pyridazin-3-yl]carbamoyl}-5-chloro-1*H*-indol-1l-yl)acetic acid;
- N-[5-(4-butyryl-5-methyl-1*H*-pyrazol-1-yl)pyrazin-2-yl]-5-chloro-1-[2-(4-methylpiperazin-1-yl)-2-oxoethyl]-1*H*-indole-3-carboxamide;
- N-[6-(4-butyryl-5-methyl-1*H*-pyrazol-1-yl)pyridazin-3-yl]-5-chloro-1-[2-(4-methylpiperazin-1-yl)-2-oxoethyl]-1*H*-indole-3-carboxamide;
- N-[6-(4-butyryl-5-methyl-1*H*-pyrazol-1-yl)pyridazin-3-yl]-5-chloro-1-{2-[4-(2-methoxyethyl)piperazin-1-yl]-2-oxoethyl}-1*H*-indole-3-carboxamide;
- N-[6-(4-butyryl-5-methyl-1*H*-pyrazol-1-yl)pyridazin-3-yl]-5-chloro-1-{2-oxo-2-[4-(3,3,3-trifluoropropyl)piperazin-1-yl]ethyl}-1*H*-indole-3-carboxamide;
- N-[6-(4-butyryl-5-methyl-1*H*-pyrazol-1-yl)pyridazin-3-yl]-5-chloro-1-[2-(methylamino)-2-oxoethyl]-1*H*-indole-3-carboxamide;
- N-[6-(4-butyryl-5-methyl-1*H*-pyrazol-1-yl)pyridazin-3-yl]-5-chloro-1-(2-morpholin-4-yl-2-oxoethyl)-1*H*-indole-3-carboxamide;
- N-[6-(4-butyryl-5-methyl-1H-pyrazol-1-yl)pyridazin-3-yl]-5-chloro-1-[2-(dimethylamino)-2-oxoethyl]-1*H-*indole-3-carboxamide;
- N-[6-(4-butyryl-5-methyl-1*H*-pyrazol-1-yl)pyridazin-3-yl]-5-chloro-1-{2-[(2S)-2-(methoxymethyl)pyrrolidin-1-yl]-2-oxoethyl}-1*H*-indole-3-carboxamide;
- N-[6-(4-butyryl-5-methyl-1*H*-pyrazol-1-yl)pyridazin-3-yl]-5-chloro-1-[2-(4-ethylpiperazin-1-yl)-2-oxoethyl]-1*H*-indole-3-carboxamide;
- N-[6-(4-butyryl-5-methyl-1*H*-pyrazol-1-yl)pyridazin-3-yl]-5-chloro-1-[2-(4-propylpiperazin-1-yl)-2-oxoethyl]-1*H*-indole-3-carboxamide;
- N-[6-(4-butyryl-5-methyl-1*H*-pyrazol-1-yl)pyridazin-3-yl]-5-chloro-1-{2-[(2-methoxyethyl)(methyl)amino]-2-oxoethyl}-1*H*-indole-3-carboxamide;
- N-[6-(4-butyryl-5-methyl-1*H*-pyrazol-1-yl)pyridazin-3-yl]-5-chloro-1-{2-oxo-2-[4-(tetrahydrofuran-2-ylmethyl)piperazin-1-yl]ethyl}-1*H*-indole-3-carboxamide;
- N-[6-(4-butyryl-5-methyl-1*H*-pyrazol-1-yl)pyridazin-3-yl]-5-chloro-1-[2-(5,6-dihydro[1,2,4]triazolo[4,3-a]pyrazin-7 (8H)-yl)-2-oxoethyl]-1*H*-indole-3-carboxamide;
- N-[6-(4-butyryl-5-methyl-1*H*-pyrazol-1-yl)pyridazin-3-yl]-5-chloro-1-[2-(4-cyclobutylpiperazin-1-yl)-2-oxoethyl]-1*H*-indole-3-carboxamide;
- N-[6-(4-butyryl-5-methyl-1*H*-pyrazol-1-yl)pyridazin-3-yl]-5-chloro-1-(2-{4-[(5-methyl-1,2,4-oxadiazol-3-yl)methyl]piperazin-1-yl}-2-oxoethyl)-1*H*-indole-3-carboxamide;
- {4-[(3-([6-(4-butyryl-5-methyl-1*H*-pyrazol-1-yl)pyridazin-3-yl]carbamoyl}-5-chloro-1*H*-indol-1-yl)acetyl]piperazin-1-yl}methyl acetate;
- N-[6-(4-butyryl-5-methyl-1*H*-pyrazol-1-yl)pyridazin-3-yl]-5-chloro-1-{2-[cyclopropyl(methyl)amino]-2-oxoethyl}-1*H*-indole-3-carboxamide;
- N-[6-(4-butyryl-5-methyl-1*H*-pyrazol-1-yl)pyridazin-3-yl]-5-chloro-1-{2-[4-(4-fluorobenzyl)piperazin-1-yl]-2-oxoethyl}-1*H*-indole-3-carboxamide;
- N-[6-(4-butyryl-5-methyl-1*H*-pyrazol-1-yl)pyridazin-3-yl]-5-chloro-1-{2-[4-(2-ethoxyethyl)piperazin-1-yl]-2-oxoethyl}-1*H*-indole-3-carboxamide;
- N-[6-(4-butyryl-5-methyl-1*H*-pyrazol-1-yl)pyridazin-3-yl]-5-chloro-1-{2-[4-(2-hydroxyethyl)piperazin-1-yl]-2-oxoethyl}-1*H*-indole-3-carboxamide;
- N-[6-(4-butyryl-5-methyl-1*H*-pyrazol-1-yl)pyridazin-3-yl]-5-chloro-1-(2-{[2-(dimethylamino)ethyl](methyl)amino}-2-oxoethyl)-1*H-*indole-3-carboxamide;
- N-[6-(4-butyryl-5-methyl-1*H*-pyrazol-1-yl)pyridazin-3-yl]-5-chloro-1-[2-(4-methyl-1,4-diazepan-1-yl)-2-oxoethyl]-1*H*-indole-3-carboxamide;
- N-[4-(4-butyryl-5-methyl-1*H*-pyrazol-1-yl)phenyl]-5-chloro-1-[2-(8-methyl-9-oxooctahydro-2*H*-pyrazino[1,2-a]pyrazin-2-yl)-2-oxoethyl]-1*H*-indole-3-carboxamide;
- N-[4-(4-butyryl-5-methyl-1*H*-pyrazol-1-yl)-2-cyanophenyl]-5-chloro-1-[2-(4-methylpiperazin-1-yl)-2-oxoethyl]-1*H*-indole-3-carboxamide;
- N-[4-(4-butyryl-5-methyl-1*H*-pyrazol-1-yl)phenyl]-5-chloro-1-{2-[4-(4-fluorobenzyl)piperazin-1-yl]-2-oxoethyl}-1*H*-indole-3-carboxamide;
- N-[4-(4-butyryl-5-methyl-1*H*-pyrazol-1-yl)phenyl]-5-chloro-1-{2-[4-(2-methoxyethyl)piperazin-1-yl]-2-oxoethyl}-1*H*-indole-3-carboxamide;
- N-[4-(4-butyryl-5-methyl-1*H*-pyrazol-1-yl)phenyl]-1-{2-[4-(2-methoxyethyl)piperazin-1-yl]-2-oxoethyl}-5-methyl-1*H*-indole-3-carboxamide;
- N-[4-(4-butyryl-5-methyl-1*H*-pyrazol-1-yl)phenyl]-5-chloro-1-[2-(4-methylpiperazin-1-yl)-2-oxoethyl]-1*H-*indole-3-carboxamide;
- N-[4-(4-butyryl-5-methyl-1*H*-pyrazol-1-yl)phenyl]-5-chloro-1-{2-[4-(2-methoxy-1-methylethyl)piperazin-1-yl]-2-oxoethyl}-1*H*-indole-3-carboxamide;
- N-[4-(4-butyryl-5-methyl-1*H*-pyrazol-1-yl)phenyl]-5-methyl-1-[2-(4-methylpiperazin-1-yl)-2-oxoethyl]-1*H-*indole-3-carboxamide;
- N-[4-(4-butyryl-5-methyl-1*H*-pyrazol-1-yl)phenyl]-5-chloro-6-fluoro-1-{2-[4-(2-methoxyethyl)piperazin-1-yl]-2-oxoethyl}-1*H*-indole-3-carboxamide;
- N-[4-(4-butyryl-5-methyl-1*H*-pyrazol-1-yl)phenyl]-5-methoxy-1-[2-(4-methylpiperazin-1-yl)-2-oxoethyl]-1*H-*indole-3-carboxamide;
- N-[4-(4-butyryl-5-methyl-1*H*-pyrazol-1-yl)phenyl]-5-chloro-1-{2-[4-methyl-3-(methylcarbamoyl)piperazin-1-yl]-2-oxoethyl}-1*H*-indole-3-carboxamide;
- N-[4-(4-butyryl-5-methyl-1*H*-pyrazol-1-yl)phenyl]-5,6-dimethyl-1-[2-(4-methylpiperazin-1-yl)-2-oxoethyl]-1*H-*indole-3-carboxamide;
- N-[4-(4-butyryl-5-methyl-1*H*-pyrazol-1-yl)phenyl]-5-methyl-1-[2-(4-methylpiperazin-1-yl)-2-oxoethyl]-1*H-*indazole-3-carboxamide;
- N-[4-(4-butyryl-5-methyl-1*H*-pyrazol-1-yl)phenyl]-5-methyl-1-(2-oxo-2-piperazin-1-ylethyl)-1*H*-indole-3-carboxamide;
- N-[4-(4-butyryl-5-methyl-1*H*-pyrazol-1-yl)phenyl]-5-chloro-6-fluoro-1-[2-(4-methylpiperazin-1-yl)-2-oxoethyl]-1*H*-indole-3-carboxamide;
- N-[4-(4-butyryl-5-methyl-1*H*-pyrazol-1-yl)phenyl]-5-chloro-1-(2-{4-[(5-methylisoxazol-3-yl)methyl]piperazin-1-yl}-2-oxoethyl)-1*H*-indole-3-carboxamide;
- N-[4-(4-butyryl-5-methyl-1*H*-pyrazol-1-yl)phenyl]-5-methyl-1-(2-morpholin-4-yl-2-oxoethyl)-1*H*-indole-3-carboxamide;
- N-[4-(4-butyryl-5-methyl-1*H*-pyrazol-1-yl)phenyl]-5-chloro-1-(2-oxo-2-piperazin-1-ylethyl)-1*H*-indole-3-carboxamide;
- N-[4-(4-butyryl-5-methyl-1*H*-pyrazol-1-yl)phenyl]-5-chloro-1-{2-[4-(2-methoxy-1,1-dimethylethyl)piperazin-1-yl]-2-oxoethyl}-1*H*-indole-3-carboxamide;
- N-[4-(4-butyryl-5-methyl-1*H*-pyrazol-1-yl)phenyl]-5-chloro-1-{2-[4-(2-hydroxy-1,1-dimethylethyl)piperazin-1-yl]-2-oxoethyl}-1*H*-indole-3-carboxamide;
- N-[4-(4-butyryl-5-methyl-1*H*-pyrazol-1-yl)phenyl]-5-chloro-1-[2-(8-methyl-3,8-diazabicyclo[3.2.1]oct-3-yl)-2-oxoethyl]-1*H*-indole-3-carboxamide;
- N-[4-(4-butyryl-5-methyl-1*H*-pyrazol-1-yl)phenyl]-1-[2-(4-methylpiperazin-1-yl)-2-oxoethyl]-5-(trifluoromethyl)-1*H*-indole-3-carboxamide;
- N-[4-(4-butyryl-5-methyl-1*H*-pyrazol-1-yl)phenyl]-5-chloro-1-[2-oxo-2-(9-oxooctahydro-2H-pyrazino[1,2-a]pyrazin-2-yl)ethyl]-1*H*-indole-3-carboxamide;
in the form of a base or salt of addition with an acid or a base.

12. Method of preparation of compounds of formula (I) according to Claim 1 in which R₃ = -OH (compound IA), **characterized in that**:
a compound of formula (II): 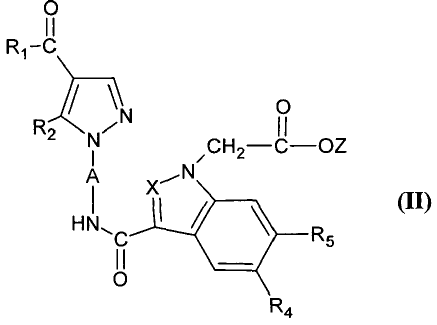 in which A, X, R₁, R₂, R₄ and R₅ are as defined for a compound of formula (I) in Claim 1 and Z represents a (C₁-C₄) alkyl, is hydrolyzed in an acid or basic medium.

13. Method of preparation of the compounds of formula (I) according to Claim 1 in which R₃ = -NR₇R₈ (compound IB), **characterized in that**:
a compound of formula (IA): 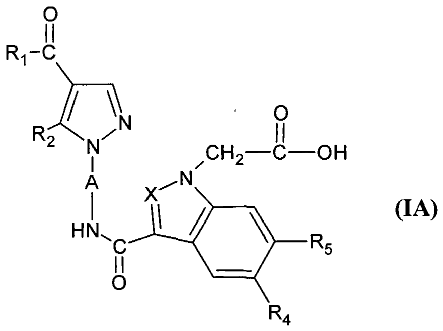 in which A, X, R₁, R₂, R₄ and R₅ are as defined for a compound of formula (I) in Claim 1, is reacted with an amine of formula (III):
HNR₇R₈ **(III)**
in which R₇ and R₈ are as defined for a compound of formula (I) in Claim 1

14. Compound of formula: 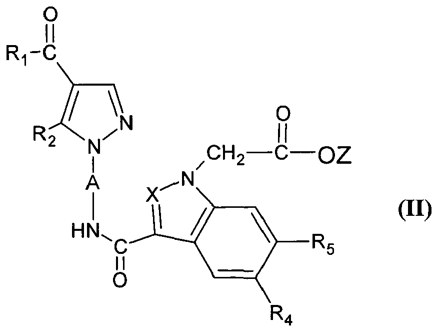 in which:
- A represents a divalent aromatic radical selected from: 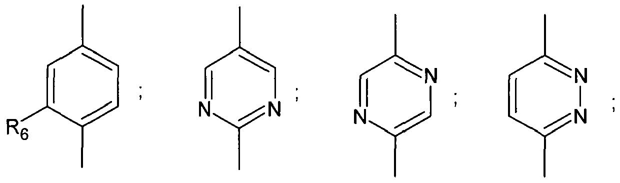
- X represents a -CH- group or a nitrogen atom;
- R₁ represents a (C₁-C₄)alkyl or a (C₁-C₄)alkoxy;
- R₂ represents a group Alk;
- R₄ represents a hydrogen atom, a halogen atom, a cyano, a phenyl, a group Alk, a group OAlk or a group - NR₉R₁₀;
- R₅ represents a hydrogen atom, a halogen atom or a group Alk;
- R₆ represents a hydrogen atom, a halogen atom, a cyano, a group
- COOAlk or a -CONH₂ group;
- R₉ and R₁₀ represent, each independently, a hydrogen atom or a (C₁-C₄) alkyl;
- Z represents a (C₁-C₄) alkyl;
- Alk represents a (C₁-C₄)alkyl, unsubstituted or substituted one or more times with a fluorine atom.

15. Medicament, **characterized in that** it contains a compound of formula (I) according to any one of Claims 1 to 11, or a pharmaceutically acceptable salt of the compound of formula (I).

16. Pharmaceutical composition, **characterized in that** it comprises a compound of formula (I) according to any one of Claims 1 to 11, a pharmaceutically acceptable salt of said compound, as well as at least one pharmaceutically acceptable excipient.

17. Compound of formula (I) according to any one of Claims 1 to 11 for the treatment and prevention of unstable angina, percutaneous transluminal coronary angioplasty (PTCA), of myocardial infarction, thrombotic arterial complications of atherosclerosis such as embolic or thrombotic cerebrovascular accidents, transient ischemic attacks, peripheral vascular disease, arterial complications of atherosclerosis due to surgical interventions such as angioplasty, endarterectomy, placement of stents, coronary vascular grafts, thrombotic complications of surgery or of mechanical damage such as recovery of tissues after accidental or surgical trauma, reconstructive surgery (including skin and muscle flaps), thrombotic thrombocytopenic purpura, antiphospholipid syndrome, heparin-induced thrombocytopenia and pre-eclampsia/eclampsia; or venous thromboses such as deep vein thrombosis, venoocclusive disease; or in the prevention of platelet activation induced mechanically *in vivo*, such as during cardiopulmonary bypass and extracorporeal oxygenation (prevention of micro-thromboembolisms), in the prevention of platelet activation induced mechanically *in vitro* (use in the storage of blood products (platelet concentrates), use during shunts such as renal dialysis and plasmapheresis), thrombosis secondary to a vascular lesion / inflammation such as angiitis, arteritis, glomerulonephritis, and organ graft rejection, conditions in which platelets can contribute to the process of the underlying inflammatory disease in the vessel wall such as formation / progression of atheromatous plaques, stenosis / restenosis.
